# EUROPEAN PATENT SPECIFICATION

(11) **EP 4 426 688 B1**
(45) Date of publication and mention of the grant of the patent: **03.12.2025**
(21) Application number: 22802689.4
(22) Date of filing: 03.11.2022
(51) Int. Cl.: C07D 403/12, C07D 403/14, C07D 413/14, C07D 471/04, A61P 35/00, A61P 29/00, A61K 31/416

(54) **DDR1 AND DDR2 INHIBITORS FOR THE TREATEMENT OF CANCER AND FIBROTIC DISEASES**
DDR1- UND DDR2-INHIBITOREN ZUR BEHANDLUNG VON KREBS UND FIBROTISCHEN ERKRANKUNGEN
INHIBITEURS DE DDR1 ET DE DDR2 POUR LE TRAITEMENT DU CANCER ET DE MALADIES FIBROTIQUES

(30) Priority: 04.11.2021 GB 202115838
(43) Date of publication of application: 11.09.2024
(73) Proprietor: Redx Pharma Limited, Macclesfield, Cheshire SK10 4TG GB (GB)
(72) Inventor: BHAMRA, Inder, Macclesfield Cheshire SK10 4TG (GB); JONES, Clifford D., Macclesfield Cheshire SK10 4TG (GB); VARELA RODRÍGUEZ, Ana, Macclesfield Cheshire SK10 4TG (GB); GUISOT, Nicolas E.S., Macclesfield Cheshire SK10 4TG (GB)
(74) Representative: HGF
(86) International application number: PCT/GB2022/052778
(87) International publication number: WO 2023/079291

(56) References cited:
- WO-A1-2017/137334
- US-A1- 2005 054 670
- US-A1- 2011 152 273

## Description

This invention relates to novel compounds and pharmaceutical compositions comprising the novel compounds. More specifically, the invention relates to compounds useful as inhibitors of discoidin domain receptor 1 (DDR1) and discoidin domain receptor 2 (DDR2). The compounds are particularly useful in the treatment of cancer and fibrotic diseases

### BACKGROUND

Discoidin Domain Receptors (DDRs) DDR1 and DDR2 are type 1 transmembrane Receptor Tyrosine Kinases (RTKs) with collagen receptor functionality (Vogel et al, Mol. Cell, 1997). DDRs contain characteristic collagen binding discoidin domains in the N-terminal extracellular domain. These domains are proceeded by an extracellular juxtamembrane domain, a single transmembrane domain, a cytosolic juxtamembrane domain and a catalytic kinase domain prior to a short C-terminal tail. Five isoforms of DDR1 (DRR1a-e) have been identified which arise from alternative splicing of the cytoplasmic region. No alternative isoforms of DDR2 have been identified. DDR1 and DDR2 have broadly (but not completely) mutually exclusive expression profiles in epithelial cell and stroma respectively. DDRs are activated by binding to collagens with broad specificity but with distinct preference for certain collagen types. Upon activation DDRs are known to regulate cell adhesion, proliferation and remodelling of the extracellular matrix. It is recognised that DDRs are upregulated in response to cellular activity and many forms of tissue injury and as such DDRs are implicated in diseases including cancer, atherosclerosis as well as diseases characterised by fibrosis and inflammation. Inhibitors of DDR kinase activity may be of benefit as therapeutic agents in these disease areas.

DDR1 and DDR2 overexpression and/or activation has been linked to multiple forms of cancer as summarised in a recent review (Elkamhawy et al, Int. J. Mol. Sci., 2021). Studies have shown that elevated DDR expression levels and/or mutations can be found in a number of cancer cell lines as well as primary tumour tissues including lung, pancreas, prostate, breast, brain, ovary, liver and others. DDR1 was found to be a prognostic marker for non-small-cell lung carcinoma (NSCLC) patients. A recent study demonstrated that siRNA-mediated downregulation of DDR1 suppressed melanoma cell malignancy, migration, invasion, and survival. DDR1 protein was also found to be expressed in 63% of serous ovarian cancer tissue, but not in normal ovarian surface epithelium. Involvement of DDR1 in glioblastoma cell invasion and epithelial-mesenchymal transition (EMT) has also been demonstrated. DDR1 expression was found in 50.5% of gastric cancer tissues . DDR1 was found to control triple-negative breast cancer growth by modulating tumuor-infiltrating CD4+ and CD8+ T cells . There is also strong evidence indicating that DDR2 could be a potential biomarker and a molecular target for a variety of cancers. For instance, DDR2 overexpression was reported to contribute to NSCLC, thyroid carcinoma, Hodgkin's lymphoma, nasopharyngeal carcinomas, prostate cancer, as well as to head and neck squamous cell carcinoma. According to studies DDR2 contributes to breast cancer metastasis by stabilizing the SNAIL1 protein. DDR2 has also been shown to be a favourable independent predictor of recurrence and outcome in primary breast cancers. In addition to the essential roles of the wild type of DDR in cancer pathology and prognosis, various mutations of DDR1 and/or DDR2 have also been reported in numerous types of cancer cells, for instance, G1486T(DDR1) and A496S(DDR1) in lung cancer, N502S(DDR1), A533S(DDR1), and A803V(DDR1) in acute myeloid leukemia (AML), and S768R(DDR2) in squamous cell carcinoma. DDRs also play a role in cancer growth by controlling how tumour cells interact with their surrounding collagen matrix. This role of DDRs becomes more prominent when considering their role as extracellular matrix receptors. The extracellular matrix (ECM) confers structural properties to tissues around the tumour, as well as regulating cell proliferation, survival, migration, and invasion. The physiological interactions between tumour cells and their immediate microenvironment, represented by the extracellular matrix, are disrupted in metastatic cancers. As a key component of the tumour extracellular matrix, type I collagen shows high density and distorted architecture in malignant cancer, linking it to tumour formation and metastasis. Therefore, the discovery of DDRs as collagen receptors represents a new target in the regulation of tumour progression.

DDRs also appear to play a central role in the modulation of inflammation and fibrosis. Modulation of fibrosis and inflammation has been demonstrated in several organs including lung and kidney. In lung DDR-1 deficient mice show reduced bleomycin induced pulmonary injury (Vogel et al, Am. J. Respir. Crit. Care Med., 2006) and both DDR1 and DDR2 have been demonstrated to have increased expression in patients with fibrotic lung disease (Bian et al, ERJ Open Res., 2016). In kidney, DDR1 expression is elevated in patients with lupus nephritis and Goodpasture's syndrome as well as mouse models of glomerulonephritis (Kerroch et al, FASEB journal, 2012) and in the tubules of mice that have undergone unilatereal ureteral obstruction (UUO) (Guerrot et al, Am. J. Pathol., 2011). Several studies have demonstrated that DDR1-null mice are protected from angiotensin II-mediated proteinuria, glomerular fibrosis and inflammation as well as showing reduced collagen deposition, tubular macrophage infiltration and pro-inflamatory cytokine levels following the UUO procedure.. Finally, COL3A3 KO mice (the mouse model for human Alport syndrome, crossed on to DDR1-null mice have mice have reduced renal fibrosis and inflammation as a consequence of reduced TGF-β mediated signalling and reduced levels of the pro-inflammatory cytokine IL-6 (Dorison, Cell Adhesion and Migration, 2018).

Small molecule inhibitors of DDR1 and DDR2 kinase activity have been disclosed in the prior art (for instance WO2017137334) and inhibitory activity of DDR1 and/or DDR2 has been demonstrated to give rise to efficacious effects in mouse models of cancer and fibrotic disease (Richter et al, ACS Chem. Biol., 2019; Wang et al, J. Med. Chem., 2018; Zhu et al, J. Med. Chem., 2019). Such reports support the hypothesis that inhibitors of DDR kinase activity may be of benefit as therapeutic agents for the treatment of human cancer and fibrotic disease.

Furthermore, it is an aim of certain embodiments of this invention to provide new compounds useful in treating diseases such as cancer and fibrotic diseases. The compounds may be inhibitors of DDR1 and/or DDR2. It is an aim of certain embodiments of this invention to provide compounds which have comparable activity to existing DDR1 and/or DDR2 inhibitors. It is an aim of certain embodiments of this invention to provide compounds which have increased activity relative to existing DDR1 and/or DDR2 inhibitors.

Certain embodiments of the present invention satisfy some or all of the above aims.

### BRIEF SUMMARY OF THE DISCLOSURE

The present invention provides a compound of formula (I) and pharmaceutically acceptable salts thereof: 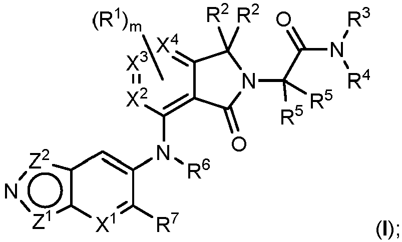 wherein
Z¹ and Z² are each selected from -CR^{8a}- and -NR^{8b}-, wherein one of Z¹ and Z² is -CR^{8a}- and the other is -NR^{8b}-; and wherein the ring comprising Z¹ and Z² is a pyrazole;
X¹ is independently selected from CR^{7a} and N;
X², X³ and X⁴ are each independently selected from carbon and nitrogen, wherein at least one of X², X³ and X⁴ is carbon;
R¹ is independently selected at each occurrence from halo, nitro, cyano, NR⁹ R¹⁰, OR¹¹, SR⁹, SO₂NR⁹R⁹, SO₂R⁹, CO₂R⁹, C(O)R⁹, CONR⁹R⁹, C₁-C₄-alkyl, C₁-C₄-alkyl substituted with NR⁹R¹⁰, C₁-C₄-alkyl substituted with OR¹¹, C₂-C₄-alkenyl, C₂-C₄-alkynyl, C₁-C₄-haloalkyl and cyclopropyl;
R² is independently selected at each occurrence from H, fluoro, C₁-C₄-alkyl, C₁-C₄-haloalkyl and cyclopropyl, or the two R² groups and the carbon atom to which they are attached together form a C₃-C₆-cycloalkyl ring;
R³ is independently selected from H and C₁-C₄-alkyl;
R⁴ is independently selected from C₁-C₆-alkyl, C₁-C₆-haloalkyl, C₀-C₄-alkylene-R^{4a}; wherein R^{4a} is independently selected from: C₃-C₈-cycloalkyl, phenyl, 5-, 6-, 9- or 10-membered heteroaryl and 4- to 10-membered heterocycloalkyl; wherein said heterocycloalkyl or heteroaryl group may be monocyclic or bicyclic; wherein said cycloalkyl or heterocycloalkyl group is optionally substituted with a single R¹² group and/or from 1 to 4 R¹³ groups and wherein said phenyl or heteroaryl group is optionally substituted with a single R¹² group and/or from 1 to 3 R¹⁴ groups;
or R³ and R⁴, together with the nitrogen atom to which they are attached together form a 4- to 10-membered heterocycloalkyl group or a 5-, or 9-membered heteroaryl group; wherein said heterocycloalkyl or heteroaryl group may be monocyclic or bicyclic; wherein said heterocycloalkyl group is optionally substituted with a single R¹² group and/or from 1 to 4 R¹³ groups and wherein said heteroaryl group is optionally substituted with a single R¹² group and/or from 1 to 3 R¹⁴ groups;
R⁵ is independently at each occurrence selected from H, halo and C₁-C₄-alkyl, or the two R⁵ groups and the carbon atom to which they are attached may together form a C₃-C₆ cycloalkyl ring;
R⁶ is independently selected from H, C₁-C₄-alkyl, C₁-C₄-haloalkyl and cyclopropyl;
R⁷ and R^{7a} are each independently selected from H, halo, nitro, cyano, NR⁹ R¹⁰, OR¹¹, SR⁹, SO₂NR⁹R⁹, SO₂R⁹, CO₂R⁹, C(O)R⁹, CONR⁹R⁹, C₁-C₄-alkyl, C₁-C₄-alkyl substituted with NR⁹R¹⁰, C₁-C₄-alkyl substituted with OR¹¹, C₂-C₄-alkenyl, C₂-C₄-alkynyl, C₁-C₄-haloalkyl and cyclopropyl;
R^{8a} is independently selected from H, halo, nitro, cyano, NR⁹ R¹⁰, OR¹¹, SR⁹, SO₂NR⁹R⁹, SO₂R⁹, CO₂R⁹, C(O)R⁹, CONR⁹R⁹, C₁-C₄-alkyl, C₁-C₄-alkyl substituted with NR⁹R¹⁰, C₁-C₄-alkyl substituted with OR¹¹, C₂-C₄-alkenyl, C₂-C₄-alkynyl, C₁-C₄-haloalkyl and C₀-C₄-alkylene-R^{8c};
R^{8b} is independently selected from H, C₁-C₄-alkyl, C₂-C₄-alkyl substituted with NR⁹ R¹⁰, C₂-C₄-alkyl substituted with OR¹¹, C₃-C₄-alkenyl, C₃-C₄-alkynyl, C₁-C₄-haloalkyl and C₀-C₄-alkylene-R^{8c};
R^{8c} is independently selected from C₃-C₆-cycloalkyl and 3- to 7-membered heterocycloalkyl; wherein said heterocycloalkyl group is attached to the C₀-C₄-alkylene via a carbon atom in the heterocycloalkyl ring; wherein said cycloalkyl or heterocycloalkyl group is optionally substituted with from 1 to 4 R¹³ groups;
R⁹ is independently at each occurrence selected from H and C₁-C₄-alkyl; or two R⁹ groups, together with the nitrogen atom to which they are attached together form a C₅-C₈-heterocycloalkyl group optionally substituted with from 0 to 4 R¹³ groups;
R¹⁰ is independently at each occurrence selected from H, C₁-C₄-alkyl, C(O)-C₁-C₄-alkyl and S(O)₂-C₁-C₄-alkyl; or R⁹ and R¹⁰, together with the nitrogen atom to which they are attached together form a C₅-C₈-heterocycloalkyl group optionally substituted with from 0 to 4 R¹³ groups;
R¹¹ is independently at each occurrence selected from H, C₁-C₄-alkyl, C(O)-C₁-C₄-alkyl and C₁-C₄-haloalkyl;
R¹² is independently selected from C₃-C₆-cycloalkyl, phenyl, 5- or 6- membered heteroaryl and 3- to 6-membered-heterocycloalkyl; wherein said cycloalkyl or heterocycloalkyl group is optionally substituted with from 1 to 4 R¹³ groups and wherein said phenyl or heteroaryl group is optionally substituted with from 1 to 3 R¹⁴ groups;
R¹³ is independently at each occurrence selected from =O, halo, nitro, cyano, NR⁸R⁹, OR¹⁴, SR⁸, SO₂NR⁸R⁸, CO₂R⁸, C(O)R⁸, CONR⁸R⁸, C₁-C₄-alkyl, C₁-C₄-alkyl substituted with OR¹¹, C₁-C₄-alkyl substituted with NR⁹R¹⁰, C₂-C₄-alkenyl, C₂-C₄-alkynyl, C₁-C₄-haloalkyl, C₆-C₁₀-aryl, and C₃-C₆-cycloalkyl;
R¹⁴ is independently at each occurrence selected from halo, nitro, cyano, NR⁸R⁹, OR¹⁰, SR⁸, SO₂R⁸, SO₂NR⁸R⁸, CO₂R⁸, C(O)R⁸, CONR⁸R⁸, C₁-C₄-alkyl, C₂-C₄-alkenyl, C₂-C₄-alkynyl, C₁-C₄-haloalkyl, C₁-C₄--alkyl substituted with OR¹¹, C₁-C₄-alkyl substituted with NR⁸R⁹ and cyclopropyl;
m is an integer selected from 0, 1, 2 and 3;
wherein any of the aforementioned alkyl, alkylene or cyclopropyl groups is optionally substituted, where chemically possible, by 1 to 5 substituents which are each independently at each occurrence selected from the group consisting of: halo, oxo, fluoro, nitro, cyano, NR^{a}R^{b}, OR^{a}, SR^{a}, CO₂R^{a}, C(O)R^{a}, CONR^{a}R^{a}, C₁-C₄-alkyl, C₁-C₄-haloalkyl and cyclopropyl; wherein R^{a} is independently at each occurrence selected from H, C₁-C₄-alkyl and C₁-C₄-haloalkyl; and R^{b} is independently at each occurrence selected from H, C₁-C₄-alkyl, C(O)-C₁-C₄-alkyl and S(O)₂-C₁-C₄-alkyl.

In an embodiment, the compound of formula (I) is a compound of formula (**II**): 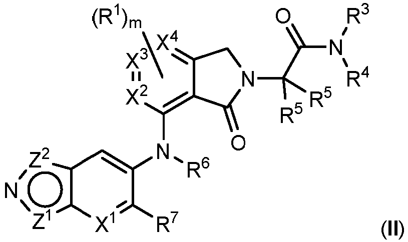 wherein Z¹, Z², X¹, X², X³, X⁴, R¹, R³, R⁴, R⁵, R⁶, R⁷ and m are as described above for compounds of formula (I).

In an embodiment, the compound of formula (I) is a compound of formula (**III**): 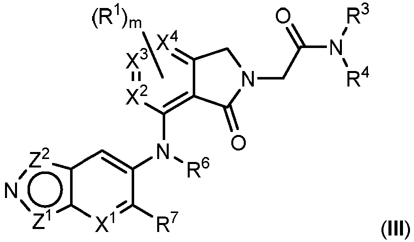 wherein Z¹, Z², X¹, X², X³, X⁴, R¹, R³, R⁴, R⁷ and m are as described above for compounds of formula (I).

In an embodiment, the compound of formula (I) is a compound of formula (**IV**): 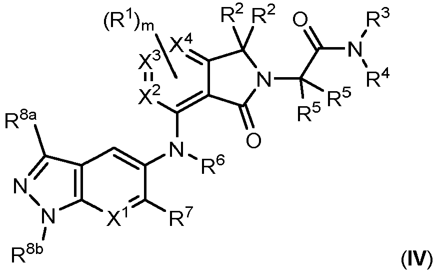 wherein X¹, X², X³, X⁴, R¹, R², R³, R⁴, R⁵, R⁶, R⁷, R^{8a}, R^{8b}, m and n are as described above for compounds of formula (**I**).

In an embodiment, the compound of formula (**I**) is a compound of formula (V): 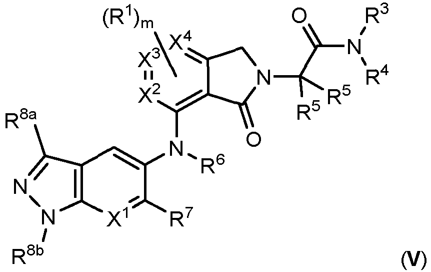 wherein X¹, X², X³, X⁴, R¹, R³, R⁴, R⁵, R⁶, R⁷, R^{8a}, R^{8b} and m are as described above for compounds of formula (I).

In an embodiment, the compound of formula (**I**) is a compound of formula (**VI**): 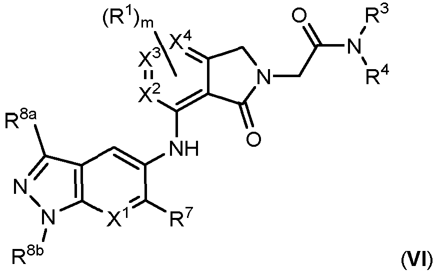 wherein Z¹, Z², X¹, X², X³, X⁴, R¹, R³, R⁴, R⁷, R^{8a}, R^{8b} and m are as described above for compounds of formula (I).

In an embodiment, the compound of formula (**I**) is a compound of formula (**VII**): 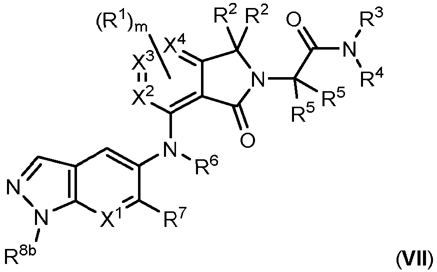 wherein X¹, X², X³, X⁴, R¹, R², R³, R⁴, R⁵, R⁶, R⁷, R^{8b}, m and n are as described above for compounds of formula (I).

In an embodiment, the compound of formula (**I**) is a compound of formula (**VIII**): 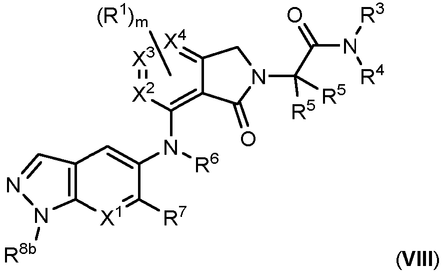 wherein X¹, X², X³, X⁴, R¹, R³, R⁴, R⁵, R⁶, R⁷, R^{8b} and m are as described above for compounds of formula (I).

In an embodiment, the compound of formula (**I**) is a compound of formula (**IX**): 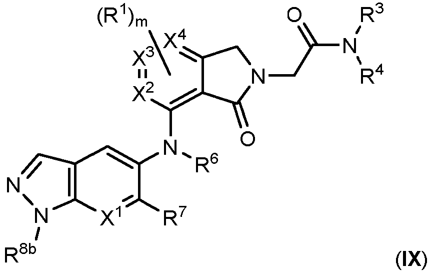 wherein X¹, X², X³, X⁴, R¹, R³, R⁴, R⁷, R^{8b} and m are as described above for compounds of formula (I).

In an embodiment, the compound of formula (**I**) is a compound of formula (**X**): 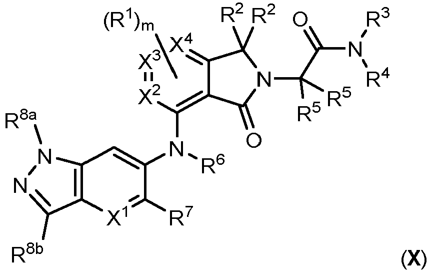 wherein X¹, X², X³, X⁴, R¹, R², R³, R⁴, R⁵, R⁶, R⁷, R^{8a,} R^{8b}, m and n are as described above for compounds of formula (I).

In an embodiment, the compound of formula (**I**) is a compound of formula (**XI**): 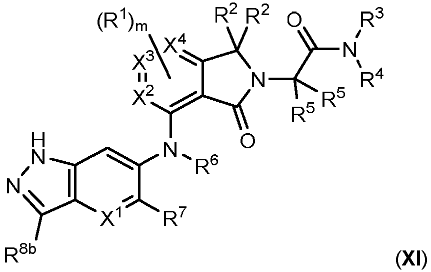 wherein X¹, X², X³, X⁴, R¹, R², R³, R⁴, R⁵, R⁶, R⁷, R^{8a}, m and n are as described above for compounds of formula (I).

In an embodiment, the compound of formula (**I**) is a compound of formula (**XII**): 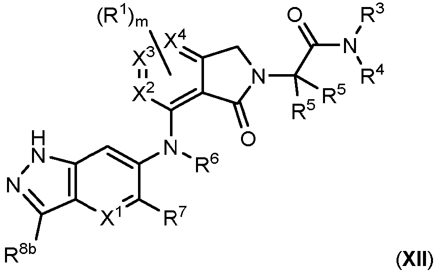 wherein X¹, X², X³, X⁴, R¹, R³, R⁴, R⁵, R⁶, R⁷, R^{8a} and m are as described above for compounds of formula (I).

In an embodiment, the compound of formula (**I**) is a compound of formula (**XIII**): 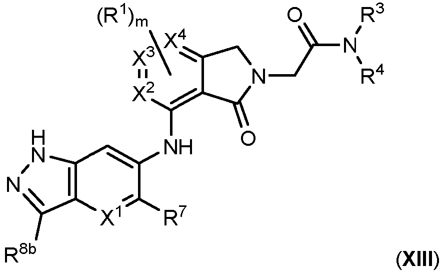 wherein X¹, X², X³, X⁴, R¹, R³, R⁴, R⁷, R^{8a} and m are as described above for compounds of formula (I).

In an embodiment, the compound of formula (**I**) is a compound of formula (**XIV**): 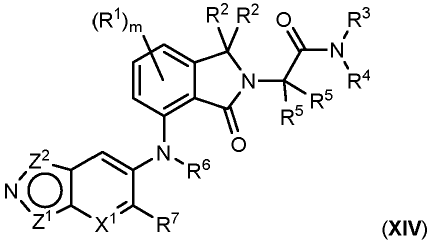 wherein Z¹, Z², X¹, R¹, R², R³, R⁴, R⁵, R⁶, R⁷ and m are as described above for compounds of formula (I).

In an embodiment, the compound of formula (**I**) is a compound of formula (**XV**): 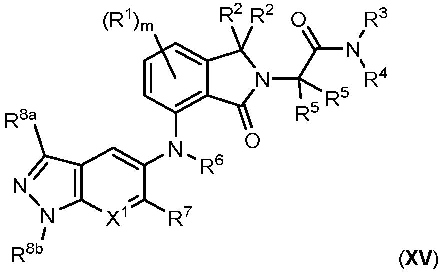 wherein X¹, R¹, R², R³, R⁴, R⁵, R⁶, R⁷, R^{8a,} R^{8b}, m and n are as described above for compounds of formula (I).

In an embodiment, the compound of formula (**I**) is a compound of formula (**XVI**): 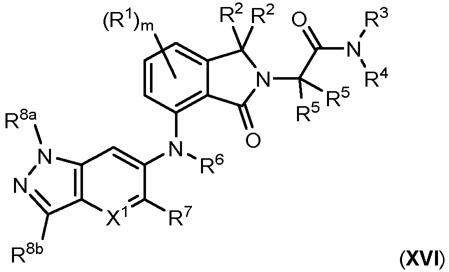 wherein X¹, X², X³, X⁴, R¹, R², R³, R⁴, R⁵, R⁶, R⁷, R^{8a,} R^{8b}, m and n are as described above for compounds of formula (I).

In an embodiment, the compound of formula (**I**) is a compound of formula (**XVII**): 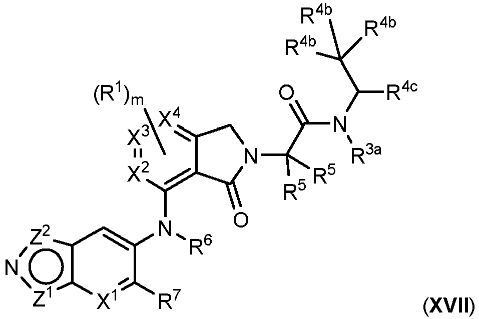
wherein Z¹, Z², X¹, X², X³, X⁴, R¹, R⁵, R⁶, R⁷ and m are as described above for compounds of formula (I) and wherein
R^{4b} is at each occurence selected from H and F; wherein at least two R^{4b} groups are F;
R^{3a} is independently selected from H and C₁-C₄-alkyl;
R^{4c} is independently selected from H, C₁-C₄-alkyl and C₄-C₆-cycloalkyl; or
R^{3a} and R^{4c}, together with the carbon and nitrogen to which they are attached, form a 4- to 6-membered heterocycloalkyl group

In an embodiment, the compound of formula (**I**) is a compound of formula (**XVIII**): 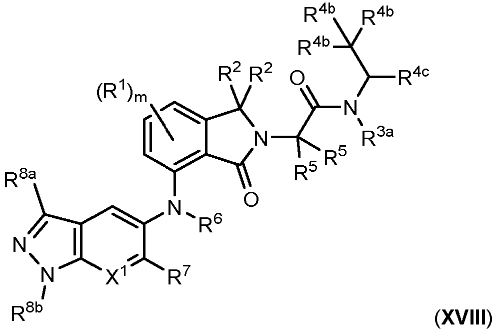 wherein X¹, R¹, R², R⁵, R⁶, R⁷, R^{8a,} R^{8b} and m are as described above for compounds of formula (**I**); and wherein R^{3a}, R^{4b} and R^{4c} are as described above for formula (**XVII**).

In an embodiment, the compound of formula (**I**) is a compound of formula (**XIX**): 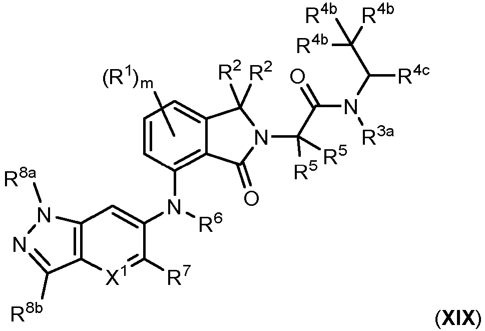 wherein X¹, R¹, R², R⁵, R⁶, R⁷, R^{8a,} R^{8b} and m are as described above for compounds of formula (I); and wherein R^{3a}, R^{4b} and R^{4c} are as described above for formula (**XVII**).

In an embodiment, the compound of formula (I) is a compound of formula (**XX**): 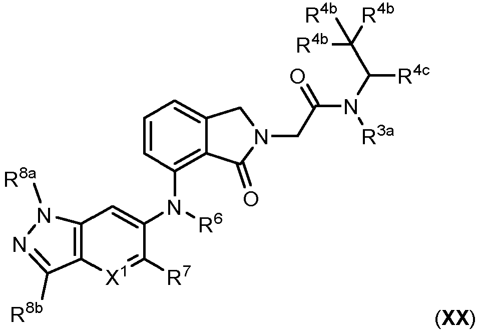 wherein X¹, R⁶, R⁷, R^{8a} and R^{8b} are as described above for compounds of formula (I); and wherein R^{3a}, R^{4b} and R^{4c} are as described above for formula (**XVII**)**.** In an embodiment, the compound of formula (**I**) is a compound of formula (**XXI**): 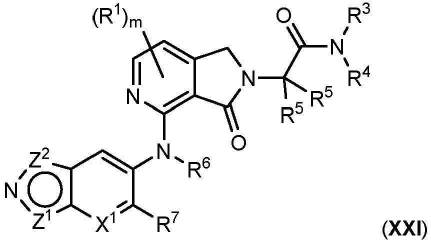 wherein Z¹, Z², X¹, R¹, R³, R⁴, R⁵, R⁶, R⁷ and m are as described above for compounds of formula (I).

The following embodiments apply to compounds of any of formulae (**I**)-(**XXI**). These embodiments are independent and interchangeable. Any one embodiment may be combined with any other embodiment, where chemically allowed. In other words, any of the features described in the following embodiments may (where chemically allowable) be combined with the features described in one or more other embodiments. In particular, where a compound is exemplified or illustrated in this specification, any two or more of the embodiments listed below, expressed at any level of generality, which encompass that compound may be combined to provide a further embodiment which forms part of the present disclosure.

It may be either that Z¹ is NR^{8b} and Z² is CH or that Z¹ is CR^{8a} and Z² is NH.

It may be that Z¹ is NR^{8b} and Z² is CR^{8a}. It may be that Z¹ is NR^{8b} and Z² is CH. It may be that Z¹ is NMe and Z² is CR^{8a}. It may be that Z¹ is NMe and Z² is CH.

It may be that Z¹ is CR^{8a} and Z² is NR^{8b}. It may be that Z¹ is CR^{8a} and Z² is NH.

It may be that X¹ is N. It may be that X¹ is CR^{7a}.

It may be that X² is carbon. It may be that X³ is carbon. It may be that X⁴ is carbon. It may be that X² and X³ are both carbon. It may be that X³ and X⁴ are both carbon. It may be that X² and X⁴ are both carbon. It may be that X², X³ and X⁴ are each carbon.

It may be that no more than one of X², X³ and X⁴ are nitrogen. It may be that a single one of X², X³ and X⁴ is nitrogen. It may be that X² is nitrogen. It may be that X³ is nitrogen. It may be that X⁴ is nitrogen. It may be that X² is nitrogen, X³ is carbon and X⁴ is carbon.

m may be 0.

m may be 1. Where m is 1, it may be that the single R¹ group is situated ortho to NR⁶. Where m is 1, it may be that the single R¹ group is situated para to NR⁶. Where m is 1, it may be that the single R¹ group is situated para to NR⁸.

m may be 2.

R¹ may be independently at each occurrence selected from halo, nitro, cyano, OR¹¹, C₁-C₄-alkyl, C₁-C₄-alkyl substituted with NR⁹R¹⁰, C₁-C₄-alkyl substituted with OR¹¹, C₁-C₄-haloalkyl and cyclopropyl. R¹ may be independently at each occurrence selected from halo, OR¹¹, C₁-C₄-alkyl, C₁-C₄-haloalkyl and cyclopropyl. R¹ may be independently at each occurrence selected from halo and C₁-C₄-alkyl. R¹ may be independently at each occurrence halo, e.g. fluoro. R¹ may be independently at each occurrence C₁-C₄-alkyl, e.g. methyl. R¹ may be H.

It may be that m is 1, the single R¹ group is F and is situated ortho to NR⁶.

It may be that m is 0, R² at each occurrence is H and R⁵ at each occurrence is H.

R² may be independently at each occurrence selected from H, fluoro, nitro, cyano, OR¹¹, C₁-C₄-alkyl, C₁-C₄-alkyl substituted with NR⁹R¹⁰, C₁-C₄-alkyl substituted with OR¹¹, C₁-C₄-haloalkyl and cyclopropyl. R² may be independently at each occurrence selected from H, fluoro, OR¹¹, C₁-C₄-alkyl, C₁-C₄-haloalkyl and cyclopropyl. R² may be independently at each occurrence selected from H, fluoro and C₁-C₄-alkyl. R² may be independently at each occurrence selected from fluoro and C₁-C₄-alkyl. R² may be independently at each occurrence selected from H and C₁-C₄-alkyl. R² may be at each occurrence H. R² may be at each occurrence fluoro. R² may be at each occurrence C₁-C₄-alkyl, e.g. methyl. It may be that the R² groups and the carbon atom to which they are attached together form a C₃-C₆-cycloalkyl ring, e.g. cyclopropyl.

R⁵ may be independently at each occurrence selected from H, fluoro and C₁-C₄-alkyl. R⁵ may be independently at each occurrence selected from H, fluoro and C₁-C₄-alkyl, or the two R⁵ groups and the carbon atom to which they are attached may together form a C₃-C₆ cycloalkyl ring. R⁵ may be independently at each occurrence selected from H and C₁-C₄-alkyl, or the two R⁵ groups and the carbon atom to which they are attached may together form a C₃-C₆ cycloalkyl ring. R⁵ may be independently at each occurrence selected from H and C₁-C₄-alkyl, e.g. methyl.

It may be that R⁵ is at each occurrence H. It may be that R⁵ is at one occurrence H and at the other occurrence C₁-C₄-alkyl, e.g. methyl. It may be that R⁵ is at each occurrence C₁-C₄-alkyl, e.g. methyl.

R⁶ may be H. R⁶ may be C₁-C₄-alkyl, e.g. methyl.

It may be that R⁷ is H. R⁷ may be independently selected from halo, nitro, cyano, OR¹¹, C₁-C₄-alkyl, C₁-C₄-alkyl substituted with NR⁹R¹⁰, C₁-C₄-alkyl substituted with OR¹¹, C₁-C₄-haloalkyl and cyclopropyl. R⁷ may be independently selected from halo, OR¹¹, C₁-C₄-alkyl, C₁-C₄-haloalkyl and cyclopropyl. R⁷ may be independently selected from halo and C₁-C₄-alkyl. If present, R⁷ may be halo, e.g. fluoro. R⁷ may be C₁-C₄-alkyl, e.g. methyl.

It may be that R^{7a} is H. R^{7a} may be independently selected from halo, nitro, cyano, OR¹¹, CO₂R⁹, CONR⁹R⁹, C₁-C₄-alkyl, C₁-C₄-alkyl substituted with NR⁹R¹⁰, C₁-C₄-alkyl substituted with OR¹¹, C₁-C₄-haloalkyl and cyclopropyl. R^{7a} may be independently selected from halo, OR¹¹, C₁-C₄-alkyl, C₁-C₄-haloalkyl and cyclopropyl.

It may be that R⁷ is H and X¹ is N. It may be that R⁷ is H and X¹ is CR^{7a}. It may be that R⁷ is H and X¹ is CH. It may be that R⁷ is H and X¹ is CR^{7a}, wherein R^{7a} is independently selected from halo, nitro, cyano, OR¹¹, CO₂R⁹, CONR⁹R⁹, C₁-C₄-alkyl, C₁-C₄-alkyl substituted with NR⁹ R¹⁰, C₁-C₄-alkyl substituted with OR¹¹, C₁-C₄-haloalkyl and cyclopropyl.

R^{8a} may be H. R^{8a} may be selected from halo, C₁-C₄-alkyl, C₁-C₄-alkyl substituted with NR⁹R¹⁰, C₁-C₄-alkyl substituted with OR¹¹, C₁-C₄-haloalkyl and C₀-C₄-alkylene-R⁸°. R^{8a} may be selected from H, halo, C₁-C₄-alkyl, C₁-C₄-haloalkyl and cyclopropyl. R^{8a} may be selected from H, C₁-C₄-alkyl, C₁-C₄-haloalkyl and cyclopropyl. R^{8a} may be selected from C₁-C₄-alkyl (e.g. methyl) and C₁-C₄-haloalkyl (e.g. CF₃). R^{8a} may be C₁-C₄-alkyl (e.g. methyl). R^{8a} may be halo e.g., F.

R^{8b} may be H. R^{8b} may be selected from C₁-C₄-alkyl, C₂-C₄-alkyl substituted with NR⁹R¹⁰, C₂-C₄-alkyl substituted with OR¹¹, C₁-C₄-haloalkyl and C₀-C₄-alkylene-R⁸°. R^{8b} may be selected from H, C₁-C₄-alkyl and cyclopropyl. R^{8b} may be C₁-C₄-alkyl (e.g. methyl).

R^{8b} is independently selected from H, C₁-C₄-alkyl, C₂-C₄-alkyl substituted with NR⁹R¹⁰, C₂-C₄-alkyl substituted with OR¹¹, C₃-C₄-alkenyl, C₃-C₄-alkynyl, C₁-C₄-haloalkyl and C₀-C₄-alkylene-R^{8c}.

It may be that R^{8a} is H and R^{8b} is C₁-C₄-alkyl (e.g. methyl). It may be that R^{8a} is C₁-C₄-alkyl (e.g. methyl) and R^{8b} is C₁-C₄-alkyl (e.g. methyl). It may be that R^{8a} and R^{8b} are both methyl.

It may be that R⁹ is independently at each occurrence selected from H and C₁-C₄-alkyl.

It may be that R¹⁰ is independently at each occurrence selected from H, C₁-C₄-alkyl, C(O)-C₁-C₄-alkyl and S(O)₂-C₁-C₄-alkyl. It may be that R¹⁰ is independently at each occurrence selected from H and C₁-C₄-alkyl.

It may be that R¹¹ is independently at each occurrence selected from H, C₁-C₄-alkyl and C₁-C₄-haloalkyl. It may be that R¹¹ is independently at each occurrence selected from H and C₁-C₄-alkyl. It may be that R¹¹ is independently at each occurrence C₁-C₄-alkyl, e.g. methyl.

R¹² may be independently selected from 5- or 6- membered heteroaryl, wherein said heteroaryl group is optionally substituted with from 1 to 3 R¹⁴ groups. R¹² may be independently selected from 5-membered heteroaryl, e.g. imidazole, wherein said heteroaryl group is optionally substituted with from 1 to 3 R¹⁴ groups.

R¹³ may be independently at each occurrence selected from oxo, fluoro, OR¹¹, CO₂R⁹, CO₂NR⁹R⁹, C₁-C₄-alkyl, C₁-C₄-alkyl substituted with NR⁹R¹⁰, C₁-C₄-alkyl substituted with OR¹¹, C₁-C₄-haloalkyl and cyclopropyl. R¹³ may be independently at each occurrence selected from oxo, OR¹¹, C₁-C₄-alkyl and cyclopropyl. R¹³ may be independently at each occurrence selected from oxo and C₁-C₄-alkyl. R¹³ may be independently at each occurrence C₁-C₄-alkyl, e.g. methyl.

R¹⁴ may be independently at each occurrence selected from halo, nitro, cyano, OR¹¹, C₁-C₄-alkyl, C₁-C₄-alkyl substituted with NR⁸R⁹, C₁-C₄-alkyl substituted with OR¹¹, C₁-C₄-haloalkyl and cyclopropyl. R¹⁴ may be independently at each occurrence selected from halo, OR¹¹, C₁-C₄-alkyl, C₁-C₄-haloalkyl and cyclopropyl. R¹⁴ may be independently at each occurrence selected from halo and C₁-C₄-alkyl. R¹⁴ may be independently at each occurrence halo, e.g. fluoro. R¹⁴ may be independently at each occurrence C₁-C₄-alkyl, e.g. methyl.

It may be that:
R³ is H; and
R⁴ is independently selected from C₁-C₆-alkyl, C₁-C₆-haloalkyl, C₀-C₄-alkylene-R^{4a}; wherein R^{4a} is independently selected from: C₃-C₈-cycloalkyl, phenyl, 5-, 6-, 9- or 10-membered heteroaryl and 4- to 10-membered heterocycloalkyl; wherein said heterocycloalkyl or heteroaryl group may be monocyclic or bicyclic; wherein said cycloalkyl or heterocycloalkyl group is optionally substituted with a single R¹² group and/or from 1 to 4 R¹³ groups and wherein said phenyl or heteroaryl group is optionally substituted with a single R¹² group and/or from 1 to 3 R¹⁴ groups;
or R³ and R⁴, together with the nitrogen atom to which they are attached together form a 4- to 10-membered heterocycloalkyl group or a 5-, or 9-membered heteroaryl group; wherein said heterocycloalkyl group is optionally substituted with a single R¹² group and/or from 1 to 4 R¹³ groups and wherein said heteroaryl group is optionally substituted with a single R¹² group and/or from 1 to 3 R¹⁴ groups.

It may be that:
R³ is H; and
R⁴ is independently selected from C₁-C₆-alkyl, C₁-C₆-haloalkyl, C₀-C₄-alkylene-R^{4a}; wherein R^{4a} is independently selected from: C₃-C₈-cycloalkyl, phenyl, 5-, 6-, 9- or 10-membered heteroaryl and 4- to 10-membered heterocycloalkyl; wherein said heterocycloalkyl or heteroaryl group may be monocyclic or bicyclic; wherein said cycloalkyl or heterocycloalkyl group is optionally substituted with from 1 to 4 R¹³ groups and wherein said phenyl or heteroaryl group is optionally substituted with from 1 to 3 R¹⁴ groups;
or R³ and R⁴, together with the nitrogen atom to which they are attached together form a 4- to 10-membered heterocycloalkyl group or a 5-, or 9-membered heteroaryl group; wherein said heterocycloalkyl group is optionally substituted with from 1 to 4 R¹³ groups and wherein said heteroaryl group is optionally substituted with from 1 to 3 R¹⁴ groups.

It may be that R³ is H.

It may be that R⁴ is selected from C₁-C₆-alkyl, C₁-C₆-haloalkyl and C₀-C₄-alkylene-R^{4a}.

It may be that R⁴ is selected from C₁-C₆-alkyl and C₁-C₆-haloalkyl. It may be that R⁴ is selected from C₂-C₃-alkyl and C₂-C₃-haloalkyl. It may be that R⁴ is C₁-C₄-haloalkyl. It may be that R⁴ is C₂-C₃-haloalkyl. It may be that R⁴ is 2,2,2-trifluoroethyl.

Illustrative R⁴ groups include:

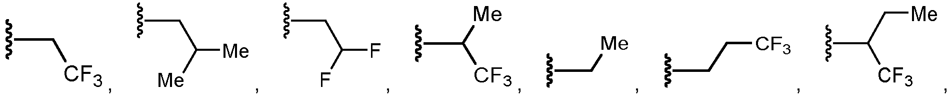

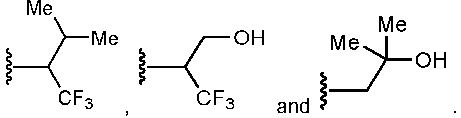

It may be that R⁴ is C₀-C₄-alkylene-R^{4a}. It may be that R⁴ is CH₂-R^{4a}. It may be that R⁴ is R^{4a}.

It may be that R^{4a} is independently selected from C₃-C₈-cycloalkyl, phenyl, 5-, 6-, 9- or 10-membered heteroaryl, 4- to 10-membered heterocycloalkyl, wherein said heterocycloalkyl or heteroaryl group may be monocyclic or bicyclic; wherein said cycloalkyl or heterocycloalkyl group is optionally substituted with from 1 to 4 R¹³ groups and wherein said phenyl or heteroaryl group is optionally substituted with from 1 to 3 R¹⁴ groups.

It may be that R^{4a} is selected from C₃-C₈-cycloalkyl and 4- to 10-membered heterocycloalkyl, wherein said cycloalkyl or heterocycloalkyl group is optionally substituted with a single R¹² group and/or from 1 to 4 R¹³ groups. It may be that R^{4a} is selected from C₃-C₈-cycloalkyl and 4- to 10-membered heterocycloalkyl, wherein said cycloalkyl or heterocycloalkyl group is optionally substituted with from 1 to 4 R¹³ groups.

It may be that R⁴ is selected from CH₂-C₃-C₈-cycloalkyl and CH₂-4- to 10-membered heterocycloalkyl, wherein said cycloalkyl or heterocycloalkyl group is optionally substituted with a single R¹² group and/or from 1 to 4 R¹³ groups. It may be that R⁴ is selected from CH₂-C₃-C₈-cycloalkyl and CH₂-4- to 10-membered heterocycloalkyl, wherein said cycloalkyl or heterocycloalkyl group is optionally substituted with from 1 to 4 R¹³ groups.

It may be that R⁴ is selected from C₃-C₈-cycloalkyl and 4- to 10-membered heterocycloalkyl, wherein said cycloalkyl or heterocycloalkyl group is optionally substituted with a single R¹² group and/or from 1 to 4 R¹³ groups. It may be that R⁴ is selected from C₃-C₈-cycloalkyl and 4- to 10-membered heterocycloalkyl, wherein said cycloalkyl or heterocycloalkyl group is optionally substituted with from 1 to 4 R¹³ groups.

Illustrative R⁴ groups include: 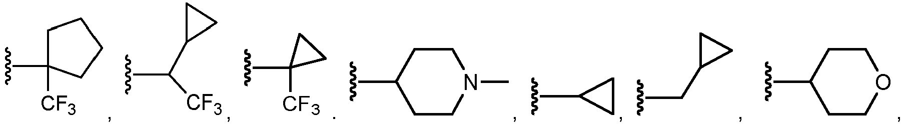 and

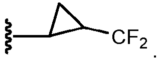

It may be that R^{4a} is independently selected from: phenyl and 5- or 6- membered heteroaryl; wherein said phenyl or heteroaryl group is optionally substituted with a single R¹² group and/or from 1 to 3 R¹⁴ groups. It may be that R^{4a} is independently selected from: phenyl and 5- or 6- membered heteroaryl; wherein said phenyl or heteroaryl group is optionally substituted with from 1 to 3 R¹⁴ groups. It may be that R^{4a} is independently phenyl; wherein said phenyl group is optionally substituted with a single R¹² group and/or from 1 to 3 R¹⁴ groups. It may be that R^{4a} is independently phenyl; wherein said phenyl group is optionally substituted with from 1 to 3 R¹⁴ groups. It may be that R^{4a} is independently 5- or 6- membered heteroaryl; wherein said heteroaryl group is optionally substituted with a single R¹² group and/or from 1 to 3 R¹⁴ groups. It may be that R^{4a} is independently 5- or 6- membered heteroaryl; wherein said heteroaryl group is optionally substituted with from 1 to 3 R¹⁴ groups.

It may be that R⁴ is independently selected from CH₂-phenyl or CH₂-5- or 6- membered heteroaryl wherein said phenyl or heteroaryl group is optionally substituted with a single R¹² group and/or from 1 to 3 R¹⁴ groups. It may be that R⁴ is independently selected from CH₂-phenyl or CH₂-5- or 6-membered heteroaryl wherein said phenyl or heteroaryl group is optionally substituted with from 1 to 3 R¹⁴ groups. It may be that R⁴ is independently CH₂-phenyl wherein said phenyl is optionally substituted with a single R¹² group and/or from 1 to 3 R¹⁴ groups. It may be that R⁴ is independently CH₂-phenyl wherein said phenyl group is optionally substituted with from 1 to 3 R¹⁴ groups. It may be that R⁴ is independently CH₂-5- or 6- membered heteroaryl wherein said heteroaryl group is optionally substituted with a single R¹² group and/or from 1 to 3 R¹⁴ groups. It may be that R⁴ is independently CH₂-5- or 6-membered heteroaryl wherein said heteroaryl group is optionally substituted with from 1 to 3 R¹⁴ groups.

It may be that R⁴ is independently selected from phenyl or 5- or 6- membered heteroaryl wherein said phenyl or heteroaryl group is optionally substituted with a single R¹² group and/or from 1 to 3 R¹⁴ groups. It may be that R⁴ is independently selected from phenyl or 5- or 6- membered heteroaryl wherein said phenyl or heteroaryl group is optionally substituted with from 1 to 3 R¹⁴ groups. It may be that R⁴ is independently phenyl wherein said phenyl group is optionally substituted with a single R¹² group and/or from 1 to 3 R¹⁴ groups. It may be that R⁴ is independently phenyl wherein said phenyl group is optionally substituted with from 1 to 3 R¹⁴ groups. It may be that R⁴ is independently 5- or 6-membered heteroaryl wherein said heteroaryl group is optionally substituted with a single R¹² group and/or from 1 to 3 R¹⁴ groups. It may be that R⁴ is independently 5- or 6- membered heteroaryl wherein said heteroaryl group is optionally substituted with from 1 to 3 R¹⁴ groups.

It may be that R⁴ is independently selected from phenyl or 6-membered heteroaryl wherein said phenyl or 6-membered heteroaryl group is substituted at the meta position with 1 R¹⁴ group. It may be that the R¹⁴ group is selected from C₁-C₄-alkyl substituted with OR¹¹, C₁-C₄-alkyl and C₁-C₄-haloalkyl. It may be that the R¹⁴ group at the meta position is C₁-C₄-alkyl substituted with OR¹¹ e.g. -(CH₃)₂-OH. It may be that the R¹⁴ group at the meta position is C₁-C₄-haloalkyl e.g. CF₃.

It may be that R⁴ is a 6-membered heteroaryl group. It may be that R⁴ is phenyl. It may be that R⁴ is phenyl substituted at the meta position with 1 R¹⁴ group. It may be that R¹⁴ is R^{14a}. Illustrative R⁴ groups include: 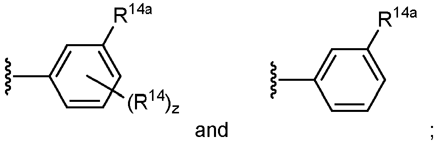 wherein R^{14a} is selected from halo, nitro, cyano, NR⁸R⁹, OR¹⁰, SR⁸, SO₂R⁸, SO₂NR⁸R⁸, CO₂R⁸, C(O)R⁸, CONR⁸R⁸, C₁-C₄-alkyl, C₂-C₄-alkenyl, C₂-C₄-alkynyl, C₁-C₄-haloalkyl, C₁-C₄-alkyl substituted with OR¹¹, C₁-C₄-alkyl substituted with NR⁸R⁹ and cyclopropyl; and wherein z is an integer selected from 1 and 2.

Illustrative R⁴ groups include: 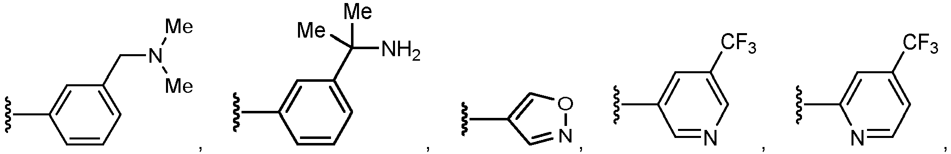 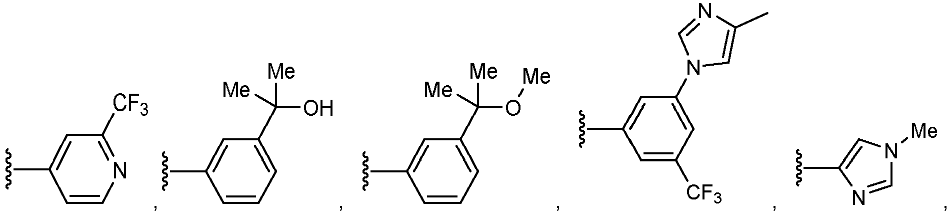 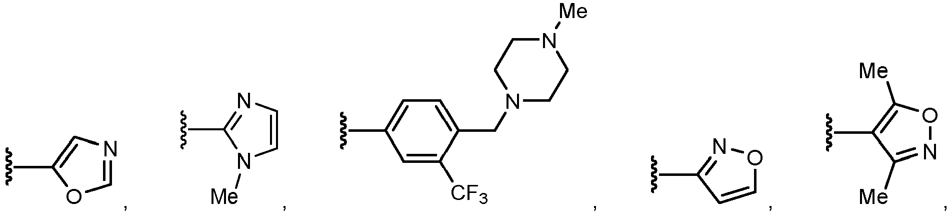 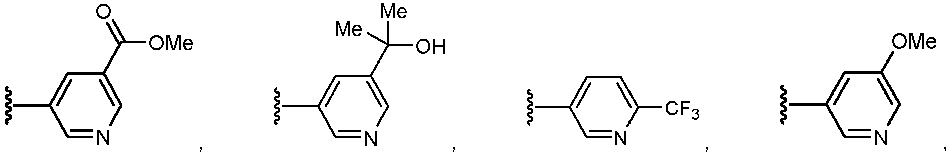 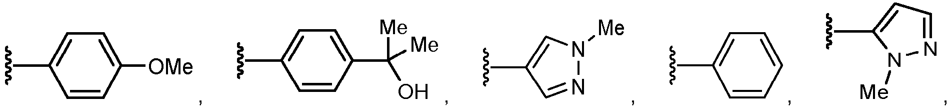 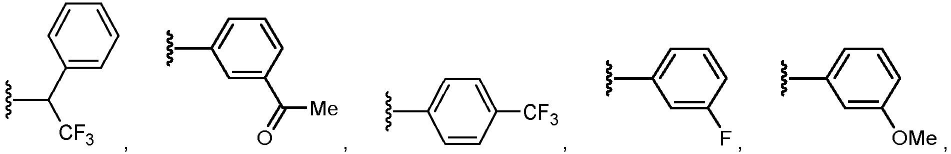 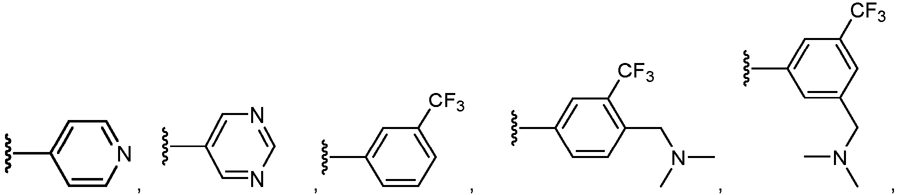 and

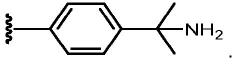

It may be that R³ and R⁴, together with the nitrogen atom to which they are attached together form a 4- to 10-membered heterocycloalkyl group or a 5-, or 9-membered heteroaryl group; wherein said heterocycloalkyl group is optionally substituted with a single R¹² group and/or from 1 to 4 R¹³ groups and wherein said heteroaryl group is optionally substituted with a single R¹² group and/or from 1 to 3 R¹⁴ groups. It may be that R³ and R⁴, together with the nitrogen atom to which they are attached together form a 4- to 10-membered heterocycloalkyl group or a 5-, or 9- membered heteroaryl group; wherein said heterocycloalkyl group is optionally substituted with from 1 to 4 R¹³ groups and wherein said heteroaryl group is optionally substituted with from 1 to 3 R¹⁴ groups.

It may be that R³ and R⁴, together with the nitrogen atom to which they are attached together form a 5-, or 9-membered heteroaryl group; wherein heteroaryl group is optionally substituted with a single R¹² group and/or from 1 to 3 R¹⁴ groups. It may be that R³ and R⁴, together with the nitrogen atom to which they are attached together form a 5-, or 9-membered heteroaryl group; wherein said heteroaryl group is optionally substituted with from 1 to 3 R¹⁴ groups. It may be that R³ and R⁴, together with the nitrogen atom to which they are attached together form a 5- membered heteroaryl group; wherein heteroaryl group is optionally substituted with a single R¹² group and/or from 1 to 3 R¹⁴ groups. It may be that R³ and R⁴, together with the nitrogen atom to which they are attached together form a 5-membered heteroaryl group; wherein said heteroaryl group is optionally substituted with from 1 to 3 R¹⁴ groups.

Illustrative NR³R⁴ groups include:

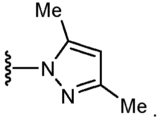

It may be that R³ and R⁴, together with the nitrogen atom to which they are attached together form a 4- to 10-membered heterocycloalkyl group; wherein said heterocycloalkyl group is optionally substituted with a single R¹² group and/or from 1 to 4 R¹³ groups. It may be that R³ and R⁴, together with the nitrogen atom to which they are attached together form a 4- to 10-membered heterocycloalkyl group; wherein said heterocycloalkyl group is optionally substituted with from 1 to 4 R¹³ groups. It may be that said heterocycloalkyl group is 7- to 10-membered bicyclic heterocycloalkyl group. It may be that said heterocyclic group is 7- to 10-membered bridged bicyclic heterocycloalkyl group. It may be that said heterocyclic group is a monocyclic 4- to 7- membered heterocycloalkyl group. It may be that said heterocyclic group is a monocyclic 5- to 6- membered heterocycloalkyl group. It may be that said heterocyclic group is pyrrolidine. It may be that said heterocyclic group is piperidine. It may be that said heterocyclic group is morpholine. It may be that said heterocyclic group is piperazine. For the absence of doubt the heterocycloalkyl groups mentioned in this paragraph are optionally substituted with a single R¹² group and/or from 1 to 4 R¹³ groups. It may be that the heterocycloalkyl groups mentioned in this paragraph are optionally substituted with from 1 to 4 R¹³ groups. It may be that said heterocyclic group is pyrrolidine and is substituted at the 2 position with 1 R¹³ group. It may be that said heterocyclic group is pyrrolidine and is substituted at the 3 position with 1 R¹³ group. It may be that said heterocyclic group is piperidine and is substituted at the 2 position with 1 R¹³ group. It may be that said heterocyclic group is piperidine and is substituted at the 3 position with 1 R¹³ group. It may be that said heterocyclic group is piperidine and is substituted at the 4 position with 1 R¹³ group. It may be that said heterocyclic group is morpholine and is substituted at the 2 position with 1 R¹³ group. It may be that said heterocyclic group is morpholine and is substituted at the 3 position with 1 R¹³ group.. It may be that the R¹³ group is selected from C₁-C₄-alkyl, C₁-C₄-alkyl substituted with OR¹¹ e.g. -(CH₃)₂-OH, and C₁-C₄-haloalkyl, e.g. - CF₃. It may be that the R¹³ group is C₁-C₄-haloalkyl, e.g. -CHF₂ or -CF₃.

Illustrative NR³R⁴ groups include:

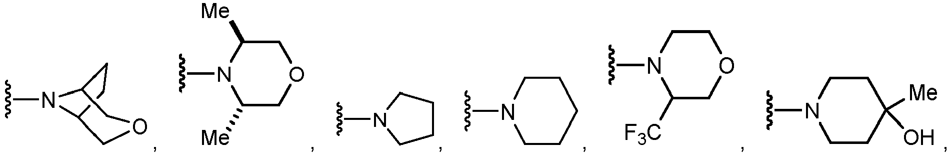

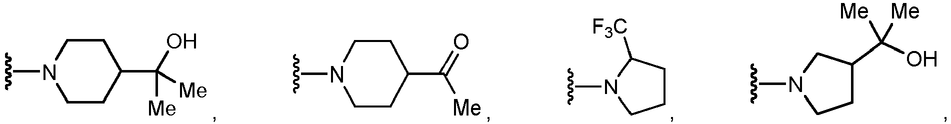

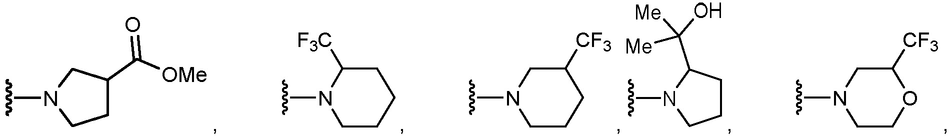

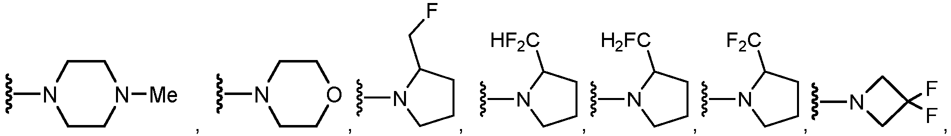

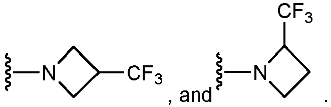

It may be that R³ and R⁴ are selected such that NR³R⁴ comprises a CHF₂ group or a CF₃ group.

It may be that NR³R⁴ has the formula 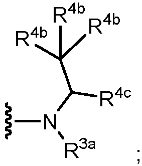 wherein
R^{4b} is at each occurence selected from H and F; wherein at least one R^{4b} group is F; R^{3a} is independently selected from H and C₁-C₄-alkyl; R^{4c} is independently selected from H, C₁-C₄-alkyl and C₄-C₆-cycloalkyl; or R^{3a} and R^{4c}, together with the carbon and nitrogen to which they are attached, form a 4- to 6-membered heterocycloalkyl group.

It may be that at least two R^{4b} groups are F. It may be that two R^{4b} groups are F and one R^{4b} group is H. It may be that each R^{4b} group is F.

It may be that R^{3a} is independently selected from H and C₁-C₄-alkyl; and R^{4c} is independently selected from H, C₁-C₄-alkyl and C₄-C₆-cycloalkyl. It may be that R^{3a} is H; and R^{4c} is independently selected from H, C₁-C₄-alkyl and C₄-C₆-cycloalkyl. Said alkyl or cycloalkyl group may be unsubstituted.

It may be that R^{3a} and R^{4c}, together with the carbon and nitrogen to which they are attached, form a 4- to 6-membered heterocycloalkyl group. It may be that R^{3a} and R^{4c}, together with the carbon and nitrogen to which they are attached, form a 5-membered heterocycloalkyl group. Said heterocycloalkyl group may be unsubstituted.

The compound of formula (I) may be selected from:

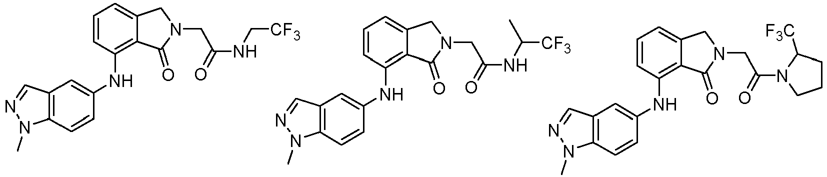

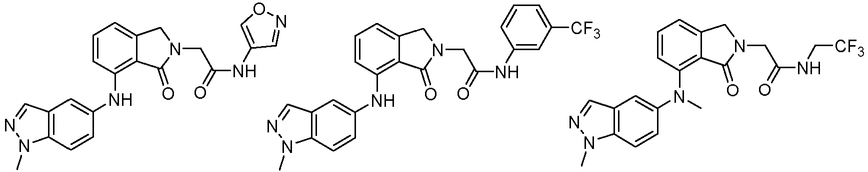

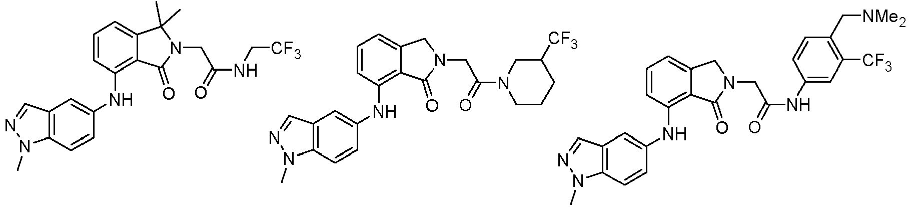

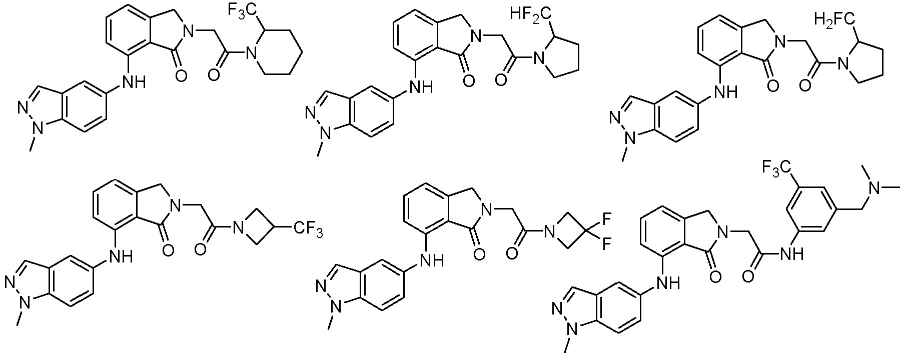

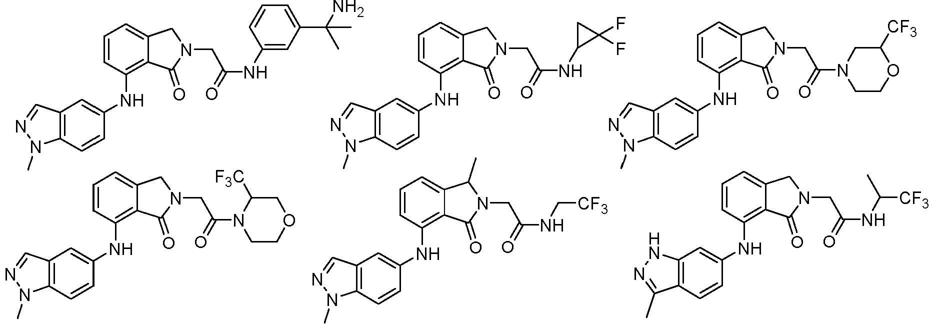

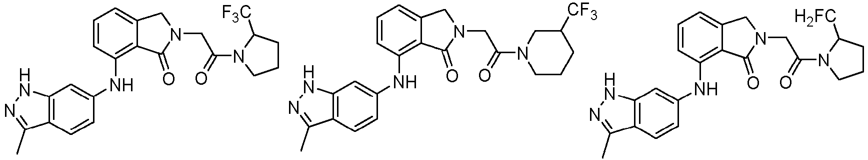

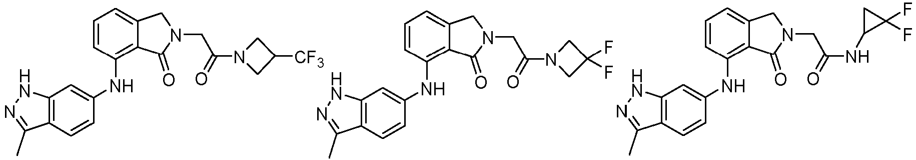

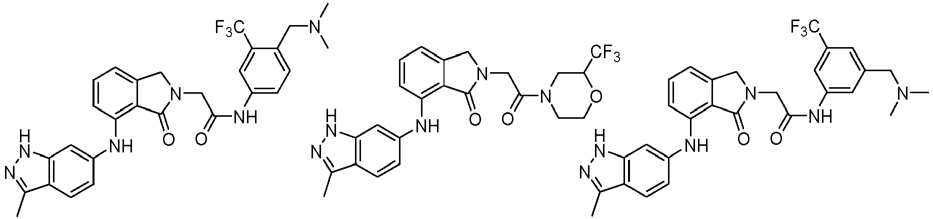

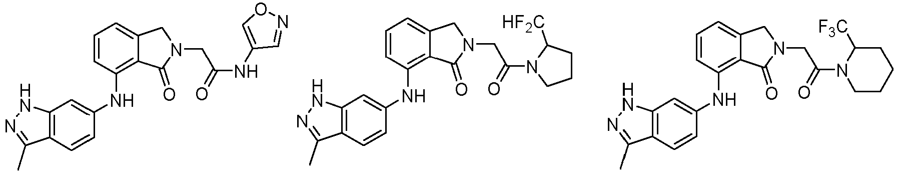

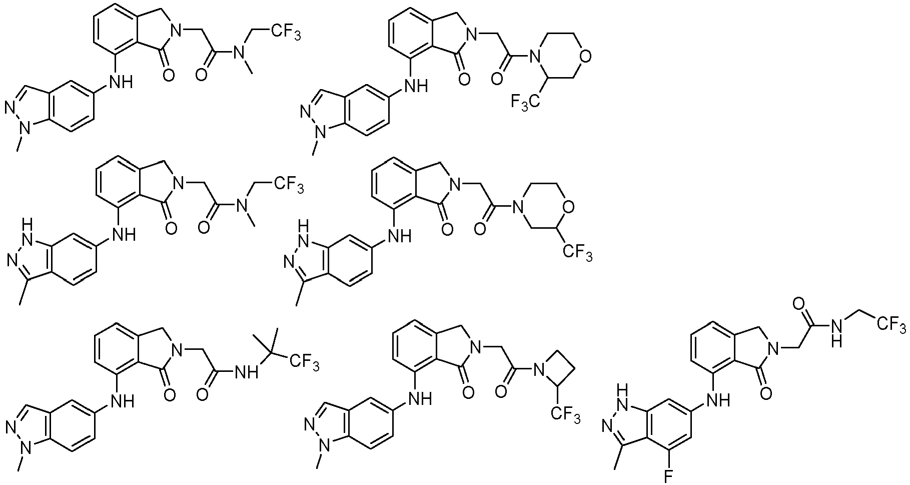

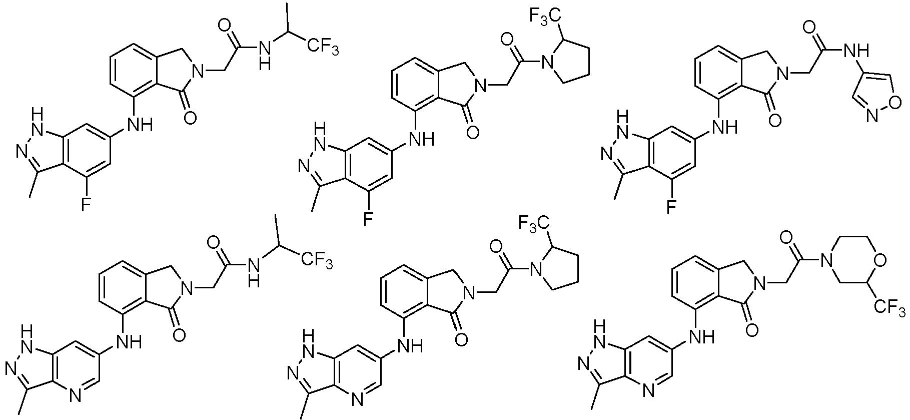

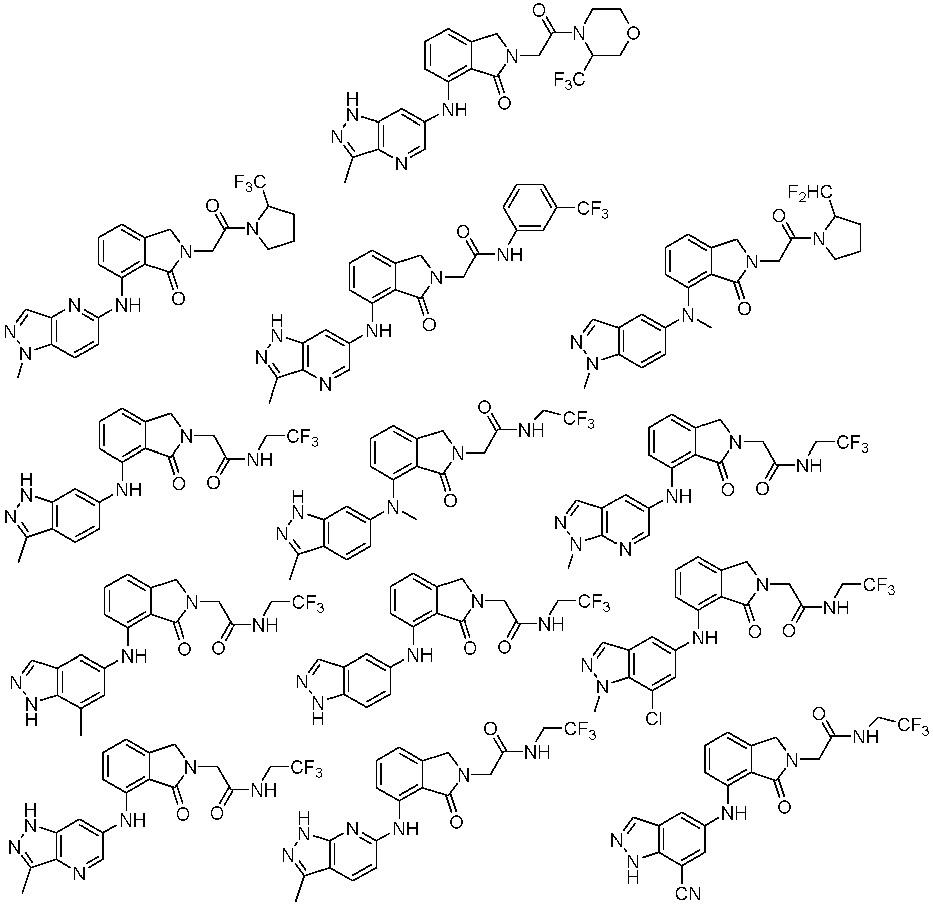

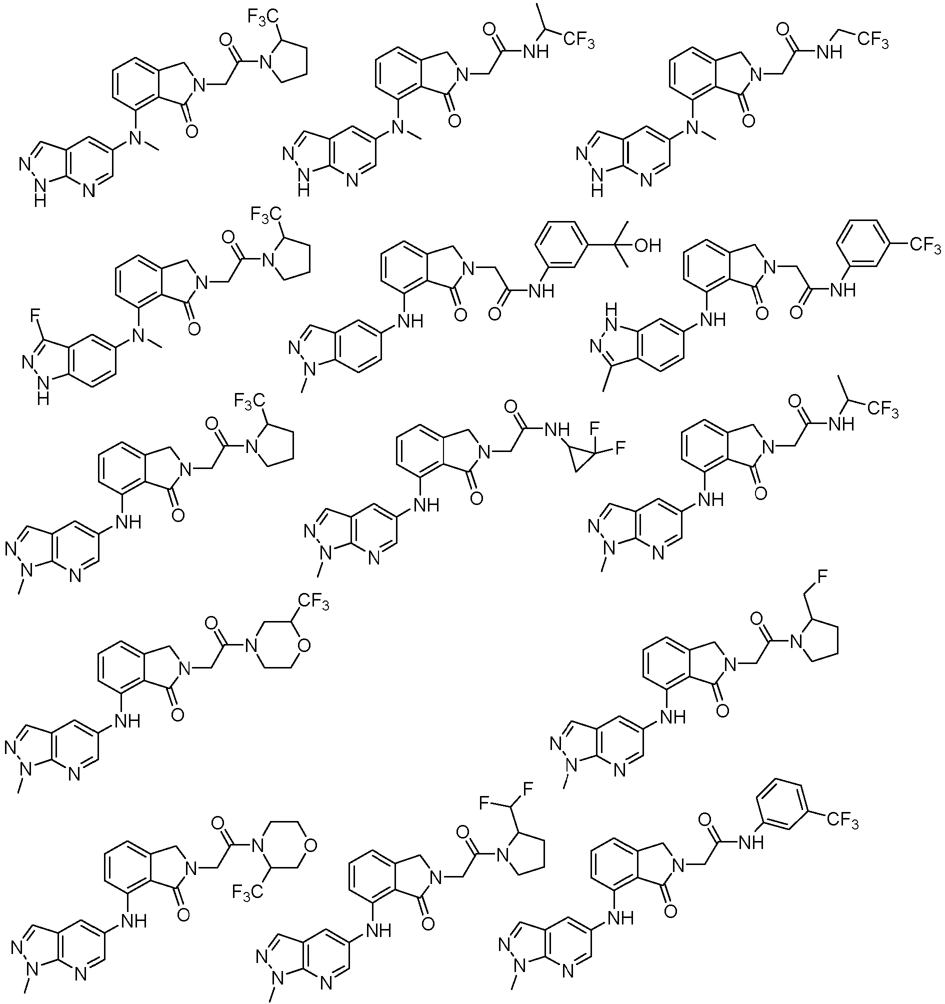

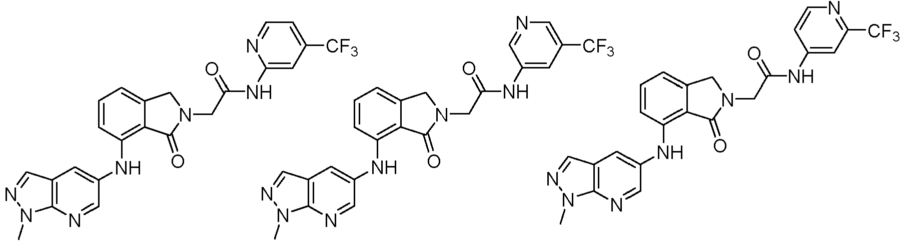

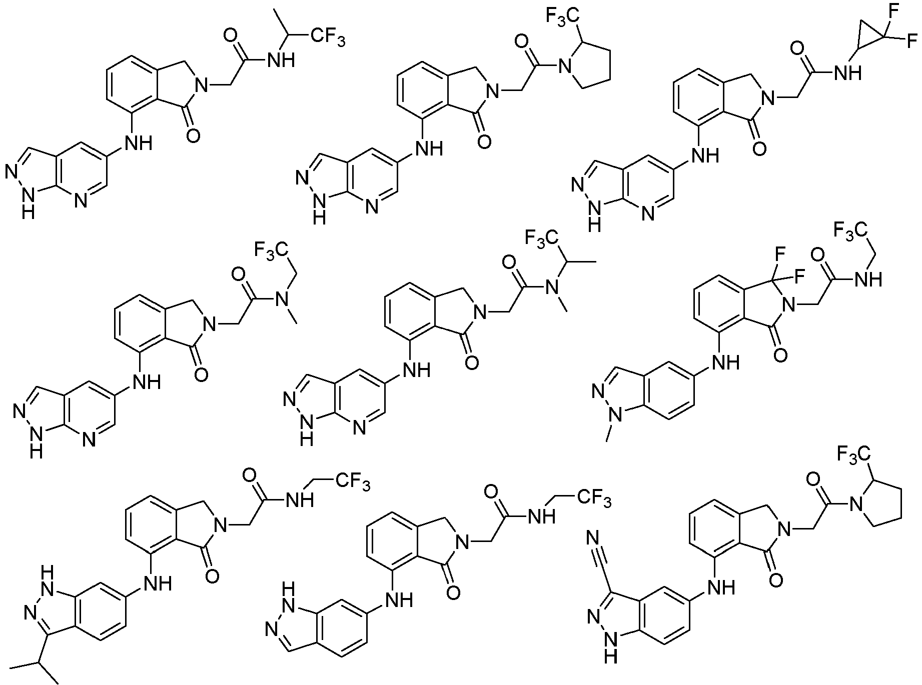

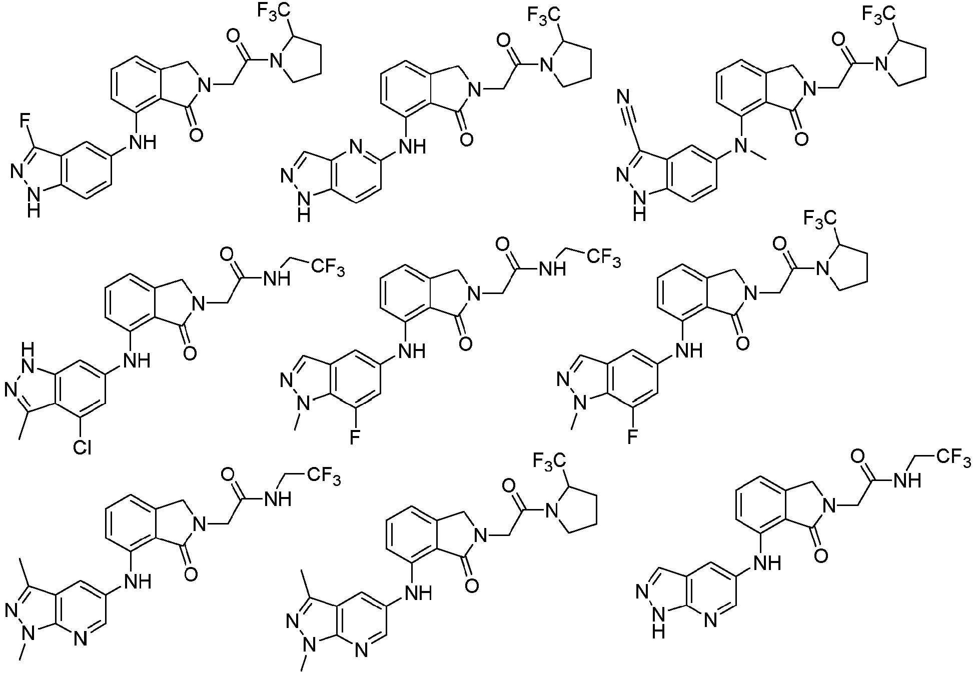

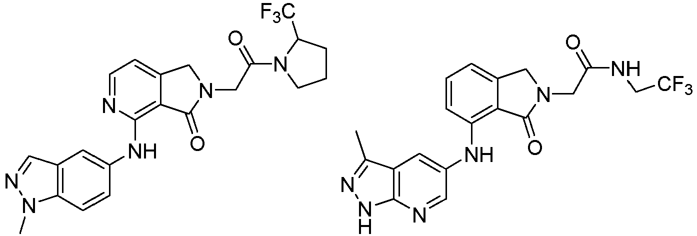

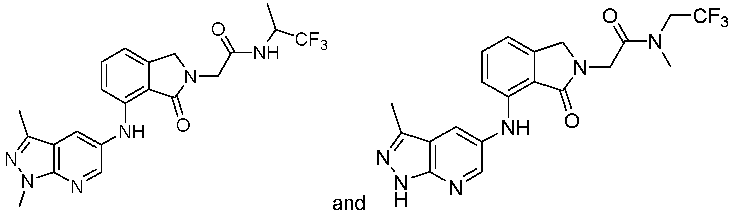

The compound of formula (I) may be selected from:

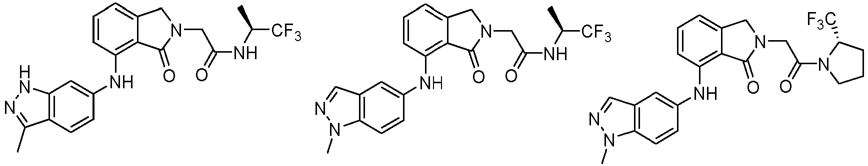

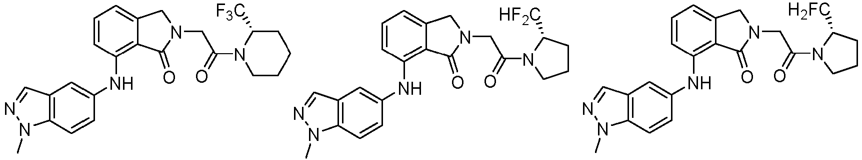

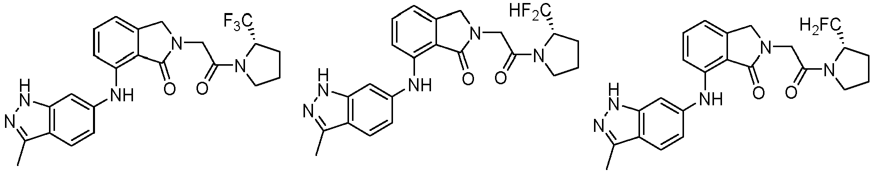

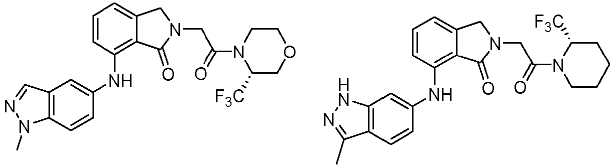

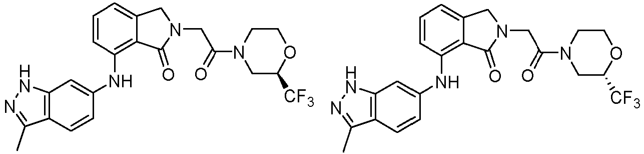

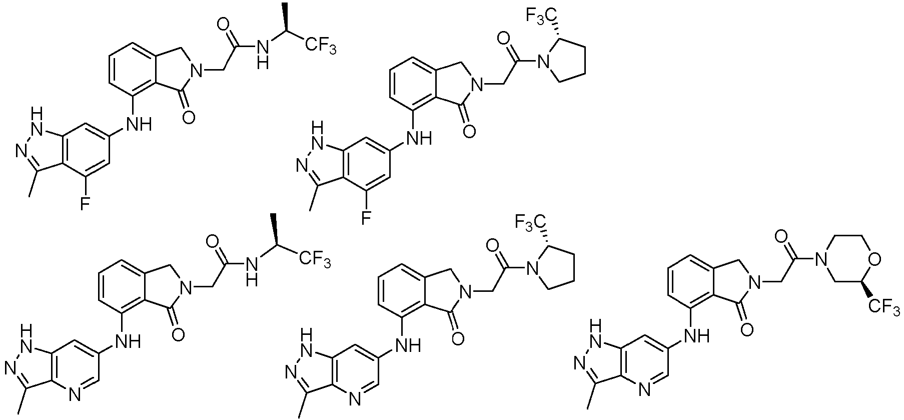

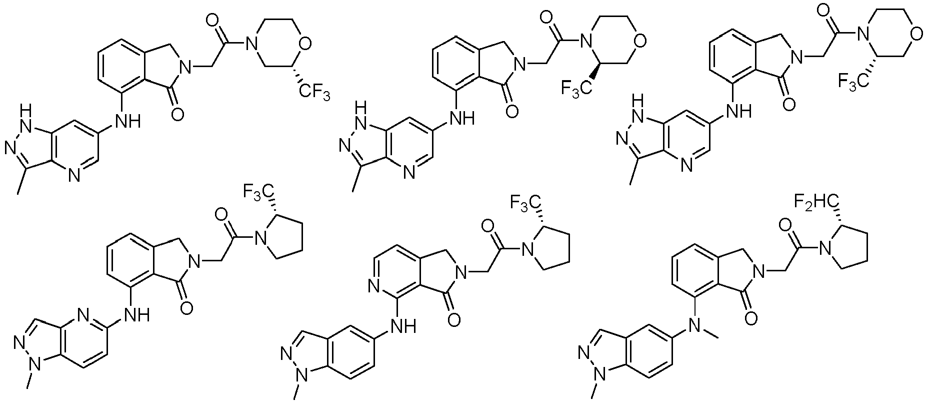

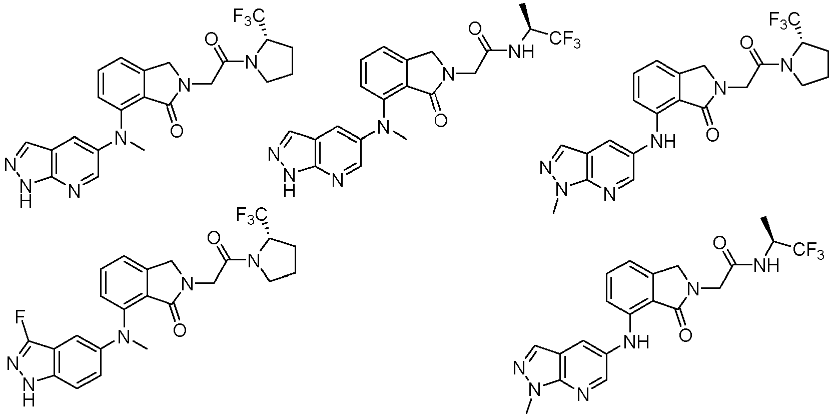

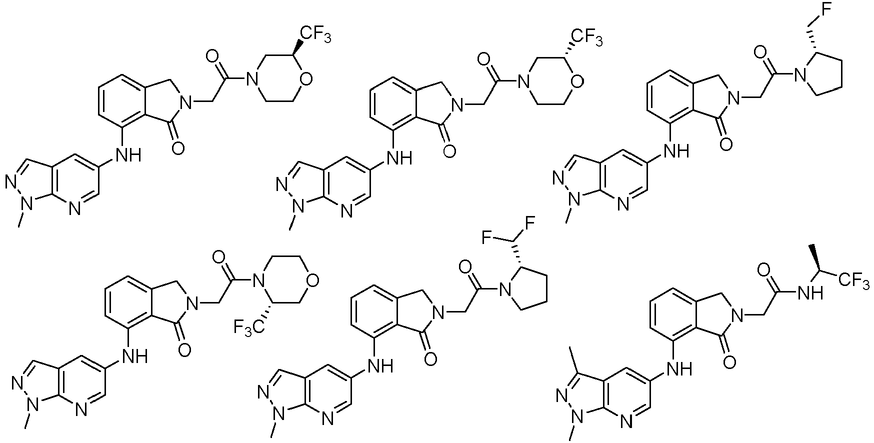

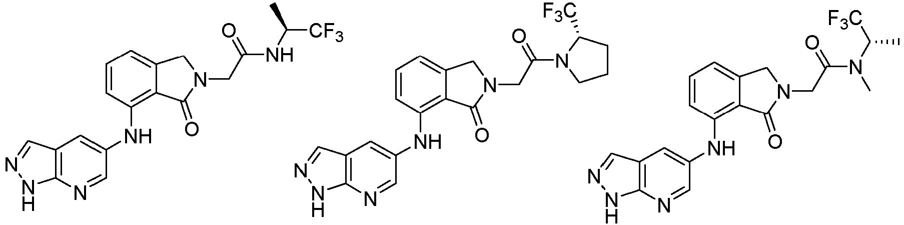

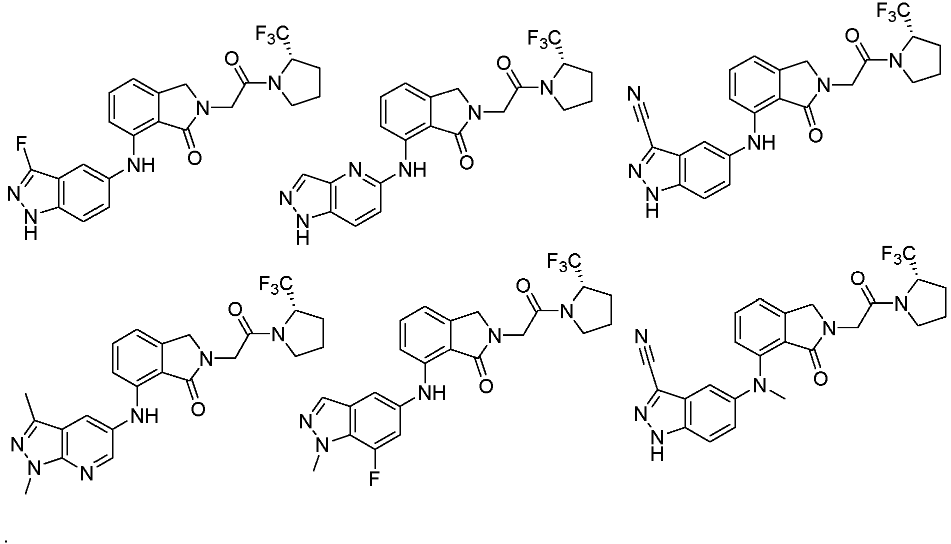

In an aspect of the invention there is provided the compounds of the present invention for use as a medicament.

In accordance with another aspect, there is provided a compound of the present invention for use in the treatment of a condition which is modulated by DDR1 and/or DDR2. A compound of any formula disclosed herein may be for use in the treatment of a condition treatable by the inhibition of DDR1 and/or DDR2.

In another aspect of the invention, there is provided a compound of the present invention for use in the treatment of a disease or disorder selected from: renal conditions, liver conditions, inflammatory conditions, cardiovascular conditions, acute and chronic organ transplant rejection, fibrotic diseases and cancer.

In an aspect of the invention there is provided a method of treating a disease or disorder which is modulated by DDR1 and/or DDR2 wherein the method comprises administering a therapeutic amount of a compound of the invention, to a patient in need thereof.

The method of treatment may be a method of treating a condition treatable by the inhibition of DDR1 and/or DDR2.

The invention also provides a method of treating a disease or disorder selected from: renal conditions, liver conditions, inflammatory conditions, cardiovascular conditions, acute and chronic organ transplant rejection, fibrotic diseases and cancer wherein the method comprises administering a therapeutic amount of a compound of any formula disclosed herein, to a patient in need thereof.

Renal conditions include, acute kidney injury and chronic renal disease with and without proteinuria including end-stage renal disease (ESRD). This includes decreased creatinine clearance and decreased glomerular filtration rate, micro albuminuria, albuminuria and proteinuria, glomerulosclerosis with expansion of reticulated mesangial matrix with or without significant hypercellularity (particularly diabetic nephropathy and amyloidosis), focal thrombosis of glomerular capillaries (particularly thrombotic microangiopathies), global fibrinoid necrosis, ischemic lesions, malignant nephrosclerosis (such as ischemic retraction, reduced renal blood flow and renal arteriopathy), swelling and proliferation of intracapillary (endothelial and mesangial) and/or extracapillary cells (crescents) like in glomerular nephritis entities, focal segmental glomerular sclerosis, IgA nephropathy, vasculitis / systemic diseases as well as acute and chronic kidney transplant rejection. Early and advanced Alport syndrome are also included amongst renal conditions.

Inflammatory conditions include, arthritis, osteoarthritis, multiple sclerosis, systemic lupus erythematodes, inflammatory bowel disease, abnormal evacuation disorder and the like as well as inflammatory airways diseases such as idiopathic pulmonary fibrosis (IPF), chronic obstructive pulmonary disease (COPD) or chronic asthma. Further conditions of the respiratory system include other diffuse parenchymal lung diseases of different etiologies including iatrogenic drug-induced fibrosis, occupational and/or environmental induced fibrosis, systemic diseases and vasculitis, granulomatous diseases (sarcoidosis, hypersensitivity pneumonia), collagen vascular disease, radiation induced fibrosis.

Vascular conditions include atherosclerosis, thrombotic vascular disease as well as thrombotic microangiopathies, proliferative arteriopathy (such as swollen myointimal cells surrounded by mucinous extracellular matrix and nodular thickening), atherosclerosis, decreased vascular compliance (such as stiffness, reduced ventricular compliance and reduced vascular compliance), endothelial dysfunction and the like.

Cardiovascular conditions include acute coronary syndrome, coronary heart disease, myocardial infarction, arterial and pulmonary hypertension, cardiac arrhythmia such as atrial fibrillation, stroke and other vascular damage.

Fibrotic diseases include, but are not limited to myocardial and vascular fibrosis, renal fibrosis, liver fibrosis, pulmonary fibrosis, skin fibrosis, scleroderma and encapsulating peritonitis, systemic sclerosis, Alport syndrome, Chronic kidney disease, NASH, Interstitial lung diseases and Systemic Sclerosis.

In certain embodiments compounds of the invention are for use in the treatment of or are used in a method of treatment of cancer. Examples include but are not limited to: liver cancer, bladder cancer, hepatoma, squamous carcinoma of the lung, non-small cell lung cancer, adenocarcinoma of the lung, small-cell lung cancer, various types of head and neck cancer, breast cancer, colon cancer, colorectal cancer, cancer of the peritoneum, hepatocellular cancer, gastrointestinal cancer, esophageal cancer, endometrial or uterine carcinoma, salivary gland carcinoma, squamous cell cancer, pituitary cancer, astrocytoma, soft tissue sarcoma, pancreatic cancer, glioblastoma, cervical cancer, ovarian cancer, kidney cancer, liver cancer, prostate cancer, vulval cancer, thyroid cancer, hepatic carcinoma, brain cancer, endometrial cancer, testis cancer, cholangiocarcinoma, gallbladder carcinoma, gastric cancer and melanoma. In certain embodiments, the cancer is selected from bladder cancer, pancreatic cancer, breast cancer, lung cancer, ovarian cancer and glioblastoma.

In another aspect of the invention there is provided a pharmaceutical composition, wherein the composition comprises a compound of the invention and pharmaceutically acceptable excipients.

In an embodiment the pharmaceutical composition may be a combination product comprising an additional pharmaceutically active agent.

In an aspect of the present invention there is provided the use of a compound of the invention in the manufacture of a medicament for use in the treatment of any condition disclosed herein.

### DETAILED DESCRIPTION

Given below are definitions of terms used in this application. Any term not defined herein takes the normal meaning as the skilled person would understand the term.

The term "halo" refers to one of the halogens, group 17 of the periodic table. In particular, the term refers to fluorine, chlorine, bromine and iodine. Preferably, the term refers to chlorine or fluorine.

The term "alkyl" refers to a linear or branched hydrocarbon chain. For example, the term "C₁₋₆ alkyl" refers to a linear or branched hydrocarbon chain containing 1, 2, 3, 4, 5 or 6 carbon atoms, for example methyl, ethyl, n-propyl, iso-propyl, n-butyl, sec-butyl, tert-butyl, n-pentyl and n-hexyl. "Alkylene" groups may likewise be linear or branched and is divalent, i.e. it attached at two positions to other portions of the molecule. Furthermore, an alkylene group may, for example, correspond to one of those alkyl groups listed in this paragraph. The alkyl and alkylene groups may be unsubstituted or substituted by one or more substituents.

The term "haloalkyl" refers to a hydrocarbon chain substituted with at least one halogen atom independently chosen at each occurrence, for example fluorine, chlorine, bromine and iodine. For example, the term "C₁₋₆ haloalkyl" refers to a linear or branched hydrocarbon chain containing 1, 2, 3, 4, 5 or 6 carbon atoms substituted with at least one halogen. The halogen atom may be present at any position on the hydrocarbon chain. For example, C₁₋₆ haloalkyl may refer to chloromethyl, fluoromethyl, trifluoromethyl, chloroethyl e.g. 1-chloromethyl and 2-chloroethyl, trichloroethyl e.g. 1,2,2-trichloroethyl, 2,2,2-trichloroethyl, fluoroethyl e.g. 1-fluoromethyl and 2-fluoroethyl, trifluoroethyl e.g. 1,2,2-trifluoroethyl and 2,2,2-trifluoroethyl, chloropropyl, trichloropropyl, fluoropropyl, trifluoropropyl. The term "fluoroalkyl" refers to a hydrocarbon chain substituted with at least one fluorine atom..

The term "alkenyl" refers to a branched or linear hydrocarbon chain containing at least one double bond. For example, the term "C₂₋₆ alkenyl" refers to a branched or linear hydrocarbon chain containing at least one double bond and having 2, 3, 4, 5 or 6 carbon atoms. The double bond(s) may be present as the *E* or *Z* isomer. The double bond may be at any possible position of the hydrocarbon chain. For example, the "C₂₋₆ alkenyl" may be ethenyl, propenyl, butenyl, butadienyl, pentenyl, pentadienyl, hexenyl and hexadienyl.

The term "alkynyl" refers to a branched or linear hydrocarbon chain containing at least one triple bond. For example, the term "C₂₋₆ alkynyl" refers to a branched or linear hydrocarbon chain containing at least one triple bond and having 2, 3, 4, 5 or 6 carbon atoms. The triple bond may be at any possible position of the hydrocarbon chain. For example, the "C₂₋₆ alkynyl" may be ethynyl, propynyl, butynyl, pentynyl and hexynyl.

The term "heteroalkyl" refers to a branched or linear hydrocarbon chain containing at least one heteroatom selected from N, O and S positioned between any carbon in the chain or at an end of the chain. For example, the term "C₁₋₆ heteroalkyl" refers to a branched or linear hydrocarbon chain containing 1, 2, 3, 4, 5, or 6 carbon atoms and at least one heteroatom selected from N, O and S positioned between any carbon in the chain or at an end of the chain. For example, the hydrocarbon chain may contain one or two heteroatoms. The C₁₋₆ heteroalkyl may be bonded to the rest of the molecule through a carbon or a heteroatom. For example, the "C₁₋₆ heteroalkyl" may be C₁₋₆ N-alkyl, C₁₋₆ N,N-alkyl, or C₁₋₆ O-alkyl.

The term "heterocycle" refers to a saturated, unsaturated or aromatic ring system containing at least one heteroatom selected from N, O or S. A "heterocyclic" system may contain 1, 2, 3 or 4 heteroatoms, for example 1 or 2. A "heterocyclic" system may be monocyclic or a fused polycyclic ring system, for example, bicyclic or tricyclic. A "heterocyclic" moiety may contain from 3 to 14 carbon atoms, for example, 3 to 8 carbon atoms in a monocyclic system and 7 to 14 carbon atoms in a polycyclic system. "Heterocyclic" encompasses heterocycloalkyl moieties, heterocycloalkenyl moieties and heteroaryl moieties. For example, the heterocyclic group may be: oxirane, aziridine, azetidine, oxetane, tetrahydrofuran, pyrrolidine, imidazolidine, succinimide, pyrazolidine, oxazolidine, isoxazolidine, thiazolidine, isothiazolidine, piperidine, morpholine, thiomorpholine, piperazine, and tetrahydropyran. Heteroaryl includes groups such as pyridones and N-alkyl-pyridones.

The term "C₃₋₈ cycloalkyl" refers to a saturated hydrocarbon ring system containing 3, 4, 5, 6, 7 or 8 carbon atoms. For example, the "C₃₋₈ cycloalkyl" may be cyclopropyl, cyclobutyl, cyclopentyl, cyclohexyl, cycloheptyl and cyclooctyl.

The term "C₃₋₈ cycloalkenyl" refers to an unsaturated hydrocarbon ring system containing 3, 4, 5, 6, 7 or 8 carbon atoms that is not aromatic. The ring may contain more than one double bond provided that the ring system is not aromatic. For example, the "C₃₋₈ cycloalkyl" may be cyclopropenyl, cyclobutenyl, cyclopentenyl, cyclopentadienyl, cyclohexenyl, cyclohexadienly, cycloheptenyl, cycloheptadiene, cyclooctenyl and cycloatadienyl.

The term "heterocycloalkyl" refers to a saturated hydrocarbon ring system containing carbon atoms and at least one heteroatom within the ring selected from N, O and S. For example, there may be 1, 2 or 3 heteroatoms, optionally 1 or 2. The "heterocycloalkyl" may be bonded to the rest of the molecule through any carbon atom or heteroatom. The "heterocycloalkyl" may have one or more, e.g. one or two, bonds to the rest of the molecule: these bonds may be through any of the atoms in the ring. For example, the "heterocycloalkyl" may be a "C₃₋₈ heterocycloalkyl". The term "C₃₋₈ heterocycloalkyl" refers to a saturated hydrocarbon ring system containing 3, 4, 5, 6, 7 or 8 atoms at least one of the atoms being a heteroatom within the ring selected from N, O and S. The "heterocycloalkyl" may be oxirane, aziridine, azetidine, oxetane, tetrahydrofuran, pyrrolidine, imidazolidine, succinimide, pyrazolidine, oxazolidine, isoxazolidine, thiazolidine, isothiazolidine, piperidine, morpholine, thiomorpholine, piperazine, and tetrahydropyran.

The term "aromatic" when applied to a substituent as a whole means a single ring or polycyclic ring system with 4n + 2 electrons in a conjugated π system within the ring or ring system where all atoms contributing to the conjugated π system are in the same plane.

The term "aryl" refers to an aromatic hydrocarbon ring system. The ring system has 4n +2 electrons in a conjugated π system within a ring where all atoms contributing to the conjugated π system are in the same plane. For example, the "aryl" may be phenyl and naphthyl. The aryl system itself may be substituted with other groups.

The term "heteroaryl" refers to an aromatic hydrocarbon ring system with at least one heteroatom within a single ring or within a fused ring system, selected from O, N and S. The ring or ring system has 4n +2 electrons in a conjugated π system where all atoms contributing to the conjugated π system are in the same plane. For example, the "heteroaryl" may be imidazole, oxazole, isoxazole, thiazole, isothiazole, thiene, furan, thianthrene, pyrrole, benzimidazole, pyrazole, pyrazine, pyridine, pyrimidine and indole.

A bond terminating in a " 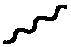 " represents that the bond is connected to another atom that is not shown in the structure. A bond terminating inside a cyclic structure and not terminating at an atom of the ring structure represents that the bond may be connected to any of the atoms in the ring structure where allowed by valency.

A bond drawn as a solid line and a dotted line represents a bond which can be either a single bond or a double bond, where chemically possible. For example, the bond drawn below could be a single bond or a double bond.

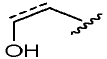

Where a moiety is substituted, it may be substituted at any point on the moiety where chemically possible and consistent with atomic valency requirements. The moiety may be substituted by one or more substituents, e.g. 1, 2, 3 or 4 substituents; optionally there are 1 or 2 substituents on a group. Where there are two or more substituents, the substituents may be the same or different.

Substituents are only present at positions where they are chemically possible, the person skilled in the art being able to decide (either experimentally or theoretically) without inappropriate effort which substitutions are chemically possible and which are not.

Ortho, meta and para substitution are well understood terms in the art. For the absence of doubt, "ortho" substitution is a substitution pattern where adjacent carbons possess a substituent, whether a simple group, for example the fluoro group in the example below, or other portions of the molecule, as indicated by the bond ending in " 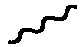 "

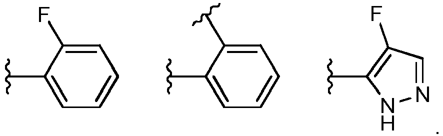

"Meta" substitution is a substitution pattern where two substituents are on carbons one carbon removed from each other, i.e with a single carbon atom between the substituted carbons. In other words there is a substituent on the second atom away from the atom with another substituent. For example the groups below are meta substituted.

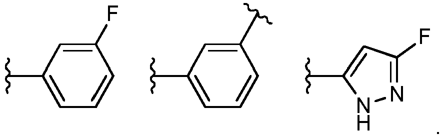

"Para" substitution is a substitution pattern where two substituents are on carbons two carbons removed from each other, i.e with two carbon atoms between the substituted carbons. In other words there is a substituent on the third atom away from the atom with another substituent. For example the groups below are para substituted.

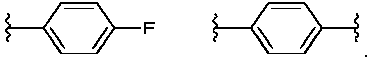

Throughout the description the disclosure of a compound also encompasses pharmaceutically acceptable salts, solvates and stereoisomers thereof.

Where a compound has a stereocentre, both (R) and (S) stereoisomers are contemplated by the invention, equally mixtures of stereoisomers or a racemic mixture are completed by the present application. Where a compound of the invention has two or more stereocentres any combination of (R) and (S) stereoisomers is contemplated. The combination of (R) and (S) stereoisomers may result in a diastereomeric mixture or a single diastereoisomer. The compounds of the invention may be present as a single stereoisomer or may be mixtures of stereoisomers, for example racemic mixtures and other enantiomeric mixtures, and diasteroemeric mixtures. Where the mixture is a mixture of enantiomers the enantiomeric excess may be any of those disclosed above. Where the compound is a single stereoisomer the compounds may still contain other diasteroisomers or enantiomers as impurities. Hence a single stereoisomer does not necessarily have an enantiomeric excess (e.e.) or diastereomeric excess (d.e.) of 100% but could have an e.e. or d.e. of about at least 85%, at least 60% or less. For example, the e.e. or d.e. may be 90% or more, 90% or more, 80% or more, 70% or more, 60% or more, 50% or more, 40% or more, 30% or more, 20% or more, or 10% or more.

The invention contemplates pharmaceutically acceptable salts of the compounds of the invention. These may include the acid addition and base salts of the compounds. These may be acid addition and base salts of the compounds. In addition the invention contemplates solvates of the compounds. These may be hydrates or other solvated forms of the compound.

Suitable acid addition salts are formed from acids which form non-toxic salts. Examples include the acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 1,5-naphthalenedisulfonate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulfate and hemicalcium salts. For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (I) may be prepared by one or more of three methods:
(i) by reacting the compound of the invention with the desired acid or base;
(ii) by removing an acid- or base-labile protecting group from a suitable precursor of the compound of the invention or by ring-opening a suitable cyclic precursor, for example, a lactone or lactam, using the desired acid or base; or
(iii) by converting one salt of the compound of the invention to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non- ionised. For a review of such complexes, see J Pharm Sci, 64 (8), 1269-1288 by Haleblian (August 1975).

Hereinafter all references to compounds of any formula include references to salts, solvates and complexes thereof and to solvates and complexes of salts thereof.

The compounds of the invention include compounds of a number of formula as herein defined, including all polymorphs and crystal habits thereof, prodrugs and isomers thereof (including optical, geometric and tautomeric isomers) as hereinafter defined and isotopically-labelled compounds of the invention.

The present invention also includes all pharmaceutically acceptable isotopically-labelled compounds of the invention wherein one or more atoms are replaced by atoms having the same atomic number, but an atomic mass or mass number different from the atomic mass or mass number most commonly found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds, for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances.

Before purification, the compounds of the present invention may exist as a mixture of enantiomers depending on the synthetic procedure used. The enantiomers can be separated by conventional techniques known in the art. Thus the invention covers individual enantiomers as well as mixtures thereof.

For some of the steps of the process of preparation of the compounds of the invention, it may be necessary to protect potential reactive functions that are not wished to react, and to cleave said protecting groups in consequence. In such a case, any compatible protecting radical can be used. In particular methods of protection and deprotection such as those described by T.W. GREENE (Protective Groups in Organic Synthesis, A. Wiley- Interscience Publication, 1981) or by P. J. Kocienski (Protecting groups, Georg Thieme Verlag, 1994), can be used. All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.

Also, the compounds of the present invention as well as intermediates for the preparation thereof can be purified according to various well-known methods, such as for example crystallization or chromatography.

One or more compounds of the invention may be combined with one or more pharmaceutical agents, for example anti-inflammatory agents, anti-fibrotic agents, chemotherapeutics, anti cancer agents, immunosuppressants, anti-tumour vaccines, cytokine therapy, or tyrosine kinase inhibitors, for the treatment of conditions modulated by the inhibition of ROCK, for example fibrotic diseases, autoimmune, inflammatory-fibrotic conditions, inflammatory conditions, central nervous system disorders, or cancer.

The method of treatment or the compound for use in the treatment of renal conditions, liver conditions, inflammatory conditions, cardiovascular conditions, acute and chronic organ transplant rejection, fibrotic diseases and cancer as defined hereinbefore may be applied as a sole therapy or be a combination therapy with an additional active agent.

The method of treatment or the compound for use in the treatment of renal conditions, liver conditions, inflammatory conditions, cardiovascular conditions, acute and chronic organ transplant rejection, fibrotic diseases and cancer. The additional active agents may be one or more active agents used to treat the condition being treated by the compound of the invention and additional active agent. The additional active agents may include one or more of the following active agents:-
(i) steroids such as corticosteroids, including glucocorticoids and mineralocorticoids, for example aclometasone, aclometasone dipropionate, aldosterone, amcinonide, beclomethasone, beclomethasone dipropionate, betamethasone, betamethasone dipropionate, betamethasone sodium phosphate, betamethasone valerate, budesonide, clobetasone, clobetasone butyrate, clobetasol propionate, cloprednol, cortisone, cortisone acetate, cortivazol, deoxycortone, desonide, desoximetasone, dexamethasone, dexamethasone sodium phosphate, dexamethasone isonicotinate, difluorocortolone, fluclorolone, flumethasone, flunisolide, fluocinolone, fluocinolone acetonide, fluocinonide, fluocortin butyl, fluorocortisone, fluorocortolone, fluocortolone caproate, fluocortolone pivalate, fluorometholone, fluprednidene, fluprednidene acetate, flurandrenolone, fluticasone, fluticasone propionate, halcinonide, hydrocortisone, hydrocortisone acetate, hydrocortisone butyrate, hydrocortisone aceponate, hydrocortisone buteprate, hydrocortisone valerate, icomethasone, icomethasone enbutate, meprednisone, methylprednisolone, mometasone paramethasone, mometasone furoate monohydrate, prednicarbate, prednisolone, prednisone, tixocortol, tixocortol pivalate, triamcinolone, triamcinolone acetonide, triamcinolone alcohol and their respective pharmaceutically acceptable derivatives. A combination of steroids may be used, for example a combination of two or more steroids mentioned in this paragraph;
(ii) TNF inhibitors for example etanercept; monoclonal antibodies (e.g. infliximab (Remicade), adalimumab (Humira), certolizumab pegol (Cimzia), golimumab (Simponi)); fusion proteins (e.g. etanercept (Enbrel)); and 5-HT_{2A} agonists (e.g. 2,5-dimethoxy-4-iodoamphetamine, TCB-2, lysergic acid diethylamide (LSD), lysergic acid dimethylazetidide);
(iii) anti-inflammatory drugs, for example non-steroidal anti-inflammatory drugs;
(iv) dihydrofolate reductase inhibitors/antifolates, for example methotrexate, trimethoprim, brodimoprim, tetroxoprim, iclaprim, pemetrexed, ralitrexed and pralatrexate; and
(v) immunosuppressants for example cyclosporins, tacrolimus, sirolimus pimecrolimus, angiotensin II inhibitors (e.g. Valsartan, Telmisartan, Losartan, Irbesatan, Azilsartan, Olmesartan, Candesartan, Eprosartan) and ACE inhibitors e.g. sulfhydryl-containing agents (e.g. Captopril, Zofenopril), dicarboxylate-containing agents (e.g. Enalapril, Ramipril, Quinapril, Perindopril, Lisinopril, Benazepril, Imidapril, Zofenopril, Trandolapril), phosphate-containing agents (e.g. Fosinopril), casokinins, lactokinins and lactotripeptides.
(vi)Anti-fibrotic agents for example: Pirfenidone, Nintedanib, Anti-IL-13 monoclonal antibodies (e.g. Tralokinumab, QAX576, Lebrikizumab), simtuzumab, FG-3019, lysophosphatidic acid receptor antagonists (e.g. BMS-986020, AM966), LOXL2 inhibitors, BET bromodomain inhibitors (e.g. JQ1), HDAC inhibitors (e.g. Vorinostat), thrombin inhibitors (e.g. Dabigatran), FactorXa inhibitors (e.g. Apixban, Rivaroxaban) 15PGDH inhibitors, anti-avβ6 monoclonal antibodies (e.g. BG00011), Anti-CTGF monoclonal antibodies (e.g. FG-3019), PAR1 inhibitors, Nox4 inhibitors and PAI-1 inhibitors.

The method of treatment or the compound for use in the treatment of cancer may involve, in addition to the compound of the invention, conventional surgery or radiotherapy or chemotherapy. Such chemotherapy may include one or more of the following categories of anti-tumor agents:
(i) antiproliferative/antineoplastic drugs and combinations thereof, such as alkylating agents (for example cis-platin, oxaliplatin, carboplatin, cyclophosphamide, nitrogen mustard, uracil mustard, bendamustin, melphalan, chlorambucil, chlormethine, busulphan, temozolamide, nitrosoureas, ifosamide, melphalan, pipobroman, triethylene-melamine, triethylenethiophoporamine, carmustine, lomustine, stroptozocin and dacarbazine); antimetabolites (for example gemcitabine and antifolates such as fluoropyrimidines like 5-fluorouracil and tegafur, raltitrexed, methotrexate, pemetrexed, cytosine arabinoside, floxuridine, cytarabine, 6-mercaptopurine, 6-thioguanine, fludarabine phosphate, pentostatine, and gemcitabine and hydroxyurea); antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); proteasome inhibitors, for example carfilzomib and bortezomib; interferon therapy; and topoisomerase inhibitors (for example epipodophyllotoxins like etoposide and teniposide, amsacrine, topotecan, mitoxantrone and camptothecin); bleomcin, dactinomycin, daunorubicin, doxorubicin, epirubicin, idarubicin, ara-C, paclitaxel (Taxol^{™}), nabpaclitaxel, docetaxel, mithramycin, deoxyco-formycin, mitomycin-C, L-asparaginase, interferons (especially IFN-a), etoposide, and teniposide;
(ii) cytostatic agents such as antiestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride; and navelbene, CPT-II, anastrazole, letrazole, capecitabine, reloxafme, cyclophosphamide, ifosamide, and droloxafine;
(iii) anti-invasion agents, for example dasatinib and bosutinib (SKI-606), and metalloproteinase inhibitors, inhibitors of urokinase plasminogen activator receptor function or antibodies to Heparanase;
(iv) inhibitors of growth factor function: for example such inhibitors include growth factor antibodies and growth factor receptor antibodies, for example the anti-erbB2 antibody trastuzumab [Herceptin^{™}], the anti-EGFR antibody panitumumab, the anti-erbB1 antibody cetuximab, tyrosine kinase inhibitors, for example inhibitors of the epidermal growth factor family (for example EGFR family tyrosine kinase inhibitors such as gefitinib, erlotinib, 6-acrylamido-N-(3-chloro-4-fluorophenyl)-7-(3-morpholinopropoxy)-quinazolin-4-amine (Cl 1033), erbB2 tyrosine kinase inhibitors such as lapatinib) and antibodies to costimulatory molecules such as CTLA-4, 4-IBB and PD-I, or antibodies to cytokines (IL-I0, TGF-beta); inhibitors of the hepatocyte growth factor family; inhibitors of the insulin growth factor family; modulators of protein regulators of cell apoptosis (for example Bcl-2 inhibitors); inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib , tipifarnib and lonafarnib), inhibitors of cell signalling through MEK and/or AKT kinases, c-kit inhibitors, abl kinase inhibitors, PI3 kinase inhibitors, PIt3 kinase inhibitors, CSF-1 R kinase inhibitors, IGF receptor, kinase inhibitors; aurora kinase inhibitors and cyclin dependent kinase inhibitors such as CDK2 and/or CDK4 inhibitors; and CCR2, CCR4 or CCR6 modulator;
(v) antiangiogenic agents such as those which inhibit the effects of vascular endothelial growth factor, for example the anti-vascular endothelial cell growth factor antibody bevacizumab (Avastin^{™}); thalidomide; lenalidomide; and for example, a VEGF receptor tyrosine kinase inhibitor such as vandetanib, vatalanib, sunitinib, axitinib and pazopanib;
(vi) gene therapy approaches, including for example approaches to replace aberrant genes such as aberrant p53 or aberrant BRCA1 or BRCA2;
(vii) immunotherapy approaches, including for example antibody therapy such as alemtuzumab, rituximab, ibritumomab tiuxetan (Zevalin^{®}) and ofatumumab; interferons such as interferon α; interleukins such as IL-2 (aldesleukin); interleukin inhibitors for example IRAK4 inhibitors; cancer vaccines including prophylactic and treatment vaccines such as HPV vaccines, for example Gardasil, Cervarix, Oncophage and Sipuleucel-T (Provenge); gp100;dendritic cell-based vaccines (such as Ad.p53 DC); and toll-like receptor modulators for example TLR-7 or TLR-9 agonists; and
(viii) cytotoxic agents for example fludaribine (fludara), cladribine, pentostatin (Nipent^{™});
(ix) steroids such as corticosteroids, including glucocorticoids and mineralocorticoids, for example aclometasone, aclometasone dipropionate, aldosterone, amcinonide, beclomethasone, beclomethasone dipropionate, betamethasone, betamethasone dipropionate, betamethasone sodium phosphate, betamethasone valerate, budesonide, clobetasone, clobetasone butyrate, clobetasol propionate, cloprednol, cortisone, cortisone acetate, cortivazol, deoxycortone, desonide, desoximetasone, dexamethasone, dexamethasone sodium phosphate, dexamethasone isonicotinate, difluorocortolone, fluclorolone, flumethasone, flunisolide, fluocinolone, fluocinolone acetonide, fluocinonide, fluocortin butyl, fluorocortisone, fluorocortolone, fluocortolone caproate, fluocortolone pivalate, fluorometholone, fluprednidene, fluprednidene acetate, flurandrenolone, fluticasone, fluticasone propionate, halcinonide, hydrocortisone, hydrocortisone acetate, hydrocortisone butyrate, hydrocortisone aceponate, hydrocortisone buteprate, hydrocortisone valerate, icomethasone, icomethasone enbutate, meprednisone, methylprednisolone, mometasone paramethasone, mometasone furoate monohydrate, prednicarbate, prednisolone, prednisone, tixocortol, tixocortol pivalate, triamcinolone, triamcinolone acetonide, triamcinolone alcohol and their respective pharmaceutically acceptable derivatives. A combination of steroids may be used, for example a combination of two or more steroids mentioned in this paragraph;
(x) targeted therapies, for example PI3Kd inhibitors, for example idelalisib and perifosine; PD-1, PD-L1, PD-L2 and CTL4-A modulators, antibodies and vaccines; other IDO inhibitors (such as indoximod); anti-PD-1 monoclonal antibodies (such as MK-3475 and nivolumab); anti-PD-L1 monoclonal antibodies (such as MEDI-4736 and RG-7446); anti-PD-L2 monoclonal antibodies; and anti-CTLA-4 antibodies (such as ipilimumab);
(xii) chimeric antigen receptors, anticancer vaccines and arginase inhibitors.

Such combination treatment may be achieved by way of the simultaneous, sequential or separate dosing of the individual components of the treatment. Such combination products employ the compounds of this invention within a therapeutically effective dosage range described hereinbefore and the other pharmaceutically-active agent within its approved dosage range.

Compounds of the invention may exist in a single crystal form or in a mixture of crystal forms or they may be amorphous. Thus, compounds of the invention intended for pharmaceutical use may be administered as crystalline or amorphous products. They may be obtained, for example, as solid plugs, powders, or films by methods such as precipitation, crystallization, freeze drying, or spray drying, or evaporative drying. Microwave or radio frequency drying may be used for this purpose.

For the above-mentioned compounds of the invention the dosage administered will, of course, vary with the compound employed, the mode of administration, the treatment desired and the disorder indicated. For example, if the compound of the invention is administered orally, then the daily dosage of the compound of the invention may be in the range from 0.01 micrograms per kilogram body weight (µg/kg) to 100 milligrams per kilogram body weight (mg/kg).

A compound of the invention, or pharmaceutically acceptable salt thereof, may be used on their own but will generally be administered in the form of a pharmaceutical composition in which the compounds of the invention, or pharmaceutically acceptable salt thereof, is in association with a pharmaceutically acceptable adjuvant, diluent or carrier. Conventional procedures for the selection and preparation of suitable pharmaceutical formulations are described in, for example, "Pharmaceuticals - The Science of Dosage Form Designs", M. E. Aulton, Churchill Livingstone, 1988.

Depending on the mode of administration of the compounds of the invention, the pharmaceutical composition which is used to administer the compounds of the invention will preferably comprise from 0.05 to 99 %w (per cent by weight) compounds of the invention, more preferably from 0.05 to 80 %w compounds of the invention, still more preferably from 0.10 to 70 %w compounds of the invention, and even more preferably from 0.10 to 50 %w compounds of the invention, all percentages by weight being based on total composition.

The pharmaceutical compositions may be administered topically (e.g. to the skin) in the form, e.g., of creams, gels, lotions, solutions, suspensions, or systemically, e.g. by oral administration in the form of tablets, capsules, syrups, powders or granules; or by parenteral administration in the form of a sterile solution, suspension or emulsion for injection (including intravenous, subcutaneous, intramuscular, intravascular or infusion); by rectal administration in the form of suppositories; or by inhalation in the form of an aerosol.

For oral administration the compounds of the invention may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum and titanium dioxide. Alternatively, the tablet may be coated with a suitable polymer dissolved in a readily volatile organic solvent.

For the preparation of soft gelatine capsules, the compounds of the invention may be admixed with, for example, a vegetable oil or polyethylene glycol. Hard gelatine capsules may contain granules of the compound using either the above-mentioned excipients for tablets. Also liquid or semisolid formulations of the compound of the invention may be filled into hard gelatine capsules. Liquid preparations for oral application may be in the form of syrups or suspensions, for example, solutions containing the compound of the invention, the balance being sugar and a mixture of ethanol, water, glycerol and propylene glycol. Optionally such liquid preparations may contain colouring agents, flavouring agents, sweetening agents (such as saccharine), preservative agents and/or carboxymethylcellulose as a thickening agent or other excipients known to those skilled in art.

For intravenous (parenteral) administration the compounds of the invention may be administered as a sterile aqueous or oily solution.

The size of the dose for therapeutic purposes of compounds of the invention will naturally vary according to the nature and severity of the conditions, the age and sex of the animal or patient and the route of administration, according to well-known principles of medicine.

Dosage levels, dose frequency, and treatment durations of compounds of the invention are expected to differ depending on the formulation and clinical indication, age, and co-morbid medical conditions of the patient.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

The compounds of the invention may be prepared according to or analogously to the General Schemes 1 - 4 and Examples 1 to 14.

### EXAMPLES AND SYNTHESIS

### Experimental Procedures

Solvents, reagents and starting materials were purchased from commercial vendors and used as received unless otherwise described. All reactions were performed at room temperature unless otherwise stated. Compound identity and purity confirmations were performed by LCMS. Retention times RT are reported in minutes. The following methods were also used on occasions when described throughout the experimental section, gradients are detailed in table 1. Method 1 utilised a Shimadzu 2020 series spectrometer equipped with a binary pump and diode array detector (acquisition wavelength 214 and 254 nm) and the MS was in positive and negative electrospray mode *(m*/*z:* 100-900). 2µL Aliquot were injected onto an Agilent Poroshell 120 EC-C18 column (2.7 µm, 4.6×50 mm) maintained at 35°C and eluted at 1.0ml/min using mobile phase consisting of: A: 0.05% Formic acid in water (v/v), B: 0.05% Formic acid in ACN(v/v). Method 2 utilised an Agilent Technologies 1290 series spectrometer equipped with a binary pump and diode array detector (acquisition wavelength 214 and 254 nm) and the MS was in positive electrospray mode (m/z: 70-1000). 2µL Aliquots were injected onto an Agilent Eclipse Plus RRHD C18, (1.8µm, 3.0×50 mm) column maintained at 40°C and eluted at 0.8ml/min using mobile phase consisting of: A: 0.05% Formic acid in water (v/v), B: 0.05% Formic acid in ACN(v/v).

**Table 1**

| Method 1 | | |
|---|---|---|
| Time (min) | %A | %B |
| 0.01 | 85 | 15 |
| 1.0 | 85 | 15 |
| 4.0 | 0 | 100 |
| 4.5 | 0 | 100 |
| 4.51 | 85 | 15 |
| 5.0 | 85 | 15 |

| Method 2 | | |
|---|---|---|
| Time (min) | %A | %B |
| 0.00 | 80 | 20 |
| 2.65 | 20 | 80 |
| 3.00 | 20 | 80 |

NMR was also used to characterise final compounds. NMR spectra were obtained on a Bruker AVIII 400 Nanobay with 5mm BBFO probe. Optionally, compound Rf values on silica thin layer chromatography (TLC) plates were measured.

Compound purification was performed by flash column chromatography on silica or by preparative LCMS. LCMS purification was performed using a Waters 3100 Mass detector in positive and negative electrospray mode *(m*/*z:* 150-800) with a Waters 2489 UV/Vis detector. Samples were eluted at a flow rate of 20mL/min on a XBridge^{™} prep C18 5µM OBD 19x100mm column with a mobile phase system composed of A (0.1% (v/v) Formic Acid in Water) and B (0.1% (v/v) Formic Acid in Acetonitrile) according to the gradient outlined in Table 2 below.

**Table 2**

| Time (min) | %A | %B |
|---|---|---|
| 0 | 90 | 10 |
| 1.5 | 90 | 10 |
| 11.7 | 5 | 95 |
| 13.7 | 5 | 95 |
| 14 | 90 | 10 |
| 15 | 90 | 10 |

### General Syntheitic Routes

Examples conforming to formula (I) can be prepared by one or more of the synthetic routes described in general schemes to 4 below. Starting materials and reagents used in the routes described in gerneral schemes 1 to 4 are either commercially available or prepared using procedures described in chemical literature, as identified using typical chemical structure and reaction searching tools. The order of steps may also be changed. In cases where reactive groups are present, for example amines or alcohols, suitable protecting groups can be used to mask the reactivity of these groups if necessary, for example carbamates, tosylates, hemi-aminals, acteals and esters.

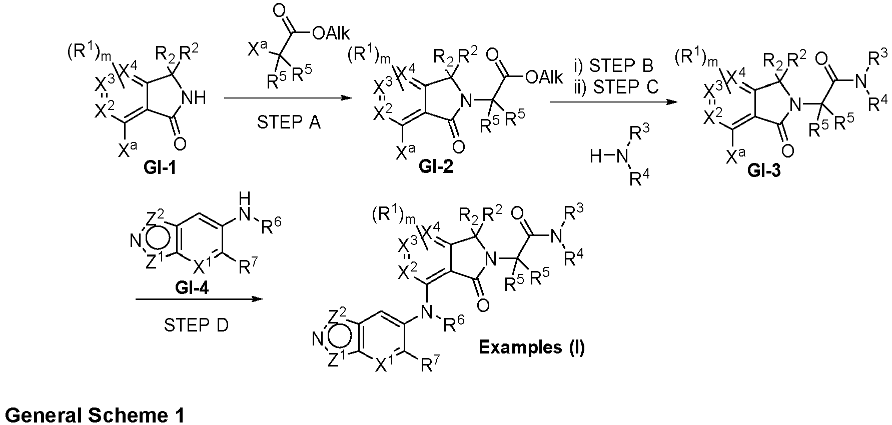

In general scheme 1 (where X^{a} is a halogen, typically bromide but can also be chloride or iodide) a lactam (GI-1) is N-alkylated with an alpha-halo ester such as ethylbromoacetate in the presence of a base such as triethylamine or cesium carbonate in an organic solvent such as acetonitrile or MeCN to afford an intermediate GI-2. The ester group of GI-2 can be hydrolysed in step B using LiOH in MeOH, THG and water and the resulting acid may form an amide bond in step 3 when reacted with an appropriate amine in the presence of an activating agent such as HATU to afford an amide represented by GI-3. In step D GI-3 undergoes a transition metal catalsed amination such a Buchwald-Hartwig reaction with an appropriate amino indazole derivate (GI-4), to afford examples of formula (I). If Z¹ or Z² is NH the corresponding NH in GI-4 can be protected with a a suitable group such as THP or tosyl to facilitate step D. In these cases an additional deprotection step (typically using TFA or HCI) is required to ultimately produce examples of formula (I).

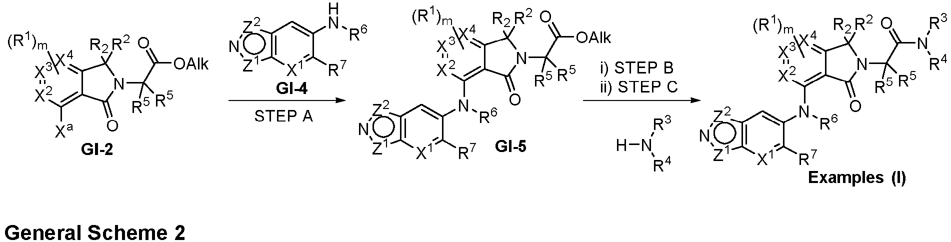

General scheme 2 is a variation of general scheme 1 in which N-alklated lactam GI-2 undergoes amination in step A via a Buchwald-Hartwig reaction with an appropriate amino indazole derivate (GI-4) to afford an alkly ester (typically ethyl or t-butyl) of formula GI-5. Steps B and C represent hydrolysis of the ester and amide formation in a similar manner to general scheme 1 to afford examples of formula (I). Once again, if Z¹ or Z² is NH the corresponding NH in GI-4 can be protected with a asuitable group such as THP or tosyl to facilitate step A. In these cases an additional deprotection step (typically using TFA or HCI) is required to ultimately produce examples of formula (I).

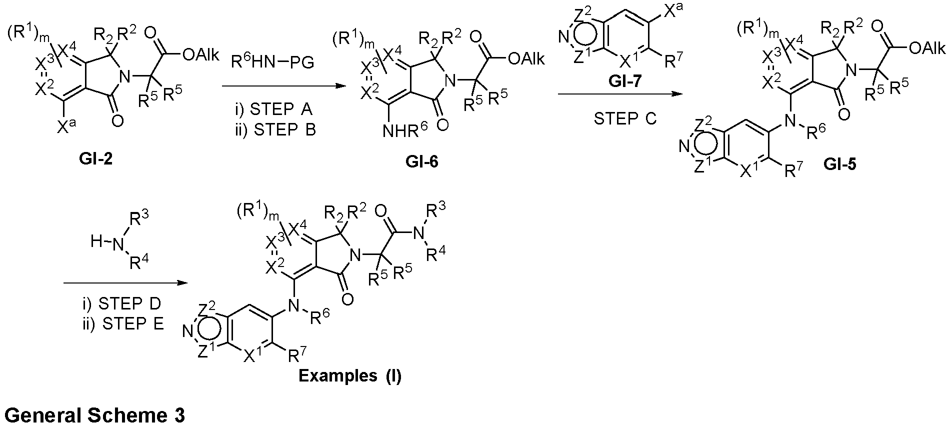

According to general scheme 3, N-alklated lactam GI-2 (where X^{a} is a halogen, typlically bromide) is converted to an amino lactam of formula GI-6. Step A involves a transition metal catalsed amination such a Buchwald-Hartwig reaction with an appropriate amine bearing an acid labile protecting group (PG) for example t-butyl carbamate and step B is removal of the protecting group using HCI or TFA in an organinc solvent such as DCM or MeOH. GI-6 can then undergoe a second transition metal catalsed amination (step C) such a Buchwald-Hartwig reaction with a halogen bearing indazole derivate (GI-7) in the presence of a palladium catalyst such as Pd₂(dba)₃ , a ligand such as Xantphos and a base, for example cesium carbonate in 1,4-dioxane as solvent, GI-5 can then undergo hydrolysis and amide formation in steps C and D as previously described in steps B and C in general schemes 1 and 2. As previously described, if Z¹ or Z² is NH in examples of formula (I) the corresponding NH in GI-7 can be protected with a suitable group such as THP or tosyl to facilitate step C. In these cases an additional final deprotection step (typically using TFA or HCI) is required to ultimately produce examples of formula (I).

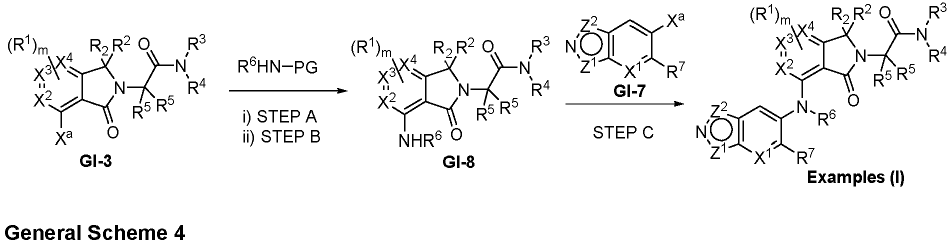

General scheme 4 represents a variation of general scheme 3. In steps A and B GI-3 (described in general scheme 1) undergoes a comparable amination to that described in steps A and B of general scheme 3 to afford GI-8 which then in step C undergoes reaction with GI-7 under conditions described in step C of general scheme 3 to afford examples of formula (I). . As previously described, if Z¹ or Z² in examples of formula (I) is NH the corresponding NH in GI-7 can be protected with a asuitable group such as THP or tosyl to facilitate step C. In these cases an additional deprotection step (typically using TFA or HCI) is required to ultimately produce examples of formula (I).

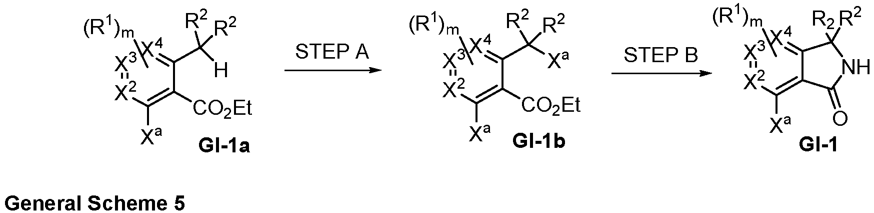

Intermediates of structure GI-1 where not commercially available could be prepared using the methodology outlined in general scheme 5. An appropriately substituted aryl or heteroaryl ethyl ester (where X^{a} is a halogen, typically a bromide) represented by GI-1a undergoes halogenation in step A, for example with NBS in the presence of a radical initiator such as benzoyl peroxide using CCl₄ as a solvent and is then cyclised in step B with concentrated aqueous ammonia.

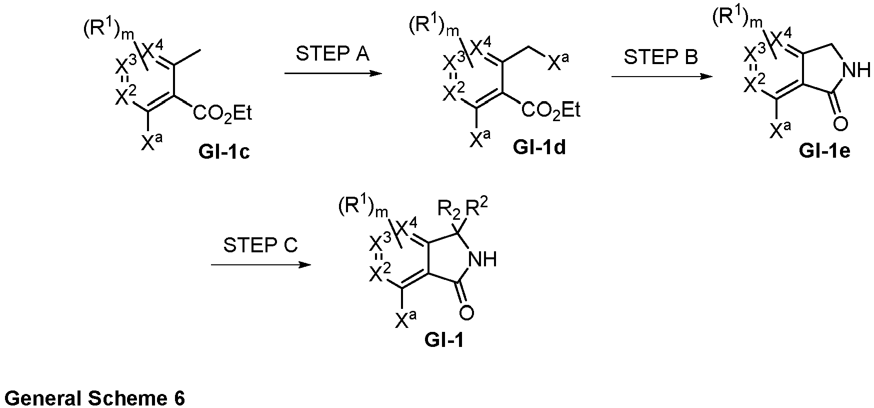

Alternatively intermediates of structure GI-I (in which one R² group is not H) can be prepared according to general scheme 6. GI-1c Is subject to the same steps A and B described in general scheme 5 to afford GI-1e. GI-1e is then alkylated in step C in the presence of a base such as NaHMDS and an appropriate alkyl iodide or bromide in THF as a solvent. If the two R² groups are not equivalent and neither are H), step C is repeated with the appropriate alkly iodide or bromide to furnish the second R² group.

Where not commercially available or described in the chemical literature halo-indazoles of the formula GI-7 could be prepared by analogy with methods reported in the literature (Gaikwad et al. Eur. J. Med. Chem., 90, 2015, p707-731). General schemes 7 and 8 describe the methods used herein.

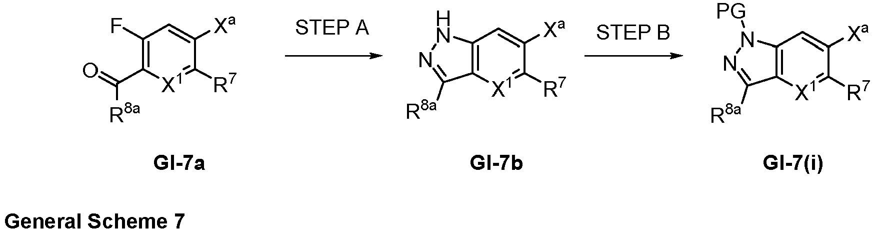

General scheme 7 describes the preparation of halo indazoles (GI-7b and GI-7(i)) used in the synthesis of examples of formula (I) in which Z¹ is CR^{8a} and Z² is NH. In step A an appropriate ortho-fluoro carbonyl heteroaryl halide of formula Gl-7a (typically halogen X^{a} is bromide) was reacted with hydrazine hydrate in iPrOH at 80°C. In step B a protecting group could be optionally incorporated to negate any reactivity of the free NH group in subsequent steps. The protecting group used was either a THP group, incorporated using 3,4-dihydropyran with catalytic para-TsOH in THF at 50°C, or a tosyl group, incorporated using tosyl chloride and triethylamine in DCM at room temperature.

Halo indazoles (GI-7d and GI-7(ii)) used in the synthesis of examples of formula (I) in which Z² is CR^{8a} and Z¹ is NH were prepared in an identical fashion starting from the isomeric fluoro carbonyl heteroaryl halides of formula GI-7c, as shown in general scheme 8.

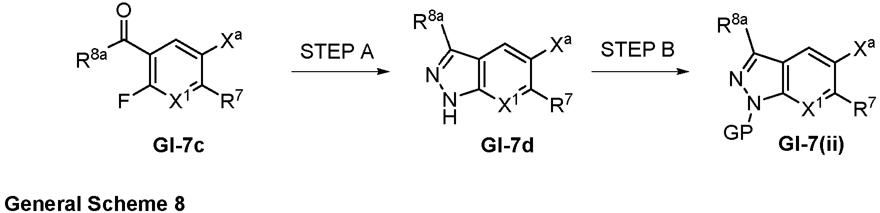

Where not commercially available or described in the chemical literature amino-indazoles of the formula GI-4 could be prepared by analogy with methods reported in the literature. General schemes 9 and 10 describe the methods used.

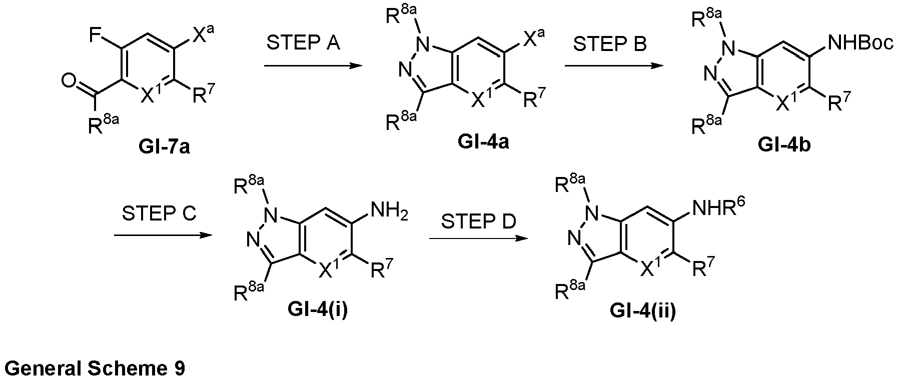

General scheme 9 describes the preparation of amino indazoles (Gl-4(i) and GI-4(ii)) used in the synthesis of examples of formula (I) in which Z¹ is CR^{8a} and Z² is not NH. In step A an appropriate ortho-fluoro carbonyl heteroaryl halide of formula Gl-7a (typically halogen X^{a} is bromide) is cyclised with an alkly hydrazine (where the the alkyl substituent of the hydrazine corresponds to R^{8a}) in iPrOH at 80°C affording halo indazoles of formulaGl-4a In step B halo indazole GI-4a undergoes a Buchwald Hartwig amination with t-butyl carbamate, catalysed by Pd₂(dba)₃ and Xantphos (or a comparable catalytic system) in the presence of a base such as cesium carbonate in a solvent such as 1,4-dioxane. Gl-4b can be Boc-deprotected in step C with HCI in dioxane or in TFA with or without a cosolvent such as DCM to produce amino indazoles of formula Gl-4(i). Finally if R⁶ is not H, R⁶ can be introduced by reductive amination using an appropriate aldehyde (R⁶CHO) and NaBH₄ in MeOH or alternatively by alkylation with R⁶X^{a} (where X^{a} is chloro, bromo or iodo-) in the presence of NaH in THF to produce amino indazoles of formula GI-4(ii) .

Amino indazoles (GI-4(iii) and Gl-4(iv)) used in the synthesis of examples of formula (I) in which Z² is CR^{8a} and Z¹ is not NH were prepared in an identical fashion starting from the isomeric fluoro carbonyl heteroaryl halides of formula GI-7c, as shown in general scheme 10.

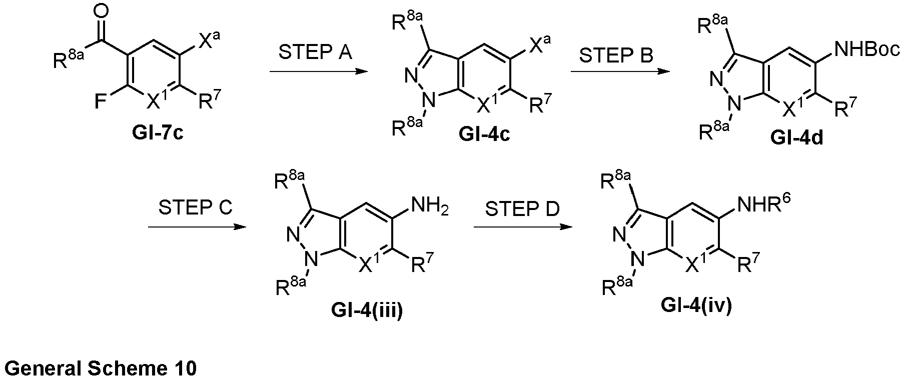

### Synthesis of Intermediates

Scheme 1 below describes the synthesis of several intermediates that are common to certain examples and indicates a synthetic approach that could be adopted to prepare other examples by selecting an appropriately functionalised building block in place of one or more of those in Scheme 1, as detailed in the description of the preparation of individual examples.

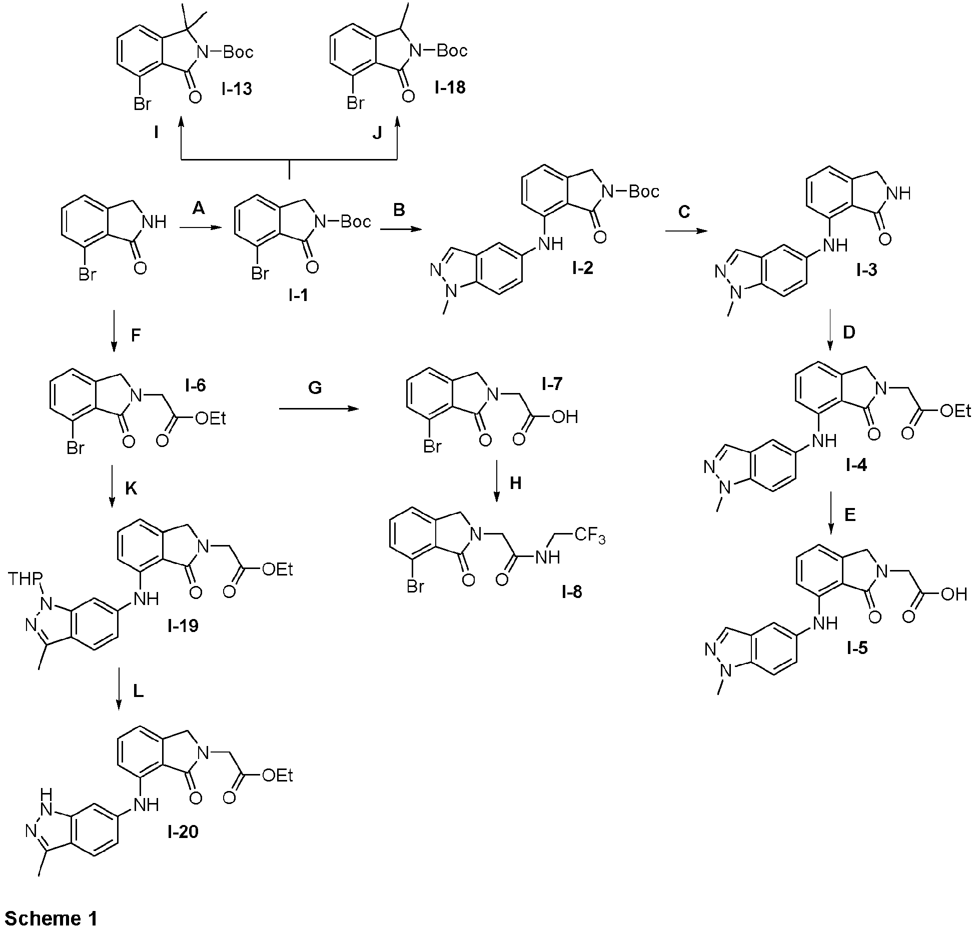

### STEP A. Synthesis of Intermediate I-1, tert-butyl 7-bromo-1-oxo-isoindoline-2-carboxylate

A mixture of 7-bromoisoindolin-1-one (1.0 g, 4.7 mmol), Boc_{z}O (1.5 g, 7.1 mmol) and DMAP (57 mg, 0.47 mmol) in MeCN (20 mL) was stirred at room temperature for 16 h. The mixture was concentrated under reduced pressure and purified by normal phase chromatography (25 g column, 10 to 50% EtOAc in petrol) to give tert-butyl 7-bromo-1-oxo-isoindoline-2-carboxylate (Intermediate I-1, 1.4 g, 4.5 mmol, 95% yield) as a white solid.

UPLC-MS (ES⁺, Method 1): 4.05 min, m/z 256.0/258.0 [M-tBu+H]⁺.

¹H NMR (400 MHz, DMSO-d₆) δ (ppm) 7.75 (d, J=7.2 Hz, 1H), 7.71 - 7.60 (m, 2H), 4.78 (s, 2H), 1.57 (s, 9H).

### STEP B. Synthesis of Intermediate I-2, tert-butyl 7-[(1-methylindazol-5-yl)amino]-1-oxo-isoindoline-2-carboxylate

A mixture of tert-butyl 7-bromo-1-oxo-isoindoline-2-carboxylate (Intermediate I-1, 1.0 g, 3.2 mmol), 1-methyl-1H-indazol-5-amine (519 mg, 3.5 mmol), Pd₂(dba)₃ (293 mg, 0.32 mmol), XantPhos (185 mg, 0.32 mmol) and Cs₂CO₃ (2.1 g, 6.4 mmol) in 1,4-dioxane (20 mL) was heated to 100 °C and stirred for 16 h. The mixture was allowed to cool to room temperature, concentrated under reduced pressure and purified by normal phase chromatography (25 g column, 5 to 20% EtOAc in petrol) to give tert-butyl 7-[(1-methylindazol-5-yl)amino]-1-oxo-isoindoline-2-carboxylate (Intermediate 1-2, 1.0 g, 2.6 mmol, 82% yield) as an off-white solid.

UPLC-MS (ES⁺, Method 1): 4.39 min, m/z 379.2 [M+H]⁺.

¹H NMR (400 MHz, DMSO-d₆) δ (ppm) 8.68 (s, 1H), 8.02 (s, 1H), 7.72 - 7.68 (m, 2H), 7.45 (t, J=8.3 Hz, 1H), 7.37 (dd, J=1.7, 8.9 Hz, 1H), 6.98 (d, J=8.3 Hz, 1H), 6.87 (d, J=7.2 Hz, 1H), 4.77 (s, 2H), 4.08 (s, 3H), 1.57 (s, 9H).

### STEP C. Synthesis of Intermediate I-3, 7-[(1-methylindazol-5-yl)amino]isoindolin-1-one

7-[(1-Methylindazol-5-yl)amino]-1-oxo-isoindoline-2-carboxylate (Intermediate 1-2, 150 mg, 0.39 mmol) was dissolved in dichloromethane (2 mL), trifluoroacetic acid (0.5 mL) was added and the mixture was stirred at room temperature for 2 h, then it was concentrated under reduced pressure to give crude 7-[(1-methylindazol-5-yl)amino]isoindolin-1-one (Intermediate 1-3, 100 mg, 0.36 mmol, 92% yield) which was used in the next step without further purification.

UPLC-MS (ES⁺, Method 2): 1.50 min, m/z 279.2 [M+H]⁺.

### STEP D. Synthesis of Intermediate I-4, ethyl 2-[7-[(1-methylindazol-5-yl)amino]-1-oxo-isoindolin-2-yl]acetate

A mixture of 7-[(1-methylindazol-5-yl)amino]isoindolin-1-one (Intermediate I-3, 418 mg, 1.5 mmol), ethyl 2-bromoacetate (276 mg, 1.6 mmol) and Cs₂CO₃ (978 mg, 3.0 mmol) in MeCN (20 mL) was heated to 50°C and stirred for 16 h. The mixture was concentrated under reduced pressure and purified by normal phase chromatography (12 g column, 10 to 35% EtOAc in petrol) to give ethyl 2-[7-[(1-methylindazol-5-yl)amino]-1-oxo-isoindolin-2-yl]acetate (Intermediate 1-4, 428 mg, 78% yield) as an off-white solid.

UPLC-MS (ES⁺, Method 1): 4.39 min, m/z 379.2 [M+H]⁺.

¹H NMR (400 MHz, DMSO-d₆) δ (ppm) 8.62 (s, 1H), 8.02 (s, 1H), 7.71 - 7.69 (m, 2H), 7.42 (t, J=7.8 Hz, 1H), 7.37 (dd, J=1.8, 8.9 Hz, 1H), 7.05 (d, J=8.2 Hz, 1H), 6.93 (d, J=7.2 Hz, 1H), 4.54 (s, 2H), 4.41 (s, 2H), 4.22 (q, J=7.1 Hz, 2H), 4.10 (s, 3H), 1.28 (t, J=7.0 Hz, 3H).

### STEP E. Synthesis of Intermediate I-5, 2-[7-[(1-methylindazol-5-yl)amino]-1-oxo-isoindolin-2-yl]acetic acid

To a solution of ethyl 2-[7-[(1-methylindazol-5-yl)amino]-1-oxo-isoindolin-2-yl]acetate (428 mg, 1.2 mmol) in THF (8 mL) and MeOH (5 mL) was added a solution of LiOH monohydrate (247 mg, 5.9 mmol) and the mixture was stirred at room temperature for 16 h. The mixture was acidified with 10% aqueous HCl until pH 6, and the resulting precipitate was collected by filtration, dissolved in MeOH and concentrated under reduced pressure to give 2-[7-[(1-methylindazol-5-yl)amino]-1-oxo-isoindolin-2-yl]acetic acid (Intermediate 1-5, 300 mg, 0.89 mmol, 76% yield) as a white solid.

UPLC-MS (ES⁺, Method 1): 3.48 min, m/z 337.2 [M+H]⁺.

¹H NMR (400 MHz, DMSO-d₆) δ (ppm) 8.62 (s, 1H), 8.00 (s, 1H), 7.70 - 7.66 (m, 2H), 7.42 - 7.33 (m, 2H), 7.03 (d, J=9.2 Hz, 1H), 6.90 (d, J=7.3 Hz, 1H), 4.51 (s, 2H), 4.29 (s, 2H), 4.07 (s, 3H).

### STEP F. Synthesis of Intermediate I-6, ethyl 2-(7-bromo-1-oxo-isoindolin-2-yl)acetate

A solution of 7-bromoisoindolin-1-one (400 mg, 1.9 mmol), ethyl 2-bromoacetate (472 mg, 2.8 mmol) and Cs₂CO₃ (1.8 g, 5.6 mmol) in MeCN (10 mL) was stirred at room temperature for 2 h. The mixture was diluted with water (50 mL), dichloromethane (50 mL) was added and the layers were separated. The aqueous layer was extracted three times with dichloromethane, the organic layers were combined, dried over Na₂SO₄ and concentrated under reduced pressure to give ethyl 2-(7-bromo-1-oxo-isoindolin-2-yl)acetate (Intermediate I-6, 465 mg, 1.6 mmol, 83% yield) as an off-white solid.

UPLC-MS (ES⁺, Method 2): 0.95 min, m/z 285.5 [M+H]⁺ - Me ester (sample run in MeOH, transesterified)

¹H NMR (400 MHz, CDCl₃) δ (ppm) 7.62 (d, J=6.0 Hz, 1H), 7.43 - 7.36 (m, 2H), 4.50 (s, 2H), 4.40 (s, 2H), 4.24 (q, J=6.9 Hz, 2H), 1.31 (t, J=7.0 Hz, 3H).

### STEP G. Synthesis of Intermediate I-7, 2-(7-bromo-1-oxo-isoindolin-2-yl)acetic acid

To a solution of ethyl 2-(7-bromo-1-oxo-isoindolin-2-yl)acetate (Inermediate 1-6, 465 mg, 1.6 mmol) in THF (2 mL) and MeOH (2 mL) was added a solution of LiOH monohydrate (197 mg, 4.7 mmol) in water (2mL) and the solution was stirred at room temperature for 2 h. The mixture was acidified with 1 M HCI until pH 5-6, the resulting precipitate was collected by filtration, dissolved in MeOH and concentrated under reduced pressure to give crude 2-(7-bromo-1-oxo-isoindolin-2-yl)acetic acid (Intermediate 1-7, 421 mg, quantitative yield) which was used in the next step without further purification.

UPLC-MS (ES⁺, Method 2): 0.72 min, m/z 269.7/271.5 [M+H]⁺.

### STEP H. Synthesis of Intermediate I-8, 2-(7-bromo-1-oxo-isoindolin-2-yl)-N-(2,2,2-trifluoroethyl) acetamide

A mixture of 2-(7-bromo-1-oxo-isoindolin-2-yl)acetic acid (420 mg, 1.6 mmol), trifluoroethylamine (186 mg, 1.9 mmol), EDCI (292 mg, 1.9 mmol), HOBT (24 mg, 1.9 mmol) and DIPEA (0 mg, 4.7 mmol) in DMF (5 mL) was stirred at room temperature overnight. The mixture was diluted with water (50 mL), dichloromethane (100 mL) was added and the layers were separated. The aqueous layer was extracted with dichloromethane (2 x 100 mL), the organic layers were combined, dried over Na₂SO₄ and concentrated under reduced pressure to give 2-(7-bromo-1-oxo-isoindolin-2-yl)-N-(2,2,2-trifluoroethyl) acetamide (Intermediate 1-8, 400 mg, 73% yield) as an off-white solid.

UPLC-MS (ES⁺, Method 2): 1.30 min, m/z 350.9/352.9 [M+H]⁺.

¹H NMR (400 MHz, DMSO-d₆) δ (ppm) 8.81 (t, J=6.2 Hz, 1H), 7.71 - 7.61 (m, 2H), 7.58 - 7.52 (m, 1H), 4.50 (s, 2H), 4.28 (s, 2H), 4.00 - 3.90 (m, 2H).

### STEP I. Synthesis of Intermediate I-13, tert-butyl 4-bromo-1,1-dimethyl-3-oxo-isoindoline-2-carboxylate

A solution of tert-butyl 7-bromo-1-oxo-isoindoline-2-carboxylate (Intermediate 1-1, 500 mg, 1.6 mmol) in THF (5 mL) was cooled to 0 °C and NaHMDS (2 M in THF, 2.4 mL, 4.8 mmol) was added. The mixture was stirred at 0 °C for 30 min, then methyl iodide (0.3 mL, 4.8 mmol) was added dropwise. The mixture was slowly warmed to room temperature, concentrated under reduced pressure and purified by normal phase chromatography (25 g column, 10% EtOAc in petrol) to give tert-butyl 4-bromo-1,1-dimethyl-3-oxo-isoindoline-2-carboxylate (Intermediate 1-13, 380 mg, 70% yield) as a clear oil.

UPLC-MS (ES⁺, Method 2): 2.10 min, m/z 284.0/286.0 [M-tBu+H]⁺.

### STEP J. Synthesis of Intermediate I-18, tert-butyl 4-bromo-1-methyl-3-oxo-isoindoline-2-carboxylate

A solution of tert-butyl 7-bromo-1-oxo-isoindoline-2-carboxylate (Intermediate 1-1, 500 mg, 1.6 mmol) in THF (10 mL) at -60 °C under N₂ was stirred for 5 min, then KHMDS (1.6 mL, 1.6 mmol) was added dropwise and the mixture was stirred for 1 h. Mel (159 mg, 1.12 mmol) in THF (10 mL) was added dropwise and the mixture was stirred for 2 h. The mixture was warmed to room temperature, concentrated *in vacuo* and purified by prep-TLC to give tert-butyl 4-bromo-1-methyl-3-oxo-isoindoline-2-carboxylate (Intermediate I-18, 229 mg, 0.7 mmol, 43% yield) as a clear oil.

UPLC-MS (ES⁺, Method 1): 4.25 min, m/z 270.0/272.0 [M-tBu+H]⁺.

¹H NMR (400 MHz, DMSO-d₆) δ (ppm) 7.83 - 7.69 (m, 3H), 5.14 (q, J=6.4 Hz, 1H), 1.66 - 1.62 (m, 12H).

### STEP K. Synthesis of Intermediate I-19, ethyl 2-[7-[(3-methyl-1-tetrahydropyran-2-yl-indazol-6-yl)amino]-1-oxo-isoindolin-2-yl]acetate

A degassed solution of 3-methyl-1-tetrahydropyran-2-yl-indazol-6-amine (6 g, 26 mmol), Cs₂CO₃ (13 g, 39 mmol), Xantphos (3 g, 5.2 mmol), Pd₂(dba)₃ (4.8 g, 5.2 mmol) and ethyl 2-(7-bromo-1-oxo-isoindolin-2-yl)acetate (7.7 g, 26 mmol) in 1,4-dioxane (50 mL) was stirred at 100 °C for 16 h. The mixture was concentrated *in vacuo* and purified by normal phase chromatography (hexane:EtOAc=3:1) to give ethyl 2-[7-[(3-methyl-1-tetrahydropyran-2-yl-indazol-6-yl)amino]-1-oxo-isoindolin-2-yl]acetate (Intermediate 1-19, 6.2 g, 14 mmol, 53% yield) as an off-white solid.

UPLC-MS (ES⁺, Method 1): 4.32 min, m/z 449.2 [M+H]⁺.

¹H NMR (400 MHz, DMSO-d₆) δ (ppm) 8.90 (s, 1H), 7.67 (d, J=8.4 Hz, 1H), 7.54 - 7.43 (m, 2H), 7.32 (d, J=8.2 Hz, 1H), 7.09 (d, J=8.2 Hz, 1H), 6.99 (d, J=7.1 Hz, 1H), 5.79 - 5.70 (m, 1H), 4.52 (s, 2H), 4.39 (s, 2H), 4.19 (q, J=7.0 Hz, 2H), 3.93 - 3.83 (m, 1H), 3.78 - 3.69 (m, 1H), 2.47 (s, 3H), 2.44 - 2.33 (m, 1H), 2.08 - 1.99 (m, 2H), 1.96 - 1.89 (m, 1H), 1.81 - 1.67 (m, 1H), 1.62 - 1.52 (m, 2H), 1.25 (t, J=7.0 Hz, 3H).

### STEP L. Synthesis of Intermediate I-20, ethyl 2-[7-[(3-methyl-1H-indazol-6-yl)amino]-1-oxo-isoindolin-2-yl]acetate

A solution of ethyl 2-[7-[(3-methyl-1-tetrahydropyran-2-yl-indazol-6-yl)amino]-1-oxo-isoindolin-2-yl]acetate (Intermediate 1-19, 6 g , 13 mmol) in trifluoroacetic acid (30 mL) was stirred under microwave irradiation at 80 °C for 2 h. The mixture was concentrated in vacuo and purified by reverse phase chromatography (MeCN:water=60:40) to give ethyl 2-[7-[(3-methyl-1H-indazol-6-yl)amino]-1-oxo-isoindolin-2-yl]acetate (Intermediate 1-20, 1.8 g, 4.9 mmol, 37% yield) as an off-white solid.

UPLC-MS (ES⁺, Method 1): 3.65 min, m/z 365.1 [M+H]⁺.

¹H NMR (400 MHz, DMSO-d₆) δ (ppm) 12.41 (s, 1H), 8.81 (s, 1H), 7.66 (d, J=8.9 Hz, 1H), 7.47 (t, J=7.8 Hz, 1H), 7.33 (s, 1H), 7.29 (d, J=8.4 Hz, 1H), 7.00 - 6.96 (m, 2H), 4.52 (s, 2H), 4.38 (s, 2H), 4.18 (q, J=7.1 Hz, 2H), 2.47 (s, 3H), 1.25 (t, J=7.1 Hz, 3H).

Table 3 describes intermediates which were synthesised in an analogous fashion to 1-2, tert-butyl 7-[(1-methylindazol-5-yl)amino]-1-oxo-isoindoline-2-carboxylate (Step B, Scheme 1), replacing tert-butyl 7-bromo-1-oxo-isoindoline-2-carboxylate (Intermediate 1-1) or 1-methyl-1H-indazol-5-amine with the appropriate building block.

**Table 3**

| Building Block | Structure/Name | LCMS | ¹H NMR (400 MHz, DMSO-d₆) δ (ppm) |
|---|---|---|---|
| **Intermediate I-1** and N,1-dimethylindazol-5-amine | 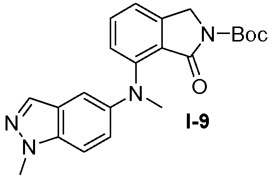 | 4.18 min, m/z, 393.3 [M+H]⁺ (Method 1) | 7.89 (s, 1H), 7.58 (t, J=7.8 Hz, 1H), 7.46 (d, J=9.0 Hz, 1H), 7.28 (d, J=7.0 Hz, 1H), 7.20 (d, J=1.8 Hz, 1H), 7.05 (d, J=8.2 Hz, 1H), 6.98 (dd, J=2.4, 8.9 Hz, 1H), 4.76 (s, 2H), 4.01 (s, 3H), 1.51 (s, 9H). One CH₃ signal under solvent peak. |
| | *tert*-Butyl 7-[methyl-(1-methylindazol-5-yl)amino]-1-oxo-isoindoline-2-carboxylate | | |
| | **Intermediate I-9** | | |
| **Intermediate I-13** and 1-methyl-1H-indazol-5-amine | 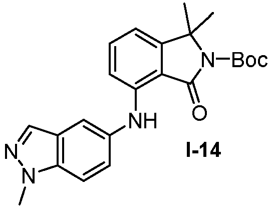 | 2.39 min, m/z, 307.1 [M-Boc+H]⁺ (Method 2) | 8.77 (s, 1H), 8.01 (s, 1H), 7.69 (d, J=9.1 Hz, 2H), 7.46 (t, J=7.9 Hz, 1H), 7.37 (dd, J=1.6, 9.0 Hz, 1H), 6.96 - 6.89 (m, 2H), 4.07 (s, 3H), 1.69 (s, 6H), 1.57 (s, 9H). |
| | *tert*-butyl 1,1-dimethyl-4-[(1-methylindazol-5-yl)amino]-3-oxo-isoindoline-2-carboxylate | | |
| | **Intermediate I-14** | | |
| **Intermediate I-18** and 1-methyl-1H-indazol-5-amine | 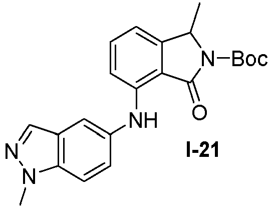 | 4.52 min, m/z, 293.2 [M-Boc+H]⁺ (Method 1) | |
| | tert-butyl 1-methyl-4-[(1-methylindazol-5-yl)amino]-3-oxo-isoindoline-2-carboxylate | | |
| | **Intermediate I-21** | | |

Table 4 describes intermediates which were synthesised in an analogous fashion to 1-3, 7-[(1-methylindazol-5-yl)amino]isoindolin-1-one (Step C, Scheme 1), replacing 7-[(1-methylindazol-5-yl)amino]-1-oxo-isoindoline-2-carboxylate with the appropriate building block. The intermediates were carried to the next step without further purification.

**Table 4**

| Building Block | Structure/Name | LCMS |
|---|---|---|
| *tert*-Butyl 7-[methyl-(1-methylindazol-5-yl)amino]-1-oxo-isoindoline-2-carboxylate | 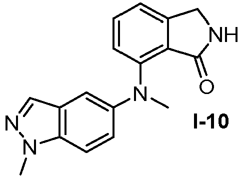 | 3.28 min, m/z, 293.2 [M+H]⁺ (Method 1) |
| **Intermediate I-9** | 7-[methyl-(1-methylindazol-5-yl)amino]isoindolin-1-one | |
| | **Intermediate I-10** | |
| *tert*-butyl 1,1-dimethyl-4-[(1-methylindazol-5-yl)amino]-3-oxo-isoindoline-2-carboxylate | 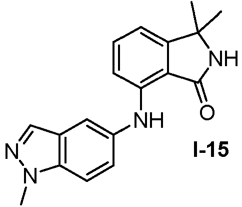 | 1.18 min, m/z, 307.1 [M+H]⁺ (Method 2) |
| **Intermediate I-14** | 3,3-dimethyl-7-[(1-methylindazol-5-yl)amino]isoindolin-1-one | |
| | **Intermediate I-15** | |
| tert-butyl 1-methyl-4-[(1-methylindazol-5-yl)amino]-3-oxo-isoindoline-2-carboxylate | 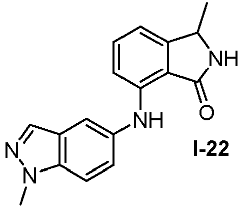 | Not characterised (used directly in subsequent steps see 1-23 for characterisation) |
| **Intermediate I-21** | Intermediate 1-22 | |

Table 5 describes intermediates which were synthesised in an analogous fashion to 1-4, ethyl 2-[7-[(1-methylindazol-5-yl)amino]-1-oxo-isoindolin-2-yl]acetate (Step D, Scheme 1), replacing 7-[(1-methylindazol-5-yl)amino]isoindolin-1-one with the appropriate building block.

**Table 5**

| Building Block | Structure/Name | LCMS | ¹H NMR (400 MHz, DMSO-d₆) δ (ppm) |
|---|---|---|---|
| 7-[methyl-(1-methylindazol-5-yl)amino]isoindolin-1-one | 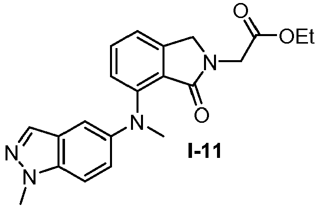 | 3.78 min, m/z, 379.2 [M+H]⁺ (Method 1) | 7.88 (s, 1H), 7.51 (t, J=7.7 Hz, 1H), 7.44 (d, J=9.2 Hz, 1H), 7.30 (d, J=7.4 Hz, 1H), 7.17 (d, J=1.8 Hz, 1H), 7.04 (d, J=8.2 Hz, 1H), 6.96 (dd, J=2.1, 9.1 Hz, 1H), 4.51 (s, 2H), 4.31 (s, 2H), 4.22 (s, 3H), 4.15 (q, J=7.3 Hz, 2H), 4.00 (s, 3H), 1.22 (t, J=7.1 Hz, 3H). |
| **Intermediate I-10** | | | |
| | Ethyl 2-[7-[methyl-(1-methylindazol-5-yl)amino]-1-oxo-isoindolin-2-yl]acetate | | |
| | **Intermediate I-11** | | |
| 3,3-dimethyl-7-[(1-methylindazol-5-yl)amino]isoindolin-1-one | 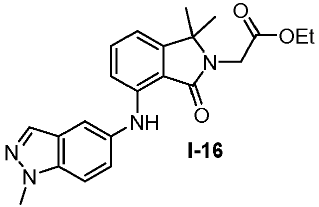 | 4.15 min, m/z, 393.3 [M+H]⁺ (Method 1) | N/D |
| **Intermediate I-15** | | | |
| | Ethyl 2-[1,1-dimethyl-4-[(1-methylindazol-5-yl)amino]-3-oxo-isoindolin-2-yl]acetate | | |
| | **Intermediate I-16** | | |
| 3-methyl-7-[(1-methylindazol-5-yl)amino]isoindolin-1-one | 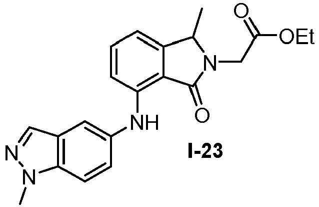 | 4.08 min, m/z, 379.2 [M+H] ⁺ (Method 1) | N/D |
| **Intermediate I-22** | | | |
| | Ethyl 2-[1-methyl-4-[(1-methylindazol-5-yl)amino]-3-oxo-isoindolin-2-yl]acetate | | |
| | **Intermediate I-23** | | |

Table 6 describes intermediates which were synthesised in an analogous fashion to **I-5,** 2-[7-[(1-methylindazol-5-yl)amino]-1-oxo-isoindolin-2-yl]acetic acid (Step E, Scheme 1), replacing ethyl 2-[7-[(1-methylindazol-5-yl)amino]-1-oxo-isoindolin-2-yl]acetate with the appropriate building block.

**Table 6**

| Building Block | Structure/Name | LCMS | ¹H NMR (400 MHz, DMSO-d₆) δ (ppm) |
|---|---|---|---|
| Ethyl 2-[7-[methyl-(1-methylindazol-5-yl)amino]-1-oxo-isoindolin-2-yl]acetate | 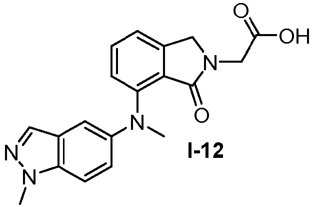 | 3.32 min, m/z, 351.2 [M+H]⁺ (Method 1) | N/D |
| **Intermediate I-11** | 2-[7-[methyl-(1-methylindazol-5-yl)amino]-1-oxo-isoindolin-2-yl]acetic acid | | |
| | **Intermediate I-12** | | |
| Ethyl 2-[1,1-dimethyl-4-[(1-methylindazol-5-yl)amino]-3-oxo-isoindolin-2-yl]acetate | 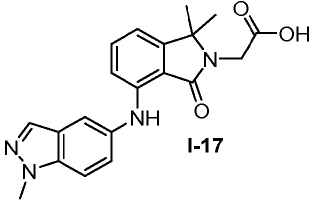 | 3.62 min, m/z, 365.2 [M+H]⁺ | 7.99 (s, 1H), 7.69 - 7.65 (m, 2H), 7.38 (t, J=7.9 Hz, 1H), 7.33 (dd, J=1.9, 9.0 Hz, 1H), 6.99 (d, J=8.2 Hz, 1H), 6.92 (d, J=7.2 Hz, 1H), 4.16 (s, 2H), 4.06 (s, 3H), 1.44 (s, 6H). OH and NH not observed. |
| | | (Method 1) | |
| **Intermediate I-16** | 2-[1,1-dimethyl-4-[(1-methylindazol-5-yl)amino]-3-oxo-isoindolin-2-yl]acetic acid | | |
| | **Intermediate I-17** | | |
| 2-[1-methyl-4-[(1-methylindazol-5-yl)amino]-3-oxo-isoindolin-2-yl]acetic acid | 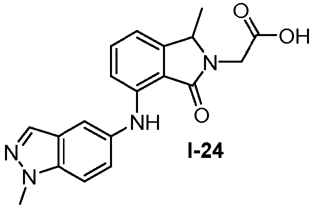 | N/D used directly in next step (see example 15 for characterisation) | N/D |
| **Intermediate I-23** | | | |
| ethyl 2-[7-[(3-methyl-1-tetrahydropyran-2-yl-indazol-6-yl)amino]-1-oxo-isoindolin-2-yl]acetate | 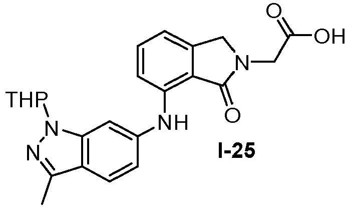 | N/D used directly in next step (see example 25 for characterisation) | N/D |
| **Intermediate I-19** | 2-[7-[(3-methyl-1-tetrahydropyran-2-yl-indazol-6-yl)amino]-1-oxo-isoindolin-2-yl]acetic acid | | |
| | **Intermediate I-25** | | |
| ethyl 2-[7-[(3-methyl-1 H-indazol-6-yl)amino]-1-oxo-isoindolin-2-yl]acetate | 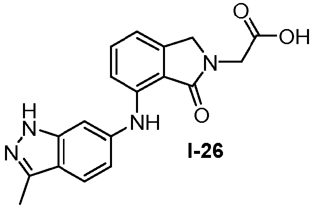 | 3.35 min, m/z, 337.1 [M+H]⁺ (Method 1) | 7.71 (d, J=8.6 Hz, 1H), 7.48 (t, J=7.8 Hz, 1H), 7.37 - 7.29 (m, 2H), 7.04 - 6.99 (m, 2H), 4.52 (s, 2H), 4.29 (s, 2H), 2.53 (s, 3H). 2 x NH and CO2H not observed. |
| **Intermediate I-20** | 2-[7-[(3-methyl-1H-indazol-6-yl)amino]-1-oxo-isoindolin-2-yl]acetic acid | | |
| | **Intermediate I-26** | | |

Table 7 describes intermediates prepared from intermediate 1-6, ethyl 2-(7-bromo-1-oxo-isoindolin-2-yl)acetate, by analogy with intermediate 1-19, ethyl 2-[7-[(3-methyl-1-tetrahydropyran-2-yl-indazol-6-yl)amino]-1-oxo-isoindolin-2-yl]acetate (step K, scheme 1), replacing 3-methyl-1-tetrahydropyran-2-yl-indazol-6-amine with the appropriate amine building block.

**Table 7**

| Building Block | Structure/Name | LCMS | ¹H NMR (400 MHz, DMSO-d₆) δ (ppm) |
|---|---|---|---|
| 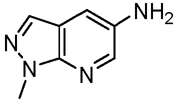 1-methylpyrazolo[3,4-b]pyridin-5-amine | 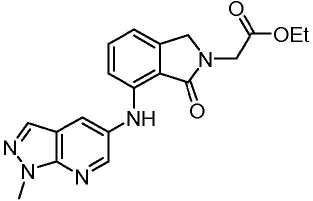 ethyl 2-[7-[(1-methylpyrazolo[3,4-b]pyridin-5-yl)amino]-1-oxo-isoindolin-2-yl]acetate | 1.30 min, m/z, 366.0 [M+H] ⁺ (Method 2) | 8.59 (s, 1H), 8.52 (s, 1H), 8.17 (s, 1H), 8.08 (s, 1H), 7.38 - 7.34 (m, 1H), 6.92 (d, 2H), 4.50 (s, 2H), 4.36 (s, 2H), 4.17 (q, J=7.0 Hz, 2H), 1.25 (t, J=7.0 Hz, 3H). |
| | Intermediate **I-27** | | |
| 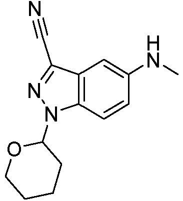 5-(methylamino)-1-tetrahydropyran-2-yl-indazole-3-carbonitrile | 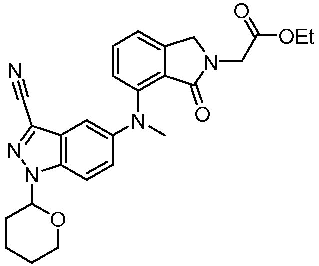 ethyl 2-[7-[(3-cyano-1-tetrahydropyran-2-yl-indazol-5-yl)-methyl-amino]-1-oxo-isoindolin-2-yl]acetate | 4.31 min, m/z, 474.2 [M+H]⁺ (Method 1) | |
| | Intermediate **I-41** | | |

Further intermediates of use in the preparation of examples were derived from intermediate I-6 according to scheme 2 below.

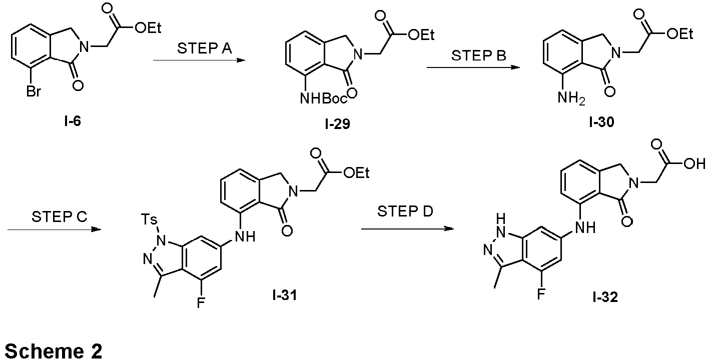

STEP A. Intermediate 1-29, ethyl 2-[7-(tert-butoxycarbonylamino)-1-oxo-isoindolin-2-yl]acetate. A mixture of ethyl 2-(7-bromo-1-oxoisoindolin-2-yl)acetate (4 g, 13.417 mol), tert-butyl carbamate (3.14 g , 26.8 mol), Pd₂(dba)₃ (1.3 g, 1.34 mol, 0.1 eq), Xantphos (775 mg, 1.34 mol) and Cs₂CO₃ (8.72 g, 26.8 mol) in 1,4-dioxane (30 mL) was stirred at 100°C under N₂ overnight. The mixture was concentrated under vacuum and purified by silica gel column chromatography (pet. ether/ EtOAc, 30/1 to pet. ether / EtOAc, 20/1, v/v) to give ethyl 2-[7-(tert-butoxycarbonylamino)-1-oxo-isoindolin-2-yl]acetate (I-29) (2.73 g, 60.8 % yield) as a colourless oil.

UPLC-MS (ES⁺, Method 1): 4.38 min, m/z 334.4 [M+H]⁺.

¹H NMR (400 MHz, DMSO-d₆) δ (ppm) 9.42 (s, 1H), 8.02-8.00 (m, 1H), 7.57-7.53 (m, 1H), 7.21-7.19 (m, 1H), 4.51 (s, 2H), 4.35, (s, 2H), 4.15 (q, *J* = 7.3 Hz, 2H), 1.49 (s, 9H), 1.21 (t, *J* = 7.3 Hz, 3H).

STEP B. Intermediate I-30, ethyl 2-(7-amino-1-oxo-isoindolin-2-yl)acetate. A mixture of ethyl 2-[7-(tert-butoxycarbonylamino)-1-oxo-isoindolin-2-yl]acetate (I-29) (500 mg, 1.5 mmol) in DCM (5 mL) and 4 M HCI in dioxane (3 mL) was stirred at room temperature for 2 h.The reaction was concentrated to give ethyl 2-(7-amino-1-oxo-isoindolin-2-yl)acetate (I-30) (530 mg, 90 % yield) as a crude solid used without further purification.

UPLC-MS (ES⁺, Method 2): 1.00 min, m/z 235.1 [M+H]⁺.

STEP C. Intermediate 1-31, ethyl 2-[7-[[4-fluoro-3-methyl-1-(p-tolylsulfonyl)indazol-6-yl]amino]-1-oxo-isoindolin-2-yl]acetate. A solution of 6-bromo-4-fluoro-3-methyl-1-(p-tolylsulfonyl)indazole (410 mg, 1.07 mmol), ethyl 2-(7-amino-1-oxo-isoindolin-2-yl)acetate (I-30) (276 mg, 1.181 mmol), Cs₂CO₃ (700 mg, 2.14 mmol), Xantphos(124. mg, 0.22 mmol), and Pd₂(dba)₃(98 mg, 0.11 mmol), in 1,4-dioxane (5.0 mL) was stirred at 100 °C under N₂ overnight. The mixture was diluted with EtOAc (10 mL) and washed with H₂O (10 mL x 3). The organic layers were washed with EtOAc (20 mL × 3), combined, dried over anhydrous Na₂SO₄ and concentrated to give a yellow solid analysed as ethyl 2-[7-[[4-fluoro-3-methyl-1-(p-tolylsulfonyl)indazol-6-yl]amino]-1-oxo-isoindolin-2-yl]acetate (I-31) (400 mg, 69 % yield) which was used without further purification.

UPLC-MS (ES⁺, Method 1): 4.62 min, m/z 537.0 [M+H]⁺.

STEP D. Intermediate I-32, 2-[7-[(4-fluoro-3-methyl-1H-indazol-6-yl)amino]-1-oxo-isoindolin-2-yl]acetic acid. A mixture of ethyl 2-[7-[[4-fluoro-3-methyl-1-(p-tolylsulfonyl)indazol-6-yl]amino]-1-oxo-isoindolin-2-yl]acetate (I-31) (400 mg, 0.75 mmol), and LiOH.H₂O (93.8 mg, 2.23 mmol) in THF (3 mL), MeOH (3 mL) and H₂O (2 mL) was stirred at 25°C overnight. The mixture was diluted with H₂O (10 mL) and 10% HCI in water (25 mL) was added to reach a pH of 2 - 3. The mixture was further diluted with water (20 mL x 3) and extracted with DCM (30 mL x 3). After drying over Na₂SO₄, the mixture was filtered and concentrated *in vacuo* to obtain 2-[7-[(4-fluoro-3-methyl-1H-indazol-6-yl)amino]-1-oxo-isoindolin-2-yl]acetic acid (I-32) (200 mg, 76 % yield) as a white solid.

UPLC-MS (ES⁺, Method 1): 3.52 min, m/z 355.0 [M+H]⁺.

### Intermediate I-33, 2-[7-[(3-methyl-1H-pyrazolo[4,3-b]pyridin-6-yl)amino]-1-oxo-isoindolin-2-yl]acetic acid

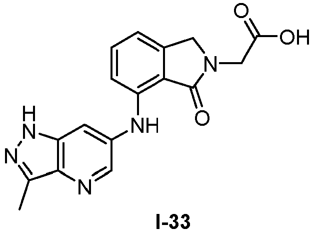

Intermediated I-33 was prepared following an analogous synthetic route to that used for I-32 (scheme 2). In step C, 6-bromo-4-fluoro-3-methyl-1-(p-tolylsulfonyl)indazole was replaced with 6-bromo-3-methyl-1-(p-tolylsulfonyl)pyrazolo[4,3-b]pyridine.

UPLC-MS (ES⁺, Method 1): 2.88 min, m/z 338.1 [M+H]⁺.

¹H NMR (400 MHz, DMSO-d₆) δ (ppm) 8.49 (s, 1H), 7.81 - 7.78 (m, 2H), 7.48 - 7.09 (m, 6H), 4.51 (s, 2H), 4.23 (s, 2H), 2.30 (s, 3H).

### Intermediate I-34, 2-[1-oxo-7-[(1-tetrahydropyran-2-ylpyrazolo[3,4-b]pyridin-5-yl)amino]isoindolin-2-yl]acetic acid.

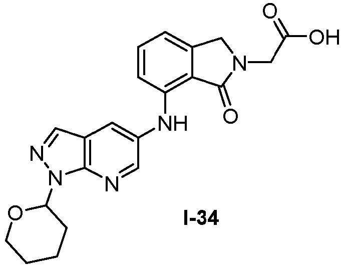

Intermediated I-34 was prepared following an analogous synthetic route to that used for I-32 (scheme 2). In step C, 6-bromo-4-fluoro-3-methyl-1-(p-tolylsulfonyl)indazole was replaced with 1-tetrahydropyran-2-ylpyrazolo[3,4-b]pyridin-5-amine.

UPLC-MS (ES⁺, Method 2): 1.22 min, m/z 408.4 [M+H]⁺.

¹H NMR (400 MHz, DMSO-d₆) δ (ppm) 8.67 (s, 1H), 8.54 (s, 1H), 8.20 (s, 1H), 8.15 (s, 1H), 7.39 - 7.34 (m, 1H), 6.96 - 6.91 (m, 2H), 6.02 - 5.99 (m, 1H), 4.49 (s, 2H), 4.24 (s, 2H), 3.97 - 3.94 (m, 1H), 3.73 - 3.71 (m, 1H), 2.25 - 1.57 (m, 6H).

### Intermediate I-35. 2-[7-[(3-isopropyl-1H-indazol-6-yl)amino]-1-oxo-isoindolin-2-yl]acetic acid.

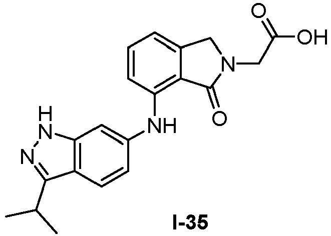

Intermediate I-35 was prepared by analogy with Intermediate I-32 (scheme 2). In step C, 6-bromo-4-fluoro-3-methyl-1-(p-tolylsulfonyl)indazole was replaced with 6-bromo-3-isopropyl-1-(p-tolylsulfonyl)indazole.

UPLC-MS (ES⁺, Method 2): 1.27 min, m/z, 364.8 [M+H]⁺.

### Intermediate I-36. 2-[7-(1H-indazol-6-ylamino)-1-oxo-isoindolin-2-yl]acetic acid.

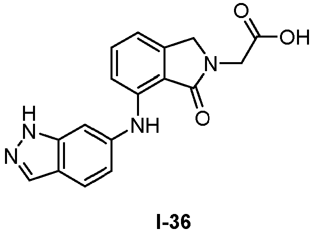

Intermediate I-36 was prepared by analogy with Intermediate I-32 (scheme 2). In step C, 6-bromo-4-fluoro-3-methyl-1-(p-tolylsulfonyl)indazole was replaced with 6-bromo-1-tetrahydropyran-2-yl-indazole.

UPLC-MS (ES⁺, Method 1): 3.23 min, m/z, 323.1 [M+H]⁺.

### Intermediate I-37. 2-[7-[(3-cyano-1-tetrahydropyran-2-yl-indazol-5-yl)amino]-1-oxo-isoindolin-2-yl]acetic acid.

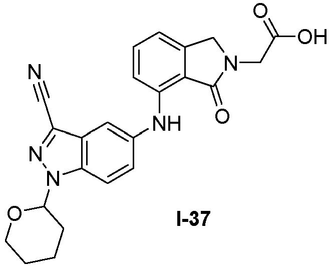

Intermediate I-37 was prepared by analogy with Intermediate I-32 (scheme 2). In step C, 6-bromo-4-fluoro-3-methyl-1-(p-tolylsulfonyl)indazole was replaced with 5-bromo-1-tetrahydropyran-2-yl-indazole-3-carbonitrile.

UPLC-MS (ES⁺, Method 1): 4.11 min, m/z, 432.2 [M+H]⁺.

### Intermediate I-38. 2-[7-[(3-fluoro-1H-indazol-5-yl)amino]-1-oxo-isoindolin-2-yl]acetic acid.

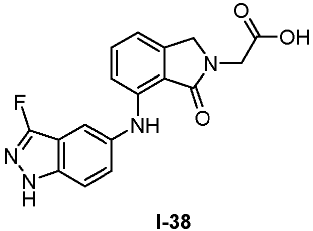

Intermediate I-38 was prepared by analogy with Intermediate I-32 (scheme 2). In step C, 6-bromo-4-fluoro-3-methyl-1-(p-tolylsulfonyl)indazole was replaced with 5-bromo-3-fluoro-1-(p-tolylsulfonyl)indazole.

UPLC-MS (ES⁺, Method 1): 3.45 min, m/z, 351.1 [M+H]⁺.

### Intermediate I-39. 2-[1-oxo-7-(1H-pyrazolo[4,3-b]pyridin-5-ylamino)isoindolin-2-yl]acetic acid.

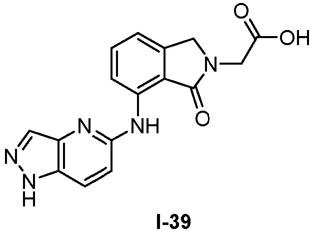

Intermediate I-39 was prepared by analogy with Intermediate I-32 (scheme 2). In step C, 6-bromo-4-fluoro-3-methyl-1-(p-tolylsulfonyl)indazole was replaced with 5-bromo-1-tetrahydropyran-2-yl-pyrazolo[4,3-b]pyridine.

UPLC-MS (ES⁺, Method 2): 0.71 min, m/z, 323.6 [M+H]⁺.

### Intermediate I-40. 2-[7-[(1-methylpyrazolo[4,3-b]pyridin-5-yl)amino]-1-oxo-isoindolin-2-yl]acetic acid.

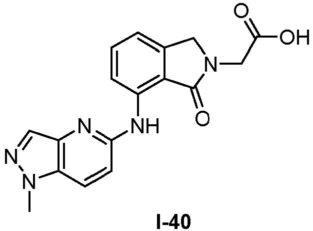

Intermediate I-40 was prepared by analogy with Intermediate I-32 (scheme 2). In step C, 6-bromo-4-fluoro-3-methyl-1-(p-tolylsulfonyl)indazole was replaced with 5-bromo-1-methyl-pyrazolo[4,3-b]pyridine.

UPLC-MS (ES⁺, Method 1): 3.58 min, m/z 338.2 [M+H]⁺.

¹H NMR (400 MHz, DMSO-d₆) δ (ppm) 9.82 (s, 1H), 8.78 (d, *J* = 8.3 Hz, 1H), 8.15 - 7.96 (m, 2H), 7.63 - 7.51 (m, 1H), 7.05 (dd, J = 26.4, 8.2 Hz, 2H), 4.52 (s, 2H), 4.41 (s, 2H), 4.04 (s, 3H), 3.70 (s, 3H).

### Intermediate I-42. 2-[7-[(1-methylpyrazolo[3,4-b]pyridin-5-yl)amino]-1-oxo-isoindolin-2-yl]acetic acid

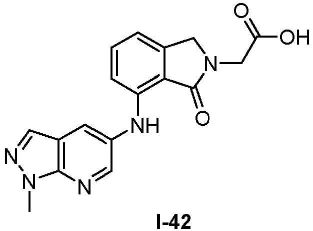

Intermediate I-42 was prepared by analogy with Intermediate I-32 (scheme 2). In step C, 6-bromo-4-fluoro-3-methyl-1-(p-tolylsulfonyl)indazole was replaced with 5-bromo-1 N-methylpyrazolo[3,4-b]pyridine.

UPLC-MS (ES⁺, Method 1): 3.51 min, m/z 338.2 [M+H]⁺.

### Intermediate I-47. 2-[7-[(3-methyl-1H-pyrazolo[4,3-b]pyridin-6-yl)amino]-1-oxo-isoindolin-2-yl]acetic acid.

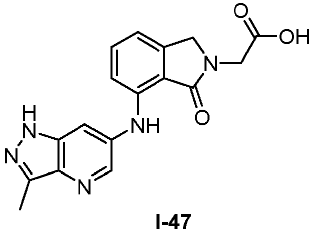

Intermediate I-47 was prepared by analogy with Intermediate I-32 (scheme 2). In step C, 6-bromo-4-fluoro-3-methyl-1-(p-tolylsulfonyl)indazole was replaced with 6-bromo-3-methyl-1-(p-tolylsulfonyl)pyrazolo[4,3-b]pyridine.

UPLC-MS (ES⁺, Method 1): 2.91 min, m/z 338.2 [M+H]⁺.

### Intermediate I-43. 7-bromo-2-[2-oxo-2-[(2S)-(trifluoromethyl)pyrrolidin-1-yl]ethyl]isoindolin-1-one.

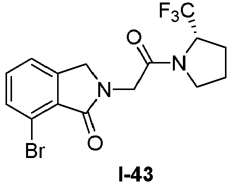

Intermediate I-43 was prepared by analogy with intermediate I-8 (scheme 1), replacing trifluoroethylamine in step H with (2S)-(trifluoromethyl)pyrrolidine.

UPLC-MS (ES⁺, Method 1): 3.72 min, m/z, 391.0/393.0 [M+H]⁺.

### Intermediate I-44. 2-(7-bromo-1-oxo-isoindolin-2-yl)-N-[(1S)-2,2,2-trifluoro-1-methyl-ethyl]acetamide

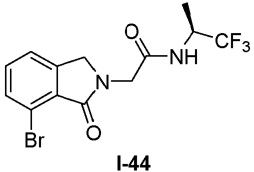

Intermediate I-44 was prepared by analogy with intermediate I-8 (scheme 1), replacing trifluoroethylamine in step H with (2S)-amino-1,1,1-trifluoropropane hydrochloride.

UPLC-MS (ES⁺, Method 1): 3.52 min, m/z, 365.1/367.1 [M+H]⁺.

Further examples could be prepared from intermediates I-48 and I-49, the synthesis of which is described in scheme 3 and the methods below.

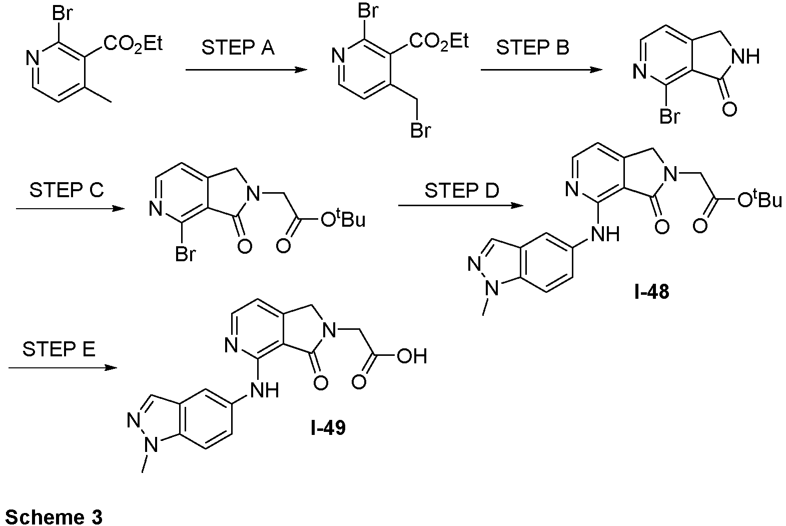

STEP A. Ethyl 2-bromo-4-(bromomethyl)pyridine-3-carboxylate. A mixture of ethyl 2-bromo-4-methylpyridine-3-carboxylate (2 g, 8.19 mmol), NBS (2.92 g, 16.38 mmol) , benzoyl peroxide (396mg, 1.63 mmol) in CCl₄ (20 mL) was stirred at 90°C under N₂ overnight. The mixture was diluted with water (100 mL) and extracted with EtOAc (100 mL x 3) and was dried over Na₂CO₃. The mixture was purified by silica gel chromatography (pet. ether/EtOAc, 100/1 to pet. ether/EtOAc, 50/1) to afford ethyl 2-bromo-4-(bromomethyl)pyridine-3-carboxylate (1.14g, 21% yield).

UPLC-MS (ES⁺, Method 1): 3.98 min, m/z 323.9 [M+H]⁺.

STEP B. 4-Bromo-1,2-dihydropyrrolo[3,4-c]pyridin-3-one. A mixture of ethyl 2-bromo-4-(bromomethyl)pyridine-3-carboxylate (1.14g, 1.76 mmol and concentrated ammonium hydroxide (6 mL) in THF (10 mL) was stirred at 25°C under N₂. The mixture was partitioned between DCM and water and the combined organics dried over Na2SO4 and concentrated before purifying by silica gel chromatography (pet. ether/EtOAc, 5:1 to pet. ether/EtOAc, 1:2) to give 4-bromo-1,2-dihydropyrrolo[3,4-c]pyridin-3-one (320 mg, 43% yield) as a yellow solid.

UPLC-MS (ES⁺, Method 1): 1.34 min, m/z 213.1 [M+H]⁺.

STEP C. tert-Butyl 2-(4-bromo-3-oxo-1H-pyrrolo[3,4-c]pyridin-2-yl)acetate. A mixture of 4-bromo-1,2-dihydropyrrolo[3,4-c]pyridin-3-one (200 mg, 0.94 mmol) Cs₂CO₃ (610 mg, 1.88 mmol) tert-butyl bromoacetate (274 mg, 1.41 mmol) in THF (5mL) was stirred at 50°C for 2 hours. The mixture was diluted with water (20 mL) and extracted with EtOAc (15 mL x 3) and was dried by Na₂CO₃. The mixture was purified by silica gel chromatography (pet. ether/EtOAc, 1/2) to afford tert-butyl 2-(4-bromo-3-oxo-1H-pyrrolo[3,4-c]pyridin-2-yl)acetate (130 mg, 42% yield) as a brown solid.

UPLC-MS (ES⁺, Method 1): 3.48 min, m/z 329.1 [M+H]⁺.

STEP D. tert-Butyl 2-[4-[(1-methylindazol-5-yl)amino]-3-oxo-1H-pyrrolo[3,4-c]pyridin-2-yl]acetate (Intermediate 1-48). A mixture of tert-butyl 2-(4-bromo-3-oxo-1H-pyrrolo[3,4-c]pyridin-2-yl)acetate (200 mg, 0.61 mmol), 1-methyl-1H-indazol-5-amine (89.9 mg, 0.61 mmol), Pd₂(dba)₃ (55.9 mg, 0.061 mmol) , Xantphos (35.3 mg, 0.061 mmol) and Cs₂CO₃ (397mg, 1.22 mmol) in 1,4-dioxane (3 mL) was stirred at 100°C under N₂ overnight. The mixture was diluted with saturated NH₄Cl (20 mL) and extracted with EtOAc (30mL x 3). The mixture was concentrated under vacuum and purified by silica gel chromatography (DCM methanol, 20/1) to afford tert-butyl 2-[4-[(1-methylindazol-5-yl)amino]-3-oxo-1H-pyrrolo[3,4-c]pyridin-2-yl]acetate (I-48) (50 mg, 21%) as a white solid.

UPLC-MS (ES⁺, Method 1): 3.82 min, m/z 394.2 [M+H]⁺.

STEP E. 2-[4-[(1-Methylindazol-5-yl)amino]-3-oxo-1H-pyrrolo[3,4-c]pyridin-2-yl]acetic acid (Intermediate 1-49). A mixture of tert-butyl 2-[4-[(1-methylindazol-5-yl)amino]-3-oxo-1H-pyrrolo[3,4-c]pyridin-2-yl]acetate (I-48) (40 mg, 0.1017 mmol, 1.0 eq) and TFA (1 mL) in DCM (2 mL) was stirred at 25°C for 4 hours. The mixture and was concentrated under vacuum to afford 2-[4-[(1-Methylindazol-5-yl)amino]-3-oxo-1H-pyrrolo[3,4-c]pyridin-2-yl]acetic acid (Intermediate I-49) (40 mg) as a brown solid containing residual TFA and used without further purification.

UPLC-MS (ES⁺, Method 1): 2.55 min, m/z 338.2 [M+H]⁺.

### Synthesis of Examples

### Example 1, 2-[7-[(1-Methylindazol-5-yl)amino]-1-oxo-isoindolin-2-yl]-N-(2,2,2-trifluoroethyl) acetamide

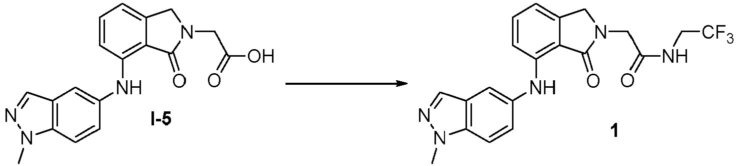

A mixture of 2-[7-[(1-methylindazol-5-yl)amino]-1-oxo-isoindolin-2-yl]acetic acid (Intermediate **I-5,** 60 mg, 0.18 mmol), trifluoroethylamine (27 mg, 0.27 mmol), T3P (170 mg, 0.3 mmol) and DIPEA (69 mg, 0.53 mmol) in dichloromethane (2 mL) was stirred at room temperature for 16 h. The mixture was concentrated under reduced pressure and purified by prep LCMS to give 2-[7-[(1-methylindazol-5-yl)amino]-1-oxo-isoindolin-2-yl]-N-(2,2,2-trifluoroethyl)acetamide (Example 1, 12 mg, 16% yield) as a yellow solid.

UPLC-MS (ES⁺, Method 1): 3.74 min, m/z 418.2 [M+H]⁺.

¹H NMR (400 MHz, DMSO-d₆) δ (ppm) 8.88 (t, J=6.4 Hz, 1H), 8.68 (s, 1H), 8.05 (s, 1H), 7.75 - 7.71 (m, 2H), 7.46 - 7.37 (m, 2H), 7.07 (d, J=8.3 Hz, 1H), 6.94 (d, J=7.3 Hz, 1H), 4.54 (s, 2H), 4.32 (s, 2H), 4.12 (s, 3H), 4.02 (dq, J=6.6, 9.7 Hz, 2H).

Examples synthesised following the same procedure as Example 1 replacing 2-[7-[(1-methylindazol-5-yl)amino]-1-oxo-isoindolin-2-yl]acetic acid (Intermediate I-5) and/or trifluoroethylamine with the appropriate building block are described in table 8.

**Table 8**

| Building Block | Structure/Name | LCMS | ¹H NMR (400 MHz, DMSO-d₆) δ (ppm) |
|---|---|---|---|
| **Intermediate I-5** and (2S)-Amino-1,1,1-trifluoropropane hydrochloride 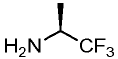 | 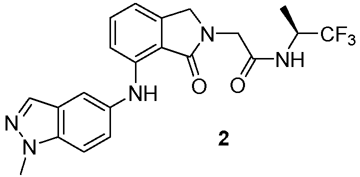 2-[7-[(1-Methylindazol-5-yl)amino]-1-oxo-isoindolin-2-yl]-N-[(1S)-2,2,2-trifluoro-1-methyl-ethyl]acetamide | 3.82 min, m/z 432.2 [M+H]⁺ | 8.75 (d, J=8.9 Hz, 1H), 8.62 (s, 1H), 7.99 (s, 1H), 7.69 - 7.65 (m, 2H), 7.41 - 7.31 (m, 2H), 7.02 (d, J=8.4 Hz, 1H), 6.89 (d, J=7.3 Hz, 1H), 4.69 - 4.58 (m, 1H), 4.49 (s, 2H), 4.28 (d, J=16.8 Hz, 1H), 4.22 (d, J=16.9 Hz, 1H), 4.06 (s, 3H), 1.30 (d, J=6.8 Hz, 3H). |
| | | (Method 1) | |
| | **Example 2** | | |
| **Intermediate I-5** and (2S) - Trifluoromethyl pyrrolidine 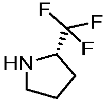 | 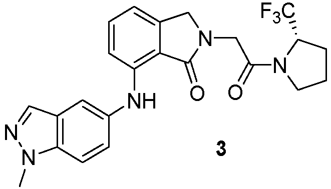 7-[(1-Methylindazol-5-yl)amino]-2-[2-oxo-2-[(2S)-2-(trifluoromethyl)pyrrolidin-1-yl]ethyl]isoindolin-1-one | 4.02 min, m/z 458.3 [M+H]⁺ | 8.64 (s, 1H), 8.00 (s, 1H), 7.69 - 7.66 (m, 2H), 7.41 - 7.32 (m, 2H), 7.03 (d, J=8.2 Hz, 1H), 6.90 (d, J=7.2 Hz, 1H), 4.84 - 4.75 (m, 1H), 4.53 - 4.42 (m, 4H), 4.07 (s, 3H), 3.74 - 3.66 (m, 2H), 2.17 - 1.95 (m, 4H). |
| | | (Method 1) | |
| | **Example 3** | | |
| **Intermediate I-5** and Isoxazol-4-amine 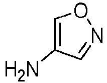 | 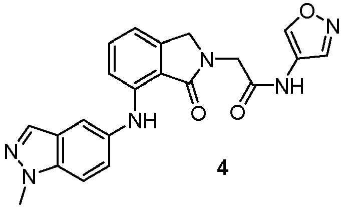 N-isoxazol-4-yl-2-[7-[(1-methylindazol-5-yl)amino]-1-oxo-isoindolin-2-yl]acetamide | 3.58 min, m/z 403.2 [M+H]⁺ | 10.47 (s, 1H), 9.12 (s, 1H), 8.65 (s, 1H), 8.61 (s, 1H), 8.00 - 7.95 (m, 1H), 7.68 - 7.62 (m, 2H), 7.37 (t, *J* = 7.8 Hz, 1H), 7.31 (dd, *J* = 8.9, 2.0 Hz, 1H), 7.01 (d, *J* = 8.3 Hz, 1H), 6.88 (d, *J* = 7.4 Hz, 1H), 4.52 (s, 2H), 4.37 (s, 2H), 4.04 (s, 3H). |
| | | (Method 1) | |
| | **Example 4** | | |
| **Intermediate I-5** and 3-(Trifluoro methyl)aniline 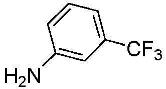 | 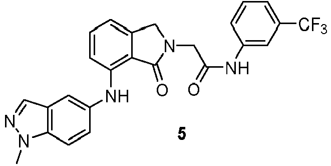 2-{7-[(1-methyl-1H-indazol-5-yl)amino]-1-oxo-2,3-dihydro-1H-isoindol-2-yl}-N-[3-(trifluoromethyl)phenyl]acetamide | 4.25 min, m/z 480.2 [M+H]⁺ | 10.56 (s, 1H), 8.62 (s, 1H), 8.11 (s, 1H), 7.97 (s, 1H), 7.79 (d, *J =* 8.2 Hz, 1H), 7.68 - 7.63 (m, 2H), 7.57 (t, *J* = 8.0 Hz, 1H), 7.42 (d, *J* = 7.8 Hz, 1H), 7.37 (t, *J =* 7.8 Hz, 1H), 7.32 (dd, *J* = 8.9, 2.0 Hz, 1H), 7.01 (d, *J = 8.3* Hz, 1H), 6.88 (d, *J =* 7.4 Hz, 1H), 4.55 (s, 2H), 4.41 (s, 2H), 4.04 (s, 3H). |
| | | (Method 1) | |
| | **Example 5** | | |
| **Intermediate I-12** and trifluoroethylamine 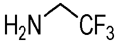 | 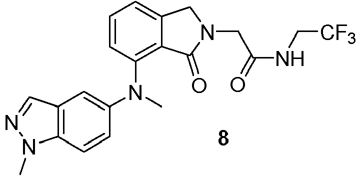 2-[7-[methyl-(1-methylindazol-5-yl)amino]-1-oxo-isoindolin-2-yl]-N-(2,2,2-trifluoroethyl)acetamide | 3.61 min, m/z 432.2 [M+H]⁺ | 8.75 (d, *J =* 5.7 Hz, 1H), 7.86 (s, 1H), 7.47 (t, *J* = 7.7 Hz, 1H), 7.41 (d, *J =* 9.1 Hz, 1H), 7.27 (d, *J* = 7.4 Hz, 1H), 7.15 (d, *J =* 2.2 Hz, 1H), 7.00 (d, *J =* 8.0 Hz, 1H), 6.93 (dd, *J* = 9.1, 2.2 Hz, 1H), 4.48 (s, 2H), 4.19 (s, 2H), 3.98 (s, 3H), 3.92 (dd, *J =* 10.1, 5.0 Hz, 2H), 3.34 (s, 3H). |
| | | (Method 1) | |
| | **Example 8** | | |
| **Intermediate I-17** and trifluoroethylamine 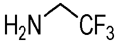 | 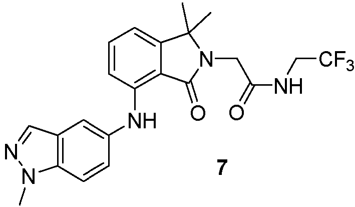 2-[1,1-dimethyl-4-[(1-methylindazol-5-yl)amino]-3-oxo-isoindolin-2-yl]-N-(2,2,2-trifluoroethyl)acetamide | 3.93 min, m/z 446.3 [M+H]⁺ | 8.69 - 8.63 (m, 2H), 7.99 (s, 1H), 7.69 - 7.66 (m, 2H), 7.39 (t, J=7.8 Hz, 1H), 7.33 (d, J=10.6 Hz, 1H), 7.01 (d, J=8.2 Hz, 1H), 6.92 (d, J=7.4 Hz, 1H), 4.14 (s, 2H), 4.07 (s, 3H), 3.96 (dq, J=6.5, 9.7 Hz, 2H), 1.44 (s, 6H). |
| | | (Method 1) | |
| | **Example 7** | | |
| **Intermediate I-5** and 3-(trifluoromethyl) piperidine 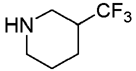 | 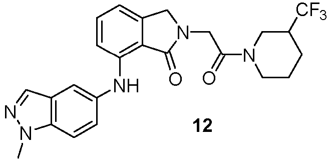 7-[(1-methylindazol-5-yl)amino]-2-[2-oxo-2-[3-(trifluoromethyl)-1-piperidyl]ethyl]isoquinolin-1-one | 4.06 min, m/z 472.3 [M+H] ⁺ | 8.63 (s, 1H), 7.98 (s, 1H), 7.68 - 7.64 (m, 2H), 7.39 - 7.29 (m, 2H), 7.01 (d, J=8.3 Hz, 1H), 6.87 (d, J=7.3 Hz, 1H), 4.50 - 4.37 (m, 5H), 4.20 - 4.16 (m, 1H), 4.05 (s, 3H), 4.01 - 3.97 (m, 1H), 3.91 - 3.86 (m, 1H), 3.20 - 3.11 (m, 1H), 2.85 - 2.76 (m, 1H), 2.00 - 1.97 (m, 1H), 1.81 - 1.71 (m, 1H), 1.59 - 1.53 (m, 1H). |
| | | (Method 1) | |
| | **Example 12** | | |
| **Intermediate I-5** and 4-((dimethylamino)m ethyl)-3-(trifluoromethyl) aniline 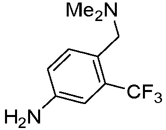 | 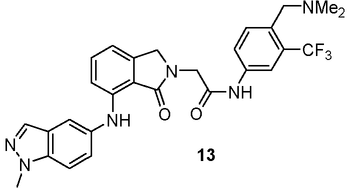 N-[4-[(dimethylamino)methyl]-3-(trifluoromethyl)phenyl]-2-[7-[(1-methylindazol-5-yl)amino]-1-oxo-isoindolin-2-yl]acetamide | 1.20 min, m/z 537.3 [M+H]⁺ | 10.52 (s, 1H), 8.64 (s, 1H), 8.07 (s, 1H), 8.00 (s, 1H), 7.81 (d, J=8.4 Hz, 1H), 7.72 - 7.65 (m, 3H), 7.39 (t, J=7.8 Hz, 1H), 7.34 (d, J=10.6 Hz, 1H), 7.03 (d, J=8.2 Hz, 1H), 6.90 (d, J=7.3 Hz, 1H), 4.56 (s, 2H), 4.42 (s, 2H), 4.06 (s, 3H), 2.18 (s, 6H). (One CH₂ signal under solvent peak) |
| | | (Method 2) | |
| | **Example 13** | | |
| **Intermediate I-5** and (2S) - (trifluoromethyl)pip eridine 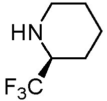 | 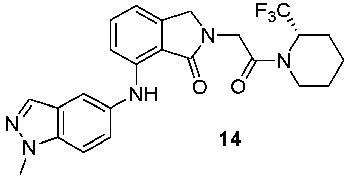 7-[(1-methylindazol-5-yl)amino]-2-[2-oxo-2-[(2S)-(trifluoromethyl)-1-piperidyl]ethyl]isoindolin-1-one | 2.07 min, m/z 472.1 [M+H]⁺ | 8.64 (s, 1H), 8.00 (s, 1H), 7.68 - 7.66 (m, 2H), 7.41 - 7.32 (m, 2H), 7.03 (d, J=8.3 Hz, 1H), 6.89 (d, J=6.7 Hz, 1H), 5.19 - 5.15 (m, 1H), 4.65 - 4.49 (m, 2H), 4.47 - 4.39 (m, 2H), 4.07 (s, 3H), 3.99 - 3.96 (m, 1H), 3.22 - 3.16 (m, 1H), 2.01 - 1.97 (m, 1H), 1.77 - 1.50 (m, 5H). |
| | | (Method 2) | |
| | **Example 14** | | |
| **Intermediate I-5** and (2S)-(difluoromethyl) pyrrolidine hydrochloride 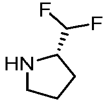 | 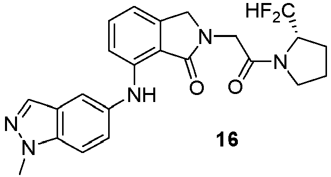 2-[2-[(2S)-(difluoromethyl)pyrrolidin-1-yl]-2-oxo-ethyl]-7-[(1-methylindazol-5-yl)amino]isoindolin-1-one | 3.88 min, m/z 440.3 [M+H] ⁺ | 8.62 (d, *J =* 4.1 Hz, 1H), 7.97 (s, 1H), 7.70 - 7.61 (m, 2H), 7.35 (t, *J =* 7.8 Hz, 1H), 7.31 (dd, *J* = 8.9, 1.9 Hz, 1H), 7.00 (d, *J* = 8.3 Hz, 1H), 6.86 (d, *J =* 7.3 Hz, 1H), 6.40 - 6.03 (m, 1H), 4.57 - 4.21 (m, 5H), 4.04 (s, 3H), 3.60 (t, *J =* 5.9 Hz, 2H), 2.10 - 1.81 (m, 4H). |
| | | (Method 1) | |
| | **Example 16** | | |
| **Intermediate I-5** and (2S)-(fluoromethyl) pyrrolidine hydrochloride 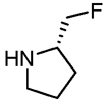 | 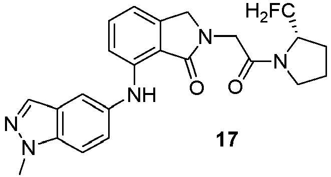 2-[2-[(2S)-(fluoromethyl)pyrrolidin-1-yl]-2-oxo-ethyl]-7-[(1-methylindazol-5-yl)amino]isoindolin-1-one | 3.75 min, m/z 422.3 [M+H] ⁺ | 8.63 (d, *J* = 3.5 Hz, 1H), 7.97 (s, 1H), 7.68 - 7.62 (m, 2H), 7.35 (t, *J =* 7.8 Hz, 1H), 7.31 (dd, *J* = 8.8, 2.0 Hz, 1H), 7.00 (d, *J* = 8.3 Hz, 1H), 6.86 (d, *J =* 7.3 Hz, 1H), 4.52 (t, *J =* 3.7 Hz, 1H), 4.45 (s, 2H), 4.42 - 4.39 (m, 1H), 4.36 (s, 1H), 4.14 (d, *J* = 22.6 Hz, 1H), 4.04 (s, 3H), 3.57 - 3.39 (m, 3H), 2.02 - 1.80 (m, 4H). |
| | | (Method 1) | |
| | **Example 17** | | |
| **Intermediate I-5** and 3-(trifluoromethyl) azetidine 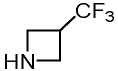 | 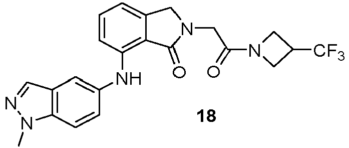 7-[(1-methylindazol-5-yl)amino]-2-[2-oxo-2-[3-(trifluoromethyl)73zetidine-1-yl]ethyl]isoindolin-1-one | 3.78 min, m/z 444.3 [M+H] ⁺ | 8.60 (s, 1H), 7.97 (s, 1H), 7.65 (dd, *J =* 5.6, 3.4 Hz, 2H), 7.38 - 7.29 (m, 2H), 6.99 (d, *J* = 8.3 Hz, 1H), 6.86 (d, *J =* 7.3 Hz, 1H), 4.50 (t, *J =* 9.1 Hz, 1H), 4.43 (s, 2H), 4.30 (dd, *J =* 9.5, 5.6 Hz, 1H), 4.23 (d, *J =* 2.7 Hz, 2H), 4.16 (t, *J =* 9.7 Hz, 1H), 4.04 (s, 3H), 3.90 (dd, *J =* 10.1, 5.4 Hz, 1H), 3.75 - 3.64 (m, 1H). |
| | | (Method 1) | |
| | **Example 18** | | |
| **Intermediate I-5** and 3,3-difluoroazetidine 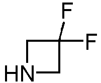 | 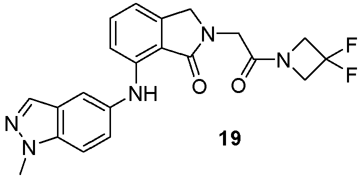 2-[2-(3,3-difluoroazetidin-1-yl)-2-oxo-ethyl]-7-[(1-methylindazol-5-yl)amino]isoindolin-1-one | 3.65 min, m/z 412.3 [M+H] ⁺ | 8.59 (s, 1H), 7.97 (s, 1H), 7.65 (d, *J* = 2.2 Hz, 2H), 7.39 - 7.29 (m, 2H), 6.99 (d, *J* = 8.3 Hz, 1H), 6.86 (d, *J* = 7.4 Hz, 1H), 4.75 (t, *J* = 12.5 Hz, 2H), 4.44 (s, 2H), 4.37 (t, *J =* 12.6 Hz, 2H), 4.29 (s, 2H), 4.04 (s, 3H). |
| | | (Method 1) | |
| | **Example 19** | | |
| **Intermediate I-5** and 3-[(dimethylamino)me thyl]-5-(trifluoromethyl) aniline 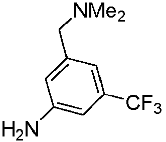 | 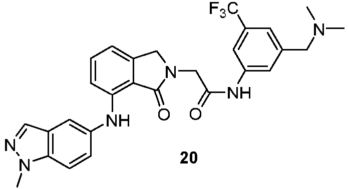 N-[3-[(dimethylamino)methyl]-5-(trifluoromethyl)phenyl]-2-[7-[(1-methylindazol-5-yl)amino]-1-oxo-isoindolin-2-yl]acetamide | 2.82 min, m/z 537.3 [M+H] ⁺ | 10.56 (s, 1H), 8.61 (s, 1H), 7.98 (d, *J* = 8.0 Hz, 2H), 7.84 (s, 1H), 7.67 - 7.63 (m, 2H), 7.40 - 7.34 (m, 2H), 7.33 - 7.29 (m, 1H), 7.05 - 6.99 (m, 1H), 6.88 (d, *J* = 7.4 Hz, 1H), 4.54 (s, 2H), 4.40 (s, 2H), 4.04 (s, 3H), 3.65 (s, 2H), 2.29 (s, 6H). |
| | | (Method 1) | |
| | **Example 20** | | |
| **Intermediate I-5** and 3-(1-amino-1-methyl-ethyl)aniline 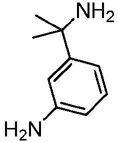 | | 2.98 min, m/z 469.3 [M+H]⁺ | 8.64 (s, 1H), 8.37 (s, 1H), 7.98 (s, 1H), 7.69 - 7.64 (m, 2H), 7.38 - 7.29 (m, 2H), 7.17 (t, J=7.8 Hz, 1H), 7.00 (d, J=8.2 Hz, 1H), 6.97 - 6.91 (m, 2H), 6.85 (d, J=7.4 Hz, 1H), 6.75 (d, J=8.1 Hz, 1H), 4.44 (s, 2H), 4.20 (s, 2H), 4.06 (s, 3H), 1.57 (s, 6H). NH2 not observed. |
| | 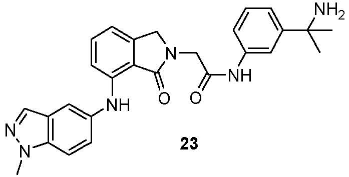 N-[3-(1-amino-1-methylethyl)phenyl]-2-[7-[(1-methylindazol-5-yl)amino]-1-oxo-isoindolin-2-yl]acetamide | (Method 1) | |
| | **Example 23** | | |
| **Intermediate I-5** and 2,2-difluorocyclopropan amine 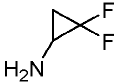 | 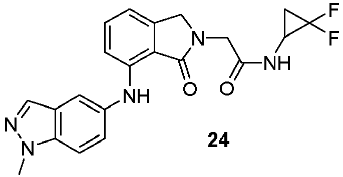 N-(2,2-difluorocyclopropyl)-2-[7-[(1-methylindazol-5-yl)amino]-1-oxo-isoindolin-2-yl]acetamide | 3.58 min, m/z 412.2 [M+H] ⁺ | 8.61 (s, 2H), 7.97 (s, 1H), 7.65 (dd, *J =* 5.6, 3.4 Hz, 2H), 7.38 - 7.28 (m, 2H), 7.00 (d, *J =* 8.2 Hz, 1H), 6.86 (d, *J =* 7.3 Hz, 1H), 4.46 (s, 2H), 4.19 (s, 2H), 4.04 (s, 3H), 3.44 (s, 1H), 1.90 (s, 1H), 1.52 (dq, *J =* 12.4, 5.7 Hz, 1H). |
| | | (Method 1) | |
| | **Example 24** | | |
| **Intermediate I-5** and 2-(trifluoromethyl)mor pholine 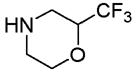 | 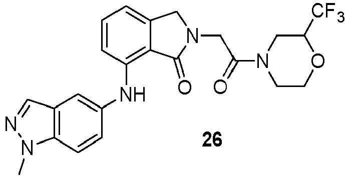 7-[(1-methylindazol-5-yl)amino]-2-[2-oxo-2-[2-(trifluoromethyl)morpholin-4-yl]ethyl]isoindolin-1-one | 3.88 min, m/z 474.3 [M+H] ⁺ | 8.62 (s, 1H), 7.97 (s, 1H), 7.65 (dd, *J* = 5.5, 3.3 Hz, 2H), 7.38 - 7.29 (m, 2H), 7.00 (d, *J = 8.3* Hz, 1H), 6.86 (d, *J =* 7.4 Hz, 1H), 4.63 - 4.53 (m, 1H), 4.43 (d, *J* = 7.2 Hz, 3H), 4.34 (t,*J* = 13.4 Hz, 1H), 4.20 (d, *J* = 31.4 Hz, 1H), 4.16 - 3.81 (m, 6H), 3.73 - 3.52 (m, 1H), 2.90 (t, *J* = 11.9 Hz, 1H), 1.24 (s, 1H). |
| | | (Method 1) | |
| | **Example 26** | | |
| **Intermediate I-5** and 3-(trifluoromethyl)mor pholine hydrochloride 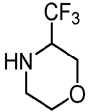 | 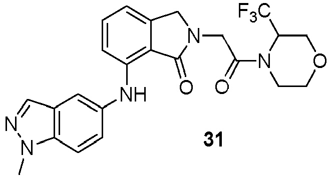 7-[(1-methylindazol-5-yl)amino]-2-[2-oxo-2-[3-(trifluoromethyl)morpholin-4-yl]ethyl]isoindolin-1-one | 3.85 min, m/z 496.2 [M+Na] ⁺ | 8.60 (s, 1H), 7.97 (s, 1H), 7.66 - 7.64 (m, 2H), 7.38 - 7.30 (m, 2H), 7.02 - 6.99 (m, 1H), 6.88 - 6.85 (m, 1H), 4.99 - 4.87 (m, 1H), 4.67 (t, *J* = 16.2 Hz, 1H), 4.51 - 4.34 (m, 3H), 4.19 - 4.10 (s, 1H), 4.04 (s, 3H), 3.93 - 3.80 (m, 2H), 3.69 - 3.41 (m, 3H). |
| | | (Method 1) | |
| | **Example 31** | | |
| **Intermediate I-24** and trifluoroethyl amine 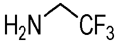 | 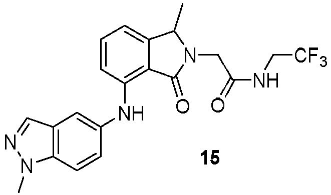 2-[1-methyl-4-[(1-methylindazol-5-yl)amino]-3-oxo-isoindolin-2-yl]-N-(2,2,2-trifluoroethyl)acetamide | 3.82 min, m/z 432.2 [M+H] ⁺ | 8.76 (t, *J* = 6.4 Hz, 1H), 8.61 (s, 1H), 7.97 (d, *J =* 0.9 Hz, 1H), 7.65 (dd, *J* = 5.6, 3.4 Hz, 2H), 7.37 (t, *J* = 7.8 Hz, 1H), 7.31 (dd, *J* = 9.0, 1.9 Hz, 1H), 6.99 (d, *J =* 8.3 Hz, 1H), 6.88 (d, *J =* 7.4 Hz, 1H), 4.62 (d, *J* = 6.7 Hz, 1H), 4.35 (d, *J* = 17.0 Hz, 1H), 4.06 (d, *J =* 9.8 Hz, 4H), 3.99 - 3.86 (m, 2H), 1.40 (d, *J* = 6.8 Hz, 3H). |
| | | (Method 1) | |
| | **Example 15** | | |
| **Intermediate I-26** and (2S)-Amino-1,1,1-trifluoropropane hydrochloride 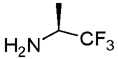 | 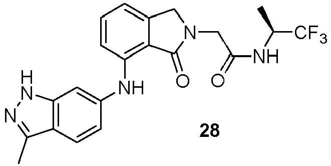 2-[7-[(3-methyl-1H-indazol-6-yl)amino]-1-oxo-isoindolin-2-yl]-N-[(1S)-2,2,2-trifluoro-1-methyl-ethyl]acetamide | 3.68 min, m/z 432.2 [M+H]⁺ | 12.37 (s, 1H), 8.83 (s, 1H), 8.73 (d, *J* = 8.8 Hz, 1H), 7.64 (d, *J* = 8.6 Hz, 1H), 7.43 (t, *J =* 7.8 Hz, 1H), 7.33 - 7.29 (m, 1H), 7.27 (d, *J* = 8.3 Hz, 1H), 6.98 - 6.93 (m, 2H), 4.68 - 4.56 (m, 1H), 4.48 (s, 2H), 4.31 - 4.17 (m, 2H), 2.45 (s, 3H), 1.27 (d, *J* = 7.1 Hz, 3H). |
| | | (Method 1) | |
| | **Example 28** | | |
| **Intermediate I-26** and (2S)-trifluoromethyl pyrrolidine 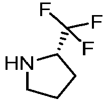 | 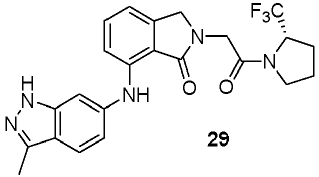 7-[(3-methyl-1H-indazol-6-yl)amino]-2-[2-oxo-2-[(2S)-(trifluoromethyl)77yrrolidine-1-yl]ethyl]isoindolin-1-one | 3.82 min, m/z 458.0 [M+H]⁺ | 12.37 (s, 1H), 8.84 (s, 1H), 7.64 (d, *J* = 8.5 Hz, 1H), 7.44 (t, *J* = 7.8 Hz, 1H), 7.31 - 7.26 (m, 2H), 6.96 - 6.94 (m, 2H), 5.07 - 4.73(m, 1H), 4.61 - 4.37 (m, 4H), 3.66 (p, *J* = 7.9, 7.3 Hz, 2H), 2.67 (s, 0.5H), 2.45 (s, 3H), 2.33 (s, 0.5H), 2.10 - 1.99 (m, 3H). |
| | | (Method 1) | |
| | **Example 29** | | |
| **Intermediate I-26** and 3-(trifluoromethyl) piperidine 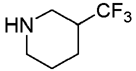 | 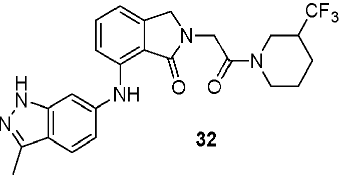 7-[(3-methyl-1H-indazol-6-yl)amino]-2-[2-oxo-2-[3-(trifluoromethyl)-1-piperidyl]ethyl]isoindolin-1-one | 3.85 min, m/z 472.2 [M+H] ⁺ | 12.37 (s, 1H), 8.86 (s, 1H), 7.64 (d, *J* = 8.5 Hz, 1H), 7.43 (t, *J =* 7.8 Hz, 1H), 7.31 (s, 1H), 7.27 (d, *J = 8.3* Hz, 1H), 6.94 (d, *J =* 7.8 Hz, 2H), 4.51 - 3.83 (m, 6H), 3.28 - 2.63 (m, 3H), 2.45 (s, 3H), 2.02 - 1.91 (m, 1H), 1.83 - 1.67 (m, 1H), 1.64 - 1.35 (m, 2H). |
| | | (Method 1) | |
| | **Example 32** | | |
| **Intermediate I-26** and (2S)-(fluoromethyl) pyrrolidine hydrochloride 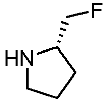 | 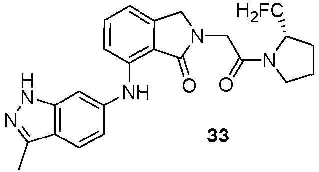 2-[2-[(2S)-(fluoromethyl)pyrrolidin-1-yl]-2-oxo-ethyl]-7-[(3-methyl-1H-indazol-6-yl)amino]isoindolin-1-one | 3.58 min, m/z 422.3 [M+H]⁺ | 12.45 (s, 1H), 8.93 (s, 1H), 7.72 (d, *J* = 8.5 Hz, 1H), 7.51 (t, *J* = 7.8 Hz, 1H), 7.39 (s, 1H), 7.34 (d, *J* = 8.3 Hz, 1H), 7.02 (d, *J* = 7.9 Hz, 2H), 4.64 - 4.17 (m, 7H), 3.62 (d, *J* = 5.5 Hz, 2H), 2.52 (s, 3H), 2.12 - 1.87 (m, 4H). |
| | | (Method 1) | |
| | **Example 33** | | |
| **Intermediate I-26** and 3-(trifluoromethyl) azetidine 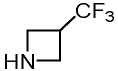 | 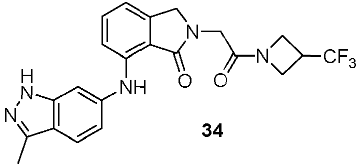 7-[(3-methyl-1 H-indazol-6-yl)amino]-2-[2-oxo-2-[3-(trifluoromethyl)azetidin-1-yl]ethyl]isoindolin-1-one | 3.60 min, m/z 444.1 [M+H]⁺ | 12.37 (s, 1H), 8.82 (s, 1H), 7.64 (d, *J* = 8.5 Hz, 1H), 7.43 (t, *J* = 7.8 Hz*,* 1H), 7.31 (s, 1H), 7.26 (d, *J* = 8.3 Hz, 1H), 6.94 (d, *J =* 6.7 Hz, 2H), 4.50 (t, *J* = 9.1 Hz, 1H), 4.45 (s, 2H), 4.32 - 4.28 (m, 1H), 4.23 (s, 2H), 4.16 (t, J = 9.8 Hz, 1H), 3.9 - 3.88 (m, 1H), 3.72 - 3.67 (m, 1H), 2.45 (s, 3H). |
| | | (Method 1) | |
| | **Example 34** | | |
| **Intermediate I-26** and 3,3-difluoroazetidine 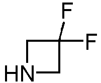 | 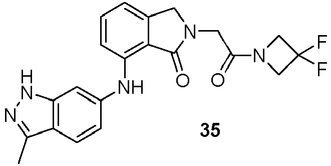 2-[2-(3,3-difluoroazetidin-1-yl)-2-oxo-ethyl]-7-[(3-methyl-1 H-indazol-6-yl)amino]isoindolin-1-one | 3.48 min, m/z 412.1 [M+H]⁺ | 12.38 (s, 1H), 8.82 (s, 1H), 7.64 (d, *J* = 8.5 Hz, 1H), 7.43 (t, *J* = 7.8 Hz, 1H), 7.31 (s, 1H), 7.26 (d, *J* = 8.2 Hz, 1H), 6.95 (d, *J =* 6.5 Hz, 2H), 4.74 (t, *J* = 12.5 Hz, 2H), 4.45 (s, 2H), 4.36 (t, *J* = 12.6 Hz, 2H), 4.30 (s, 2H), 2.45 (s, 3H). |
| | | (Method 1) | |
| | **Example 35** | | |
| **Intermediate I-26** and 2,2-difluoro cyclo propanamine 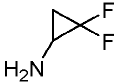 | 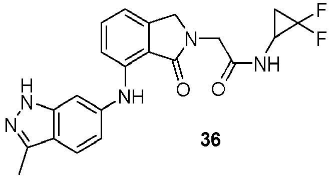 N-(2,2-difluorocyclopropyl)-2-[7-[(3-methyl-1H-indazol-6-yl)amino]-1-oxo-isoindolin-2-yl]acetamide | 3.45 min, m/z 412.1 [M+H]⁺ | 12.37 (s, 1H), 8.83 (s, 1H), 8.60 (s, 1H), 7.64 (d, *J =* 8.5 Hz, 1H), 7.44 (t, *J* = 7.8 Hz, 1H), 7.31 - 7.26F (m, 2H), 6.95 (d, *J =* 7.9 Hz, 2H), 4.47 (s, 2H), 4.20 (s, 2H), 2.45 (s, 3H), 2.03 - 1.85 (m, 1.5H), 1.62 - 1.47 (m, 1.5H). |
| | | (Method 1) | |
| | **Example 36** | | |
| **Intermediate I-26** and 4-[(dimethylamino)me thyl]-3-(trifluoromethyl) aniline 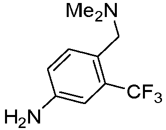 | 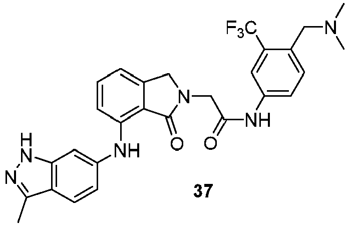 N-[4-[(dimethylamino)methyl]-3-(trifluoromethyl)phenyl]-2-[7-[(3-methyl-1H-indazol-6-yl)amino]-1-oxo-isoindolin-2-yl]acetamide | 2.74 min, m/z 537.2 [M+H]⁺ | 10.76 (s, 1H), 9.71 (s, 1H), 8.83 (s, 1H), 8.16 (d, *J* = 2.2 Hz, 1H), 7.96 (d, *J* = 8.4 Hz, 1H), 7.79 (d, *J* = 8.6 Hz, 1H), 7.64 (d, *J =* 8.5 Hz, 1H), 7.46 (t, *J* = 7.8 Hz, 1H), 7.32 (s, 1H), 7.29 (d, *J* = 8.2 Hz, 1H), 6.96 (t, *J* = 7.8 Hz, 2H), 4.56 (s, 2H), 4.44 (s, 2H), 4.41 (s, 2H), 2.80 (s, 6H), 2.45 (s, 3H). |
| | | (Method 1) | |
| | **Example 37** | | |
| **Intermediate I-26** and 2-(trifluoromethyl)mor pholine 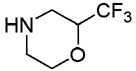 | 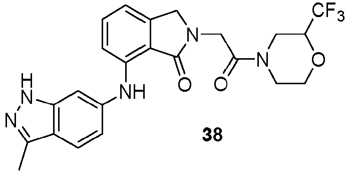 7-[(3-methyl-1H-indazol-6-yl)amino]-2-[2-oxo-2-[2-(trifluoromethyl)morpholin-4-yl]ethyl]isoindolin-1-one | 3.68 min, m/z 474.2 [M+H]⁺ | 12.37 (s, 1H), 8.85 (s, 1H), 7.64 (d, *J* = 8.5 Hz, 1H), 7.44 (t, *J* = 7.8 Hz, 1H), 7.31 (d, *J =* 1.8 Hz, 1H), 7.27 (d, *J* = 8.3 Hz, 1H), 6.95 (d, *J* = 7.9 Hz, 2H), 4.64 - 4.54 (m, 1H), 4.45 - 4.30 (m, 4H), 4.24 - 3.86 (m, 3H), 3.70 - 3.54 (m, 1H), 3.32 (s, 1H), 2.89 (t, *J* = 11.6 Hz, 1H), 2.45 (s, 3H). |
| | | (Method 1) | |
| | **Example 38** | | |
| **Intermediate I-26** and 3-[(dimethylamino)me thyl]-5-(trifluoromethyl) aniline 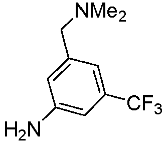 | 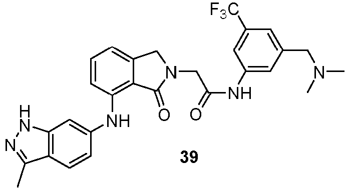 N-[3-[(dimethylamino)methyl]-5-(trifluoromethyl)phenyl]-2-[7-[(3-methyl-1H-indazol-6-yl)amino]-1-oxo-isoindolin-2-yl]acetamide | 2.80 min, m/z 537.3 [M+H]⁺ | 10.62 (s, 1H), 8.90 (s, 1H), 8.24 (s, 1H), 8.04 (s, 1H), 7.84 (s, 1H), 7.70 (d, *J* = 8.5 Hz, 1H), 7.51 (t, *J* = 7.8 Hz, 1H), 7.38 (s, 2H), 7.34 (d, *J* = 8.2 Hz, 1H), 7.05 - 6.99 (m, 2H), 4.62 (s, 2H), 4.46 (s, 2H), 3.52 (s, 2H), 2.51 (s, 3H), 2.22 (s, 6H). |
| | | (Method 1) | |
| | **Example 39** | | |
| **Intermediate I-26** and isoxazol-4-amine 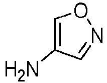 | 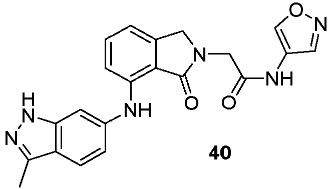 N-isoxazol-4-yl-2-[7-[(3-methyl-1H-indazol-6-yl)amino]-1-oxo-isoindolin-2-yl]acetamide | 3.42 min, m/z 402.4 [M+H]⁺ | 12.37 (s, 1H), 10.50 (s, 1H), 9.11 (s, 1H), 8.82 (s, 1H), 8.64 (s, 1H), 7.64 (d, *J* = 8.6 Hz, 1H), 7.45 (t, *J =* 7.9 Hz, 1H), 7.31 - 7.27 (m, 2H), 6.97 - 6.94 (m, 2H), 4.53 (s, 2H), 4.37 (s, 2H), 2.45 (s, 3H). |
| | | (Method 1) | |
| | **Example 40** | | |
| **Intermediate I-26** and (2S)-(difluoromethyl) pyrrolidine hydrochloride 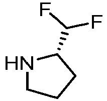 | 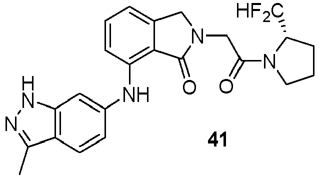 7-[(3-methyl-1H-indazol-6-yl)amino]-2-[2-oxo-2-[(2S)-(difluoromethyl)pyrrolidin-1-yl]ethyl]isoindolin-1-one | 3.70 min, m/z 440.2 [M+H]⁺ | 12.37 (s, 1H), 8.84 (s, 1H), 7.64 (d, J = 8.5 Hz, 1H), 7.44 (t, J = 7.8 Hz, 1H), 7.31 (s, 1H), 7.27 (d, J = 8.3 Hz, 1H), 6.97 - 6.92 (m, 2H), 6.20 (t, J = 56.7 Hz, 1H), 4.57 - 4.20 (m, 5H), 3.67 - 3.48 (m, 2H), 2.45 (s, 3H), 2.03 - 1.86 (m, 4H). |
| | | (Method 1) | |
| | **Example 41** | | |
| **Intermediate I-26** and (2S)-(trifluoromethyl) piperidine 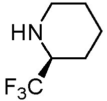 | 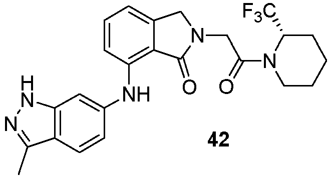 7-[(3-methyl-1H-indazol-6-yl)amino]-2-[2-oxo-2-[(2S)-(trifluoromethyl)-1-piperidyl]ethyl]isoindolin-1-one | 1.90 min, m/z 472.1 [M+H]⁺ | 12.37 (s, 1H), 8.83 (s, 1H), 7.64 (d, J = 8.6 Hz, 1H), 7.44 (t, J = 7.8 Hz, 1H), 7.31 (s, 1H), 7.27 (d, J = 8.3 Hz, 1H), 6.95 (d, J = 7.9 Hz, 2H), 5.18 - 4.88 (m, 1H), 4.66 - 4.33 (m, 5H), 3.94 (d, J = 14.1 Hz, 1H), 3.19 - 3.12 (m, 1H), 2.45 (s, 3H), 2.02 - 1.94 (m, 1H), 1.75 - 1.52 (m, 4H). |
| | | (Method 2) | |
| | **Example 42** | | |
| **Intermediate I-5** and 2,2,2-trifluoro-N-methyl-ethanamine hydrochloride 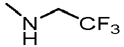 | 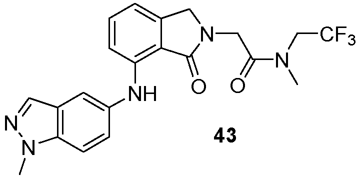 N-methyl-2-[7-[(1-methylindazol-5-yl)amino]-1-oxo-isoindolin-2-yl]-N-(2,2,2-trifluoroethyl)acetamide | 3.50 min, m/z 432.2 [M+H]⁺ | 8.61 (s, 1H), 7.97 (s, 1H), 7.64 (d, J = 6.6 Hz, 2H), 7.33 (m, 2H), 6.99 (d, J = 8.2 Hz, 1H), 6.86 (d, J = 7.3 Hz, 1H), 4.51 (d, J = 18.0 Hz, 2H), 4.43 (s, 2H), 4.20 (m, 2H), 4.04 (s, 3H), 3.07 (d, J = 90.2 Hz, 3H). |
| | | (Method 1) | |
| | **Example 43** | | |
| **Intermediate I-5** and (3S)-(trifluoromethyl)mor pholine 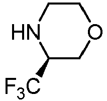 | 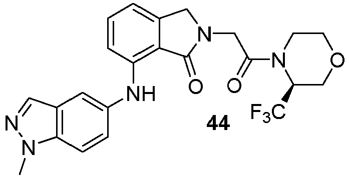 7-[(1-methylindazol-5-yl)amino]-2-[2-oxo-2-[(3S)-(trifluoromethyl)morpholin-4-yl]ethyl]isoindolin-1-one | 3.85 min, m/z 474.2 [M+H]⁺ | 8.60 (s, 1H), 7.97 (s, 1H), 7.65 (d, 2H), 7.42 - 7.26 (m, 2H), 7.00 (d, J = 8.3 Hz, 1H), 6.87 (d, J = 6.9 Hz, 1H), 5.01 - 4.87 (m, 1H), 4.72 - 4.64 (t, J = 16.0 Hz, 1H), 4.51 - 4.34 (m, 3H), 4.21 - 4.10 (m, 1H), 4.04 (s, 3H), 3.93 - 3.65 (m, 3H), 3.58 - 3.41 (m, 2H). |
| | | (Method 1) | |
| | **Example 44** | | |
| **Intermediate I-5** and (3R)-(trifluoromethyl)mor pholine 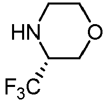 | 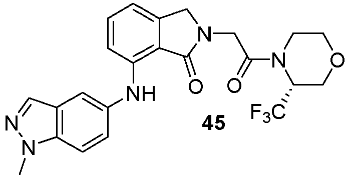 7-[(1-methylindazol-5-yl)amino]-2-[2-oxo-2-[(3R)-(trifluoromethyl)morpholin-4-yl]ethyl]isoindolin-1-one | 3.88 min, m/z 474.2 [M+H] ⁺ | 8.60 (s, 1H), 7.97 (s, 1H), 7.65 (s, 2H), 7.37 - 7.30 (m, 2H), 7.00 (d, J = 8.3 Hz, 1H), 6.87 (d, J = 7.1 Hz, 1H), 4.98 - 4.88 (m, 1H), 4.68 (t, J = 15.8 Hz, 1H), 4.48 - 4.34 (m, 3H), 4.20 - 4.10 (m, 1H), 4.04 (s, 3H), 3.93 - 3.66 (m, 3H), 3.58 - 3.44 (m, 2H). |
| | | (Method 1) | |
| | **Example 45** | | |
| **Intermediate I-26** and 2,2,2-trifluoro-N-methyl-ethanamine hydrochloride 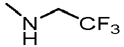 | 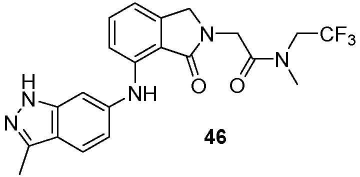 N-methyl-2-[7-[(3-methyl-1H-indazol-6-yl)amino]-1-oxo-isoindolin-2-yl]-N-(2,2,2-trifluoroethyl)acetamide | 3.73 min, m/z 454.1 [M+Na]⁺ | 12.37 (s, 1H), 8.83 (s, 1H), 7.64 (d, J = 8.5 Hz, 1H), 7.44 (t, J = 7.8 Hz, 1H), 7.31 (s, 1H), 7.27 (d, J = 8.2 Hz, 1H), 6.95 (d, J = 7.8 Hz, 2H), 4.53 - 4.39 (m, 5H), 4.19 (q, J = 9.7 Hz, 1H), 3.18 (s, 2H), 2.95 (s, 1H), 2.45 (s, 3H). |
| | | (Method 1) | |
| | **Example 46** | | |
| **Intermediate I-26** and (2R)-(trifluoromethyl)mor pholine hydrochloride 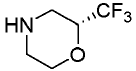 | 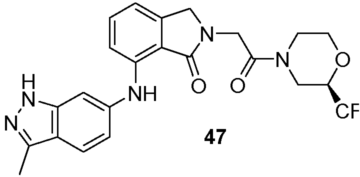 7-[(3-methyl-1H-indazol-6-yl)amino]-2-[2-oxo-2-[(2R)-(trifluoromethyl)morpholin-4-yl]ethyl]isoindolin-1-one | 3.68 min, m/z 474.2 [M+H]⁺ | 12.37 (s, 1H), 8.85 (s, 1H), 7.64 (d, J = 8.6 Hz, 1H), 7.44 (t, J = 7.8 Hz, 1H), 7.31 (d, J = 1.7 Hz, 1H), 7.27 (d, J = 8.2 Hz, 1H), 6.99 - 6.91 (m, 2H), 4.67 - 4.53 (m, 1H), 4.51 - 4.29 (m, 4H), 4.27 - 3.84 (m, 3H), 3.74 - 3.52 (m, 1H), 3.39 - 3.33 (m, 1H), 2.89 (t, J = 11.8 Hz, 1H), 2.45 (s, 3H). |
| | | (Method 1) | |
| | **Example 47** | | |
| **Intermediate I-26** and (2S)-(trifluoromethyl)mor pholine hydrochloride 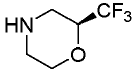 | 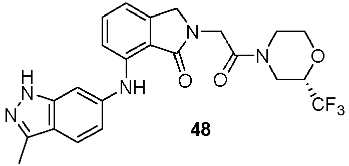 7-[(3-methyl-1H-indazol-6-yl)amino]-2-[2-oxo-2-[(2S)-(trifluoromethyl)morpholin-4-yl]ethyl]isoindolin-1-one | 3.75 min, m/z 474.1 [M+H]⁺ | 12.37 (s, 1H), 8.85 (s, 1H), 7.64 (d, J = 8.5 Hz, 1H), 7.44 (t, J = 7.8 Hz, 1H), 7.31 (d, J = 1.7 Hz, 1H), 7.27 (d, J = 8.2 Hz, 1H), 6.97 - 6.92 (m, 2H), 4.67 - 4.52 (m, 1H), 4.50 - 4.29 (m, 4H), 4.27 - 3.84 (m, 3H), 3.72 - 3.53 (m, 1H), 2.94 - 2.84 (m, 1H), 2.45 (s, 3H). |
| | | (Method 1) | |
| | **Example 48** | | |
| **Intermediate 1-5** and 1,1,1-trifluoro-2-methyl-propan-2-amine hydrochloride 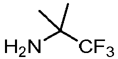 | 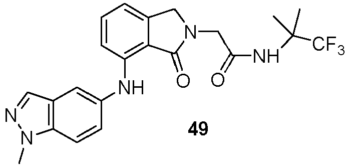 2-[7-[(1-methylindazol-5-yl)amino]-1-oxo-isoindolin-2-yl]-N-(2,2,2-trifluoro-1,1-dimethyl-ethyl)acetamide | 1.75 min, m/z 446.1 [M+H]⁺ | 8.61 (s, 1H), 8.21 (s, 1H), 7.97 (s, 1H), 7.65 (dd, J = 5.5, 3.4 Hz, 2H), 7.35 (t, J = 7.8 Hz, 1H), 7.31 (dd, J = 8.9, 2.0 Hz, 1H), 6.99 (d, J = 8.3 Hz, 1H), 6.85 (d, J = 7.4 Hz, 1H), 4.46 (s, 2H), 4.19 (s, 2H), 4.04 (s, 3H), 1.51 (s, 6H). |
| | | (Method 2) | |
| | **Example 49** | | |
| **Intermediate 1-5** and 2-(trifluoromethyl)aze tidine 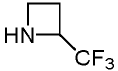 | 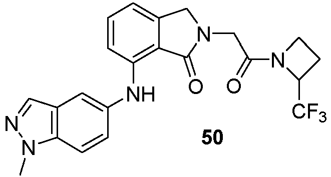 7-[(1-methylindazol-5-yl)amino]-2-[2-oxo-2-[2-(trifluoromethyl)azetidin-1-yl]ethyl]isoindolin-1-one | 1.80 min, m/z 444.0 [M+H]⁺ | 8.59 (s, 1H), 7.97 (d, J = 0.9 Hz, 1H), 7.65 (m, 2H), 7.35 (t, 1H), 7.29 - 7.32 (dd, J = 8.9, 2.0 Hz, 1H), 6.99 (d, J = 8.2 Hz, 1H), 6.86 (d, J = 7.4 Hz, 1H), 5.38 - 4.88 (m, 1H), 4.44 (s, 2H), 4.35 - 4.17 (m, 4H), 4.04 (s, 3H), 2.66 - 2.59 (m, 1H), 2.32 - 2.24 (m, 1H). |
| | | (Method 2) | |
| | **Example 50** | | |
| **Intermediate I-32** and trifluoroethylamine 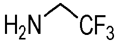 | 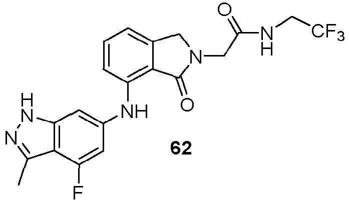 2-[7-[(4-fluoro-3-methyl-1H-indazol-6-yl)amino]-1-oxo-isoindolin-2-yl]-N-(2,2,2-trifluoroethyl)acetamide | 3.71 min, m/z 436.1 [M+H]⁺ | 12.60 (s, 1H), 8.84 - 8.79 (m, 2H), 7.47 (t, *J* = 7.8 Hz, 1H), 7.32 (d, *J =* 8.2 Hz, 1H), 7.12 (d, *J =* 1.5 Hz, 1H), 7.01 (d, *J* = 7.4 Hz, 1H), 6.76 (d, *J =* 12.2, 1.5 Hz, 1H), 4.49 (s, 2H), 4.25 (s, 2H), 3.96 - 3.92 (m, 2H), 3.30 (s, 3H). |
| | | (Method 1) | |
| | **Example 62** | | |
| **Intermediate I-32** and (2S)-Amino-1,1,1-trifluoropropane hydrochloride 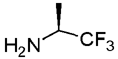 | 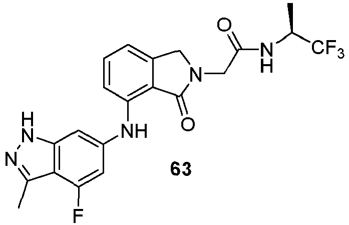 2-[7-[(4-fluoro-3-methyl-1H-indazol-6-yl)amino]-1-oxo-isoindolin-2-yl]-N-[(1S)-2,2,2-trifluoro-1-methyl-ethyl]acetamide | 3.85 min, m/z 450.1 [M+H]⁺ | 12.60 (s, 1H), 8.84 (s, 1H), 8.73 (d, J = 8.8 Hz, 1H), 7.46 (t, J = 7.8 Hz, 1H), 7.32 (d, J = 8.2 Hz, 1H),7.12 (d, J = 1.5 Hz, 1H), 7.00 (d, J = 7.4 Hz, 1H), 6.76 (dd, J = 12.2, 1.5 Hz, 1H), 4.67 - 4.56 (m, 1H), 4.48 (s, 2H), 4.29 - 4.18 (m, 2H), 2.52 (s, 3H), 1.27 (d, J = 7.1 Hz, 3H). |
| | | (Method 1) | |
| | **Example 63** | | |
| **Intermediate I-32** and (2S)-trifluoromethylpyrrol idine 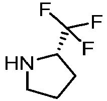 | 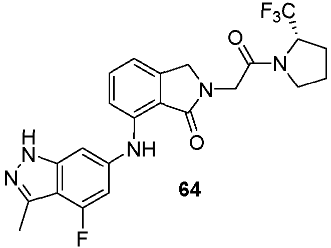 7-[(4-fluoro-3-methyl-1H-indazol-6-yl)amino]-2-[2-oxo-2-[(2S)-(trifluoromethyl)pyrrolidin-1-yl]ethyl]isoindolin-1-one | 4.01 min, m/z 476.2 [M+H]⁺ | 12.60 (s, 1H), 8.85 (s, 1H), 7.48 - 7.44 (t, *J* = 7.8 Hz, 1H), 7.34 - 7.31(d, *J* = 8.3 Hz, 1H), 7.12 (d, *J* = 1.5 Hz, 1H), 7.01 (d, *J* = 7.3 Hz, 1H), 6.77 - 6.74 (d, J = 12.1 Hz, 1H), 4.79 - 4.73 (m, 1H), 4.60 - 4.46 (m, 2H), 4.45 - 4.37(m, 2H), 3.69 - 3.62 (m, 2H), 2.51 (s, 3H), 2.10 - 2.03 (m, 2H), 2.03 - 1.99 (m, 2H). |
| | | (Method 1) | |
| | **Example 64** | | |
| **Intermediate I-32** and isoxazol-4-amine 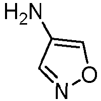 | 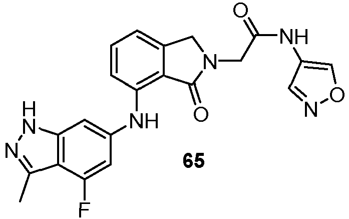 2-[7-[(4-fluoro-3-methyl-1 H-indazol-6-yl)amino]-1-oxo-isoindolin-2-yl]-N-isoxazol-4-yl-acetamide | 1.43 min, m/z 421.1 [M+H]⁺ | 10.92 (s, 1H), 8.90 (t, *J* = 6.3 Hz, 1H), 8.18 (s, 1H), 7.58 (d, *J* = 8.5 Hz, 1H), 7.53 (d, *J* = 1.9 Hz, 1H), 7.39 - 7.33 (m, 2H), 7.19 (dd, *J* = 8.5, 1.9 Hz, 1H), 6.87 (d, *J =* 8.3 Hz, 1H), 6.80 (d, *J* = 7.6 Hz, 1H), 6.51 (d, *J* = 7.3 Hz, 1H), 4.66 (s, 2H), 4.03 - 3.92 (m, 2H), 3.85 (s, 3H). |
| | | (Method 2) | |
| | **Example 65** | | |
| **Intermediate I-33** and (2S)-Amino-1,1,1-trifluoropropane hydrochloride 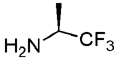 | 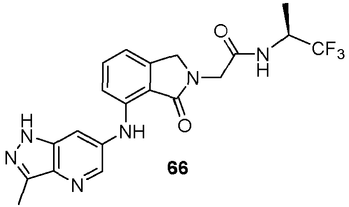 2-[7-[(3-methyl-1H-pyrazolo[4,3-b]pyridin-6-yl)amino]-1-oxo-isoindolin-2-yl]-N-[(1S)-2,2,2-trifluoro-1-methyl-ethyl]acetamide | 3.28 min, m/z 433.2 [M+H]⁺ | 12.60 (s, 1H), 8.91 (s, 1H), 8.74 (d, *J* = 8.8 Hz, 1H), 8.39 (d, *J* = 2.3 Hz, 1H), 7.75 (d, *J =* 2.3 Hz, 1H), 7.45 (t, *J* = 7.8 Hz, 1H), 7.25 (d, *J* = 8.2 Hz, 1H), 7.01 (d, *J* = 7.4 Hz, 1H), 4.65 - 4.59F (m, 1H), 4.50 (s, 2H), 4.24 (d, *J* = 3.9 Hz, 2H), 2.50 (s, 3H), 1.27 (d, *J* = 7.0 Hz, 3H). |
| | | (Method 1) | |
| | **Example 66** | | |
| **Intermediate I-33** and (2S)-trifluoromethylpyrrol idine 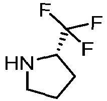 | 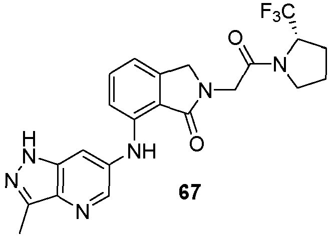 7-[(3-methyl-1H-pyrazolo[4,3-b]pyridin-6-yl)amino]-2-[2-oxo-2-[(2S)-(trifluoromethyl)pyrrolidin-1-yl]ethyl]isoindolin-1-one | 3.42 min, m/z 459.2 [M+H]⁺ | 12.60 (s, 1H), 8.92 (s, 1H), 8.39 (d, *J* = 2.3 Hz, 1H), 7.75 (d, *J* = 2.3 Hz, 1H), 7.46 (t, *J* = 7.8 Hz, 1H), 7.25 (d, *J* = 8.2 Hz, 1H), 7.02 (d, *J* = 7.2 Hz, 1H), 5.08 - 4.72 (m, 0H), 4.61 - 4.38 (m, 5H), 3.70 - 3.63 (m, 3H), 2.50 (s, 3H), 2.10 - 2.00 (m, 3H). |
| | | (Method 1) | |
| | **Example 67** | | |
| **Intermediate I-33** and (2R)-(trifluoromethyl)mor pholine hydrochloride 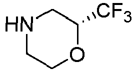 | 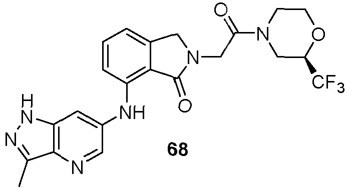 7-[(3-methyl-1H-pyrazolo[4,3-b]pyridin-6-yl)amino]-2-[2-oxo-2-[(2R)-(trifluoromethyl)morpholin-4-yl]ethyl]isoindolin-1-one | 3.18 min, m/z 475.3 [M+H]⁺ | 12.61 (s, 1H), 8.92 (s, 1H), 8.38 (d, *J* = 2.3 Hz, 1H), 7.75 (d, *J* = 2.2 Hz, 1H), 7.46 (t, *J =* 7.8 Hz, 1H), 7.25 (d, *J* = 8.2 Hz, 1H), 7.01 (d, *J* = 7.4 Hz, 1H), 4.64 - 4.55 (m, 1H), 4.47 - 4.43 (m, 3H), 4.33- 4.24 (m, 1H), 4.15 - 4.01 (m, 2H), 3.90 - 3.54 (m, 2H), 2.93 - 2.87 (m, 1H), 2.50 - 2.49 (m, 3H), 2.03 - 1.97 (m, 1H). |
| | | (Method 1) | |
| | **Example 68** | | |
| **Intermediate I-33** and (2S)-(trifluoromethyl)mor pholine hydrochloride 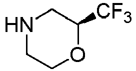 | 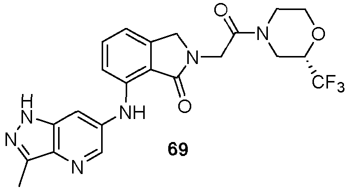 7-[(3-methyl-1H-pyrazolo[4,3-b]pyridin-6-yl)amino]-2-[2-oxo-2-[(2S)-(trifluoromethyl)morpholin-4-yl]ethyl]isoindolin-1-one | 3.18 min, m/z 475.3 [M+H]⁺ | 11.71 (s, 1H), 8.02 (s, 1H), 7.47 (d, *J* = 2.3 Hz, 1H), 6.84 (d, 1H), 6.57-6.53 (t, *J* = 7.8 Hz, 1H), 6.35-6.33 (d, *J* = 8.2 Hz, 1H), 6.11-6.09 (d, *J* = 7.4 Hz, 1H), 3.69 - 3.33 (m, 7H), 3.21 - 2.98 (m, 3H), 2.80 - 2.63 (m, 2H), 2.02-1.96 (t, 1H), 1.12 - 1.06 (m, 1H). |
| | | (Method 1) | |
| | **Example 69** | | |
| **Intermediate I-33** and (3S)-(trifluoromethyl)mor pholine hydrochloride 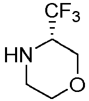 | 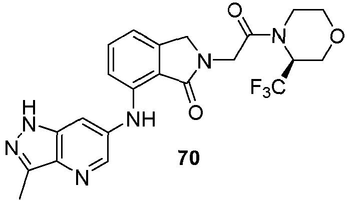 7-[(3-methyl-1H-pyrazolo[4,3-b]pyridin-6-yl)amino]-2-[2-oxo-2-[(3S)-(trifluoromethyl)morpholin-4-yl]ethyl]isoindolin-1-one | 3.30 min, m/z 475.3 [M+H]⁺ | (CD₃CN): 10.74 (s, 1H), 8.94 (s, 1H), 8.40 (s, 1H), 7.77 (s, 1H), 7.46-7.42 (m, 1H), 7.30-7.28 (m, 1H), 6.98-6.96 (m, 1H), 5.00-4.91 (m, 1H), 4.61-4.41 (m, 4H), 4.20-4.16 (m, 1H), 3.94-3.90 (m, 1H), 3.73-3.58 (m, 3H), 2.53 (s, 3H), 2.03-2.00 (m, 1H). |
| | | (Method 1) | |
| | **Example 70** | | |
| **Intermediate I-33** and (3R)-(trifluoromethyl)mor pholine hydrochloride 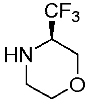 | 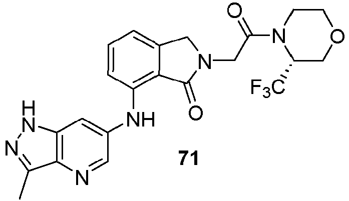 7-[(3-methyl-1H-pyrazolo[4,3-b]pyridin-6-yl)amino]-2-[2-oxo-2-[(3R)-(trifluoromethyl)morpholin-4-yl]ethyl]isoindolin-1-one | 3.28 min, m/z 475.3 [M+H]⁺ | (CD₃CN): 10.74 (s, 1H), 8.94 (s, 1H), 8.40 (s, 1H), 7.77 (s, 1H), 7.46-7.42 (m, 1H), 7.30-7.28 (m, 1H), 6.98-6.96 (m, 1H), 5.00-4.91 (m, 1H), 4.61-4.41 (m, 4H), 4.20-4.16 (m, 1H), 3.94-3.90 (m, 1H), 3.73-3.58 (m, 3H), 2.53 (s, 3H), 2.03-2.00 (m, 1H). |
| | | (Method 1) | |
| | **Example 71** | | |
| **Intermediate I-40** and (2S)-(trifluoromethyl)pyrr olidine 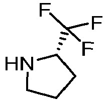 | 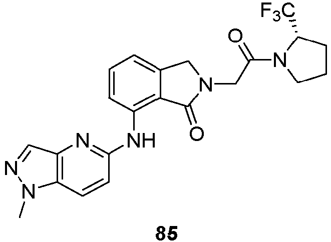 7-[(1-methylpyrazolo[4,3-b]pyridin-5-yl)amino]-2-[2-oxo-2-[ (2S)-(trifluoromethyl)pyrrolidin-1-yl]ethyl]isoindolin-1-one | 3.87 min, m/z 459.1 [M+H]⁺ | 9.88 (s, 1H), 8.77 (d, *J* = 8.3 Hz, 1H), 8.10 - 7.99 (m, 2H), 7.57 (t, *J =* 7.9 Hz, 1H), 7.08 (d, *J* = 7.4 Hz, 1H), 7.05 - 6.98 (m, 1H), 5.35 - 4.69 (m, 1H), 4.65 - 4.38 (m, 4H), 4.04 (s, 3H), 3.73 - 3.62 (m, 2H), 2.15 - 1.91 (m, 4H). |
| | | (Method 1) | |
| | **Example 85** | | |
| **Intermediate I-47** and 3-(trifluoromethyl)anili ne 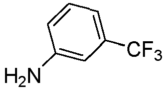 | 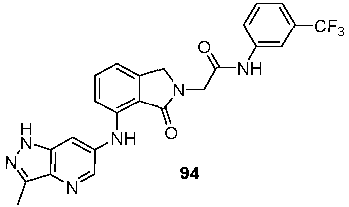 2-[7-[(3-methyl-1H-pyrazolo[4,3-b]pyridin-6-yl)amino]-1-oxo-isoindolin-2-yl]-N-[3-(trifluoromethyl)phenyl]acetamide | 1.73 min, m/z 481.2 [M+H]⁺ | 12.61 (s, 1H), 10.60 (s, 1H), 8.92 (s, 1H), 8.39 (d, *J* = 2.3 Hz, 1H), 8.10 (d, *J* = 2.0 Hz, 1H), 7.81 - 7.74 (m, 2H), 7.58 (t, *J* = 8.0 Hz, 1H), 7.49 - 7.41 (m, 2H), 7.27 (d, *J* = 8.2 Hz, 1H), 7.03 (d, *J* = 7.5 Hz, 1H), 4.58 (s, 2H), 4.43 (s, 2H), 1.23 (s, 3H). |
| | | (Method 2) | |
| | **Example 94** | | |
| **Intermediate I-12** and (2S)-(difluoromethyl)pyrr olidine 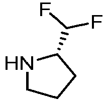 | 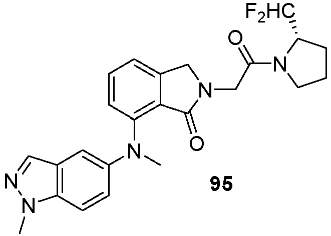 7-[methyl-(1-methylindazol-5-yl)amino]-2-[2-oxo-2-[(2S)-(difluoromethyl)pyrrolidin-1-yl]ethyl]isoindolin-1-one | 3.78 min, m/z 454.3 [M+H]⁺ | 7.85 (s, 1H), 7.48 (t, *J* = 7.6 Hz, 1H), 7.41 (d, *J* = 9.1 Hz, 1H), 7.27 (d, *J =* 7.4 Hz, 1H), 7.13 (s, 1H), 7.02 (d, *J* = 8.0 Hz, 1H), 6.93 (dd, *J* = 9.1, 2.3 Hz, 1H), 6.18 (t, *J* = 57.1 Hz, 1H), 4.46 - 4.45 (m, 2H), 4.34 (s, 2H), 4.27 - 4.21 (m, 1H), 3.97 (s, 3H), 3.55 - 3.52 (m, 2H), 3.35 (s, 3H), 2.01 - 1.89 (m, 4H). |
| | | (Method 1) | |
| | **Example 95** | | |
| **Intermediate I-49** and (2S)-(trifluoromethyl) pyrrolidine hydrochloride 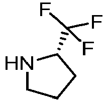 | 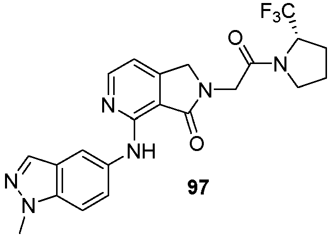 4-[(1-methylindazol-5-yl)amino]-2-[2-oxo-2-[(2S)-(trifluoromethyl)pyrrolidin-1-yl]ethyl]-1 H-pyrrolo[3,4-c]pyridin-3-one | 3.45 min, m/z 459.3 [M+H]⁺ | 9.00 (s, 1H), 8.40 (s, 1H), 8.33 (d, *J* = 5.2 Hz, 1H), 8.00 (s, 1H), 7.61 (d, *J* = 8.9 Hz, 1H), 7.50 (d, *J* = 10.4 Hz, 1H), 7.03 (d, *J* = 5.2 Hz, 1H), 4.77 - 4.73 (m, 1H), 4.52 - 4.40 (m, 4H), 4.03 (s, 3H), 3.70 - 3.66 (m, 2H), 2.05 - 2.00 (m, 4H). |
| | | (Method 1) | |
| | **Example 97** | | |

### Example 6, 2-[7-[(3-Methyl-1H-indazol-6-yl)amino]-1-oxo-isoindolin-2-yl]-N-(2,2,2-trifluoroethyl) acetamide

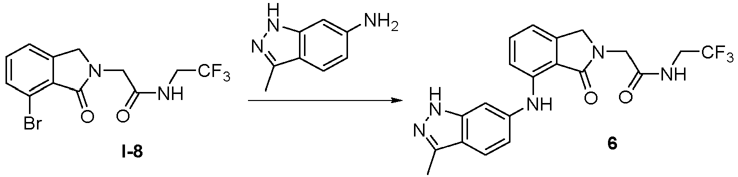

A mixture of 2-(7-bromo-1-oxo-isoindolin-2-yl)-N-(2,2,2-trifluoroethyl) acetamide (Intermediate 1-8, 50 mg, 0.14 mmol), 3-methyl-1H-indazol-6-amine (25 mg, 0.17 mmol), Pd₂(dba)₃ (13 mg, 0.014 mmol), t-BuBrettphos (14 mg, 0.028 mmol), K₂CO₃ (40 mg, 0.28 mmol) and AcOH (4.2 mg, 0.07 mmol) in t-BuOH (2 mL) was heated to 110 °C and stirred for 16 h. The mixture was concentrated under reduced pressure and the crude product was purified by prep-HPLC to give 2-[7-[(3-methyl-1H-indazol-6-yl)amino]-1-oxo-isoindolin-2-yl]-N-(2,2,2-trifluoroethyl) acetamide (Example 6, 6.5 mg, 11% yield) as a white solid.

UPLC-MS (ES⁺, Method 2): 1.58 min, m/z 418.0 [M+H]⁺.

¹H NMR (400 MHz, DMSO-d₆) δ (ppm) 12.36 (s, 1H), 8.83-8.79 (m, 2H), 7.64 (d, *J =* 8.0 Hz, 1H), 7.44 (t, *J* = 8.0 Hz, 1H), 7.31 (s, 1H), 7.27 (d, *J* = 8.0 Hz, 1H), 6.95 (d, *J* = 8.0 Hz, 2H), 4.48 (s, 2H), 4.25 (s, 2H), 3.99-3.90 (m, 2H), 2.45 (s, 3H).

### Example 78, 2-[7-[methyl-(3-methyl-1H-indazol-6-yl)amino]-1-oxo-isoindolin-2-yl]-N-(2,2,2-trifluoroethyl)acetamide

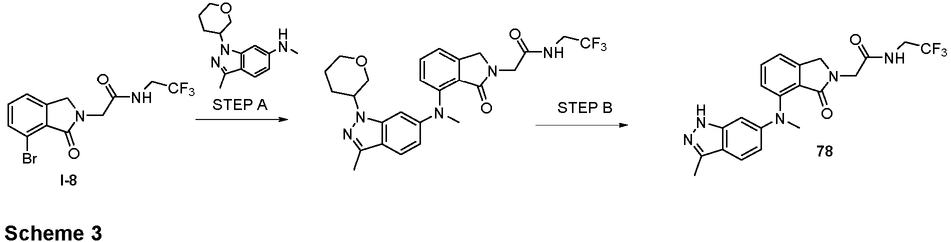

STEP A. 2-(7-bromo-1-oxo-isoindolin-2-yl)-N-(2,2,2-trifluoroethyl)acetamide (intermediate 1-8) was reacted with N,3-dimethyl-1-tetrahydropyran-2-yl-indazol-6-amine in an analogous fashion to Scheme 6 to afford 2-[7-[methyl-(3-methyl-1-tetrahydropyran-2-yl-indazol-6-yl)amino]-1-oxo-isoindolin-2-yl]-N-(2,2,2-trifluoroethyl)acetamide.

UPLC-MS (ES⁺, Method 1): 3.81 min, m/z 516.3 [M+H]⁺.

STEP B. 2-[7-[methyl-(3-methyl-1-tetrahydropyran-2-yl-indazol-6-yl)amino]-1-oxo-isoindolin-2-yl]-N-(2,2,2-trifluoroethyl)acetamide (200 mg, 0.39 mmol) was dissolved in DCM (6 mL) and TFA (2 mL) and stirred at room temperature for 30 mins. The solvents were removed *in vacuo* to leave a residue that was purified by preparative-HPLC to afford 2-[7-[methyl-(3-methyl-1H-indazol-6-yl)amino]-1-oxo-isoindolin-2-yl]-N-(2,2,2-trifluoroethyl)acetamide (21 mg, 13% yield) as a yellow solid.

UPLC-MS (ES⁺, Method 1): 3.35 min, m/z 432.2 [M+H]⁺.

¹H NMR (400 MHz, DMSO-d₆) δ (ppm) 12.12 (s, 1H), 8.79 (t, *J* = 6.4 Hz, 1H), 7.58 (t, *J* = 7.7 Hz, 1H), 7.43 (d, *J* = 7.4 Hz, 1H), 7.38 (d, *J* = 8.8 Hz, 1H), 7.21 (d, *J* = 7.9 Hz, 1H), 6.65 (d, *J* = 2.0 Hz, 1H), 6.50 (dd, *J* = 8.8, 2.0 Hz, 1H), 4.53 (s, 2H), 4.22 (s, 2H), 3.99 - 3.89 (m, 2H), 3.33 (s, 3H), 2.40 (s, 3H).

Examples synthesised following the same procedure as example 6 replacing 3-methyl-1H-indazol-6-amine and I-8 with the appropriate building blocks are described in table 9. In cases where a THP-protected building block was coupled with intermediate I-8 or analogues (as indicated in the table), a final THP deprotection step was required to afford the corresponding example. THP deprotection was carried out under standard conditions as described for example 78 in Scheme

**Table 9**

| Building Block | Structure/Name | LCMS | ¹H NMR (400 MHz, DMSO-d₆) δ (ppm) |
|---|---|---|---|
| **Intermediate I-8** and 1-methylpyrazolo[3,4-b]pyridine-5-amine 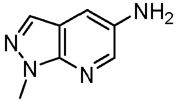 | 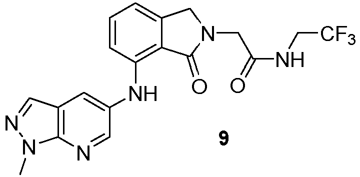 2-[7-[(1-methylpyrazolo[3,4-b]pyridin-5-yl)amino]-1-oxo-isoindolin-2-yl]-N-(2,2,2-trifluoroethyl)acetamide | 1.50 min, m/z 419.1 [M+H]⁺. | 8.80 (t, *J* = 6.3 Hz, 1H), 8.62 (s, 1H), 8.52 (d, *J =* 2.4 Hz, 1H), 8.16 (d, *J* = 2.4 Hz, 1H), 8.08 (s, 1H), 7.36 (t, *J* = 7.9 Hz, 1H), 6.91 (d, *J* = 7.8 Hz, 2H), 4.48 (s, 2H), 4.25 (s, 2H), 4.06 (s, 3H), 3.99-3.90 (m, 2H). |
| | | (Method 2) | |
| | **Example 9** | | |
| **Intermediate I-8** and 7-methyl-1 H-indazol-5-amine 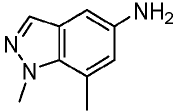 | 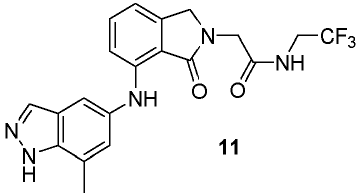 2-[7-[(7-methyl-1H-indazol-5-yl)amino]-1-oxo-isoindolin-2-yl]-N-(2,2,2-trifluoroethyl)acetamide | 3.56 min, m/z 418.2 [M+H]⁺ | 13.14 (s, 1H), 8.82 (t, J=6.1 Hz, 1H), 8.57 (s, 1H), 8.01 (s, 1H), 7.49 (s, 1H), 7.37 (t, J=7.8 Hz, 1H), 7.06 (s, 1H), 7.02 (d, J=8.4 Hz, 1H), 6.87 (d, J=7.2 Hz, 1H), 4.48 (s, 2H), 4.26 (s, 2H), 3.97 (dq, J=6.6, 9.6 Hz, 2H) (CH₃ signal under solvent peak) |
| | | (Method 1) | |
| | **Example 11** | | |
| **Intermediate I-8** and 1H-indazol-5-amine 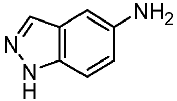 | 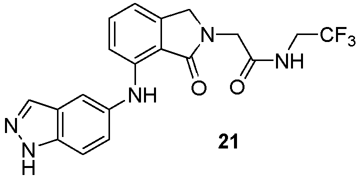 2-[7-(1H-indazol-5-ylamino)-1-oxo-isoindolin-2-yl]-N-(2,2,2-trifluoroethyl)acetamide | 1.32 min, m/z 404.1 [M+H]⁺ | 13.04 (s, 1H), 8.81 (t, *J =* 6.4 Hz, 1H), 8.58 (s, 1H), 8.01 (s, 1H), 7.65 (s, 1H), 7.56 (d, *J* = 8.8 Hz, 1H), 7.35 (t, *J* = 7.8 Hz, 1H), 7.26 (dd, *J* = 8.8, 2.0 Hz, 1H), 6.99 (d, *J* = 8.3 Hz, 1H), 6.86 (d, *J* = 7.4 Hz, 1H), 4.46 (s, 2H), 4.24 (s, 2H), 3.98-3.90 (m, 2H). |
| | | (Method 2) | |
| | **Example 21** | | |
| **Intermediate I-8** and 1-tetrahydropyran-2-ylpyrazolo[3,4-b]pyridin-5-amine 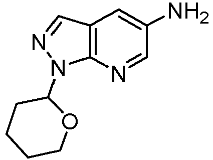 | 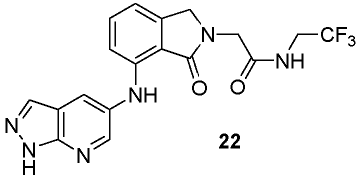 2-[1-oxo-7-(1H-pyrazolo[3,4-b]pyridin-5-ylamino)isoindolin-2-yl]-N-(2,2,2-trifluoroethyl)acetamide | 3.21 min, m/z 405.2 [M+H]⁺ | 13.63 (s, 1H), 8.82 (t, *J* = 6.4 Hz, 1H), 8.60 (s, 1H), 8.47 (d, *J =* 2.4 Hz, 1H), 8.15 (d, *J* = 2.4 Hz, 1H), 8.09 (s, 1H), 7.35 (t, *J* = 7.8 Hz, 1H), 6.91 (s, 1H), 6.89 (s, 1H), 4.48 (s, 2H), 4.25 (s, 2H), 4.00 - 3.89 (m, 2H). |
| | | (Method 1) | |
| | **Example 22** | | |
| **Intermediate I-8** and 7-chloro-1-methyl-indazol-5-amine 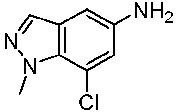 | 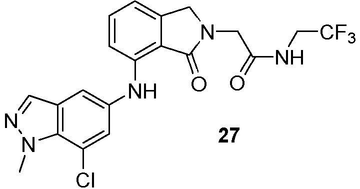 2-[7-[(7-chloro-1-methyl-indazol-5-yl)amino]-1-oxo-isoindolin-2-yl]-N-(2,2,2-trifluoroethyl)acetamide | 4.06 min, m/z 452.1 [M+H]⁺ | 8.84 (t, J=6.4 Hz, 1H), 8.65 (s, 1H), 8.07 (s, 1H), 7.68 (s, 1H), 7.44 - 7.38 (m, 2H), 7.08 (d, J=8.2 Hz, 1H), 6.94 (d, J=7.4 Hz, 1H), 4.49 (s, 2H), 4.32 (s, 3H), 4.27 (s, 2H), 3.96 (dq, J=6.4, 9.6 Hz, 2H). |
| | | (Method 1) | |
| | **Example 27** | | |
| **Intermediate I-8** and 3-methyl-1H-pyrazolo[4,3-b]pyridin-6-amine 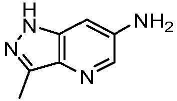 | 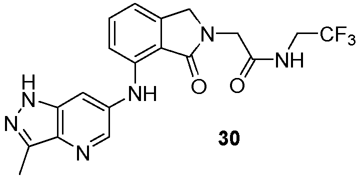 2-[7-[(3-methyl-1H-pyrazolo[4,3-b]pyridin-6-yl)amino]-1-oxo-isoindolin-2-yl]-N-(2,2,2-trifluoroethyl)acetamide | 3.15 min, m/z 419.1 [M+H]⁺ | 12.62 (s, 1H), 8.90 (s, 1H), 8.83 (t, *J* = 6.4 Hz, 1H), 8.39 (d, *J* = 2.2 Hz, 1H), 7.75 (d, *J* = 2.2 Hz, 1H), 7.45 (t, *J* = 7.8 Hz, 1H), 7.25 (d, *J* = 8.2 Hz, 1H), 7.01 (d, *J* = 7.4 Hz, 1H), 4.50 (s, 2H), 4.26 (s, 2H), 3.99- 3.90 (m, 2H), 2.52 (s, 3H). |
| | | (Method 1) | |
| | **Example 30** | | |
| **Intermediate I-8** and 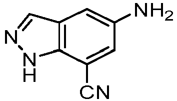 5-amino-1H-indazole-7-carbonitrile | 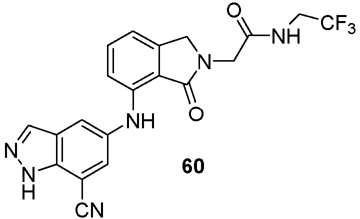 2-[7-[(7-cyano-1H-indazol-5-yl)amino]-1-oxo-isoindolin-2-yl]-N-(2,2,2-trifluoroethyl)acetamide | 1.45 min, m/z 429.1 [M+H]⁺ | 13.94 (s, 1H), 8.81 (t, *J* = 6.3 Hz, 1H), 8.65 (s, 1H), 8.23 (s, 1H), 8.07 (s, 1H), 7.89 (s, 1H), 7.38 (t, *J* = 7.8 Hz, 1H), 7.02 (d, *J* = 8.3 Hz, 1H), 6.93 (d, *J* = 7.4 Hz, 1H), 4.48 (s, 2H), 4.25 (s, 2H), 3.97-3.92 (m, 2H). |
| | | (Method 1) | |
| | **Example 60** | | |
| **Intermediate I-8** and 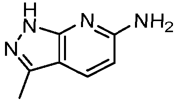 3-methyl-1H-pyrazolo[3,4-b]pyridin-6-amine | 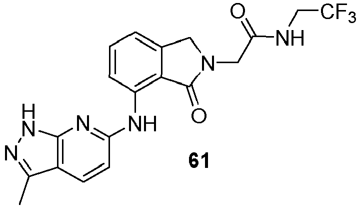 2-[7-[(3-methyl-1H-pyrazolo[3,4-b]pyridin-6-yl)amino]-1-oxo-isoindolin-2-yl]-N-(2,2,2-trifluoroethyl)acetamide | 3.45 min, m/z 419.2 [M+H]⁺ | 12.79 (s, 1H), 9.97 (s, 1H), 8.84 - 8.76 (m, 2H), 7.99 (d, *J* = 8.5 Hz, 1H), 7.57 (t, *J* = 7.9 Hz, 1H), 7.12 (d, *J* = 7.4 Hz, 1H), 6.66 (d, *J* = 8.6 Hz, 1H), 4.51 (s, 2H), 4.28 (s, 2H), 3.99 - 3.90 (m, 2H), 2.41 (s, 3H). |
| | | (Method 1) | |
| | **Example 61** | | |
| **Intermediate I-43** and 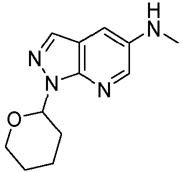 N-methyl-1-tetrahydropyran-2-yl-pyrazolo[3,4-b]pyridin-5-amine | 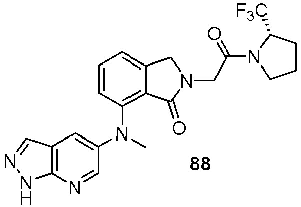 7-[methyl(1H-pyrazolo[3,4-b]pyridin-5-yl)amino]-2-[2-oxo-2-[ (2S)-(trifluoromethyl)pyrrolidin-1-yl]ethyl]isoindolin-1-one | 3.38 min, m/z 459.3 [M+H]⁺ | 13.36 (s, 1H), 8.15 - 8.10 (m, 1H), 7.94 (s, 1H), 7.59 - 7.53 (m, 2H), 7.32 (d, J = 7.5 Hz, 1H), 7.15 (d, J = 8.0 Hz, 1H), 5.02 - 4.65 (m, 1H), 4.55 - 4.19 (m, 4H), 3.72 - 3.48 (m, 2H), 3.36 (s, 3H), 2.17 - 1.85 (m, 4H). |
| | | (Method 1) | |
| | **Example 88** | | |
| **Intermediate I-44** and 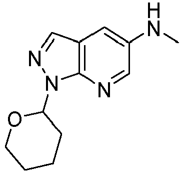 N-methyl-1-tetrahydropyran-2-yl-pyrazolo[3,4-b]pyridin-5-amine | 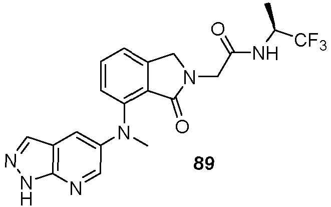 2-[7-[methyl(1H-pyrazolo[3,4-b]pyridin-5-yl)amino]-1-oxo-isoindolin-2-yl]-N-[(1S)-2,2,2-trifluoro-1-methyl-ethyl]acetamide | 1.23 min, m/z 433.1 [M+H]⁺ | 13.36 (s, 1H), 8.64 (d, *J* = 8.8 Hz, 1H), 8.12 (d, *J* = 2.6 Hz, 1H), 7.95 (s, 1H), 7.58 - 7.51 (m, 2H), 7.32 (d, *J* = 7.5 Hz, 1H), 7.12 (d, *J* = 8.0 Hz, 1H), 4.64 - 4.51 (m, 1H), 4.47 (s, 2H), 4.20 - 4.05 (m, 2H), 3.36 (s, 3H), 1.23 (d, *J =* 6.9 Hz, 3H). |
| | | (Method 2) | |
| | **Example 89** | | |
| **Intermediate I-8** and 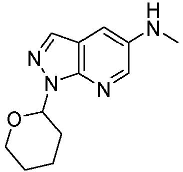 N-methyl-1-tetrahydropyran-2-yl-pyrazolo[3,4-b]pyridin-5-amine | 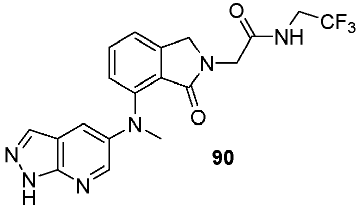 2-[7-[methyl(1 H-pyrazolo[3,4-b]pyridin-5-yl)amino]-1-oxo-isoindolin-2-yl]-N-(2,2,2-trifluoroethyl)acetamide | 3.15 min, m/z 419.2 [M+H]⁺ | 3.37 (s, 1H), 8.73 (t, *J* = 6.3 Hz, 1H), 8.12 (d, *J* = 2.6 Hz, 1H), 7.95 (s, 1H), 7.60 - 7.51 (m, 2H), 7.32 (d, *J* = 7.4 Hz, 1H), 7.12 (d, *J* = 8.0 Hz, 1H), 4.47 (s, 2H), 4.14 (s, 2H), 3.96 - 3.84 (m, 2H), 3.36 (s, 3H). |
| | | (Method 1) | |
| | **Example 90** | | |
| **Intermediate I-43** and 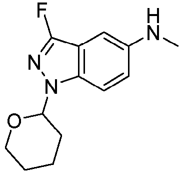 3-fluoro-N-methyl-1-tetrahydropyran-2-yl-pyrazolo[3,4-b]pyridin-5-amine | 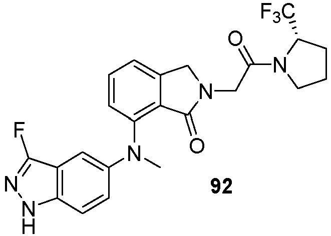 7-[(3-fluoro-1H-indazol-5-yl)-methyl-amino]-2-[2-oxo-2-[(2S)-(trifluoromethyl)pyrrolidin-1-yl]ethyl]isoindolin-1-one | 3.88 min, m/z 476.2 [M+H]⁺ | 12.25 (s, 1H), 7.52 (t, *J* = 7.7 Hz, 1H), 7.29 (d, *J* = 32.2, 8.3 Hz, 2H), 7.08 (d, *J* = 8.0 Hz, 1H), 7.01 - 6.88 (m, 2H), 5.04 - 4.67 (m, 1H), 4.57 - 4.27 (m, 4H), 3.71 - 3.54 (m, 2H), 3.32 (s, 3H), 2.10 - 1.88 (m, 4H). |
| | | (Method 1) | |
| | **Example 92** | | |
| **Intermediate I-8** and 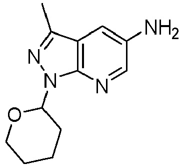 3-methyl-1-tetrahydropyran-2-yl-pyrazolo[3,4-b]pyridin-5-amine | 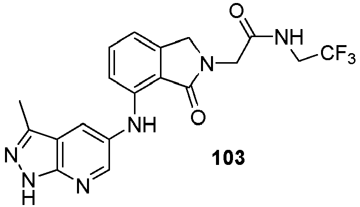 2-[7-[(3-methyl-1H-pyrazolo[3,4-b]pyridin-5-yl)amino]-1-oxo-isoindolin-2-yl]-N-(2,2,2-trifluoroethyl)acetamide | 3.31 min, m/z 419.3 [M+H]⁺ | 13.19 (s, 1H), 8.85 (t, *J* = 6.4 Hz, 1H), 8.56 (s, 1H), 8.43 (d, *J =* 2.4 Hz, 1H), 8.11 (d, *J* = 2.4 Hz, 1H), 7.35 (t, *J =* 7.8 Hz, 1H), 6.88 (t, J = 8.2 Hz, 2H), 4.48 (s, 2H), 4.26 (s, 2H), 4.00 - 3.89 (m, 2H) - *Methyl (3H) under solvent signal (2.5ppm)* |
| | | (Method 1) | |
| | **Example 103** | | |
| **Intermediate I-44** and 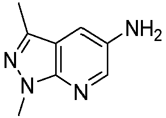 3-methyl-1-methyl-pyrazolo[3,4-b]pyridin-5-amine | 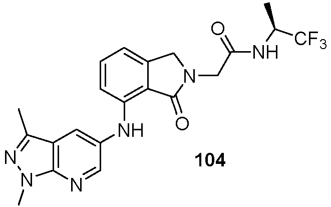 2-[7-[(1,3-dimethylpyrazolo[3,4-b]pyridin-5-yl)amino]-1-oxo-isoindolin-2-yl]-N-[(1S)-2,2,2-trifluoro-1-methyl-ethyl]acetamide | 1.59 min, m/z 447.2 [M+H]⁺ | 8.80 (s, 1H), 8.58 (s, 1H), 8.48 (s, 1H), 8.14 (s, 1H), 7.37 - 7.33 (m, 1H), 6.90 - 6.86 (m, 2H), 4.65 - 4.58 (m, 1H), 4.56 (s, 2H), 4.25 (s, 2H), 3.98 (s, 3H), 2.48 (s, 3H), 1.27 (s, 3H). |
| | | (Method 2) | |
| | **Example 104** | | |

### Example 10, N-[3-(1-hydroxy-1-methyl-ethyl)phenyl]-2-[7-[(1-methylindazol-5-yl)amino]-1-oxo-isoindolin-2-yl]acetamide

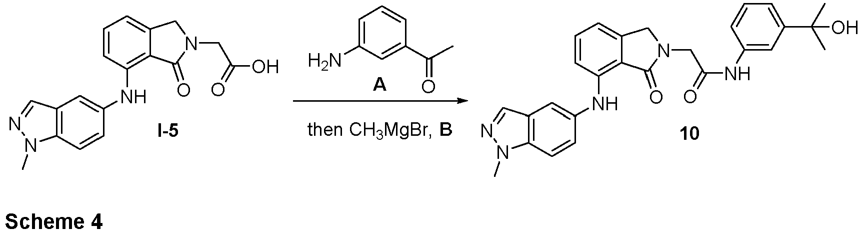

STEP A. Intermediate N-(3-acetylphenyl)-2-[7-[(1-methylindazol-5-yl)amino]-1-oxo-isoindolin-2-yl]acetamide was synthesised following the same procedure as for Example 1, using 3-aminoacetophenone as the coupling partner with Intermediate 1-5. The compound was obtained as a white solid (40 mg, 0.09 mmol, 37% yield from 80 mg of Intermediate 1-5)

UPLC-MS (ES⁺, Method 1): 3.82 min, m/z 454.2 [M+H]⁺.

¹H NMR (400 MHz, DMSO-d₆) δ (ppm) 10.45 (s, 1H), 8.64 (s, 1H), 8.21 (s, 1H), 7.99 (s, 1H), 7.87 (d, J=8.0 Hz, 1H), 7.73 - 7.65 (m, 3H), 7.51 (t, J=7.9 Hz, 1H), 7.39 (t, J=7.8 Hz, 1H), 7.34 (dd, J=1.8, 8.9 Hz, 1H), 7.03 (d, J=8.3 Hz, 1H), 6.91 (d, J=7.4 Hz, 1H), 4.57 (s, 2H), 4.42 (s, 2H), 4.06 (s, 3H), 2.58 (s, 3H).

STEP B. N-[3-(1-Hydroxy-1-methyl-ethyl)phenyl]-2-[7-[(1-methylindazol-5-yl)amino]-1-oxo-isoindolin-2-yl]acetamide (example 10). A solution of N-(3-acetylphenyl)-2-[7-[(1-methylindazol-5-yl)amino]-1-oxo-isoindolin-2-yl]acetamide (40 mg, 0.09 mmol) in THF (1 mL) was cooled to 0 °C and methylmagnesium bromide (1 M in THF, 3.5 mmol, 3.5 mL) was added dropwise. The mixture was stirred at 0 °C for 2 h. Saturated NH₄Cl (5 mL) and EtOAc (5 mL) were added, the layers were separated and the aqueous layer was extracted with EtOAc (3 x 5mL). The organic layers were combined, dried over Na₂SO₄ and concentrated in vacuo. Purification by prep HPLC afforded N-[3-(1-hydroxy-1-methyl-ethyl)phenyl]-2-[7-[(1-methylindazol-5-yl)amino]-1-oxo-isoindolin-2-yl]acetamide (5 mg, 0.1 mmol, 13% yield) as an off-white solid.

UPLC-MS (ES⁺, Method 1): 3.69 min, m/z 452.3 [M-H₂O+H]⁺.

¹H NMR (400 MHz, DMSO-d₆) δ (ppm) 10.18 (s, 1H), 8.65 (s, 1H), 8.00 (s, 1H), 7.72 - 7.65 (m, 3H), 7.50 (d, J=8.2 Hz, 1H), 7.39 (t, J=7.6 Hz, 1H), 7.34 (d, J=10.2 Hz, 1H), 7.25 (t, J=7.9 Hz, 1H), 7.17 (d, J=7.8 Hz, 1H), 7.03 (d, J=8.3 Hz, 1H), 6.90 (d, J=7.4 Hz, 1H), 5.01 (s, 1H), 4.56 (s, 2H), 4.38 (s, 2H), 4.07 (s, 3H), 1.43 (s, 6H).

### Example 25, 2-[7-[(3-methyl-1H-indazol-6-yl)amino]-1-oxo-isoindolin-2-yl]-N-[3-(trifluoromethyl) phenyl]acetamide

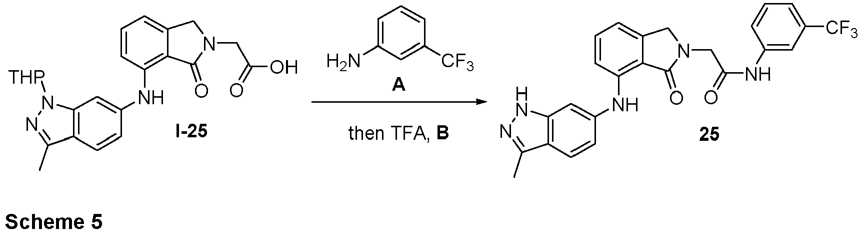

STEP A. Intermediate 2-[7-[(3-methyl-1-tetrahydropyran-2-yl-indazol-6-yl)amino]-1-oxo-isoindolin-2-yl]-N-[3-(trifluoromethyl)phenyl]acetamide was synthesised following the same procedure as for Example 1, using 3-(trifluoromethyl)aniline as the coupling partner with Intermediate 1-25. The compound was obtained as an off-white solid (60 mg, 0.1 mmol, 24% yield from 150 mg of Intermediate 1-25).

UPLC-MS (ES⁺, Method 1): 4.52 min, m/z 564.2 [M+H]⁺.

¹H NMR (400 MHz, DMSO-d₆) δ (ppm) 10.58 (s, 1H), 8.95 (s, 1H), 8.13 (s, 1H), 7.81 (d, J=8.3 Hz, 1H), 7.68 (d, J=8.7 Hz, 1H), 7.60 (t, J=7.9 Hz, 1H), 7.51 - 7.48 (m, 2H), 7.45 (d, J=7.6 Hz, 1H), 7.33 (d, J=7.9 Hz, 1H), 7.10 (d, J=7.2 Hz, 1H), 7.01 (d, J=7.1 Hz, 1H), 5.75 - 5.72 (m, 1H), 4.59 (s, 2H), 4.44 (s, 2H), 3.90 - 3.87 (m, 1H), 3.76 - 3.69 (m, 1H), 2.47 (s, 3H), 2.45 - 2.34 (m, 2H), 1.96 - 1.92 (m, 1H), 1.80 - 1.70 (m, 1H), 1.59 - 1.53 (m, 2H).

STEP B. 2-[7-[(3-methyl-1H-indazol-6-yl)amino]-1-oxo-isoindolin-2-yl]-N-[3-(trifluoromethyl)phenyl]acetamide (Example 25). To a solution of 2-[7-[(3-methyl-1-tetrahydropyran-2-yl-indazol-6-yl)amino]-1-oxo-isoindolin-2-yl]-N-[3-(trifluoromethyl)phenyl]acetamide (60 mg, 0.1 mmol) in DCM (3 mL) was added TFA (2.5 mL) dropwise. The mixture was stirred at room temperature for 2 h, then it was concentrated *in vacuo* and purified by prep-HPLC to give 2-[7-[(3-methyl-1H-indazol-6-yl)amino]-1-oxo-isoindolin-2-yl]-N-[3-(trifluoromethyl)phenyl]acetamide (Example 25, 13 mg, 0.03 mmol, 25% yield) as a yellow solid.

UPLC-MS (ES⁺, Method 1): 4.05 min, m/z 480.2 [M+H]⁺.

¹H NMR (400 MHz, DMSO-d₆) δ (ppm) 12.37 (s, 1H), 10.55 (s, 1H), 8.84 (s, 1H), 8.10 (s, 1H), 7.78 (d, *J* = 8.0 Hz, 1H), 7.64 (d, *J* = 8.4 Hz, 1H), 7.57 (t, 1H), 7.46 (d, *J* = 8.6 Hz, 1H), 7.43 (s, 1H), 7.32 (s, 1H), 7.29 (s, 1H), 6.97 (s, 1H), 4.56 (s, 2H), 4.41 (s, 2H), 2.45 (s, 3H).

### Example 51, 7-[(1-methylpyrazolo[3,4-b]pyridin-5-yl)amino]-2-[2-oxo-2-[(2S)-2-(trifluoromethyl)pyrrolidin-1-yl]ethyl]isoindolin-1-one

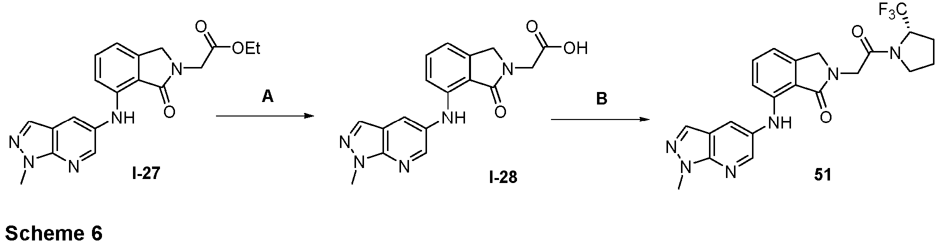

STEP A. Intermediate 1-28, 2-[7-[(1-methylpyrazolo[3,4-b]pyridin-5-yl)amino]-1-oxo-isoindolin-2-yl]acetic acid. A mixture of ethyl 2-(7-((1-methyl-1H-pyrazolo[3,4-b]pyridin-5-yl)amino)-1-oxoisoindolin-2-yl)acetate (I-27) (1.12 g, 3.067 mmol, 1.0 eq) and LiOH.H₂O (386 mg, 9.2 mmol) in H2O/THF/MeOH (1:1:1, 18 mL) was stirred at room temperature for 2 h. The reaction was diluted with water (200 mL) and was washed with DCM (100 mL x 3). The water layer was adjusted to pH~3 by slow addition of 1M HCl. The mixture was washed with DCM (200 mL x 3) and was concentrated to give 1-28, 2-[7-[(1-methylpyrazolo[3,4-b]pyridin-5-yl)amino]-1-oxo-isoindolin-2-yl]acetic acid as a yellow solid (960 mg, 93% yield).

UPLC-MS (ES⁺, Method 2): 0.82 min, m/z 338.2 [M+H]⁺.

¹H NMR (400 MHz, DMSO-d₆) δ (ppm) 8.62 (s, 1H), 8.52 (d, *J* = 5.9Hz, 1H), 8.17 (d, *J* = 5.9Hz, 1H), 8.08 (s, 1H), 7.38 - 7.34 (m, 1H), 6.91 (d, *J* = 6.9Hz, 2H), 4.27 (s, 2H), 4.06 (s, 3H).

STEP B. 7-[(1-methylpyrazolo[3,4-b]pyridin-5-yl)amino]-2-[2-oxo-2-[(2S)-2-(trifluoromethyl)pyrrolidin-1-yl]ethyl]isoindolin-1-one (Example 51). A mixture of 2-(7-((1-methyl-1H-pyrazolo[3,4-b]pyridin-5-yl)amino)-1-oxoisoindolin-2-yl)acetic acid (I-28) (237 mg, 0.70 mmol), (S)-2-(trifluoromethyl)pyrrolidine (195.48 mg, 1.40 mmol), HATU (400.7 mg, 1.05 mmol) and DIEA (272.39 mg, 2.10 mmol) in DMF (8 mL) was stirred at room temperature overnight. The mixture was diluted with 40 mL of water and was extracted with ethyl acetate (10 mL x 5). The organic solution was washed with brine (25 mL x 3), dried over Na₂SO₄ and concentrated under vacuum. The crude was purified by prep-TLC (DCM/MeOH=20/1, v/v). Methanol (2 mL) was added to the product and filtered, and the solid was concentrated and dried to give 7-[(1-methylpyrazolo[3,4-b]pyridin-5-yl)amino]-2-[2-oxo-2-[(2S)-2-(trifluoromethyl)pyrrolidin-1-yl]ethyl]isoindolin-1-one (Example 51) (140 mg, 43.4%.yield) as a yellow solid.

UPLC-MS (ES⁺, Method 2): 1.70 min, m/z, 459.15 [M+H].

¹H NMR (400 MHz, DMSO-d6) δ (ppm) 8.63 (s, 1H), 8.53 (d, J = 2.4 Hz, 1H), 8.17 (d, J = 2.4 Hz, 1H), 8.08 (s, 1H), 7.36 (t, J = 7.8 Hz, 1H), 6.91 (d, J = 7.8 Hz, 2H), 5.08 - 4.40 (m, 5H), 4.07 (s, 3H), 3.71 - 3.63 (m, 2H), 2.16 - 1.90 (m, 4H).

Examples synthesised from I-28 following the same procedure as in step B for example 51 (Scheme 6) replacing (S)-2-(trifluoromethyl)pyrrolidine with the appropriate building block are described in table 10.

**Table 10**

| Building Block | Structure/Name | LCMS | ¹H NMR (400 MHz, DMSO-d₆) δ (ppm) |
|---|---|---|---|
| 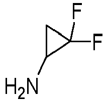 2,2-difluorocyclopropanamine | 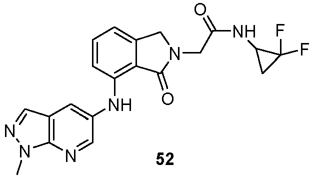 N-(2,2-difluorocyclopropyl)-2-[7-[(1-methylpyrazolo[3,4-b]pyridin-5-yl)amino]-1-oxo-isoindolin-2-yl]acetamide | 3.38 min, m/z 413.1 [M+H]⁺ (Method 1) | δ 8.63 (s, 1H), 8.59 (t, *J* = 3.3 Hz, 1H), 8.52 (d, *J* = 2.4 Hz, 1H), 8.16 (d, *J* = 2.4 Hz, 1H), 8.08 (s, 1H), 7.36 (t, *J =* 7.8 Hz, 1H), 6.91 (d, 2H), 4.48 (s, 2H), 4.20 (s, 2H), 4.07 (s, 3H), 3.33 (d, *J* = 6.3 Hz, 1H), 1.97 - 1.85 (m, 1H), 1.55 - 1.49 (m, 1H). |
| | **Example 52** | | |
| 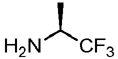 (2S)-Amino-1,1,1-trifluoropropane hydrochloride | 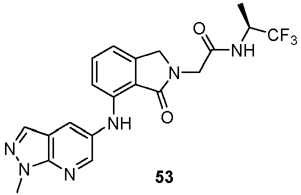 2-[7-[(1-methylpyrazolo[3,4-b]pyridin-5-yl)amino]-1-oxo-isoindolin-2-yl]-N-[(1S)-2,2,2-trifluoro-1-methyl-ethyl]acetamide | 3.61 min, m/z 433.2 [M+H]⁺ (Method 1) | δ 8.73 (d, J = 8.9 Hz, 1H), 8.63 (s, 1H), 8.52 (d, J = 2.4 Hz, 1H), 8.17 (d, J = 2.4 Hz, 1H), 8.08 (s, 1H), 7.35 (t, J = 7.8 Hz, 1H), 6.91 (d, J = 7.7 Hz, 2H), 4.67 - 4.58 (m, 1H), 4.48 (s, 2H), 4.29 - 4.19 (m, 2H), 4.07 (s, 3H), 1.27 (d, J = 7.0 Hz, 3H). |
| | **Example 53** | | |
| 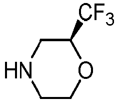 (2S)-(trifluoromethyl)morpholine | 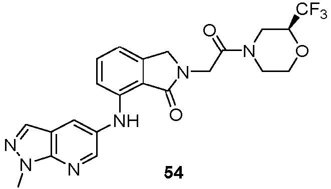 7-[(1-methylpyrazolo[3,4-b]pyridin-5-yl)amino]-2-[2-oxo-2-[(2S)-(trifluoromethyl)morpholin-4-yl]ethyl]isoindolin-1-one | 3.72 min, m/z 475.2 [M+H]⁺ (Method 1) | δ 8.63 (s, 1H), 8.52 (d, J = 2.4 Hz, 1H), 8.17 (d, J = 2.4 Hz, 1H), 8.08 (s, 1H), 7.36 (t, J = 7.8 Hz, 1H), 6.92 - 6.90 (m, 2H), 4.63 - 4.54 (m, 1H), 4.46 - 4.44 (m, 3H) , 4.38 - 4.31 (m, 1H), 4.24 - 4.13 (m, 1H), 4.07 - 3.88 (m, 1H), 3.71 - 3.54 (m, 1H), 3.38 - 3.37 (m, 1H), 2.90 (t, J = 11.8 Hz, 1H). |
| | **Example 54** | | |
| 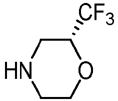 (2R)-(trifluoromethyl)morpholine | 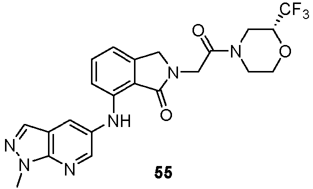 7-[(1-methylpyrazolo[3,4-b]pyridin-5-yl)amino]-2-[2-oxo-2-[(2R)-(trifluoromethyl)morpholin-4-yl]ethyl]isoindolin-1-one | 3.68 min, m/z 475.1 [M+H]⁺ (Method 1) | δ 8.63 (s, 1H), 8.52 (d, J = 2.4 Hz, 1H), 8.17 (d, J = 2.4 Hz, 1H), 8.08 (s, 1H), 7.36 (t, J = 7.8 Hz, 1H), 6.92 - 6.90 (m, 2H), 4.63 - 4.54 (m, 1H), 4.47 - 4.43 (m, 3H), 4.38 - 4.31 (m, 1H), 4.24 - 4.13 (m, 1H), 4.06 - 3.87 (m, 5H), 3.71 - 3.54 (m, 1H), 3.38 - 3.32 (m, 1H), 2.90 (t, J = 11.8 Hz, 1H). |
| | **Example 55** | | |
| 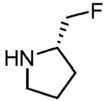 (2S)-(fluoromethyl)pyrrolidine | 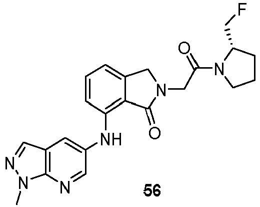 7-[(1-methylpyrazolo[3,4-b]pyridin-5-yl)amino]-2-[2-oxo-2-[(2S)-(fluoromethyl)pyrrolidin-1-yl]ethyl]isoindolin-1-one | 3.55 min, m/z 423.3 [M+H]⁺ (Method 1) | δ 8.65 (d, J = 4.6 Hz, 1H), 8.52 (d, J = 2.4 Hz, 1H), 8.17 (d, J = 2.4 Hz, 1H), 8.08 (s, 1H), 7.35 (t, J = 7.8 Hz, 1H), 6.92-6.90 (m, 2H), 4.56 - 4.32 (m, 6H), 4.15 (d, J = 23.3 Hz, 1H), 4.06 (s, 3H), 3.57 - 3.45 (m, 2H), 2.0 - 1.81 (m, 4H). |
| | **Example 56** | | |
| 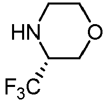 (3R)-(trifluoromethyl)morpholine | 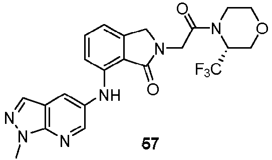 7-[(1-methylpyrazolo[3,4-b]pyridin-5-yl)amino]-2-[2-oxo-2-[ (3R)-(trifluoromethyl)morpholin-4-yl]ethyl]isoindolin-1-one | 3.65 min, m/z 475.2 [M+H]⁺ (Method 1) | δ 8.62 (s, 1H), 8.53 (d, J = 2.4 Hz, 1H), 8.17 (d, J = 2.4 Hz, 1H), 8.08 (s, 1H), 7.36 (t, J = 7.8 Hz, 1H), 6.93 - 6.90 (m, 2H), 4.99 - 4.88 (m, 1H), 4.68 (t, J = 17.0 Hz, 1H), 4.52 - 4.49 (m, 1H), 4.45 - 4.39 (m, 2H), 4.16 - 4.10 (m, 1H), 4.07 (s, 3H), 3.94 - 3.84 (m, 2H), 3.77 - 3.66 (m, 1H), 3.58 - 3.44 (m, 2H). |
| | **Example 57** | | |
| 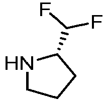 (2S)-(difluoromethyl)pyrrolidine | 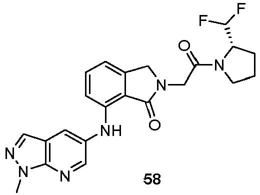 7-[(1-methylpyrazolo[3,4-b]pyridin-5-yl)amino]-2-[2-oxo-2-[(2S)-(difluoromethyl)pyrrolidin-1-yl]ethyl]isoindolin-1-one | 3.70 min, m/z 441.3 [M+H]⁺ (Method 1) | δ 8.64 (d, J = 5.9 Hz, 1H), 8.52 (d, J = 2.4 Hz, 1H), 8.17 (d, J = 2.4 Hz, 1H), 8.08 (s, 1H), 7.35 (t, J = 7.9 Hz, 1H), 6.91 (d, J = 7.9 Hz, 2H), 6.20 (ddd, J = 57.7, 55.2, 1.9 Hz, 1H), 4.56 - 4.39 (m, 4H), 4.26 (d, J = 24.8 Hz, 1H), 4.06 (s, 3H), 3.65-3.52 (m, 2H), 2.06-1.92 (m, 4H). |
| | **Example 58** | | |
| 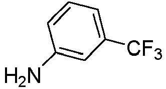 3-trifluoromethylanaline | 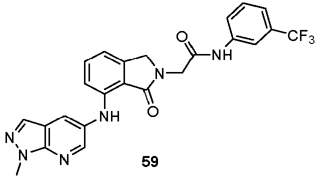 2-[7-[(1-methylpyrazolo[3,4-b]pyridin-5-yl)amino]-1-oxo-isoindolin-2-yl]-N-[3-(trifluoromethyl)phenyl]acetamide | 2.01 min, m/z 481.2 [M+H]⁺ (Method 1) | δ 10.54 (s, 1H), 8.64 (s, 1H), 8.53 (d, J = 2.4 Hz, 1H), 8.17 (d, J = 2.4 Hz, 1H), 8.10 (s, 1H), 8.09 (s, 1H), 7.83 - 7.73 (m, 1H), 7.58 (t, J = 8.0 Hz, 1H), 7.48 - 7.33 (m, 2H), 6.93 (d, J = 7.9 Hz, 2H), 4.56 (s, 2H), 4.42 (s, 2H), 4.07 (s, 3H). |
| | **Example 59** | | |
| 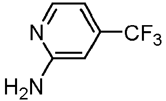 2-amino-4-(trifluoromethyl)pyridine | 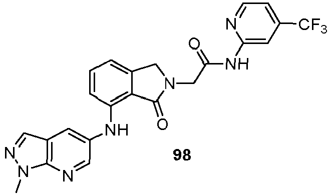 2-[7-[(1-methylpyrazolo[3,4-b]pyridin-5-yl)amino]-1-oxo-isoindolin-2-yl]-N-[4-(trifluoromethyl)-2-pyridyl]acetamide | 3.87 min, m/z 482.2 [M+H]⁺ (Method 1) | 11.30 (s, 1H), 8.63 (d, J = 5.9 Hz, 2H), 8.53 (d, J = 2.4 Hz, 1H), 8.36 (s, 1H), 8.17 (d, J = 2.4 Hz, 1H), 8.08 (s, 1H), 7.50 (d, J = 5.2 Hz, 1H), 7.37 (t, J = 7.8 Hz, 1H), 6.92 (d, J = 7.9 Hz, 2H), 4.55 (s, 2H), 4.50 (s, 2H), 4.06 (s, 3H). |
| | **Example 98** | | |
| 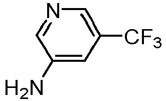 5-(trifluoromethyl)-3-aminopyridine | 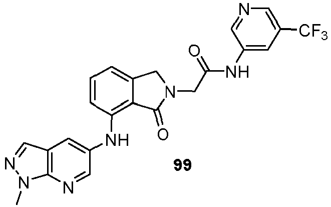 2-[7-[(1-methylpyrazolo[3,4-b]pyridin-5-yl)amino]-1-oxo-isoindolin-2-yl]-N-[5-(trifluoromethyl)-3-pyridyl]acetamide | 3.75 min, m/z 482.0 [M+H]⁺ (Method 1) | 0.87 (s, 1H), 8.97 (s, 1H), 8.66 (d, J = 21.0 Hz, 2H), 8.53 - 8.49 (m, 2H), 8.17 (d, J = 2.4 Hz, 1H), 8.08 (s, 1H), 7.38 (t, J = 7.8 Hz, 1H), 6.93 (d, J = 7.8 Hz, 2H), 4.57 (s, 2H), 4.46 (s, 2H), 4.07 (s, 3H). |
| | **Example 99** | | |
| 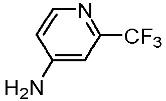 4-amino-2-trifluoromethylpyridine | 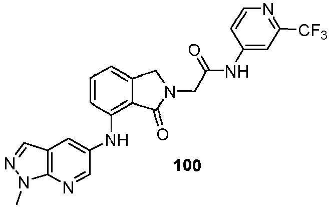 2-[7-[(1-methylpyrazolo[3,4-b]pyridin-5-yl)amino]-1-oxo-isoindolin-2-yl]-N-[2-(trifluoromethyl)-4-pyridyl]acetamide | 3.78 min, m/z 482.2 [M+H]⁺ (Method 1) | 10.98 (s, 1H), 8.65 - 8.62 (m, 2H), 8.53 (d, J = 2.4 Hz, 1H), 8.17 (d, J = 2.4 Hz, 1H), 8.13 (d, J = 1.9 Hz, 1H), 8.08 (s, 1H), 7.77 (dd, J = 5.6, 1.9 Hz, 1H), 7.38 (t, J = 7.8 Hz, 1H), 6.94 - 6.91 (m, 2H), 4.56 (s, 2H), 4.46 (s, 2H), 4.06 (s, 3H). |
| | **Example 100** | | |

### Exmaple 73, 2-[1-oxo-7-(1H-pyrazolo[3,4-b]pyridin-5-ylamino)isoindolin-2-yl]-N-[(1S)-2,2,2-trifluoro-1-methyl-ethyl]acetamide.

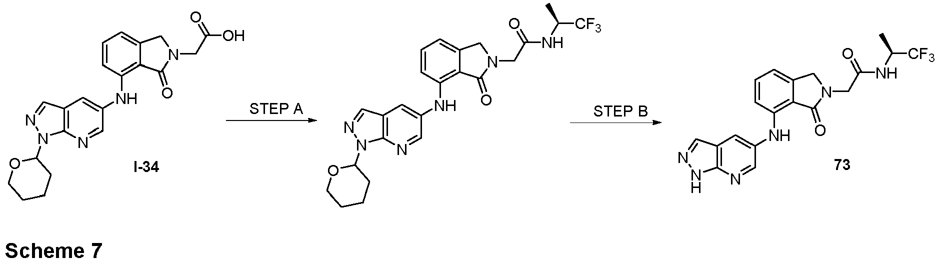

STEP A. 2-[1-oxo-7-[(1-tetrahydropyran-2-ylpyrazolo[3,4-b]pyridin-5-yl)amino]isoindolin-2-yl]-N-[(1S)-2,2,2-trifluoro-1-methyl-ethyl]acetamide. A mixture of 2-(1-oxo-7-((1-(tetrahydro-2H-pyran-2-yl)-1H-pyrazolo[3,4-b]pyridin-5-yl)amino)isoindolin-2-yl)acetic acid (80 mg, 0.19 mmol), (1S)-1,1,1-trifluoropropan-2-amine hydrochloride (44 mg, 0.29 mmol), HATU (110 mg, 0.29 mmol) and DIPEA (101 mg, 0.78 mmol) in DMF (2 mL) was stirred at room temperature overnight. The reaction was diluted with EtOAc (100 mL), washed with water (50 mL x3) and brine before drying with sodium sulfate. The crude was purified by silica gel flash column chromatography (DCM to DCM:MeOH, 50:1) and desired fractions were concentrated affording 2-[1-oxo-7-[(1-tetrahydropyran-2-ylpyrazolo[3,4-b]pyridin-5-yl)amino]isoindolin-2-yl]-N-[(1S)-2,2,2-trifluoro-1-methyl-ethyl]acetamide as a yellow solid (35 mg, 35% yield).

UPLC-MS (ES⁺, Method 2): 2.04 min, m/z 503 [M+H]⁺.

STEP B. 2-[1-oxo-7-(1H-pyrazolo[3,4-b]pyridin-5-ylamino)isoindolin-2-yl]-N-[(1S)-2,2,2-trifluoro-1-methyl-ethyl]acetamide (example 73). A mixture of 2-[1-oxo-7-[(1-tetrahydropyran-2-ylpyrazolo[3,4-b]pyridin-5-yl)amino]isoindolin-2-yl]-N-[(1S)-2,2,2-trifluoro-1-methyl-ethyl]acetamide (35 mg, 0.07 mmol) in HCl 1,4-dioxane solution (4M, 1 mL) and DCM (1 mL) and MeOH (1 mL) was stirred at room temperature for 1 h.. The mixture was concentrated to give 2-[1-oxo-7-(1H-pyrazolo[3,4-b]pyridin-5-ylamino)isoindolin-2-yl]-N-[(1S)-2,2,2-trifluoro-1-methyl-ethyl]acetamide (example 73) (29 mg, 92% yield) as a yellow solid

UPLC-MS (ES⁺, Method 2): 1.29 min, m/z, 419.05 [M+H]⁺.

¹H NMR (400 MHz, DMSO-*d*₆) δ (ppm) 8.74 (d, *J* = 8.9 Hz, 1H), 8.47 (d, *J* = 2.4 Hz, 1H), 8.16 (d, *J* = 2.4 Hz, 1H), 8.09 (s, 1H), 7.35 (t, *J* = 7.8 Hz, 1H), 6.90 (d, *J* = 7.9 Hz, 2H), 4.62 (h, *J* = 7.7 Hz, 1H), 4.48 (s, 2H), 4.29 - 4.18 (m, 2H), 1.27 (d, *J* = 6.9 Hz, 3H).

The examples in table 11 were prepared from intermediate I-34 using analogous procedures to example 73 (scheme 7) replacing (1S)-1,1,1-trifluoropropan-2-amine hydrochloride in step A with the appropriate building block.

**Table 11**

| Building Block | Structure/Name | LCMS | ¹H NMR (400 MHz, DMSO-d₆) δ (ppm) |
|---|---|---|---|
| 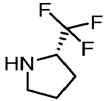 (2S)-(trifluoromethyl)pyrrolidine | 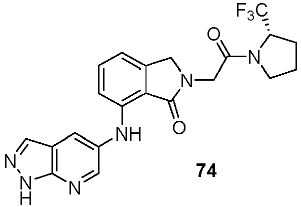 2-[2-oxo-2-[(2S)-(trifluoromethyl)pyrrolidin-1-yl]ethyl]-7-(1H-pyrazolo[3,4-b]pyridin-5-ylamino)isoindolin-1-one | 3.53 min, m/z 445.1 [M+H]⁺ (Method 1) | (CD₃OD): 8.72 (d, *J* = 2.3 Hz, 1H), 8.57 (d, *J* = 2.3 Hz, 1H), 8.37 (s, 1H), 7.42 (t, *J* = 7.8 Hz, 1H), 7.06 (d, *J* = 8.2 Hz, 1H), 6.98 (d, *J* = 7.4 Hz, 1H), 4.81 - 4.75 (m, 1H), 4.59-4.44 (m, 4H), 3.82 - 3.68 (m, 2H), 2.27 - 2.11 (m, 4H). |
| | **Example 74** | | |
| 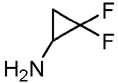 2,2-difluorocyclopropylamine | 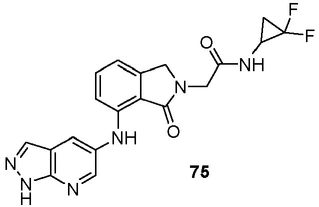 N-(2,2-difluorocyclopropyl)-2-[1-oxo-7-(1H-pyrazolo[3,4-b]pyridin-5-ylamino)isoindolin-2-yl]acetamide | 2.98 min, m/z 399.1 [M+H]⁺ (Method 1) | 13.63 (s, 1H), 8.60 (d, *J* = 4.0 Hz, 2H), 8.47 (d, *J* = 2.4 Hz, 1H), 8.15 (d, *J* = 2.4 Hz, 1H), 8.09 (s, 1H), 7.35 (t, *J* = 7.8 Hz, 1H), 6.90 (d, *J* = 7.4 Hz, 2H), 4.47 (s, 2H), 4.20 (s, 2H), 3.42 - 3.38 (m, 1H), 1.95-1.85 (m, 1H), 1.57-1.48 (m, 1H). |
| | **Example 75** | | |
| 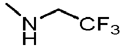 N-methyl-(2,2,2)-trifluoromethylamine | 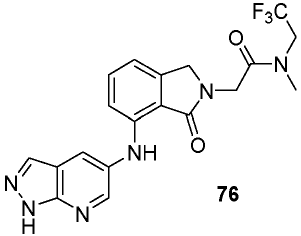 N-methyl-2-[1-oxo-7-(1H-pyrazolo[3,4-b]pyridin-5-ylamino)isoindolin-2-yl]-N-(2,2,2-trifluoroethyl)acetamide | 3.38 min, m/z 419.1 [M+H]⁺ (Method 1) | 13.62 (s, 1H), 8.60 (s, 1H), 8.47 (d, *J* = 2.4 Hz, 1H), 8.16 (d, *J* = 2.4 Hz, 1H), 8.08 (s, 1H), 7.36 (t, *J* = 7.8 Hz, 1H), 6.92 - 6.88 (m, 2H), 4.53 (s, 2H), 4.44 - 4.16 (m, 4H), 3.18 - 2.96 (d, *J* = 91.5 Hz, 3H). |
| | **Example 76** | | |
| 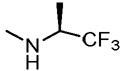 N-methyl-(1S)-1,1,1-trifluoropropan-2-amine | 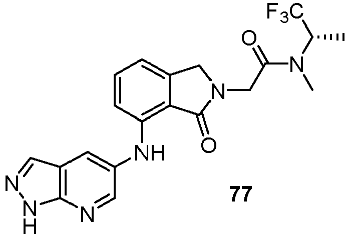 N-methyl-2-[1-oxo-7-(1H-pyrazolo[3,4-b]pyridin-5-ylamino)isoindolin-2-yl]-N-[(1S)-2,2,2-trifluoro-1-methylethyl]acetamide | 3.55 min, m/z 433.2 [M+H]⁺ (Method 1) | 13.62 (s, 1H), 8.60 (s, 1H), 8.47 (d, *J* = 2.4 Hz, 1H), 8.16 (d, *J* = 2.4 Hz, 1H), 8.13 -8.09 (s, 1H), 7.36 (t, *J* = 7.8 Hz, 1H), 6.93 - 6.89 (m, 2H), 5.29 - 4.92 (m, 1H), 4.61 - 4.44 (m, 4H), 3.03 -2.82 (d, *J* = 84.7 Hz, 3H), 1.47 -1.34 (dd, *J* = 42.3, 6.9 Hz, 3H). |
| | **Example 77** | | |

### Example 79, 2-[1,1-difluoro-4-[(1-methylindazol-5-yl)amino]-3-oxo-isoindolin-2-yl]-N-(2,2,2-trifluoroethyl)acetamide.

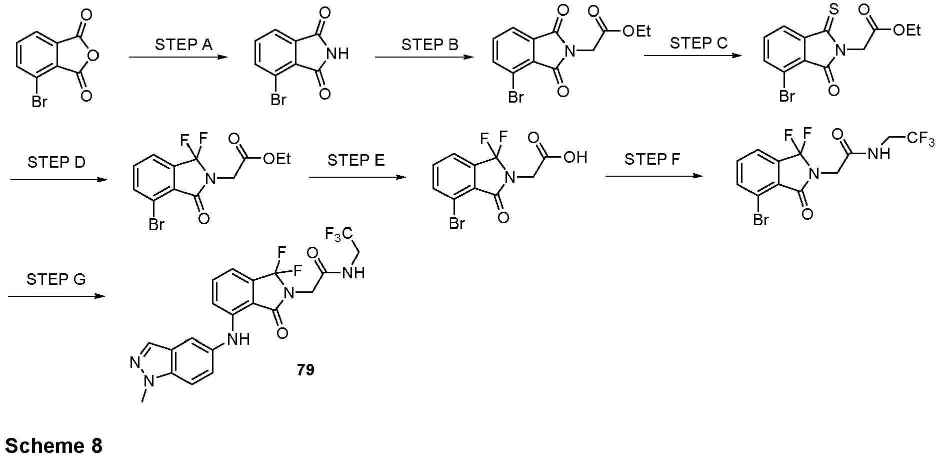

STEP A. 4-Bromoisoindoline-1,3-dione. A solution of 3-bromophthalic anhydride (1.1 g , 4.84 mmol), in formamide (4 mL) was stirred at 200 °C for 2 hrs. The resulting suspension was slurried with water and filtered to afford 4-bromoisoindoline-1,3-dione (1.0g, 91% yield) as a white solid after drying under suction.

UPLC-MS (ES⁺, Method 1): 3.25 min, m/z, 224.00 [M-H]⁺.

STEP B. Ethyl 2-(4-bromo-1,3-dioxo-isoindolin-2-yl)acetate. A solution of 4-bromoisoindoline-1,3-dione (1.0 g , 4.42 mmol), ethyl bromoacetate (886 mg, 5.3 mmol) and cesium carbonate (2.16 g, 6.6 mmol) in MeCN (10 mL) was stirred at 50 °C for 2 h. The mixture was filtered and the filter cake washed with MeCN, the combined organics were concentrated in vacuum and slurried with cold ether to give ethyl 2-(4-bromo-1,3-dioxo-isoindolin-2-yl)acetate (1.0 g, 72% yield) as an off-white semi-solid.

¹H NMR (400 MHz, DMSO-d₆) δ (ppm) 8.07 - 8.05 (d, *J* = 8 Hz, 1H), 7.95 - 7.93 (d, *J* = 8 Hz, 1H), 7.80 - 7.76 (t, *J* = 8 Hz, 1H), 4.42 (s, 2H), 4.19 - 4.14 (q, *J* = 7 Hz 2H), 1.23 - 1.19 (t, *J* = 7 Hz 3H).

STEP C. Ethyl 2-(4-bromo-3-oxo-1-thioxo-isoindolin-2-yl)acetate. A mixture of ethyl 2-(4-bromo-1,3-dioxo-isoindolin-2-yl)acetate (360 mg, 1.15 mmol) and Lawessons's reagent (513 mg, 1.28 mmol) was added to toluene (10 mL) and heated to reflux for 14 hrs. Toluene was removed in vacuo after cooling to room temperature and the resulting residue purified by silica gel flash column chromatography (loading in DCM and eluting with 10:1 hexane/EtOAc affording ethyl 2-(4-bromo-3-oxo-1-thioxo-isoindolin-2-yl)acetate (250 mg, 66% yield) as a yellow solid.

¹H NMR (400 MHz, CDCl₃) δ (ppm) 7.98 - 7.96 (d, *J* = 8 Hz, 1H), 7.86 - 7.84 (d, *J* = 8 Hz, 1H), 7.58 - 7.54 (t, *J* = 8 Hz, 1H), 4.79 (s, 2H), 4.25 - 4.20 (q, *J* = 7 Hz 2H), 1.30 - 1.26 (t, *J* = 7 Hz 3H).

STEP D. Ethyl 2-(4-bromo-1,1-difluoro-3-oxo-isoindolin-2-yl)acetate. A mixture of ethyl 2-(4-bromo-3-oxo-1-thioxo-isoindolin-2-yl)acetate (190 mg, 0.58 mmol), NBS (515 mg, 2.89 mmol) and n-Bu₄NH₂F₃ (3.5 g, 5.8 mmol) in DCM (10 mL) was stirred at room temperature for 14 hrs. The mixture was filtered and concentrated to an oily residue and purified by preparative TLC with 10:1 hexane/EtOAc) affording ethyl 2-(4-bromo-1,1-difluoro-3-oxo-isoindolin-2-yl)acetate (80 mg, 39% yield) as a white solid.

¹H NMR (400 MHz, DMSO-*d*₆) δ (ppm) 7.86 - 7.84 (d, *J* = 8 Hz, 1H), 7.71 - 7.69 (d, *J* = 8 Hz, 1H), 7.60 - 7.66 (t, *J* = 8 Hz, 1H), 4.3 (s, 2H), 4.26 - 4.21 (q, *J* = 7 Hz 2H), 1.23 - 1.19 (t, *J* = 7 Hz 3H).

STEP E. 2-(4-Bromo-1,1-difluoro-3-oxo-isoindolin-2-yl)acetic acid. A solution of LiOH.H₂O (57.8 mg, 1.37 mmol) in water (2 mL) was added to a mixture of ethyl 2-(4-bromo-1,1-difluoro-3-oxo-isoindolin-2-yl)acetate (230 mg, 0.69 mmol) in 1:1 MeOH/THF (6mL), and the resulting mixture was stirred at 25 °C for 2 hrs. The mixture was concentrated and the residue was diluted with water (2 mL) and adjusted pH to 2 with 2N HCl, then filtered, the solid was dried to afford 2-(4-bromo-1,1-difluoro-3-oxo-isoindolin-2-yl)acetic acid ( 100 mg, 46% yield) as a white solid.

UPLC-MS (ES⁺, Method 1): 3.50 min, m/z, 303.9/305.9 [M-H]⁺.

STEP F. 2-(4-Bromo-1,1-difluoro-3-oxo-isoindolin-2-yl)-N-(2,2,2-trifluoroethyl)acetamide. A solution of 2-(4-bromo-1,1-difluoro-3-oxo-isoindolin-2-yl)acetic acid (80 mg , 0.29 mmol), 2,2,2-trifluoroethan-1-amine(38.8 mg, 0.39 mmol) DIEA (168 mg, 1.30 mmol) and T3P (297 mg, 0.39 mmol) in DCM (1 mL) was stirred at room temperature for 16 h. The mixture was concentrated to an oily residue and purified by preparative-TLC to give 2-(4-bromo-1,1-difluoro-3-oxo-isoindolin-2-yl)-N-(2,2,2-trifluoroethyl)acetamide (38.6 mg, 38% yield) as a white solid.

UPLC-MS (ES⁺, Method 1): 3.51 min, m/z, 386.6/388.6 [M+H]⁺.

STEP G. 2-[1,1-difluoro-4-[(1-methylindazol-5-yl)amino]-3-oxo-isoindolin-2-yl]-N-(2,2,2-trifluoroethyl)acetamide (Example 79). A solution 2-(4-bromo-1,1-difluoro-3-oxo-isoindolin-2-yl)-N-(2,2,2-trifluoroethyl)acetamide (80 mg , 0.2mmol), cesium carbonate (101 mg, 0.31 mmol) , Xantphos (11.9 mg, 0.02 mmol), bis(dibenzylideneacetone)palladium (0) (18.9g, 0.02 mmol) , 1-methyl-1H-indazol-5-amine (30.4 mg, 0.2 mmol) in 1,4-dioxane (2 mL) was stirred at 100 °C for 16 h. The mixture was concentrated in vacuo and purified by preparative-HPLC to give 2-[1,1-difluoro-4-[(1-methylindazol-5-yl)amino]-3-oxo-isoindolin-2-yl]-N-(2,2,2-trifluoroethyl)acetamide (example 79) (21 mg, 22% yield) as a yellow solid.

UPLC-MS (ES⁺, Method 1): 3.75 min, m/z, 454.1 [M+H]⁺.

¹H NMR (400 MHz, DMSO-*d*₆) δ (ppm) 8.86 (t, J = 6.4 Hz, 1H), 8.32 (s, 1H), 8.01 (s, 1H), 7.68 (dd, *J* = 5.4, 3.4 Hz, 2H), 7.52 (t, *J* = 7.9 Hz, 1H), 7.36 (dd, *J* = 8.9, 1.9 Hz, 1H), 7.15 - 7.08 (m, 2H), 4.22 (s, 2H), 4.06 (s, 3H), 4.02 - 3.90 (m, 2H).

### Example 80, 2-[7-[(3-isopropyl-1H-indazol-6-yl)amino]-1-oxo-isoindolin-2-yl]-N-(2,2,2-trifluoroethyl)acetamide.

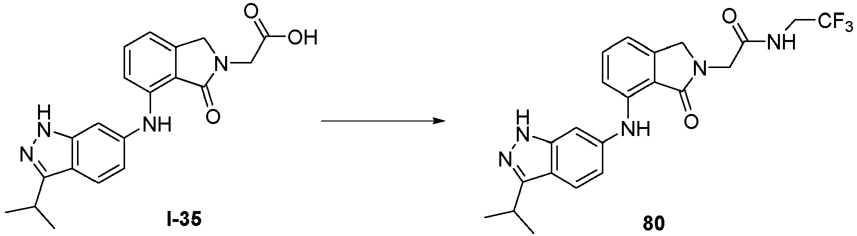

A mixture of 2-(7-((3-isopropyl-1H-indazol-6-yl)amino)-1-oxoisoindolin-2-yl)acetic acid (15 mg, 0.04 mmol), 2,2,2-trifluoroethan-1-amine (6.12 mg, 0.06 mmol), HATU (23.5 mg, 0.06 mmol,) and DIPEA (16 mg, 0.12 mmol) in DMF (1 mL) was stirred at room temperature overnight. The reaction mixture was concentrated in vacuo and was purified by preparative TLC (DCM:MeOH=20:1) to give 2-[7-[(3-isopropyl-1H-indazol-6-yl)amino]-1-oxo-isoindolin-2-yl]-N-(2,2,2-trifluoroethyl)acetamide (2.2 mg, 12% yield) as a white solid.

UPLC-MS (ES⁺, Method 2): 1.66 min, m/z, 446.1 [M+H]⁺.

¹H NMR (400 MHz, Acetonitrile-*d*₃) δ (ppm) 10.59 (s, 1H), 8.84 (s, 1H), 7.72 (d, *J* = 8.6 Hz, 1H), 7.45 - 7.41 (m, 1H), 7.40 (t, *J* = 1.7 Hz, 1H), 7.34-7.31 (m, 1H), 7.16 - 7.11 (m, 1H), 7.00 (dd, *J* = 8.7, 1.8 Hz, 1H), 6.92 (d, *J* = 8.0 Hz, 1H), 4.45 (s, 2H), 4.22 (s, 2H), 3.90 (qd, *J* = 9.5, 6.5 Hz, 2H), 3.36 (p, *J* = 7.0 Hz, 1H), 1.40 (d, *J* = 8.0 Hz, 6H).

### Example 81, 2-[7-(1H-indazol-6-ylamino)-1-oxo-isoindolin-2-yl]-N-(2,2,2-trifluoroethyl)acetamide.

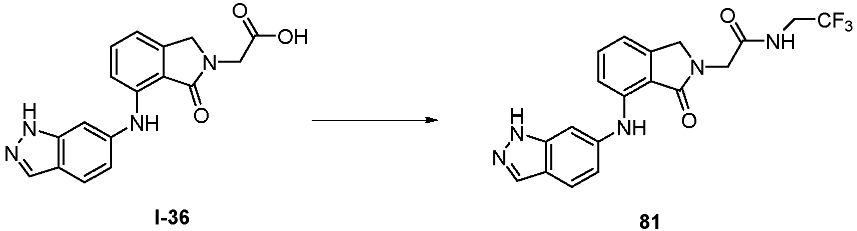

A solution of intermediate I-36 (90 mg, 0.27 mmol), trifluoroethylamine (33.19 mg, 0.33 mmol), T3P (133.27 mg, 0.41 mmol), and DIEA (180. mg, 1.39 mmol), in DCM (1.0 mL) was stirred at 25 °C for 2h.. The mixture was diluted with EtOAc (5 mL) and washed with H₂O (5 mL x 3). The organic layers were washed with EtOAc (10 mL×3), combined, dried over anhydrous Na₂SO₄ and concentrated to give a yellow solid which was purified by preparative-TLC (DCM:MeOH=20:1) to give 2-[7-(1H-indazol-6-ylamino)-1-oxo-isoindolin-2-yl]-N-(2,2,2-trifluoroethyl)acetamide (5.1 mg, 5% yield) as a white solid.

UPLC-MS (ES⁺, Method 1): 3.35 min, m/z, 404.1 [M+H]⁺.

¹H NMR (400 MHz, DMSO-*d*₆) δ (ppm) 12.81 (s, 1H), 8.84 (t, *J =* 5.2 Hz, 2H), 7.97 (s, 1H), 7.71 (d, *J* = 8.5 Hz, 1H), 7.46 - 7.40 (m, 2H), 7.29 (d, *J* = 8.3 Hz, 1H), 7.00 - 6.95 (m, 2H), 4.48 (s, 2H), 4.25 (s, 2H), 3.99 - 3.90 (m, 2H).

### Example 82, 5-[[3-oxo-2-[2-oxo-2-[(2S)-(trifluoromethyl)pyrrolidin-1-yl]ethyl]isoindolin-4-yl]amino]-1H-indazole-3-carbonitrile.

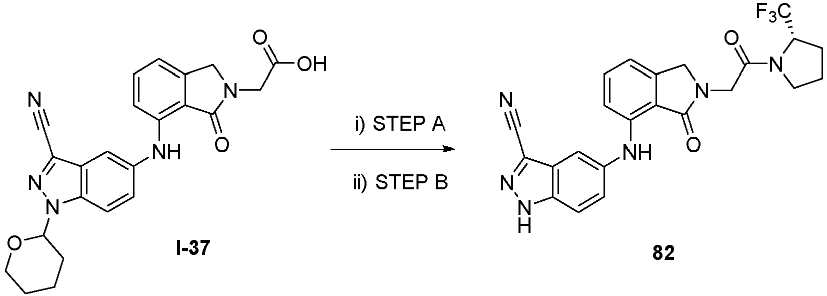

Example 82 was prepared from intermediate I-37 by analogy with the method used for example 25 (scheme 5) with I-37 in place of I-25 and replacing 3-(trifluoromethyl)aniline with (2S)-trifluoromethylpyrrolidine in step A.

UPLC-MS (ES⁺, Method 1): 3.98 min, m/z, 469.2 [M+H]⁺.

¹H NMR (400 MHz, DMSO-*d*₆) δ (ppm) 14.29 (s, 1H), 8.84 (s, 1H), 7.75 (d, *J* = 8.9 Hz, 1H), 7.68

(d, *J* = 1.9 Hz, 1H), 747 - 7.43 (m, 2H), 7.23 (d, *J* = 8.3 Hz, 1H), 6.97 (d, *J* = 7.4 Hz, 1H), 4.78 - 4.57 (m, 1H), 4.51 - 4.37 (m, 4H), 3.67 (d, *J* = 8.2 Hz, 2H), 2.14 - 1.99 (m, 3H).

### Example 83, 7-[(3-fluoro-1H-indazol-5-yl)amino]-2-[2-oxo-2-[(2S)-(trifluoromethyl)pyrrolidin-1-yl]ethyl]isoindolin-1-one.

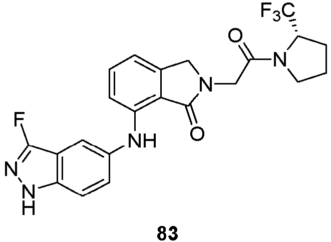

Example 83 was prepared by analogy with example 81 replacing I-36 with I-38 and replacing trifluoroethylamine with (2S)-(trifluoromethyl)pyrrolidine.

UPLC-MS (ES⁺, Method 1): 4.02 min, m/z, 462.1 [M+H]⁺.

¹H NMR (400 MHz, DMSO-d₆) δ (ppm) 12.50 (s, 1H), 8.64 (s, 1H), 7.55 (d, J = 1.9 Hz, 1H), 7.50 (dd, J = 8.9, 2.3 Hz, 1H), 7.41 - 7.33 (m, 2H), 7.04 (d, J = 8.2 Hz, 1H), 6.90 (d, J = 7.3 Hz, 1H), 4.81 - 4.70 (m, 1H), 4.51 - 4.35 (m, 4H), 3.67 (d, J = 8.6 Hz, 2H), 2.13 - 1.93 (m, 4H).

### Example 84. 2-[2-oxo-2-[(2S)-(trifluoromethyl)pyrrolidin-1-yl]ethyl]-7-(1H-pyrazolo[4,3-b]pyridin-5-ylamino)isoindolin-1-one.

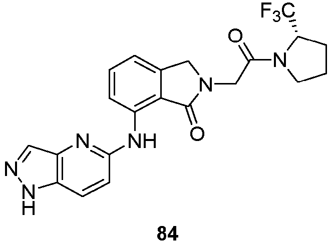

Example 83 was prepared by analogy with example 81 replacing I-38 with I-39.

UPLC-MS (ES⁺, Method 1): 3.57 min, m/z, 445.2 [M+H]⁺.

¹H NMR (400 MHz, DMSO-d₆) δ (ppm) 13.2 (bs, 1H), 9.86 (s, 1H), 8.79 - 8.77 (m, 1H), 8.13 - 7.92 (m, 2H), 7.59 - 7.51 (m, 1H), 7.09 - 6.92 (m, 2H), 4.81 - 4.70 (m, 1H), 4.54 - 4.39 (m, 4H), 3.71 - 3.63 (m, 2H), 2.12 - 1.96 (m, 4H).

### Example 86. 5-[methyl-[3-oxo-2-[2-oxo-2-[(2S)-(trifluoromethyl)pyrrolidin-1-yl]ethyl]isoindolin-4-yl]amino]-1H-indazole-3-carbonitrile.

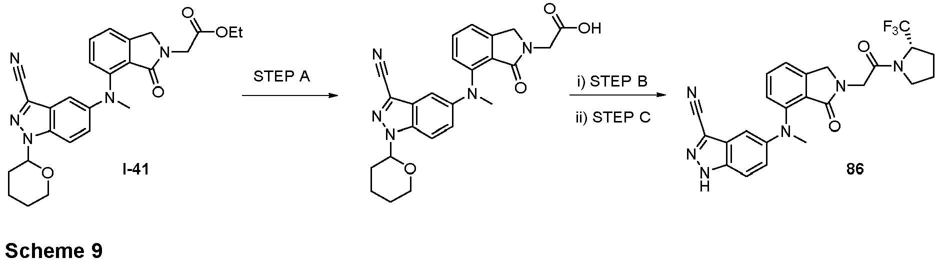

STEP A. 2-[7-[(3-cyano-1-tetrahydropyran-2-yl-indazol-5-yl)-methyl-amino]-1-oxo-isoindolin-2-yl]acetic acid. A mixture of ethyl 2-[7-[(3-cyano-1-tetrahydropyran-2-yl-indazol-5-yl)-methyl-amino]-1-oxo-isoindolin-2-yl]acetate (90 mg, 0.19 mol), LiOH (15.95 mg, 0.38 mmol), THF (2mL), MeOH (2 mL) and H₂O (0.5 mL) was stirred at 25°C for 2 hours. The mixture was diluted with H₂O and 10% HCl to achieve pH 1 - 2 and was concentrated in vacuum to afford 2-[7-[(3-cyano-1-tetrahydropyran-2-yl-indazol-5-yl)-methyl-amino]-1-oxo-isoindolin-2-yl]acetic acid (100 mg, quantitative yield) as a white solid.

UPLC-MS (ES⁺, Method 1): 4.11 min, m/z 465.2 [M+H]⁺.

STEP B. 5-[methyl-[3-oxo-2-[2-oxo-2-[(2S)-(trifluoromethyl)pyrrolidin-1-yl]ethyl]isoindolin-4-yl]amino]-1-tetrahydropyran-2-yl-indazole-3-carbonitrile. A mixture of 2-[7-[(3-cyano-1-tetrahydropyran-2-yl-indazol-5-yl)-methyl-amino]-1-oxo-isoindolin-2-yl]acetic acid (80 mg, 0.17 mmol), (2S)-trifluoromethylpyrrolidine (37.47 mg, 0.26 mmol), HATU (81.9 mg, 0.21 mmol) and DIEA (69.5 mg, 0.53 mmol) in DMF (1 mL) was stirred at 25°C overnight. The mixture was slurried with water (20 mL) and the solid was dried under vacuum to afford 5-[methyl-[3-oxo-2-[2-oxo-2-[(2S)-(trifluoromethyl)pyrrolidin-1-yl]ethyl]isoindolin-4-yl]amino]-1-tetrahydropyran-2-yl-indazole-3-carbonitrile (100 mg, 97% yield) as a yellow solid.

UPLC-MS (ES⁺, Method 1): 4.35 min, m/z 567.2 [M+H]⁺.

STEP C. 5-[methyl-[3-oxo-2-[2-oxo-2-[(2S)-(trifluoromethyl)pyrrolidin-1-yl]ethyl]isoindolin-4-yl]amino]-1H-indazole-3-carbonitrile (Example 86). A mixture of 5-[methyl-[3-oxo-2-[2-oxo-2-[(2S)-(trifluoromethyl)pyrrolidin-1-yl]ethyl]isoindolin-4-yl]amino]-1-tetrahydropyran-2-yl-indazole-3-carbonitrile (100 mg, 0.17 mmol,) and 4N HCl-dioxane (1.5 mL) in DCM (1 mL) was stirred at 25°C for 2 hours. The mixture was diluted with water (10 mL) and extracted with EtOAc (10 mL x 3)and was dried over Na₂CO₃ The mixture was purified by preparative TLC (Pet.ether/EtOAc=1/1) to give 5-[methyl-[3-oxo-2-[2-oxo-2-[(2S)-(trifluoromethyl)pyrrolidin-1-yl]ethyl]isoindolin-4-yl]amino]-1H-indazole-3-carbonitrile (example 86) (25 mg, 85% yield) as a yellow solid.

UPLC-MS (ES⁺, Method 1): 3.85 min, m/z 483.1 [M+H]⁺.

¹H NMR (400 MHz, DMSO-*d*₆) δ (ppm) 14.06 (s, 1H), 7.60 (t, *J* = 7.7 Hz, 1H), 7.48 - 7.43 (m, 2H), 7.20 (d, *J* = 7.9 Hz, 1H), 7.00 - 6.99 (m, 1H), 6.91 (dd, *J* = 9.2, 2.3 Hz, 1H), 5.02 - 4.68 (m, 1H), 4.49 - 4.31 (m, 4H), 3.66 - 3.56 (m, 2H), 3.35 (s, 3H), 2.07 - 1.97 (m, 4H).

### Example 91. 2-[7-[(4-chloro-3-methyl-1H-indazol-6-yl)amino]-1-oxo-isoindolin-2-yl]-N-(2,2,2-trifluoroethyl)acetamide.

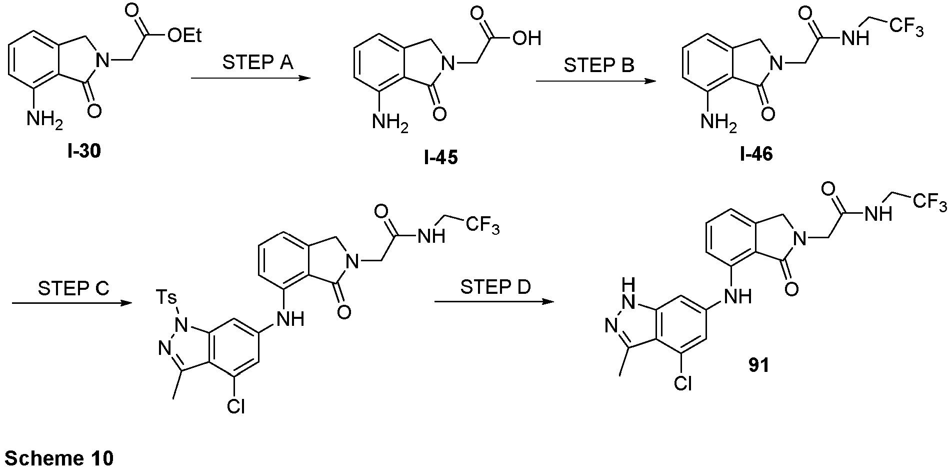

STEP A. 2-(7-Amino-1-oxo-isoindolin-2-yl)acetic acid (Intermediate I-45). A solution of ethyl 2-(7-amino-1-oxoisoindolin-2-yl)acetate (200 mg, 0.85 mmol) and LiOH (93.14 mg, 2.27 mmol) in THF (3 mL) and H₂O (1 mL) was stirred at 25 °C for 2 h. The mixture was neutralised with dilute HCl, then extracted with EtOAc (15 mL x 3), dried over anhydrous Na₂SO₄ and concentrated under vacuum to afford 2-(7-amino-1-oxo-isoindolin-2-yl)acetic acid (150 mg, 85% yield) as a white solid.

¹H NMR (400 MHz, DMSO-d₆) δ (ppm) 12.83 (s, 1H), 7.21 (dd, *J* = 8.1, 7.3 Hz, 1H), 6.67 - 6.61 (m, 1H), 6.61 - 6.54 (m, 1H), 6.03 (s, 2H), 4.36 (s, 2H), 4.18 (s, 2H).

STEP B. 2-(7-Amino-1-oxo-isoindolin-2-yl)-N-(2,2,2-trifluoroethyl)acetamide (Intermediate I-46). A mixture of 2-(7-amino-1-oxoisoindolin-2-yl)acetic acid (150 mg, 0.72 mol), HATU (414mg, 1.09 mmol) and DIPEA (279 mg, 2.18 mmol) in DMF (2 mL) was stirred at 25 °C for 30 mins before adding 2,2,2-trifluoroethan-1-amine (79 mg, 0.80 mol), the mixture was stirred at room temperature for 2h. The mixture was diluted with water (15 mL) and extracted with EtOAc (20 mL x 3). The combined organic layers were washed with brine (50 mL), dried over Na₂SO₄ and concentrated under vacuum and purified by silica gel column chromatography (pet.ether/EtOAc, 5/1 to pet.ether/EtOAc, 1/1) to give 2-(7-amino-1-oxo-isoindolin-2-yl)-N-(2,2,2-trifluoroethyl)acetamide (110 mg, 53% yield) as a clear oil.

UPLC-MS (ES⁺, Method 1): 3.12 min, m/z, 288.2 [M+H]⁺
¹H NMR (400 MHz, DMSO-d₆) δ (ppm) 8.75 (t, *J* = 6.4 Hz, 1H), 7.21 (t, *J* = 7.7 Hz, 1H), 6.60 (dd, *J* = 24.5, 7.7 Hz, 2H), 6.03 (s, 2H), 4.35 (s, 2H), 4.16 (s, 2H), 3.91 (tt, *J* = 9.9, 4.9 Hz, 2H).

STEP C. 2-[7-[[4-Chloro-3-methyl-1-(p-tolylsulfonyl)indazol-6-yl]amino]-1-oxo-isoindolin-2-yl]-N-(2,2,2-trifluoroethyl)acetamide. A mixture of 6-bromo-4-chloro-3-methyl-1-tosyl-1H-indazole (130 mg, 0.32 mol), 2-(7-amino-1-oxoisoindolin-2-yl)-N-(2,2,2-trifluoroethyl)acetamide (93 mg, 0.32 mol) t-BuBrettphos (31.5 mg, 0.065 mmol) Pd₂(dba)₃ (29.7 mg, 0.032 mol, 0.1) K₂CO₃ (8 mg, 0.65 mmol) in t-BuOH (2 mL) and AcOH (0.5 mL) was irradiated in a microwave at 100 °C for 2h. The mixture was diluted with water (5 mL) and extracted with EtOAc (20 mL x 3). The combined organic layers were washed with brine (20 mL), dried over Na₂SO₄, concentrated in vacuum and purified by silica gel column chromatography (DCM/MeOH, 100/1 to DCM/MeOH, 50/1) to give 2-[7-[[4-chloro-3-methyl-1-(p-tolylsulfonyl)indazol-6-yl]amino]-1-oxo-isoindolin-2-yl]-N-(2,2,2-trifluoroethyl)acetamide (55 mg 28% yield) as a yellow solid.

UPLC-MS (ES⁺, Method 2): 2.15 min, m/z, 607.1 [M+H]⁺

STEP D. 2-[7-[(4-chloro-3-methyl-1H-indazol-6-yl)amino]-1-oxo-isoindolin-2-yl]-N-(2,2,2-trifluoroethyl)acetamide (Example 91). A solution of ethyl 2-(7-amino-1-oxoisoindolin-2-yl)acetate (50 mg, 0.082 mmol) and LiOH (20 mg, 0.41 mmol) in THF (3 mL) and H₂O (1 mL) was stirred at 50 °C for 2 h. The mixture adjusted to pH 7.0 using dilute HCl, then concentrated to give crude material purified by preparative HPLC to give 2-[7-[(4-chloro-3-methyl-1H-indazol-6-yl)amino]-1-oxo-isoindolin-2-yl]-N-(2,2,2-trifluoroethyl)acetamide (3.3 mg, 37% yield) as a yellow solid.

UPLC-MS (ES⁺, Method 1): 3.85 min, m/z, 452.20 [M+H]⁺

¹H NMR (400 MHz, DMSO-d₆) δ (ppm) 12.63 (s, 1H), 8.89 - 8.71 (m, 2H), 7.47 (t, *J* = 7.8 Hz, 1H), 7.32 - 7.25 (m, 2H), 7.07 - 6.96 (m, 2H), 4.49 (s, 2H), 4.25 (s, 2H), 3.99 - 3.89 (m, 2H), 2.60 (s, 3H).

### Example 93. 2-[7-[(7-fluoro-1-methyl-indazol-5-yl)amino]-1-oxo-isoindolin-2-yl]-N-(2,2,2-trifluoroethyl)acetamide.

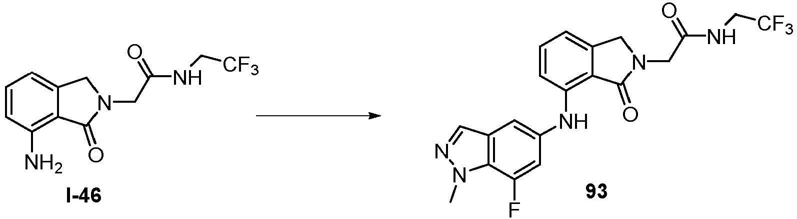

A solution of 5-bromo-7-fluoro-1-methyl-indazole (70 mg, 0.30 mmol), 2-(7-amino-1-oxo-isoindolin-2-yl)-N-(2,2,2-trifluoroethyl)acetamide (97 mg, 0.33 mmol), Cs₂CO₃ (199 mg, 0.61 mmol), Xantphos (35.3 mg, 0.061 mmol), and Pd₂(dba)₃ (28 mg, 0.031 mmol), in 1,4-dioxane (2.0 mL) was stirred at 100 °C under N₂ overnight. The mixture was diluted with EtOAc (5 mL) and washed with H₂O (5 mL x3). The organic layers were washed with EtOAc (10 mL ×3), combined, dried over anhydrous Na₂SO₄ and concentrated to give a yellow solid. This was purified by preparative TLC to afford 2-[7-[(7-fluoro-1-methyl-indazol-5-yl)amino]-1-oxo-isoindolin-2-yl]-N-(2,2,2-trifluoroethyl)acetamide (21 mg, 16% yield) as a yellow solid.

UPLC-MS (ES⁺, Method 1): 3.89 min, m/z, 436.2 [M+H]⁺

¹H NMR (400 MHz, DMSO-d₆) δ (ppm) 8.81 (t, *J* = 6.3 Hz, 1H), 8.63 (s, 1H), 8.03 (d, *J* = 2.4 Hz, 1H), 7.49 (d, *J =* 1.6 Hz, 1H), 7.39 (t, *J* = 7.9 Hz, 1H), 7.21 (dd, *J* = 13.2, 1.7 Hz, 1H), 7.09 (d, *J* = 8.2 Hz, 1H), 6.92 (d, *J* = 7.4 Hz, 1H), 4.47 (s, 2H), 4.24 (s, 2H), 4.16 (s, 3H), 3.99 - 3.90 (m, 2H).

UPLC-MS (ES⁺, Method 1): 3.89 min, m/z, 436.2 [M+H]⁺

### Example 96. 7-[(7-fluoro-1-methyl-indazol-5-yl)amino]-2-[2-oxo-2-(2S)-(trifluoromethyl)pyrrolidin-1-yl]ethyl]isoindolin-1-one.

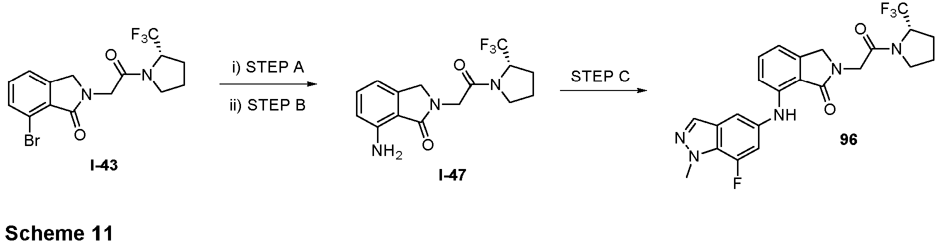

STEPS A and B. Intermediate I-47, 7-amino-2-[2-oxo-2-[(2S)-(trifluoromethyl)pyrrolidin-1-yl]ethyl]isoindolin-1-one, was prepared using the same procedure as outlined in scheme 2 for intermediate I-30, starting from I-43 in place of I-6.

UPLC-MS (ES⁺, Method 1): 3.45 min, m/z, 328.2 [M+H]⁺

STEP C. 7-[(7-Fluoro-1-methyl-indazol-5-yl)amino]-2-[2-oxo-2-[(2S)-(trifluoromethyl)pyrrolidin-1-yl]ethyl]isoindolin-1-one (Example 96). A solution of 5-bromo-7-fluoro-1-methyl-indazole (60 mg, 0.26 mmol), -amino-2-[2-oxo-2-[(2S)-(trifluoromethyl)pyrrolidin-1-yl]ethyl]isoindolin-1-one (94.3 mg, 0.28 mmol), Cs₂CO₃ (172.22mg, 0.52 mmol), Xantphos (30.58 mg, 0.052 mmol), and Pd₂(dba)₃ (24.20 mg, 0.026 mmol), in 1,4-dioxane (1.0 mL) was stirred at 100 °C under N₂ overnight. The mixture was diluted with EtOAc (10 mL) and washed with H₂O (8 mL x 3). The organic layers were washed with EtOAc (10 mL × 3), combined, dried over anhydrous Na₂SO₄ and concentrated to give of yellow solid. The mixture were purified by preparative TLC to give 7-[(7-fluoro-1-methyl-indazol-5-yl)amino]-2-[2-oxo-2-[(2S)-(trifluoromethyl)pyrrolidin-1-yl]ethyl]isoindolin-1-one (16 mg, 21% yield) as a yellow solid.

UPLC-MS (ES⁺, Method 1): 4.18 min, m/z 476.3 [M+H]⁺

¹H NMR (400 MHz, DMSO-d₆) δ (ppm) 8.64 (s, 1H), 8.03 (d, *J* = 2.4 Hz, 1H), 7.49 (d, *J* = 1.6 Hz, 1H), 7.39 (t, *J* = 7.8 Hz, 1H), 7.21 (dd, *J* = 13.3, 1.7 Hz, 1H), 7.09 (d, *J* = 8.2 Hz, 1H), 6.92 (d, *J* = 7.4 Hz, 1H), 5.08 - 4.72 (m, 1H), 4.60 - 4.36 (m, 4H), 4.16 (s, 3H), 3.70 - 3.63 (m, 2H), 2.07 - 2.00 (m, 4H).

### Example 101. 2-[7-[(1,3-dimethylpyrazolo[3,4-b]pyridin-5-yl)amino]-1-oxo-isoindolin-2-yl]-N-(2,2,2-trifluoroethyl)acetamide.

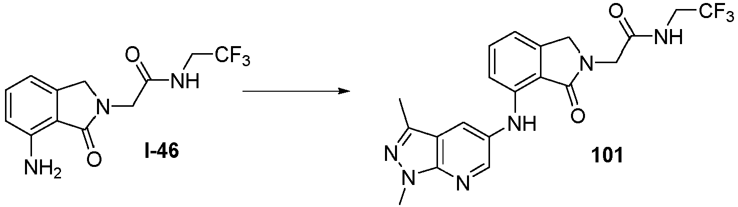

A mixture of 2-(7-amino-1-oxoisoindolin-2-yl)-N-(2,2,2-trifluoroethyl)acetamide (I-46) (80 mg, 0.28 mmol), 5-bromo-1,3-dimethyl-1H-pyrazolo[3,4-b]pyridine (75 mg, 0.33 mmol), Pd₂dba₃ (25.6 mg, 0.028 mmol), Xantphos (32.4 mg, 0.056mmol), Cs₂CO₃ (274 mg, 0.84 mmol) in 1,4-dioxane (3 mL) was stirred at 110 °C under an N₂ atmosphere overnight. The reaction was diluted with EtOAc (100 mL) and was washed with water (50 ml x 3). The combined organic layers were washed with brine (30 mL), dried over Na₂SO₄ before concentrating and purifying by silica gel chromatography (EtOAc/pet. Ether, 1/1) affording 2-[7-[(1,3-dimethylpyrazolo[3,4-b]pyridin-5-yl)amino]-1-oxo-isoindolin-2-yl]-N-(2,2,2-trifluoroethyl)acetamide (20 mg, 16% yield) as a white solid.

UPLC-MS (ES⁺, Method 1): 1.56 min, m/z 433.1 [M+H]⁺

¹H NMR (400 MHz, DMSO-d₆) δ (ppm) 8.81 (t, *J* = 6.3 Hz, 1H), 8.58 (s, 1H), 8.48 (d, *J* = 2.4 Hz, 1H), 8.13 (d, *J* = 2.4 Hz, 1H), 7.35 (t, *J* = 7.9 Hz, 1H), 6.92 - 6.86 (m, 2H), 4.48 (s, 2H), 4.25 (s, 2H), 3.98 - 3.90 (m, 5H), 2.48 (s, 3H).

### Example 102. 7-[(1,3-dimethylpyrazolo[3,4-b]pyridin-5-yl)amino]-2-[2-oxo[(2S)-(trifluoromethyl)pyrrolidin-1-yl]ethyl]isoindolin-1-one.

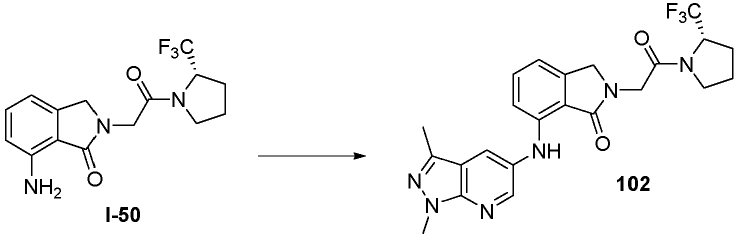

Intermediate I-50, 7-amino-2-[2-oxo-2-[(2S)-(trifluoromethyl)pyrrolidin-1-yl]ethyl]isoindolin-1-one was prepared by analogy with I-46 (scheme 10) replacing 2,2,2-trifluoroethan-1-amine in step B with (2S)-(trifluoromethyl)pyrrolidine.

UPLC-MS (ES⁺, Method 1): 3.45 min, m/z 328.2 [M+H]⁺

7-[(1,3-dimethylpyrazolo[3,4-b]pyridin-5-yl)amino]-2-[2-oxo-2-[(2S)-(trifluoromethyl)pyrrolidin-1-yl]ethyl]isoindolin-1-one (example 102) was then prepared using the same method as for example 101, replacing I-46 with I-50.

UPLC-MS (ES⁺, Method 1): 1.76 min, m/z 473.2 [M+H]⁺

¹H NMR (400 MHz, DMSO-*d*₆) δ (ppm) 8.55 (s, 1H), 8.46 (d, J = 2.4 Hz, 1H), 8.02 (d, J = 2.3 Hz, 1H), 7.33 (t, J = 7.8 Hz, 1H), 6.88 (dd, J = 11.9, 7.9 Hz, 2H), 5.36 - 4.31 (m, 5H), 4.00 (s, 3H), 3.70-3.60 (m, 2H), 2.49 (s, 3H), 2.12 - 2.09 (m, 4H).

### Example 105. N-methyl-2-[7-[(3-methyl-1H-pyrazolo[3,4-b]pyridin-5-yl)amino]-1-oxo-isoindolin-2-yl]-N-(2,2,2-trifluoroethyl)acetamide.

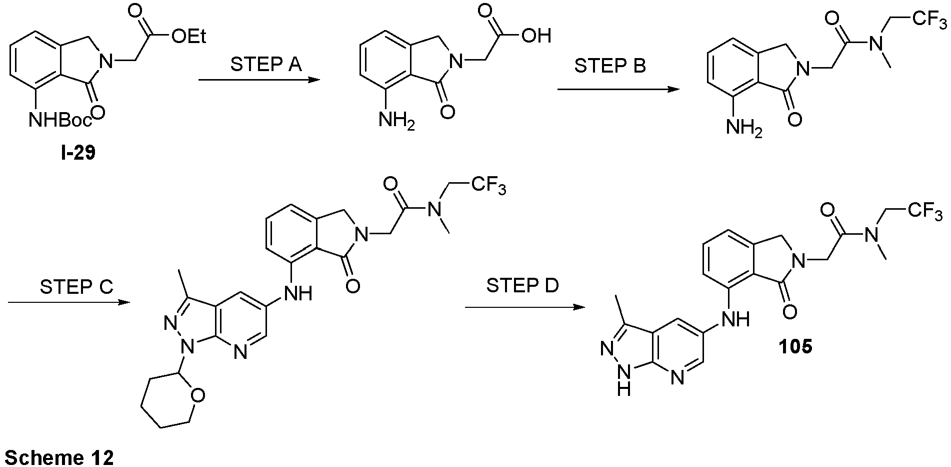

STEP A. 2-(7-amino-1-oxo-isoindolin-2-yl)acetic acid. A mixture of ethyl 2-[7-(tert-butoxycarbonylamino)-1-oxo-isoindolin-2-yl]acetate (6 g, 17.94 mmol) in DCM (10 mL) and HCl in dioxane (4M) (18 mL, 179.44 mmol was stirred at room temperature for 2 hours. The mixture was concentrated in vacuo affording 2-(7-amino-1-oxo-isoindolin-2-yl)acetic acid hydrochloride salt as a yellow solid (4.5 g, quantitative yield).

UPLC-MS (ES⁺, Method 2): 1.13 min, m/z 235.1 [M+H].

STEP B. 2-(7-amino-1-oxo-isoindolin-2-yl)-N-methyl-N-(2,2,2-trifluoroethyl)acetamide. A solution of 2-(7-amino-1-oxo-isoindolin-2-yl)acetic acid (400 mg, 1.94 mmol) HATU (1.1 g, 2.91 mmol DIEA (1.01 mL, 5.82 mmol) and 2,2,2-trifluoro-N-methyl-ethanamine hydrochloride (0.26 mL, 2.13 mmol) in DCM (5 mL) was stirred at 25 °C for 5 hours. The mixture was purified by reverse phase column chromatography on C-18 modified silca gel eluting with MeCN and water with 0.1% TFA as an additive affording 2-(7-amino-1-oxo-isoindolin-2-yl)-N-methyl-N-(2,2,2-trifluoroethyl)acetamide as a whith solid (340 mg, 58% yield).

UPLC-MS (ES⁺, Method 1): 3.34 min, m/z 302.2 [M+H].

STEP C. N-methyl-2-[7-[(3-methyl-1-tetrahydropyran-2-yl-pyrazolo[3,4-b]pyridin-5-yl)amino]-1-oxo-isoindolin-2-yl]-N-(2,2,2-trifluoroethyl)acetamide. A solution of 2-(7-amino-1-oxo-isoindolin-2-yl)-N-methyl-N-(2,2,2-trifluoroethyl)acetamide (60 mg, 0.2 mmol), 5-bromo-3-methyl-1-tetrahydropyran-2-yl-pyrazolo[3,4-b]pyridine (71 mg, 0.24 mmol) K₂CO₃ (55 mg, 0.4 mmol), Pd₂(dba)₃ (18 mg, 0.0 2mmol) and t-BuBrettphos (19 mg, 0.04 mmol) in t-butanol (1m L) was strried at 110°C in a sealed tube for 2 hours. The mixture was filtered through celite and the filter-cake washed with DCM. The combined orgaincs were dried over Na₂SO₄ and concentrated to an oily residue which was purified by preparative TLC, eluting with DCM in MeOH (20:1) to afford N-methyl-2-[7-[(3-methyl-1-tetrahydropyran-2-yl-pyrazolo[3,4-b]pyridin-5-yl)amino]-1-oxo-isoindolin-2-yl]-N-(2,2,2-trifluoroethyl)acetamide as a yellow solid (35 mg, 34% yield).

UPLC-MS (ES⁺, Method 1): 4.08 min, m/z 517.4 [M+H].

STEP D. N-methyl-2-[7-[(3-methyl-1H-pyrazolo[3,4-b]pyridin-5-yl)amino]-1-oxo-isoindolin-2-yl]-N-(2,2,2-trifluoroethyl)acetamide (example 105).

To a solution of N-methyl-2-[7-[(3-methyl-1-tetrahydropyran-2-yl-pyrazolo[3,4-b]pyridin-5-yl)amino]-1-oxo-isoindolin-2-yl]-N-(2,2,2-trifluoroethyl)acetamide (35 mg, 0.07 mmol) in DCM (1 mL) was added 4M HCl in dioxane (0.5 mL). The mixture was strried at 25°C for 2 hrs,The mixture was concentrated and purified by preparative-TLC (DCM and MeOH 20:1) to give a yellow solid analysed as N-methyl-2-[7-[(3-methyl-1H-pyrazolo[3,4-b]pyridin-5-yl)amino]-1-oxo-isoindolin-2-yl]-N-(2,2,2-trifluoroethyl)acetamide (example 105) (23 mg, 80% yield).

UPLC-MS (ES⁺, Method 1): 3.45 min, m/z 433.3 [M+H].

¹H NMR (400 MHz, DMSO-d₆) δ (ppm) 13.19 (s, 1H), 8.56 (d, J = 2.8 Hz, 1H), 8.43 (d, J = 2.4 Hz, 1H), 8.13 (d, J = 2.5 Hz, 1H), 7.35 (t, J = 7.8 Hz, 1H), 6.92 - 6.85 (m, 2H), 4.51 (d, J = 20.5 Hz, 2H), 4.44 (d, J = 2.7 Hz, 2H), 4.20 (q, J = 9.8 Hz, 2H), 3.19 (s, 3H), 2.48 (s, 3H).

### DDR1 and DDR2 Biochemical assay method

The capacity of compounds to bind to DDR1 and DDR2 was quantified using a LanthaScreen Eu Kinase Binding Assay. Recombinant human DDR1 (2.5nM; aa440-876 containing a GST tag) and DDR2 (1.75nM; aa427-855 containing a GST tag) were diluted in assay buffer (50mM HEPES pH7.3, 10mM MgCl2, 1mM EGTA and 0.01% Tween) with various concentrations of compound in a 384-well plate and a volume of 5uL. After a 30-minute incubation at room temperature, 2.5uL of Eu-anti GST antibody (diluted to 1 nM in assay buffer) plus Kinase Tracer 178 (diluted in assay buffer to 5nM for DDR1 and 10nM for DDR2) were added to the plate. Following 60-minute incubation at room temperature, time-resolved fluorescence was measured on a BMG Labtech PHERAstar plate reader. DMSO (1%) and reference compound (1µM) were used to generate the Max and Min assay signals, respectively. Data was analysed using a four-parameter logistic model to calculate IC₅₀ values, with at least two independent replicates were performed for each compound.

### Biological activity values

The following table shows the pIC₅₀ values for the above examples against the DDR1 and DDR2 kinases (A: pIC₅₀ > 8; B: 8 ≥ pIC₅₀ > 7; C: 7 ≥ pIC₅₀ > 6; D: pIC₅₀ ≤ 6; ND: not determined).

**Table 12**

| Example | DDR1 pIC₅₀ | DDR2 pIC₅₀ |
|---|---|---|
| 1 | A | B |
| 2 | A | A |
| 3 | A | A |
| 4 | A | A |
| 5 | A | B |
| 6 | A | A |
| 7 | C | D |
| 8 | B | B |
| 9 | B | C |
| 10 | A | B |
| 11 | A | A |
| 12 | A | B |
| 13 | A | B |
| 14 | A | A |
| 15 | B | B |
| 16 | A | A |
| 17 | A | A |
| 18 | B | C |
| 19 | C | C |
| 20 | A | B |
| 21 | A | B |
| 22 | B | D |
| 23 | B | B |
| 24 | A | B |
| 25 | ND | ND |
| 26 | B | B |
| 27 | A | A |
| 28 | A | A |
| 29 | A | A |
| 30 | A | B |
| 31 | A | B |
| 32 | A | A |
| 33 | A | A |
| 34 | B | B |
| 35 | ND | ND |
| 36 | A | A |
| 37 | A | B |
| 38 | A | B |
| 39 | A | B |
| 40 | A | A |
| 41 | A | A |
| 42 | A | A |
| 43 | A | B |
| 44 | B | C |
| 45 | A | B |
| 46 | A | A |
| 47 | A | B |
| 48 | A | B |
| 49 | A | A |
| 50 | A | A |
| 51 | A | A |
| 52 | B | C |
| 53 | A | B |
| 54 | C | C |
| 55 | C | C |
| 56 | B | B |
| 57 | D | D |
| 58 | A | B |
| 59 | B | C |
| 60 | A | A |
| 61 | C | D |
| 62 | A | A |
| 63 | A | A |
| 64 | A | A |
| 65 | C | D |
| 66 | A | A |
| 67 | A | A |
| 68 | B | C |
| 69 | B | C |
| 70 | C | D |
| 71 | B | B |
| 73 | A | B |
| 74 | A | A |
| 75 | B | C |
| 76 | B | B |
| 77 | B | B |
| 78 | B | C |
| 79 | B | D |
| 80 | C | C |
| 81 | B | C |
| 82 | B | D |
| 83 | B | B |
| 84 | B | C |
| 85 | B | B |
| 86 | B | B |
| 88 | B | B |
| 89 | B | B |
| 90 | B | C |
| 91 | B | ND |
| 92 | B | C |
| 93 | A | B |
| 94 | ND | ND |
| 95 | B | B |
| 96 | A | A |
| 97 | B | C |
| 98 | ND | ND |
| 99 | B | B |
| 100 | ND | ND |
| 101 | D | D |
| 102 | B | C |
| 103 | ND | ND |
| 104 | ND | ND |
| 105 | ND | ND |

## Claims

1. A compound of formula (I) or a pharmaceutically acceptable salt thereof: 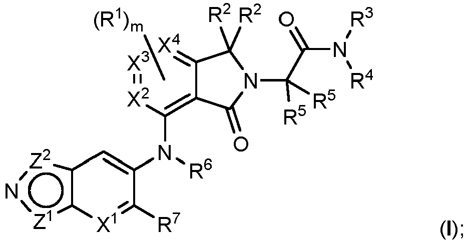 wherein
Z¹ and Z² are each selected from -CR^{8a}- and -NR^{8b}-, wherein one of Z¹ and Z² is -CR^{8a}- and the other is -NR^{8b}-; and wherein the ring comprising Z¹ and Z² is a pyrazole;
X¹ is independently selected from CR^{7a} and N;
X², X³ and X⁴ are each independently selected from carbon and nitrogen, wherein at least one of X², X³ and X⁴ is carbon;
R¹ is independently selected at each occurrence from halo, nitro, cyano, NR⁹R¹⁰, OR¹¹, SR⁹, SO₂NR⁹R⁹, SO₂R⁹, CO₂R⁹, C(O)R⁹, CONR⁹R⁹, C₁-C₄-alkyl, C₁-C₄-alkyl substituted with NR⁹R¹⁰, C₁-C₄-alkyl substituted with OR¹¹, C₂-C₄-alkenyl, C₂-C₄-alkynyl, C₁-C₄-haloalkyl and cyclopropyl;
R² is independently selected at each occurrence from H, fluoro, C₁-C₄-alkyl, C₁-C₄-haloalkyl and cyclopropyl, or the two R² groups and the carbon atom to which they are attached together form a C₃-C₆-cycloalkyl ring;
R³ is independently selected from H and C₁-C₄-alkyl;
R⁴ is independently selected from C₁-C₆-alkyl, C₁-C₆-haloalkyl, C₀-C₄-alkylene-R^{4a}; wherein R^{4a} is independently selected from: C₃-C₈-cycloalkyl, phenyl, 5-, 6-, 9- or 10-membered heteroaryl and 4- to 10-membered heterocycloalkyl; wherein said heterocycloalkyl or heteroaryl group may be monocyclic or bicyclic; wherein said cycloalkyl or heterocycloalkyl group is optionally substituted with a single R¹² group and/or from 1 to 4 R¹³ groups and wherein said phenyl or heteroaryl group is optionally substituted with a single R¹² group and/or from 1 to 3 R¹⁴ groups;
or R³ and R⁴, together with the nitrogen atom to which they are attached together form a 4- to 10-membered heterocycloalkyl group or a 5-, or 9-membered heteroaryl group; wherein said heterocycloalkyl or heteroaryl group may be monocyclic or bicyclic; wherein said heterocycloalkyl group is optionally substituted with a single R¹² group and/or from 1 to 4 R¹³ groups and wherein said heteroaryl group is optionally substituted with a single R¹² group and/or from 1 to 3 R¹⁴ groups;
R⁵ is independently at each occurrence selected from H, halo and C₁-C₄-alkyl, or the two R⁵ groups and the carbon atom to which they are attached may together form a C₃-C₆ cycloalkyl ring;
R⁶ is independently selected from H, C₁-C₄-alkyl, C₁-C₄-haloalkyl and cyclopropyl;
R⁷ and R^{7a} are each independently selected from H, halo, nitro, cyano, NR⁹R¹⁰, OR¹¹, SR⁹, SO₂NR⁹R⁹, SO₂R⁹, CO₂R⁹, C(O)R⁹, CONR⁹R⁹, C₁-C₄-alkyl, C₁-C₄-alkyl substituted with NR⁹R¹⁰, C₁-C₄-alkyl substituted with OR¹¹, C₂-C₄-alkenyl, C₂-C₄-alkynyl, C₁-C₄-haloalkyl and cyclopropyl;
R^{8a} is independently selected from H, halo, nitro, cyano, NR⁹R¹⁰, OR¹¹, SR⁹, SO₂NR⁹R⁹, SO₂R⁹, CO₂R⁹, C(O)R⁹, CONR⁹R⁹, C₁-C₄-alkyl, C₁-C₄-alkyl substituted with NR⁹R¹⁰, C₁-C₄-alkyl substituted with OR¹¹, C₂-C₄-alkenyl, C₂-C₄-alkynyl, C₁-C₄-haloalkyl and C₀-C₄-alkylene-R^{8c};
R^{8b} is independently selected from H, C₁-C₄-alkyl, C₂-C₄-alkyl substituted with NR⁹R¹⁰, C₂-C₄-alkyl substituted with OR¹¹, C₃-C₄-alkenyl, C₃-C₄-alkynyl, C₁-C₄-haloalkyl and C₀-C₄-alkylene-R^{8c};
R^{8c} is independently selected from C₃-C₆-cycloalkyl and 3- to 7-membered heterocycloalkyl; wherein said heterocycloalkyl group is attached to the C₀-C₄-alkylene via a carbon atom in the heterocycloalkyl ring; wherein said cycloalkyl or heterocycloalkyl group is optionally substituted with from 1 to 4 R¹³ groups;
R⁹ is independently at each occurrence selected from H and C₁-C₄-alkyl; or two R⁹ groups, together with the nitrogen atom to which they are attached together form a C₅-C₈-heterocycloalkyl group optionally substituted with from 0 to 4 R¹³ groups;
R¹⁰ is independently at each occurrence selected from H, C₁-C₄-alkyl, C(O)-C₁-C₄-alkyl and S(O)₂-C₁-C₄-alkyl; or R⁹ and R¹⁰, together with the nitrogen atom to which they are attached together form a C₅-C₈-heterocycloalkyl group optionally substituted with from 0 to 4 R¹³ groups;
R¹¹ is independently at each occurrence selected from **H,** C₁-C₄-alkyl, C(O)-C₁-C₄-alkyl and C₁-C₄-haloalkyl;
R¹² is independently selected from C₃-C₆-cycloalkyl, phenyl, 5- or 6- membered heteroaryl and 3- to 6-membered-heterocycloalkyl; wherein said cycloalkyl or heterocycloalkyl group is optionally substituted with from 1 to 4 R¹³ groups and wherein said phenyl or heteroaryl group is optionally substituted with from 1 to 3 R¹⁴ groups;
R¹³ is independently at each occurrence selected from =O, halo, nitro, cyano, NR⁸R⁹, OR¹⁴, SR⁸, SO₂NR⁸R⁸, CO₂R⁸, C(O)R⁸, CONR⁸R⁸, C₁-C₄-alkyl, C₁-C₄-alkyl substituted with OR¹¹, C₁-C₄-alkyl substituted with NR⁹R¹⁰, C₂-C₄-alkenyl, C₂-C₄-alkynyl, C₁-C₄-haloalkyl, C₆-C₁₀-aryl, and C₃-C₆-cycloalkyl;
R¹⁴ is independently at each occurrence selected from halo, nitro, cyano, NR⁸R⁹, OR¹⁰, SR⁸, SO₂R⁸, SO₂NR⁸R⁸, CO₂R⁸, C(O)R⁸, CONR⁸R⁸, C₁-C₄-alkyl, C₂-C₄-alkenyl, C₂-C₄-alkynyl, C₁-C₄-haloalkyl, C₁-C₄--alkyl substituted with OR¹¹, C₁-C₄-alkyl substituted with NR⁸R⁹ and cyclopropyl;
m is an integer selected from 0, 1, 2 and 3;
wherein any of the aforementioned alkyl, alkylene or cyclopropyl groups is optionally substituted, where chemically possible, by 1 to 5 substituents which are each independently at each occurrence selected from the group consisting of: halo, oxo, fluoro, nitro, cyano, NR^{a}R^{b}, OR^{a}, SR^{a}, CO₂R^{a}, C(O)R^{a}, CONR^{a}R^{a}, C₁-C₄-alkyl, C₁-C₄-haloalkyl and cyclopropyl; wherein R^{a} is independently at each occurrence selected from H, C₁-C₄-alkyl and C₁-C₄-haloalkyl; and R^{b} is independently at each occurrence selected from H, C₁-C₄-alkyl, C(O)-C₁-C₄-alkyl and S(O)₂-C₁-C₄-alkyl.

2. A compound of claim 1 wherein Z¹ is NR^{8b} and Z² is CH; or
wherein Z¹ is CR^{8a} and Z² is NH.

3. A compound of claim 1 or claim 2, wherein m is 0.

4. A compound of any one of claims 1 to 3, wherein R² is at each occurrence independently selected from C₁-C₄-alkyl and H.

5. A compound of any one of claims 1 to 4, wherein R⁵ is at each occurrence H; and/or
wherein R⁶ is H; and/or
wherein R⁷ is H.

6. A compound of any one of claims 1 to 5, wherein X¹ is N.

7. A compound of any one of claims 1 to 5, wherein X¹ is CR^{7a}; optionally
wherein R^{7a} is H; or
wherein R^{7a} is independently selected from halo, nitro, cyano, OR¹¹, CO₂R⁹, CONR⁹R⁹, C₁-C₄-alkyl, C₁-C₄-alkyl substituted with NR⁹R¹⁰, C₁-C₄-alkyl substituted with OR¹¹, C₁-C₄-haloalkyl and cyclopropyl.

8. A compound of any one of claims 1 to 7, wherein X², X³ and X⁴ are each carbon.

9. A compound of any one of claims 1 to 8, wherein R³ is H and R⁴ is selected from C₁-C₆-alkyl, C₁-C₆-haloalkyl and C₀-C₄-alkylene-R^{4a}; optionally
wherein R⁴ is selected from C₁-C₆-alkyl and C₁-C₆-haloalkyl; or
wherein R⁴ is C₀-C₄-alkylene-R^{4a}; or
wherein R⁴ is R^{4a}.

10. A compound of claim 9, wherein R^{4a} is selected from C₃-C₈-cycloalkyl and 4- to 10-membered heterocycloalkyl, wherein said cycloalkyl or heterocycloalkyl group is optionally substituted with from 1 to 4 R¹³ groups; or
wherein R^{4a} is independently selected from: phenyl and 5- or 6- membered heteroaryl; wherein said phenyl or heteroaryl group is optionally substituted with a single R¹² group and/or from 1 to 3 R¹⁴ groups.

11. A compound of any one of claims 1 to 10, wherein R³ and R⁴, together with the nitrogen atom to which they are attached together form a monocyclic 4- to 7- membered heterocycloalkyl group optionally substituted with from 1 to 4 R¹³ groups; or
wherein NR³R⁴ has the formula 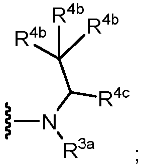
wherein
R^{4b} is at each occurence selected from H and F; wherein at least one R^{4b} group is F;
R^{3a} is independently selected from H and C₁-C₄-alkyl;
R^{4c} is independently selected from H, C₁-C₄-alkyl and C₄-C₆-cycloalkyl; or
R^{3a} and R^{4c}, together with the carbon and nitrogen to which they are attached, form a 4- to 6-membered heterocycloalkyl group.

12. A compound of claim 1 wherein the compound of formula (I) is selected from:

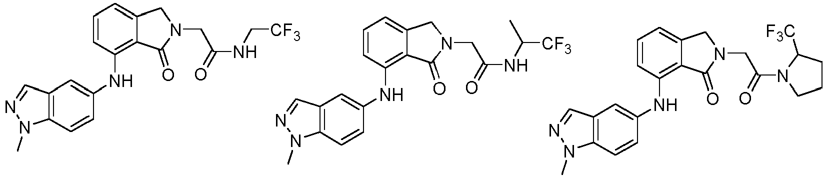

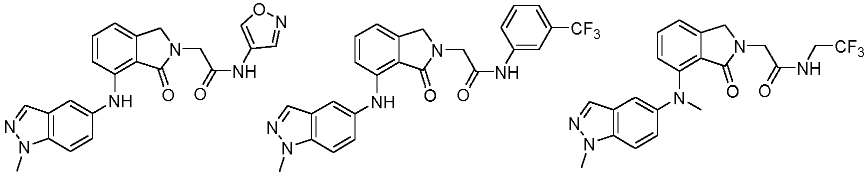

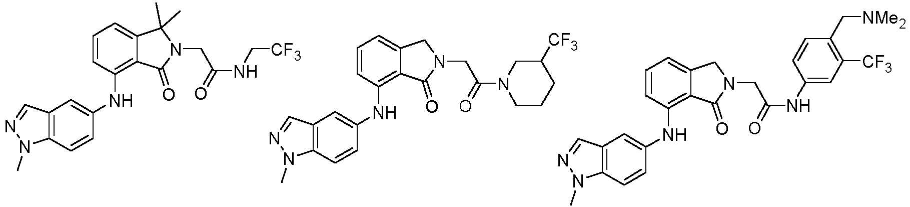

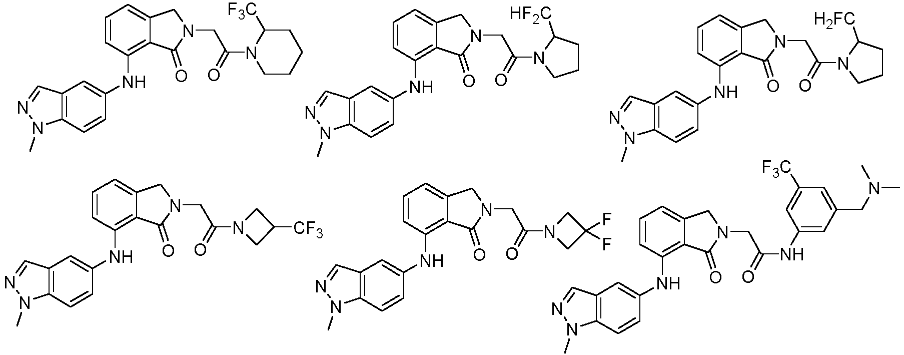

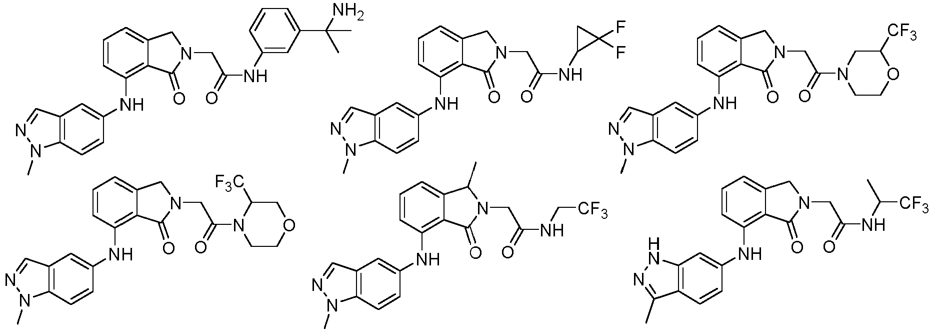

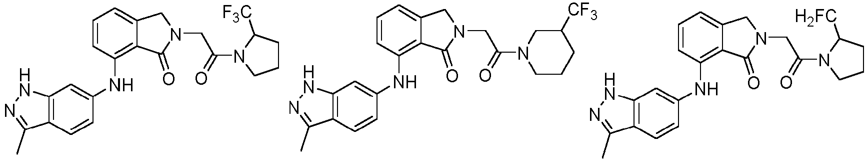

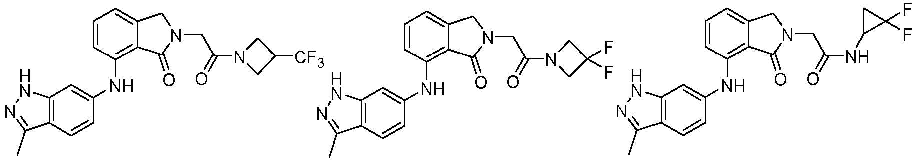

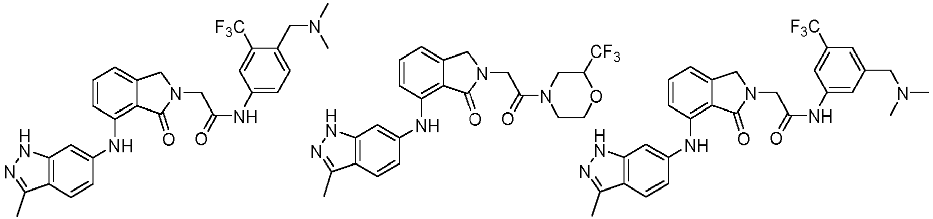

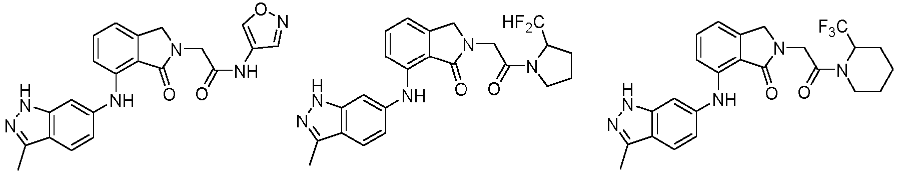

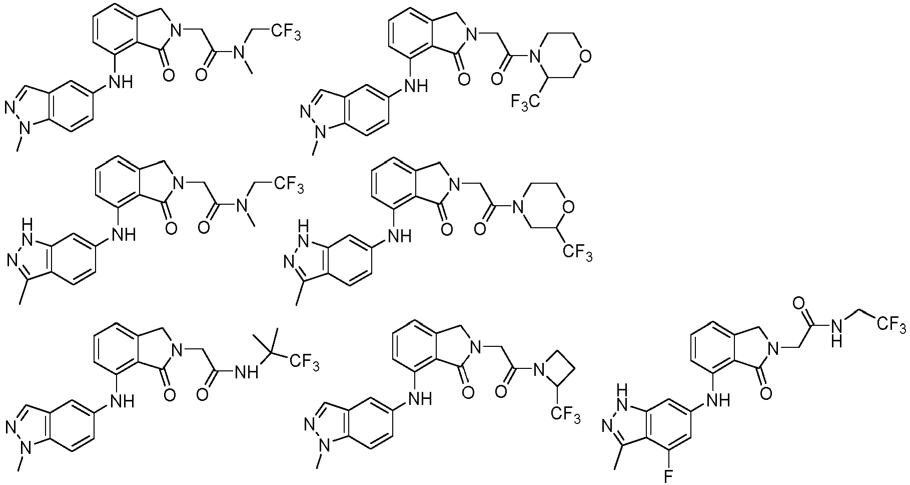

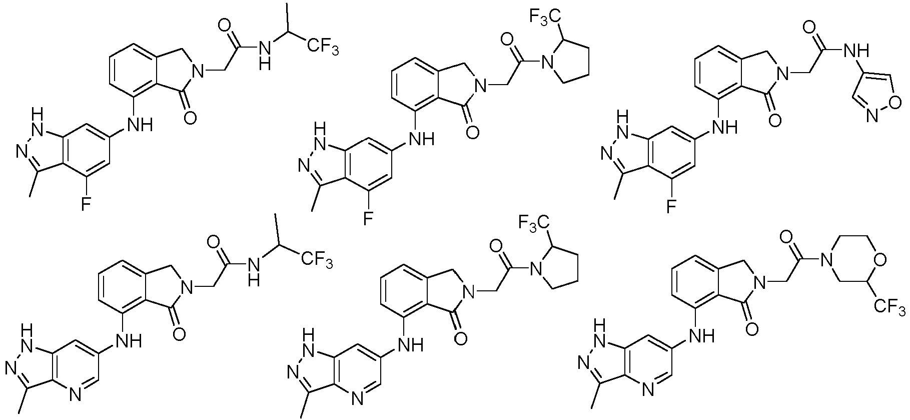

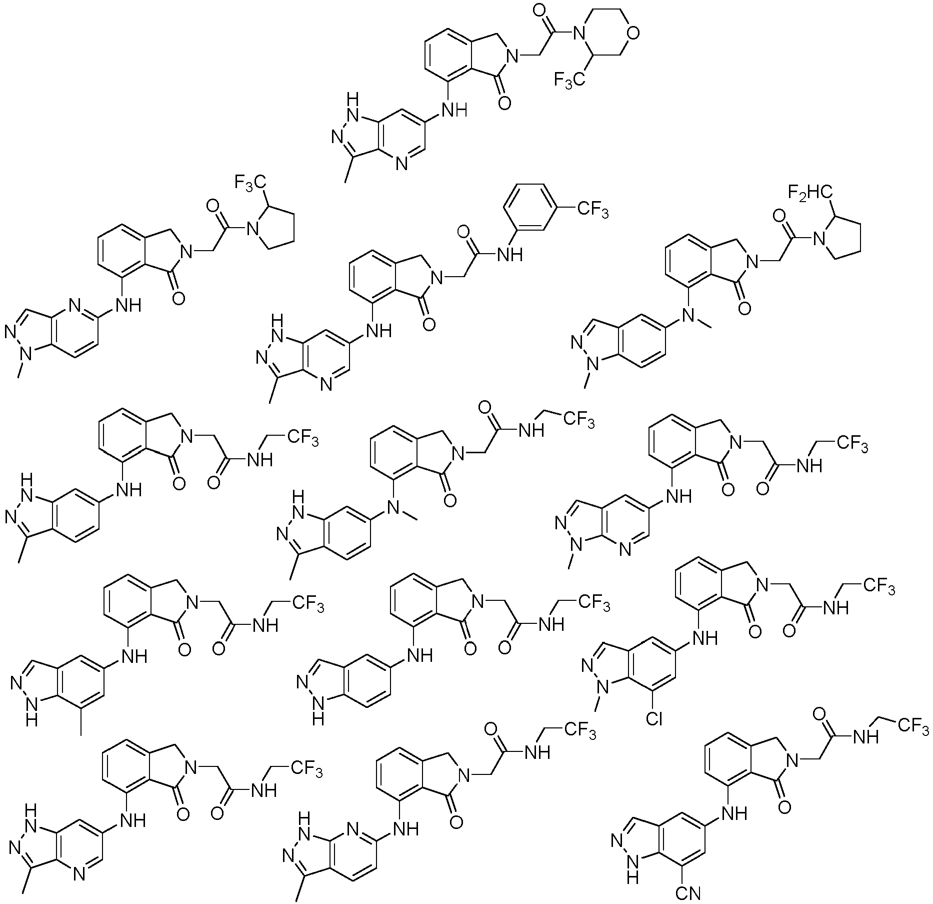

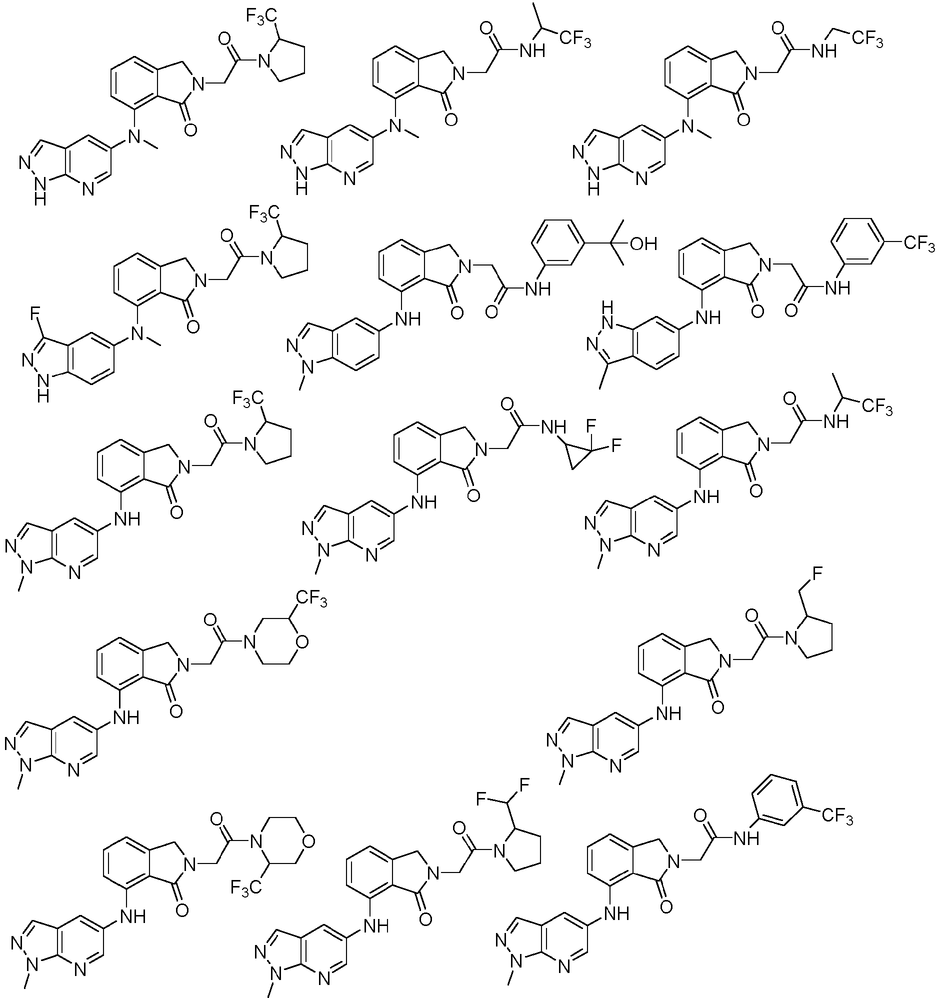

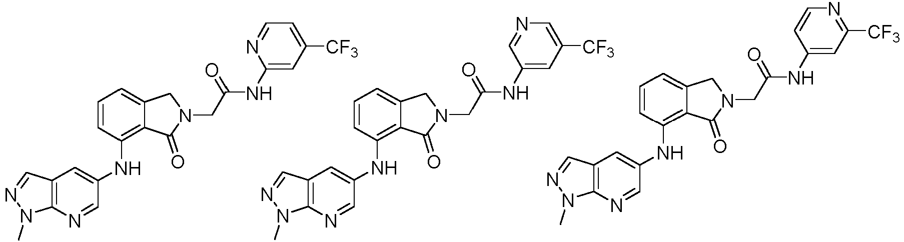

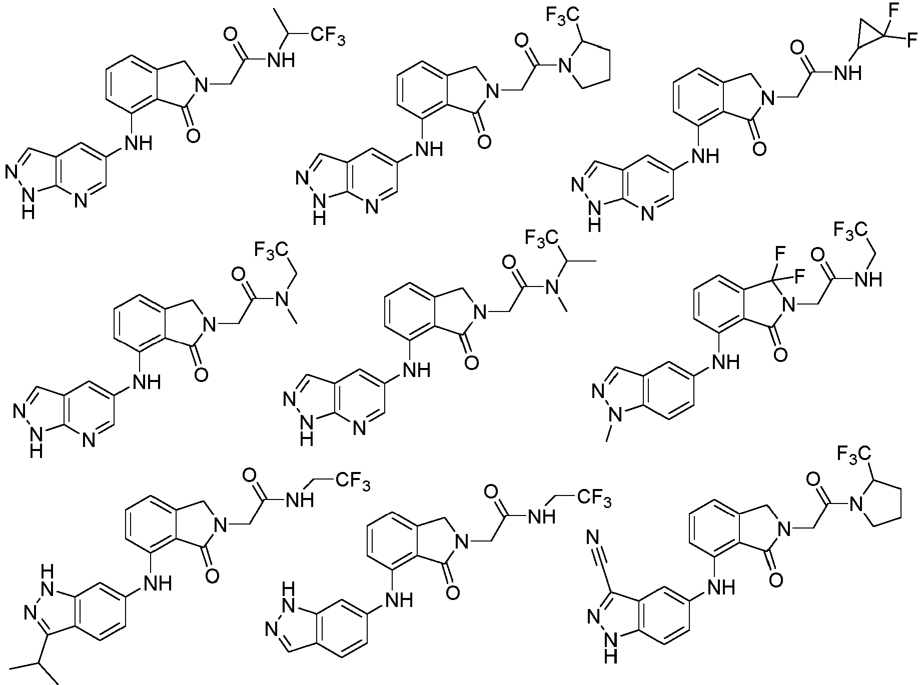

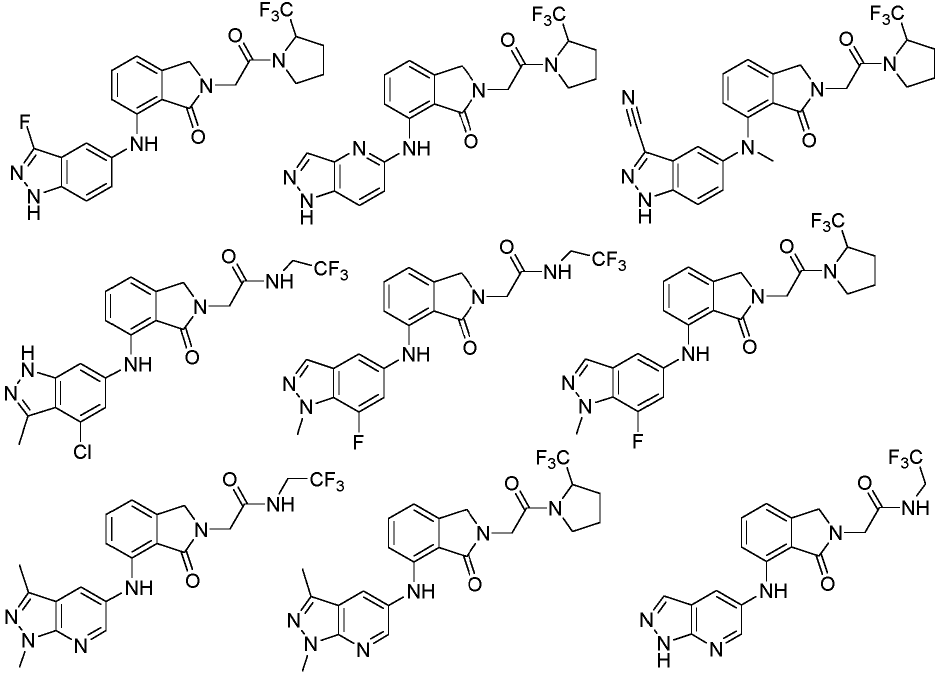

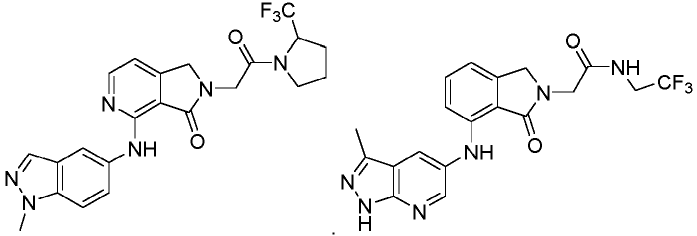

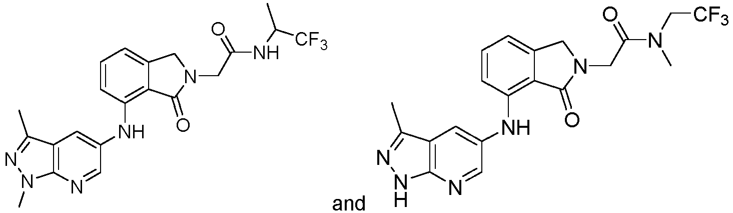

13. A pharmaceutical formulation comprising a compound of any one of claims 1 to 12 and a pharmaceutically acceptable excipient.

14. A compound of any one of claims 1 to 12 for use as a medicament.

15. A compound of any one of claims 1 to 12 for use in treating a disease or disorder selected from renal conditions, liver conditions, inflammatory conditions, cardiovascular conditions, acute and chronic organ transplant rejection, fibrotic diseases and cancer.

## Patentansprüche

1. Verbindung der Formel (I) oder pharmazeutisch verträgliches Salz davon: 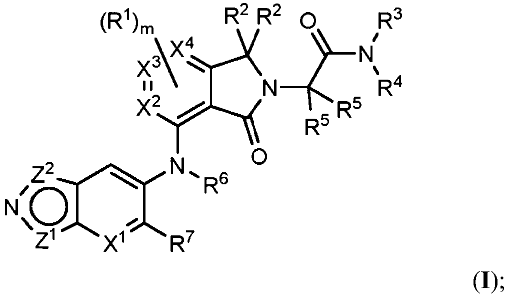 wobei
Z¹ und Z² jeweils ausgewählt sind aus -CR^{8a}- und -NR^{8b}-, wobei eines von Z¹ und Z² - CR^{8a}- ist und das andere -NR^{8b}- ist; und wobei der Ring, der Z¹ und Z² umfasst, ein Pyrazol ist;
X¹ unabhängig ausgewählt ist aus CR^{7a} und N;
X², X³ und X⁴ jeweils unabhängig ausgewählt sind aus Kohlenstoff und Stickstoff, wobei mindestens eines von X², X³ und X⁴ Kohlenstoff ist;
R¹ unabhängig bei jedem Vorkommen ausgewählt ist aus Halo, Nitro, Cyano, NR⁹R¹⁰, OR¹¹, SR⁹, SO₂NR⁹R⁹, SO₂R⁹, CO₂R⁹, C(O)R⁹, CONR⁹R⁹, C₁-C₄-Alkyl, C₁-C₄-Alkyl substituiert mit NR⁹R¹⁰, C₁-C₄-Alkyl substituiert mit OR¹¹, C₂-C₄-Alkenyl, C₂-C₄-Alkinyl, C₁-C₄-Haloalkyl und Cyclopropyl;
R² unabhängig bei jedem Vorkommen ausgewählt ist aus H, Fluor, C₁-C₄-Alkyl, C₁-C₄-Haloalkyl und Cyclopropyl oder die zwei R²-Gruppen und das Kohlenstoffatom, an das sie gebunden sind, zusammen einen C₃-C₆-Cycloalkylring bilden;
R³ unabhängig ausgewählt ist aus H und C₁-C₄-Alkyl;
R⁴ unabhängig ausgewählt ist aus C₁-C₆-Alkyl, C₁-C₆-Haloalkyl, C₀-C₄-Alkylen-R^{4a}; wobei R^{4a} unabhängig ausgewählt ist aus: C₃-C₈-Cycloalkyl, Phenyl, 5-, 6-, 9- oder 10-gliedrigem Heteroaryl und 4- bis 10-gliedrigem Heterocycloalkyl; wobei die Heterocycloalkyl- oder Heteroarylgruppe monocyclisch oder bicyclisch sein kann; wobei die Cycloalkyl- oder Heterocycloalkylgruppe optional mit einer einzelnen R¹²-Gruppe und/oder von 1 bis 4 R¹³-Gruppen substituiert ist und wobei die Phenyl- oder Heteroarylgruppe optional mit einer einzelnen R¹²-Gruppe und/oder von 1 bis 3 R¹⁴-Gruppen substituiert ist;
oder R³ und R⁴ zusammen mit dem Stickstoffatom, an das sie gebunden sind, zusammen eine 4- bis 10-gliedrige Heterocycloalkylgruppe oder eine 5- oder 9-gliedrige Heteroarylgruppe bilden; wobei die Heterocycloalkyl- oder Heteroarylgruppe monocyclisch oder bicyclisch sein kann; wobei die Heterocycloalkylgruppe optional mit einer einzelnen R¹²-Gruppe und/oder von 1 bis 4 R¹³-Gruppen substituiert ist und wobei die Heteroarylgruppe optional mit einer einzelnen R¹²-Gruppe und/oder von 1 bis 3 R¹⁴-Gruppen substituiert ist;
R⁵ unabhängig bei jedem Vorkommen ausgewählt ist aus H, Halo und C₁-C₄-Alkyl oder die zwei R⁵-Gruppen und das Kohlenstoffatom, an das sie gebunden sind, zusammen einen C₃-C₆-Cycloalkylring bilden können;
R⁶ unabhängig ausgewählt ist aus H, C₁-C₄-Alkyl, C₁-C₄-Haloalkyl und Cyclopropyl;
R⁷ und R^{7a} jeweils unabhängig ausgewählt sind aus H, Halo, Nitro, Cyano, NR⁹R¹⁰, OR¹¹, SR⁹, SO₂NR⁹R⁹, SO₂R⁹, CO₂R⁹, C(O)R⁹, CONR⁹R⁹, C₁-C₄-Alkyl, C₁-C₄-Alkyl substituiert mit NR⁹R¹⁰, C₁-C₄-Alkyl substituiert mit OR¹¹, C₂-C₄-Alkenyl, C₂-C₄-Alkinyl, C₁-C₄-Haloalkyl und Cyclopropyl;
R^{8a} unabhängig ausgewählt ist aus H, Halo, Nitro, Cyano, NR⁹R¹⁰, OR¹¹, SR⁹, SO₂NR⁹R⁹, SO₂R⁹, CO₂R⁹, C(O)R⁹, CONR⁹R⁹, C₁-C₄-Alkyl, C₁-C₄-Alkyl substituiert mit NR⁹R¹⁰, C₁-C₄-Alkyl substituiert mit OR¹¹, C₂-C₄-Alkenyl, C₂-C₄-Alkinyl, C₁-C₄-Haloalkyl und C₀-C₄-Alkylen-R^{8c};
R^{8b} unabhängig ausgewählt ist aus H, C₁-C₄-Alkyl, C₂-C₄-Alkyl substituiert mit NR⁹R¹⁰, C₂-C₄-Alkyl substituiert mit OR¹¹, C₃-C₄-Alkenyl, C₃-C₄-Alkinyl, C₁-C₄-Haloalkyl und C₀-C₄-Alkylen-R ^{8c};
R^{8c} unabhängig ausgewählt ist aus C₃-C₆-Cycloalkyl und 3- bis 7-gliedrigem Heterocycloalkyl; wobei die Heterocycloalkylgruppe über ein Kohlenstoffatom in dem Heterocycloalkylring an das C₀-C₄-Alkylen gebunden ist; wobei die Cycloalkyl- oder Heterocycloalkylgruppe optional mit von 1 bis 4 R¹³-Gruppen substituiert ist;
R⁹ unabhängig bei jedem Vorkommen ausgewählt ist aus H und C₁-C₄-Alkyl; oder zwei R⁹-Gruppen, zusammen mit dem Stickstoffatom, an das sie gebunden sind, zusammen eine C₅-C₈-Heterocycloalkylgruppe bilden, die optional mit von 0 bis 4 R¹³-Gruppen substituiert ist;
R¹⁰ unabhängig bei jedem Vorkommen ausgewählt ist aus H, C₁-C₄-Alkyl, C(O)-C₁-C₄-Alkyl und S(O)₂-C₁-C₄-Alkyl; oder R⁹ und R¹⁰ zusammen mit dem Stickstoffatom, an das sie gebunden sind, zusammen eine C₅- C₈-Heterocycloalkylgruppe bilden, die optional mit von 0 bis 4 R¹³-Gruppen substituiert ist;
R¹¹ unabhängig bei jedem Vorkommen ausgewählt ist aus H, C₁-C₄-Alkyl, C(O)-C₁-C₄-Alkyl und C₁-C₄-Haloalkyl;
R¹² unabhängig ausgewählt ist aus C₃-C₆-Cycloalkyl, Phenyl, 5- oder 6-gliedrigem Heteroaryl und 3- bis 6-gliedrigem Heterocycloalkyl; wobei die Cycloalkyl- oder Heterocycloalkylgruppe optional mit von 1 bis 4 R¹³-Gruppen substituiert ist und wobei die Phenyl- oder Heteroarylgruppe optional mit von 1 bis 3 R¹⁴-Gruppen substituiert ist;
R¹³ ist unabhängig bei jedem Vorkommen ausgewählt aus =O, Halo, Nitro, Cyano, NR⁸R⁹, OR¹⁴, SR⁸, SO₂NR⁸R⁸, CO₂R⁸, C(O)R⁸, CONR⁸R⁸, C₁-C₄-Alkyl, C₁-C₄-Alkyl substituiert mit OR¹¹, C₁-C₄-Alkyl substituiert mit NR⁹R¹⁰, C₂-C₄-Alkenyl, C₂-C₄-Alkinyl, C₁-C₄-Haloalkyl, C₆-C₁₀-Aryl und C₃-C₆-Cycloalkyl;
R¹⁴ ist unabhängig bei jedem Vorkommen ausgewählt aus Halo, Nitro, Cyano, NR⁸R⁹, OR¹⁰, SR⁸, SO₂R⁸, SO₂NR⁸R⁸, CO₂R⁸, C(O)R⁸, CONR⁸R⁸, C₁-C₄-Alkyl, C₂-C₄-Alkenyl, C₂-C₄-Alkinyl, C₁-C₄-Haloalkyl, C₁-C₄-Alkyl substituiert mit OR¹¹, C₁-C₄-Alkyl substituiert mit NR⁸R⁹ und Cyclopropyl;
m eine ganze Zahl ausgewählt aus 0, 1, 2 und 3 ist;
wobei eine beliebige der vorgenannten Alkyl-, Alkylen- oder Cyclopropylgruppen optional, falls chemisch möglich, durch 1 bis 5 Substituenten substituiert ist, die jeweils unabhängig bei jedem Vorkommen ausgewählt sind aus der Gruppe bestehend aus: Halo, Oxo, Fluor, Nitro, Cyano, NR^{a}R^{b}, OR^{a}, SR^{a}, CO₂R^{a}, C(O)R^{a}, CONR^{a}R^{a}, C₁-C₄-Alkyl, C₁-C₄-Haloalkyl und Cyclopropyl; wobei R^{a} unabhängig bei jedem Vorkommen ausgewählt ist aus H, C₁-C₄-Alkyl und C₁-C₄-Haloalkyl; und R^{b} unabhängig bei jedem Vorkommen ausgewählt ist aus H, C₁-C₄-Alkyl, C(O)-C₁-C₄-Alkyl und S(O)₂-C₁-C₄-Alkyl.

2. Verbindung nach Anspruch 1, wobei Z¹ NR^{8b} ist und Z² CH ist; oder
wobei Z¹ CR^{8a} ist und Z² NH ist.

3. Verbindung nach Anspruch 1 oder Anspruch 2, wobei m 0 ist.

4. Verbindung nach einem der Ansprüche 1 bis 3, wobei R² bei jedem Vorkommen unabhängig ausgewählt ist aus C₁-C₄-Alkyl und H.

5. Verbindung nach einem der Ansprüche 1 bis 4, wobei R⁵ bei jedem Vorkommen H ist; und/oder
wobei R⁶ H ist; und/oder
wobei R⁷ H ist.

6. Verbindung nach einem der Ansprüche 1 bis 5, wobei X¹ N ist.

7. Verbindung nach einem der Ansprüche 1 bis 5, wobei X¹ CR^{7a} ist; optional,
wobei R^{7a} H ist; oder
wobei R^{7a} unabhängig ausgewählt ist aus Halo, Nitro, Cyano, OR¹¹, CO₂R⁹, CONR⁹R⁹, C₁-C₄-Alkyl, C₁-C₄-Alkyl substituiert mit NR⁹R¹⁰, C₁-C₄-Alkyl substituiert mit OR¹¹, C₁-C₄-Haloalkyl und Cyclopropyl.

8. Verbindung nach einem der Ansprüche 1 bis 7, wobei X², X³ und X⁴ jeweils Kohlenstoff sind.

9. Verbindung nach einem der Ansprüche 1 bis 8, wobei R³ H ist und R⁴ ausgewählt ist aus C₁-C₆-Alkyl, C₁-C₆-Haloalkyl und C₀-C₄-Alkylen-R^{4a}; optional,
wobei R⁴ ausgewählt ist aus C₁-C₆-Alkyl und C₁-C₆-Haloalkyl; oder
wobei R⁴ C₀-C₄-Alkylen-R^{4a} ist; oder
wobei R⁴ R^{4a} ist.

10. Verbindung nach Anspruch 9, wobei R^{4a} ausgewählt ist aus C₃-C₈-Cycloalkyl und 4- bis 10-gliedrigem Heterocycloalkyl, wobei die Cycloalkyl- oder Heterocycloalkylgruppe optional mit von 1 bis 4 R¹³-Gruppen substituiert ist; oder
wobei R^{4a} unabhängig ausgewählt ist aus: Phenyl und 5- oder 6-gliedrigem Heteroaryl; wobei die Phenyl- oder Heteroarylgruppe optional mit einer einzelnen R¹²-Gruppe und/oder von 1 bis 3 R¹⁴-Gruppen substituiert ist.

11. Verbindung nach einem der Ansprüche 1 bis 10, wobei R³ und R⁴ zusammen mit dem Stickstoffatom, an das sie gebunden sind, zusammen eine monocyclische 4- bis 7-gliedrige Heterocycloalkylgruppe bilden, die optional mit von 1 bis 4 R¹³-Gruppen substituiert ist; oder
wobei NR³R⁴ die Formel 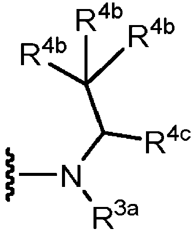 aufweist; wobei
R^{4b} bei jedem Vorkommen ausgewählt ist aus H und F; wobei mindestens eine R^{4b}-Gruppe F ist;
R^{3a} unabhängig ausgewählt ist aus H und C₁-C₄-Alkyl;
R^{4c} unabhängig ausgewählt ist aus H, C₁-C₄-Alkyl und C₄-C₆-Cycloalkyl; oder
R^{3a} und R^{4c} zusammen mit dem Kohlenstoff und Stickstoff, an den sie gebunden sind, eine 4- bis 6-gliedrige Heterocycloalkylgruppe bilden.

12. Verbindung nach Anspruch 1, wobei die Verbindung der Formel (I) ausgewählt ist aus:

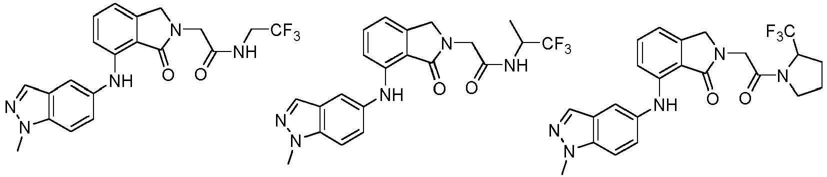

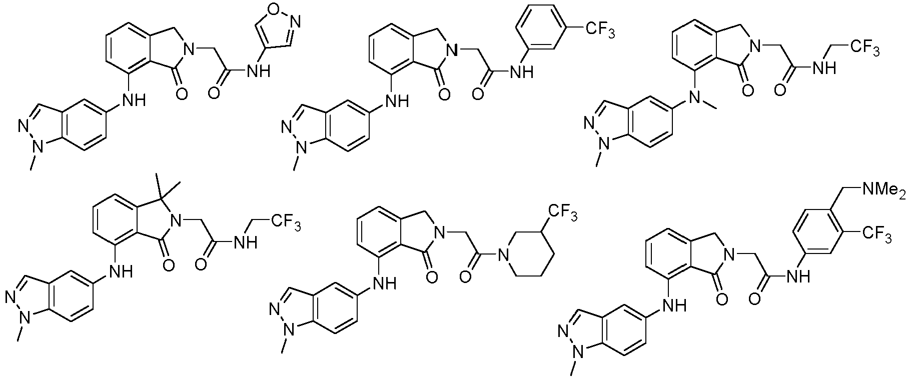

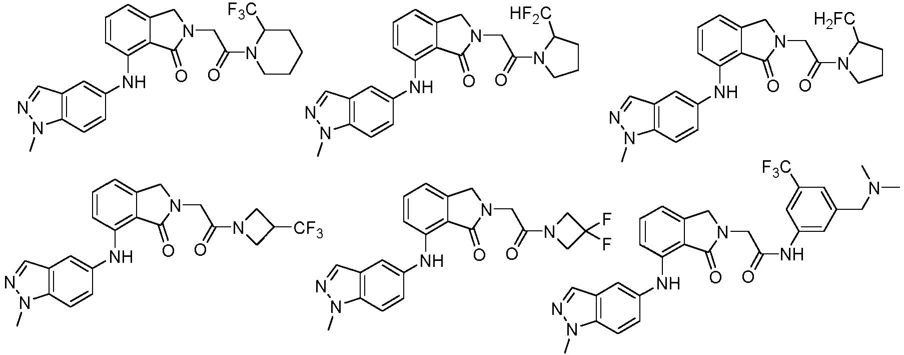

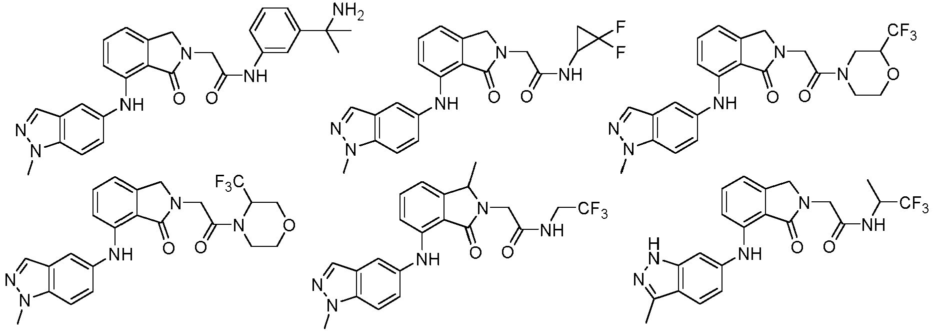

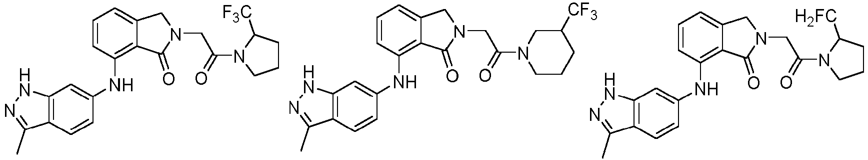

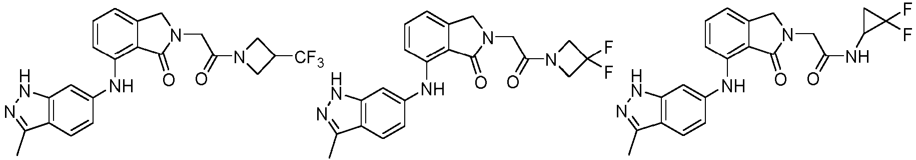

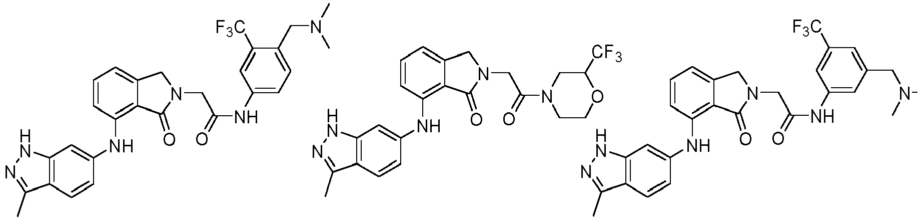

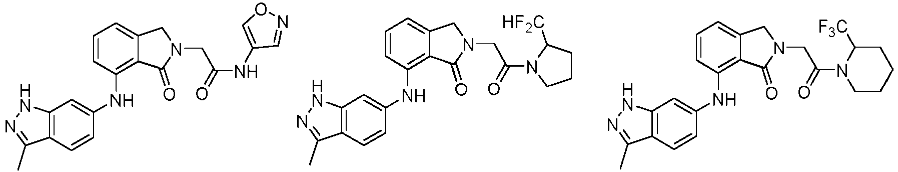

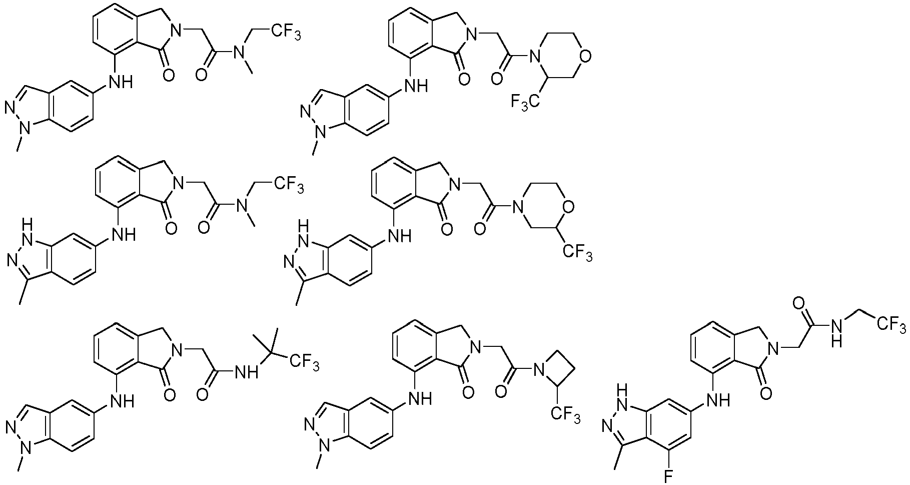

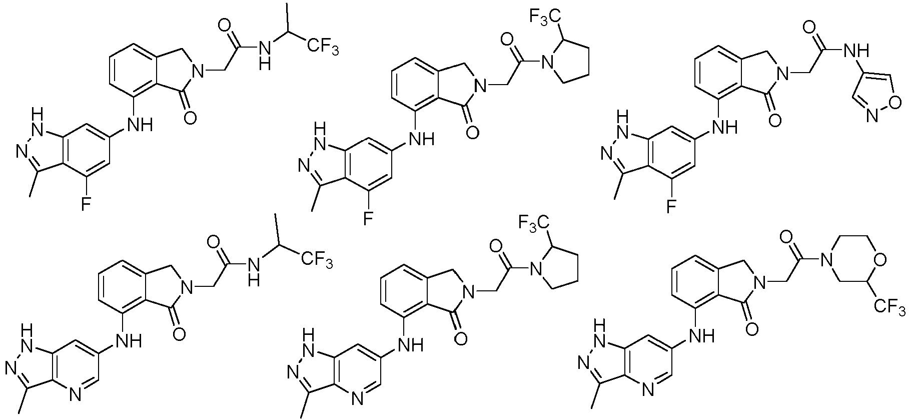

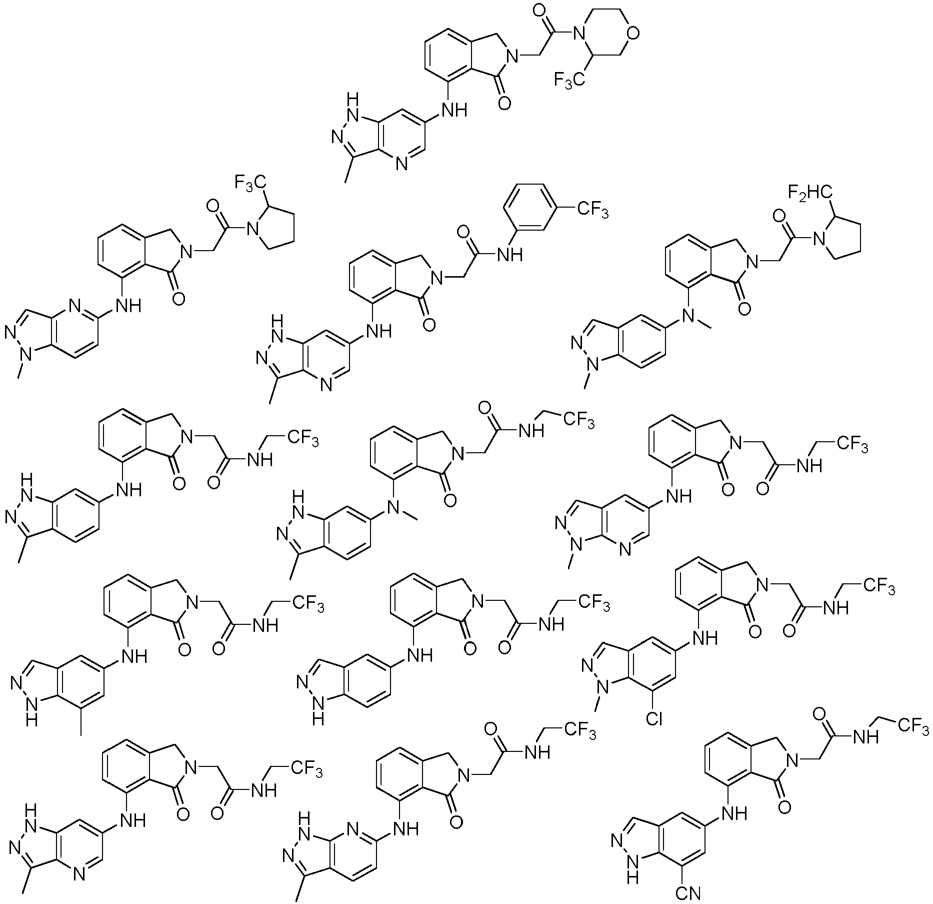

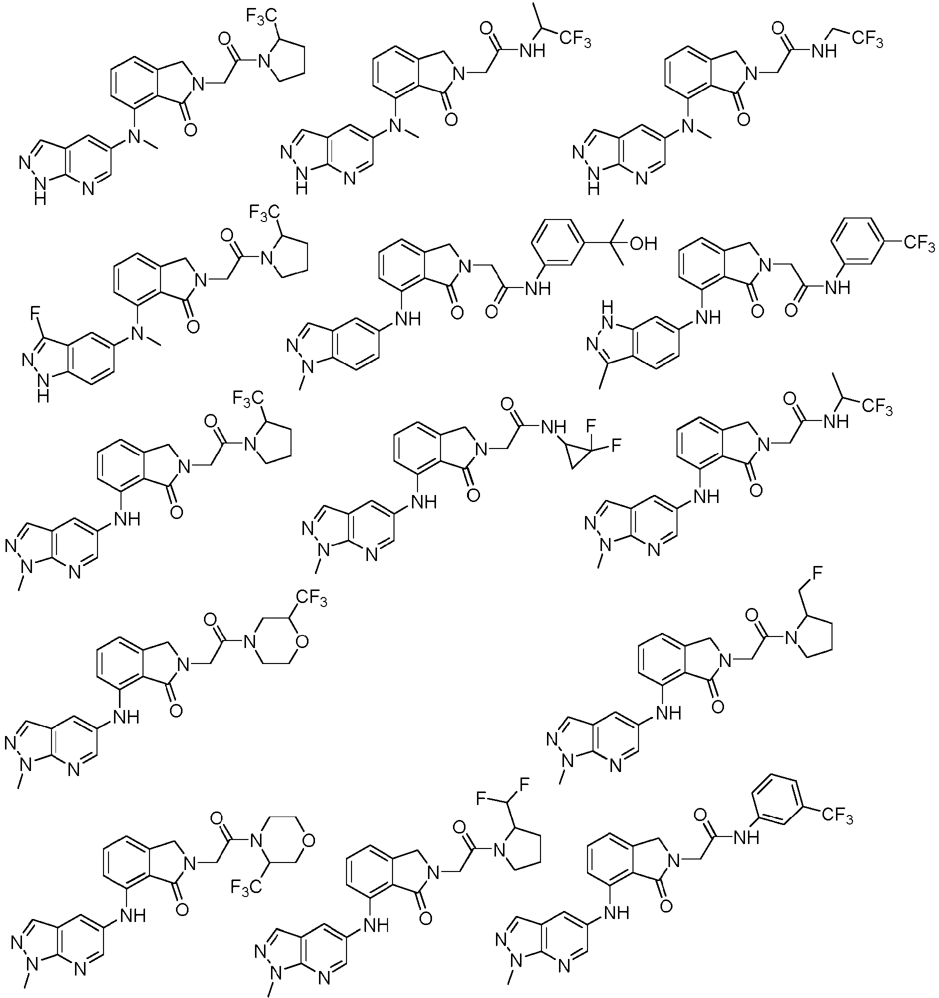

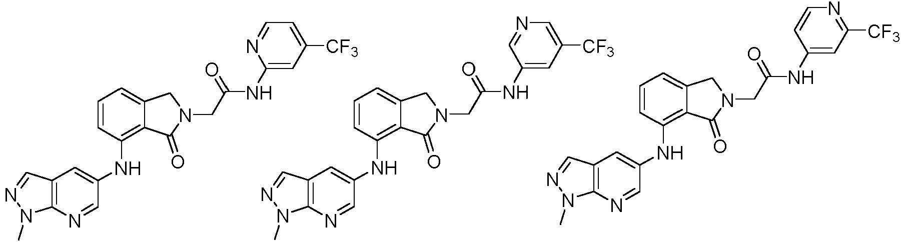

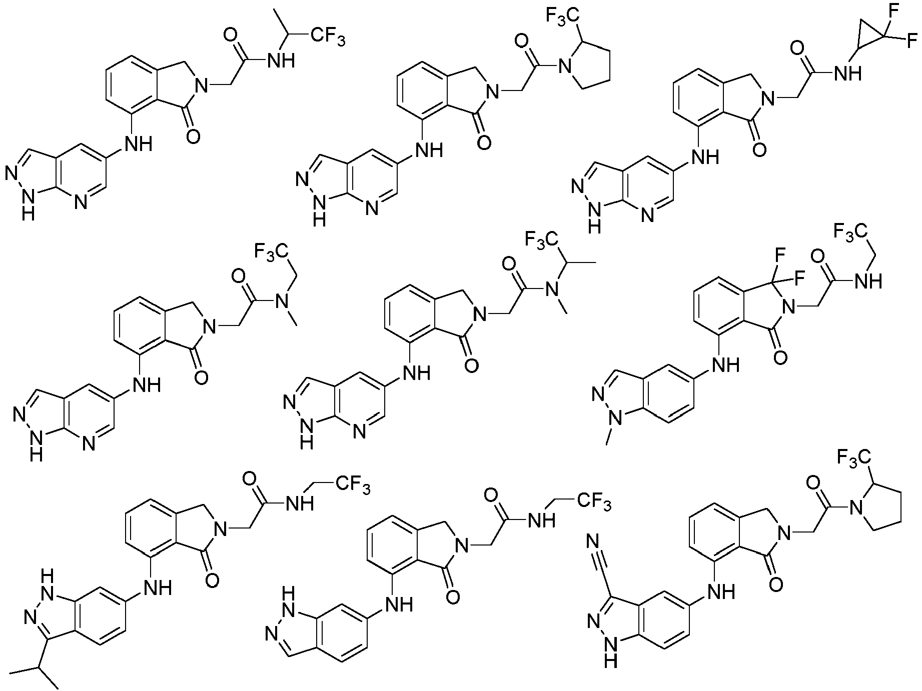

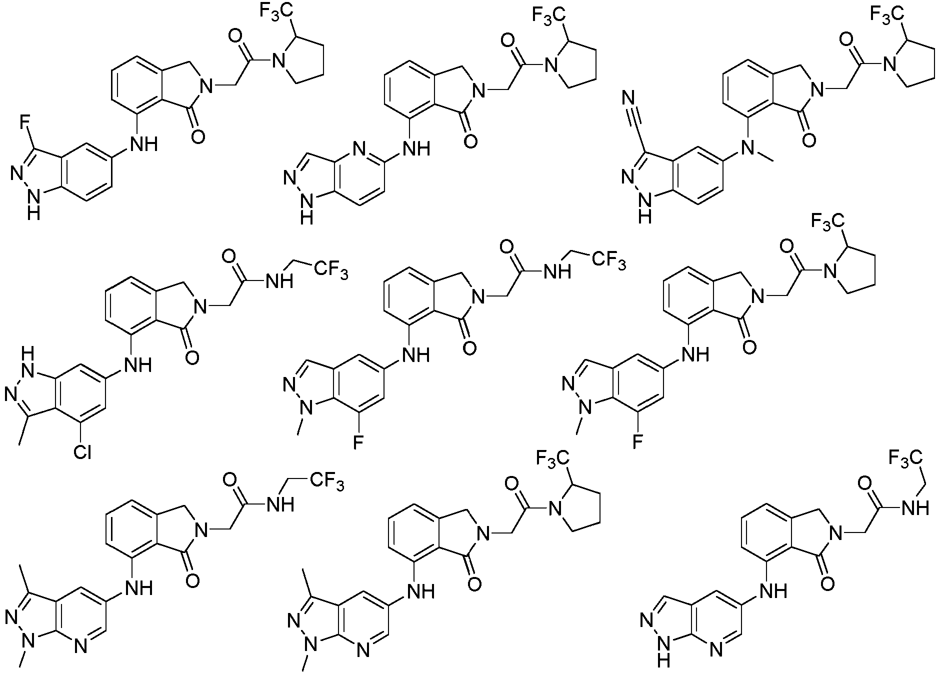

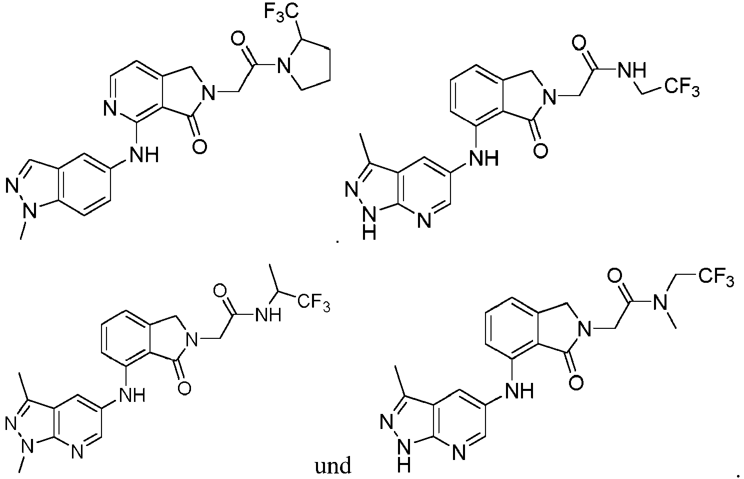

13. Pharmazeutische Formulierung, umfassend eine Verbindung nach einem der Ansprüche 1 bis 12 und einen pharmazeutisch verträglichen Hilfsstoff.

14. Verbindung nach einem der Ansprüche 1 bis 12 zur Verwendung als Medikament.

15. Verbindung nach einem der Ansprüche 1 bis 12 zur Verwendung beim Behandeln einer Erkrankung oder Störung, die ausgewählt ist aus Nierenzuständen, Leberzuständen, Entzündungszuständen, Herz-Kreislauf-Zuständen, akuter und chronischer Organtransplantatabstoßung, fibrotischen Erkrankungen und Krebs.

## Revendications

1. Composé de formule (I) ou sel acceptable pharmaceutiquement de celui-ci : 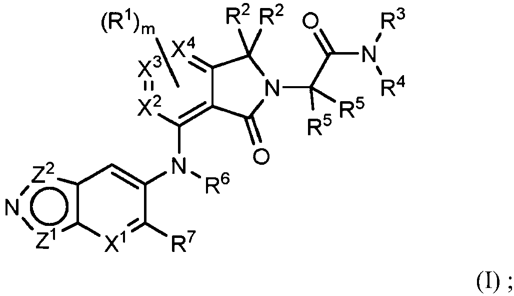 dans lequel
Z¹ et Z² sont chacun choisis parmi -CR^{8a}- et -NR^{8b}-, dans lequel l'un parmi Z¹ et Z² est - CR^{8a}- et l'autre est -NR^{8b}- ; et dans lequel le cycle comprenant Z¹ et Z² est un pyrazole ;
X¹ est indépendamment choisi parmi CR^{7a} et N ;
X², X³ et X⁴ sont chacun indépendamment choisis parmi le carbone et l'azote, au moins l'un de X², X³ et X⁴ étant le carbone ;
R¹ est indépendamment choisi à chaque occurrence parmi halo, nitro, cyano, NR⁹R¹⁰, OR¹¹, SR⁹, SO₂NR⁹R⁹, SO₂R⁹, CO₂R⁹, C(O)R⁹, CONR⁹R⁹, C₁-C₄-alkyle, C₁-C₄-alkyle substitué par NR⁹R¹⁰, C₁-C₄-alkyle substitué par OR¹¹, C₂-C₄-alcényle, C₂-C₄-alkynyle, C₁-C₄-haloalkyle et cyclopropyle ;
R² est indépendamment choisi à chaque occurrence parmi H, fluoro, C₁-C₄-alkyle, C₁-C₄-haloalkyle et cyclopropyle, ou les deux groupes R² et l'atome de carbone auquel ils sont attachés forment ensemble un cycle C₃-C₆-cycloalkyle ;
R³ est indépendamment choisi parmi H et C₁-C₄-alkyle ;
R⁴ est indépendamment choisi parmi C₁-C₆-alkyle, C₁-C₆-haloalkyle, C₀-C₄-alkylène-R^{4a} ; R^{4a} étant indépendamment choisi parmi : C₃-C₈-cycloalkyle, phényle, hétéroaryle à 5, 6, 9 ou 10 chaînons et hétérocycloalkyle à 4 à 10 chaînons ; ledit groupe hétérocycloalkyle ou hétéroaryle étant monocyclique ou bicyclique ; ledit groupe cycloalkyle ou hétérocycloalkyle étant éventuellement substitué par un seul groupe R¹² et/ou de 1 à 4 groupes R¹³ et ledit groupe phényle ou hétéroaryle étant éventuellement substitué par un seul groupe R¹² et/ou de 1 à 3 groupes R¹⁴ ;
ou R³ et R⁴, conjointement avec l'atome d'azote auquel ils sont attachés forment ensemble un groupe hétérocycloalkyle à 4 à 10 chaînons ou un groupe hétéroaryle à 5 ou 9 chaînons ; ledit groupe hétérocycloalkyle ou hétéroaryle pouvant être monocyclique ou bicyclique ; ledit groupe hétérocycloalkyle étant éventuellement substitué par un seul groupe R¹² et/ou de 1 à 4 groupes R¹³ et ledit groupe hétéroaryle étant éventuellement substitué par un seul groupe R¹² et/ou de 1 à 3 groupes R¹⁴ ;
R⁵ est indépendamment choisi à chaque occurrence parmi H, halo et C₁-C₄-alkyle, ou les deux groupes R⁵ et l'atome de carbone auquel ils sont attachés peuvent former ensemble un cycle C₃-C₆-cycloalkyle ;
R⁶ est indépendamment choisi parmi H, C₁-C₄-alkyle, C₁-C₄-haloalkyle et cyclopropyle ;
R⁷ et R^{7a} sont chacun indépendamment choisis parmi H, halo, nitro, cyano, NR⁹R¹⁰, OR¹¹, SR⁹, SO₂NR⁹R⁹, SO₂R⁹, CO₂R⁹, C(O)R⁹, CONR⁹R⁹, C₁-C₄-alkyle, C₁-C₄-alkyle substitué par NR⁹R¹⁰, C₁-C₄-alkyle substitué par OR¹¹, C₂-C₄-alcényle, C₂-C₄-alkynyle, C₁-C₄-haloalkyle et cyclopropyle ;
R^{8a} est indépendamment choisi parmi H, halo, nitro, cyano, NR⁹R¹⁰, OR¹¹, SR⁹, SO₂NR⁹R⁹, SO₂R⁹, CO₂R⁹, C(O)R⁹, CONR⁹R⁹, C₁-C₄-alkyle, C₁-C₄-alkyle substitué par NR⁹R¹⁰, C₁-C₄-alkyle substitué par OR¹¹, C₂-C₄-alcényle, C₂-C₄-alkynyle, C₁-C₄-haloalkyle et C₀-C₄-alkylène-R^{8c} ;
R^{8b} est indépendamment choisi parmi H, C₁-C₄-alkyle, C₂-C₄-alkyle substitué par NR⁹R¹⁰, C₂-C₄-alkyle substitué par OR¹¹, C₃-C₄-alcényle, C₃-C₄-alkynyle, C₁-C₄-haloalkyle et C₀-C₄-alkylène-R^{8c} ;
R^{8c} est indépendamment choisi parmi C₃-C₆-cycloalkyle et hétérocycloalkyle à 3 à 7 chaînons ; ledit groupe hétérocycloalkyle étant attaché au C₀-C₄-alkylène par l'intermédiaire d'un atome de carbone dans le cycle hétérocycloalkyle ; ledit groupe cycloalkyle ou hétérocycloalkyle étant éventuellement substitué par de 1 à 4 groupes R¹³ ;
R⁹ est indépendamment choisi à chaque occurrence parmi H et C₁-C₄-alkyle ou deux groupes R⁹, conjointement avec l'atome d'azote auquel ils sont attachés forment ensemble un groupe C₅-C₈-hétérocycloalkyle éventuellement substitué par de 0 à 4 groupes R¹³ ;
R¹⁰ est indépendamment choisi à chaque occurrence parmi H, C₁-C₄-alkyle, C(O)-C₁-C₄-alkyle et S(O)₂-C₁-C₄-alkyle ; ou R⁹ et R¹⁰, conjointement avec l'atome d'azote auquel ils sont attachés forment ensemble un groupe C₅-C₈-hétérocycloalkyle éventuellement substitué par de 0 à 4 groupes R¹³ ;
R¹¹ est indépendamment choisi à chaque occurrence parmi H, C₁-C₄-alkyle, C(O)-C₁-C₄-alkyle et C₁-C₄-haloalkyle ;
R¹² est indépendamment choisi parmi C₃-C₆-cycloalkyle, phényle, hétéroaryle à 5 ou 6 chaînons et hétérocycloalkyle à 3 à 6 chaînons ; ledit groupe cycloalkyle ou hétérocycloalkyle étant éventuellement substitué par de 1 à 4 groupes R¹³ et ledit groupe phényle ou hétéroaryle étant éventuellement substitué par de 1 à 3 groupes R¹⁴ ;
R¹³ est indépendamment choisi à chaque occurrence parmi =O, halo, nitro, cyano, NR⁸R⁹, OR¹⁴, SR⁸, SO₂NR⁸R⁸, CO₂R⁸, C(O)R⁸, CONR⁸R⁸, C₁-C₄-alkyle, C₁-C₄-alkyle substitué par OR¹¹, C₁-C₄-alkyle substitué par NR⁹R¹⁰, C₂-C₄-alcényle, C₂-C₄-alkynyle, C₁-C₄-haloalkyle, C₆-C₁₀-aryle et C₃-C₆-cycloalkyle ;
R¹⁴ est indépendamment choisi à chaque occurrence parmi halo, nitro, cyano, NR⁸R⁹, OR¹⁰, SR⁸, SO₂R⁸, SO₂NR⁸R⁸, CO₂R⁸, C(O)R⁸, CONR⁸R⁸, C₁-C₄-alkyle, C₂-C₄-alcényle, C₂-C₄-alkynyle, C₁-C₄-haloalkyle, C₁-C₄--alkyle substitué par OR¹¹, C₁-C₄-alkyle substitué par NR⁸R⁹ et cyclopropyle ;
m est un nombre entier choisi parmi 0, 1, 2 et 3 ;
l'un quelconque des groupes alkyle, alkylène ou cyclopropyle susmentionnés étant éventuellement substitué, lorsque chimiquement possible, par 1 à 5 substituants qui sont chacun indépendamment choisis à chaque occurrence dans le groupe constitué de : halo, oxo, fluoro, nitro, cyano, NR^{a}R^{b}, OR^{a}, SR^{a}, CO₂R^{a}, C(O)R^{a}, CONR^{a}R^{a}, C₁-C₄-alkyle, C₁-C₄-haloalkyle et cyclopropyle ; R^{a} étant indépendamment choisi à chaque occurrence parmi H, C₁-C₄-alkyle et C₁-C₄-haloalkyle ; et R^{b} étant indépendamment choisi à chaque occurrence parmi H, C₁-C₄-alkyle, C(O)-C₁-C₄-alkyle et S(O)₂-C₁-C₄-alkyle.

2. Composé de la revendication 1 dans lequel Z¹ est NR^{8b} et Z² est CH ; ou
dans lequel Z¹ est CR^{8a} et Z² est NH.

3. Composé de la revendication 1 ou la revendication 2, dans lequel m est 0.

4. Composé de l'une quelconque des revendications 1 à 3, dans lequel R² est indépendamment choisi à chaque occurrence parmi C₁-C₄-alkyle et H.

5. Composé de l'une quelconque des revendications 1 à 4, dans lequel R⁵ est à chaque occurrence H , et/ou
dans lequel R⁶ est H ; et/ou
dans lequel R⁷ est H.

6. Composé de l'une quelconque des revendications 1 à 5, dans lequel X¹ est N.

7. Composé de l'une quelconque des revendications 1 à 5, dans lequel X¹ est CR^{7a} ; éventuellement
dans lequel R^{7a} est H ; ou
dans lequel R^{7a} est indépendamment choisi parmi halo, nitro, cyano, OR¹¹, CO₂R⁹, CONR⁹R⁹, C₁-C₄-alkyle, C₁-C₄-alkyle substitué par NR⁹R¹⁰, C₁-C₄-alkyle substitué par OR¹¹, C₁-C₄-haloalkyle et cyclopropyle.

8. Composé de l'une quelconque des revendications 1 à 7, dans lequel X², X³ et X⁴ sont chacun carbone.

9. Composé de l'une quelconque des revendications 1 à 8, dans lequel R³ est H et R⁴ est choisi parmi C₁-C₆-alkyle, C₁-C₆-haloalkyle et C₀-C₄-alkylène-R^{4a} ; éventuellement
dans lequel R⁴ est choisi parmi C₁-C₆-alkyle et C₁-C₆-haloalkyle ; ou
dans lequel R⁴ est C₀-C₄-alkylène-R^{4a} ; ou
dans lequel R⁴ est R^{4a}.

10. Composé de la revendication 9, dans lequel R^{4a} est choisi parmi C₃-C₈-cycloalkyle et hétérocycloalkyle à 4 à 10 chaînons, ledit groupe cycloalkyle ou hétérocycloalkyle étant éventuellement substitué par de 1 à 4 groupes R¹³ ; ou
dans lequel R^{4a} est indépendamment choisi parmi : phényle et hétéroaryle à 5 ou 6 chaînons ; ledit groupe phényle ou hétéroaryle étant éventuellement substitué par un seul groupe R¹² et/ou de 1 à 3 groupes R¹⁴.

11. Composé de l'une quelconque des revendications 1 à 10, dans lequel R³ et R⁴, conjointement avec l'atome d'azote auquel ils sont attachés forment un groupe hétérocycloalkyle à 4 à 7 chaînons monocyclique, éventuellement substitué par de 1 à 4 groupes R¹³ groupes ; ou
dans lequel NR³R⁴ comporte la formule 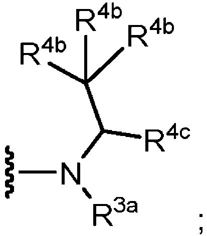 dans lequel
R^{4b} est choisi à chaque occurrence parmi H et F ; au moins un groupe R^{4b} étant F ;
R^{3a} est indépendamment choisi parmi H et C₁-C₄-alkyle ;
R^{4c} est indépendamment choisi parmi H, C₁-C₄-alkyle et C₄-C₆-cycloalkyle ; ou
R^{3a} et R^{4c}, conjointement avec le carbone et l'azote auxquels ils sont attachés, forment un groupe hétérocycloalkyle à 4 à 6 chaînons.

12. Composé de la revendication 1 dans lequel le composé de formule (**I**) est choisi parmi :

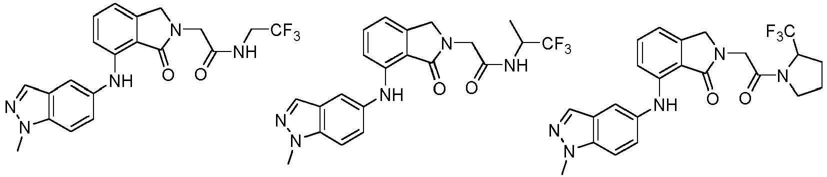

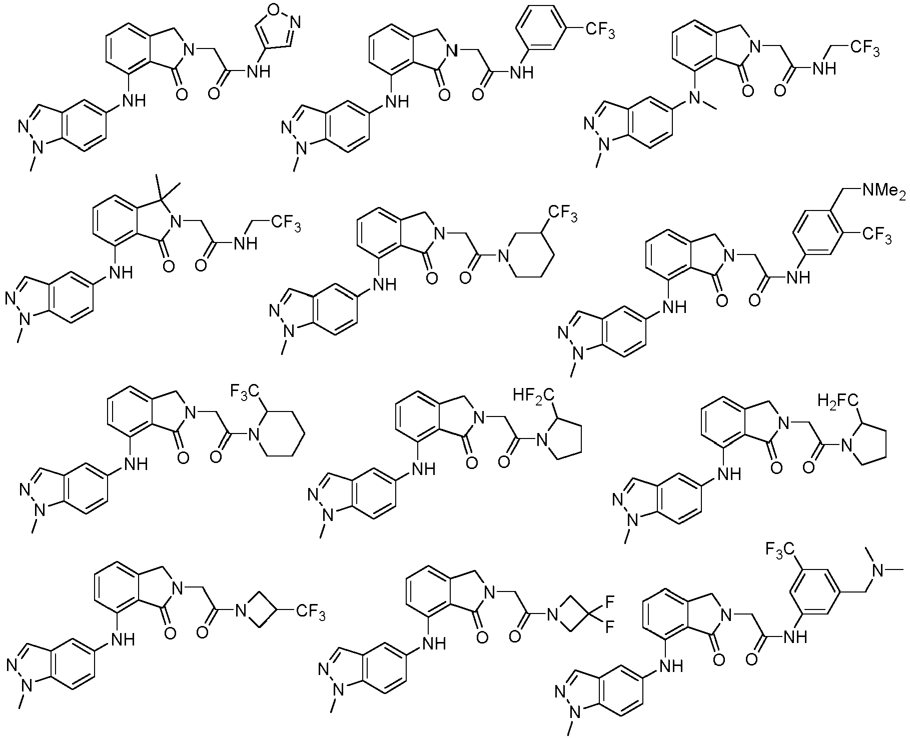

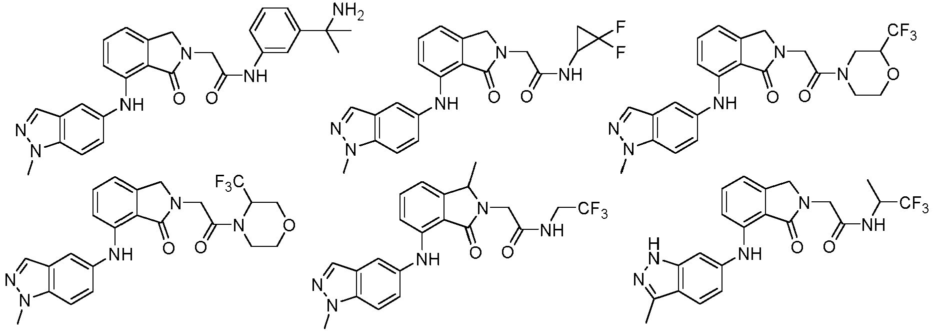

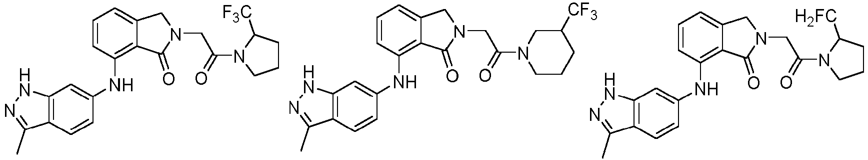

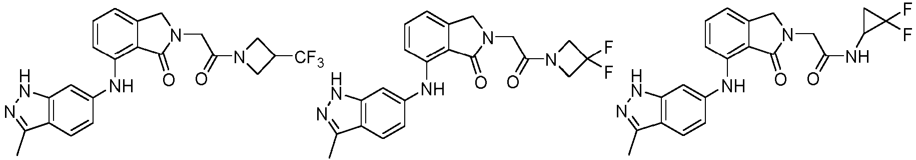

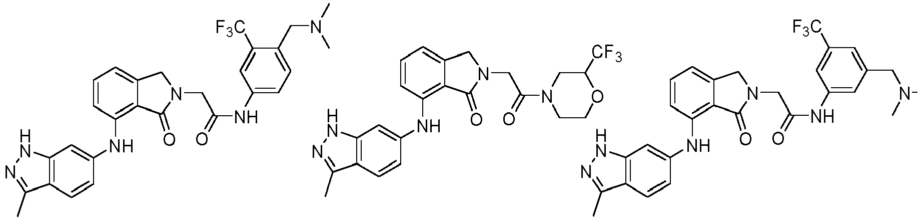

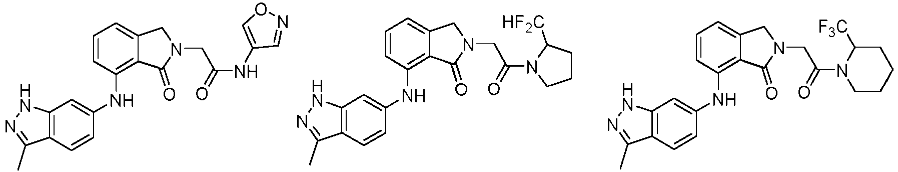

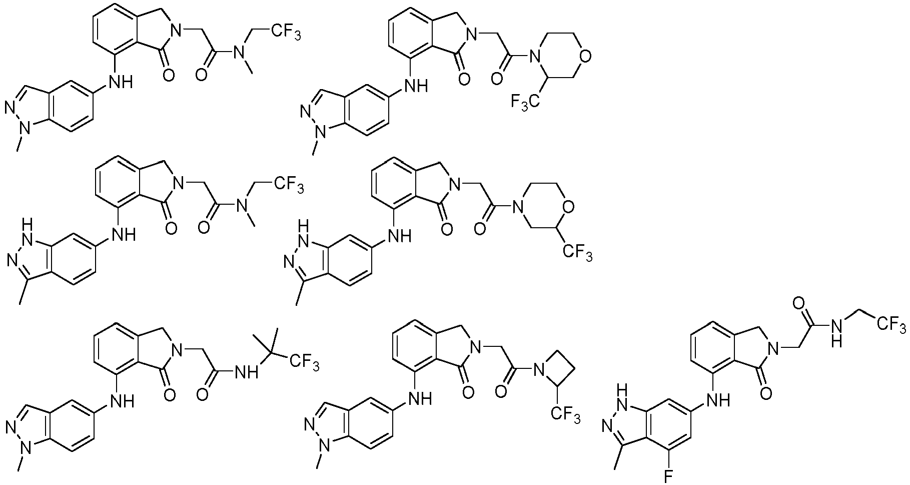

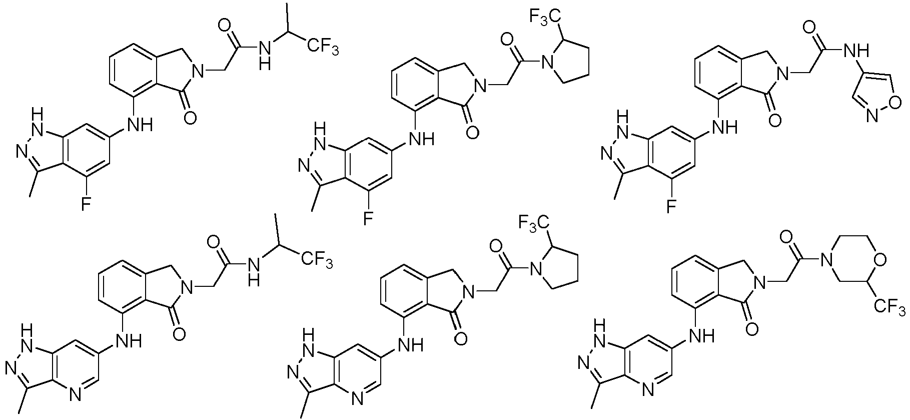

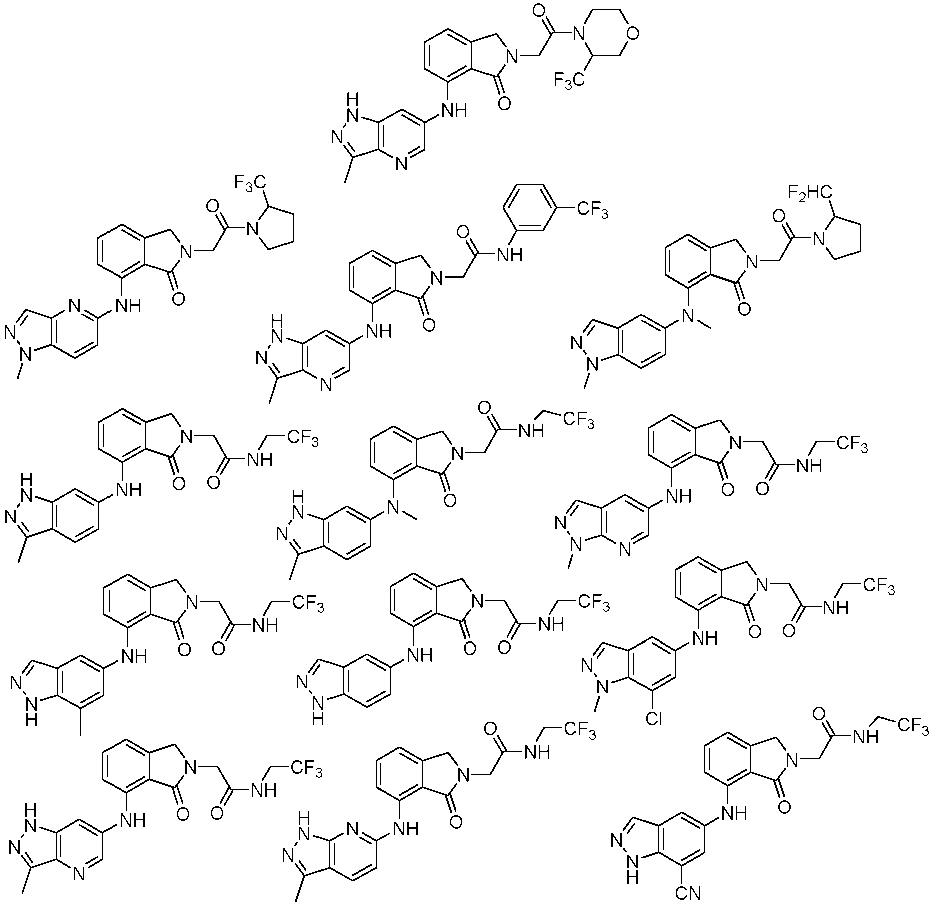

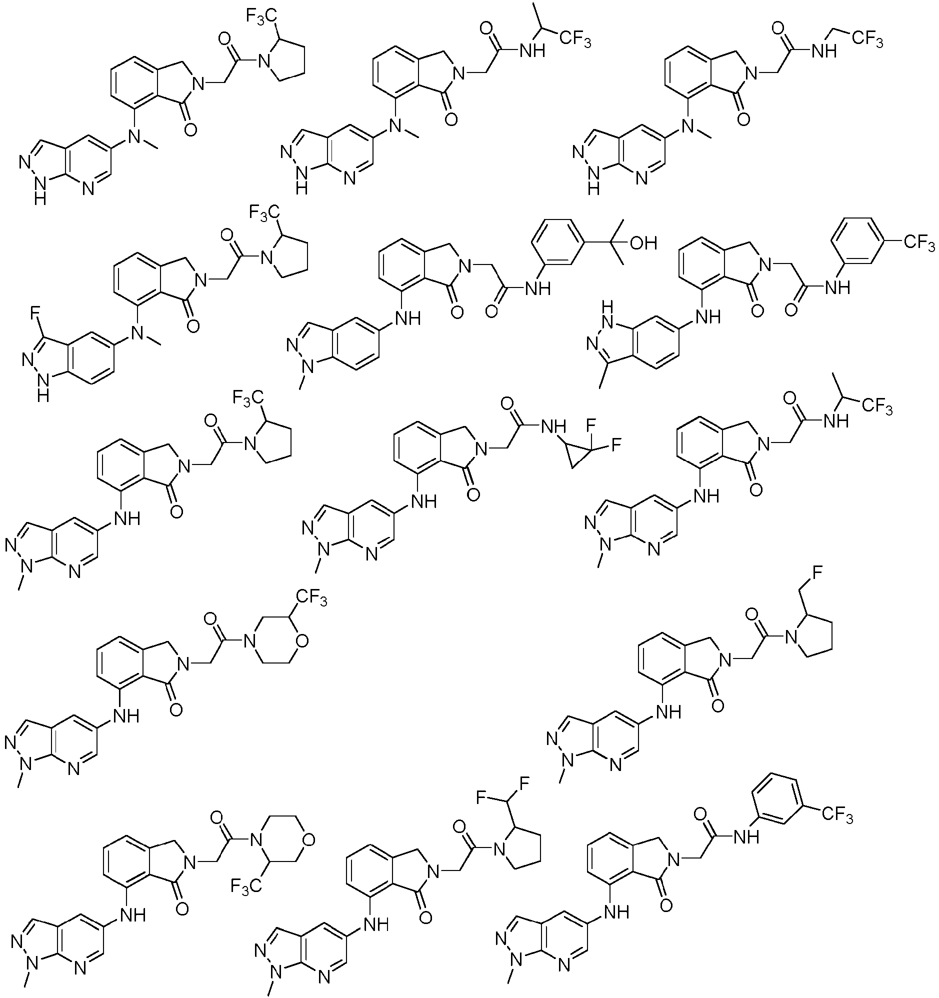

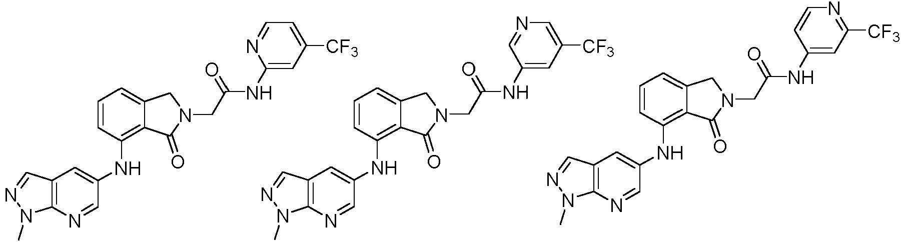

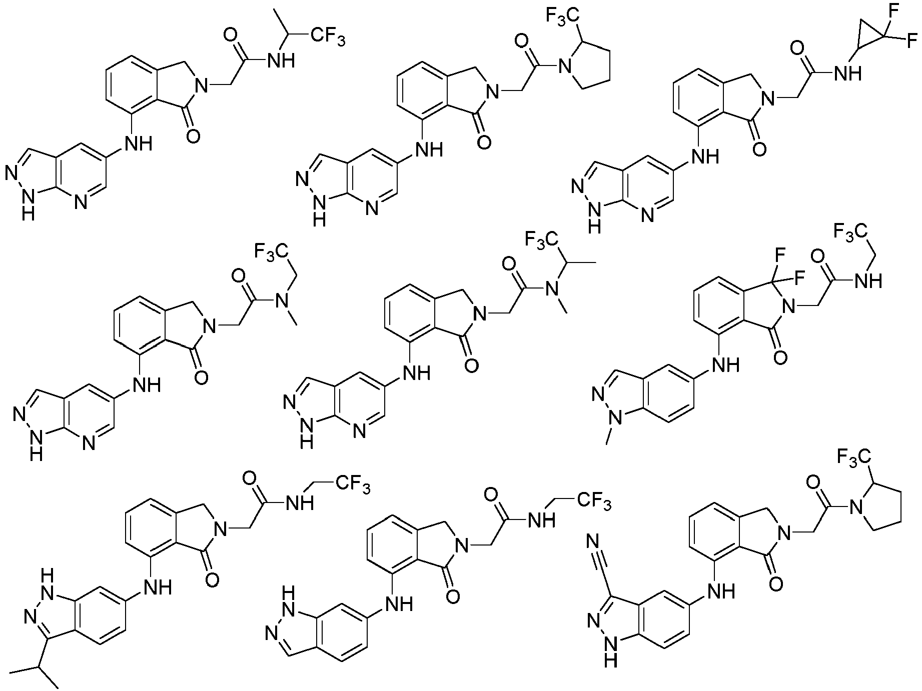

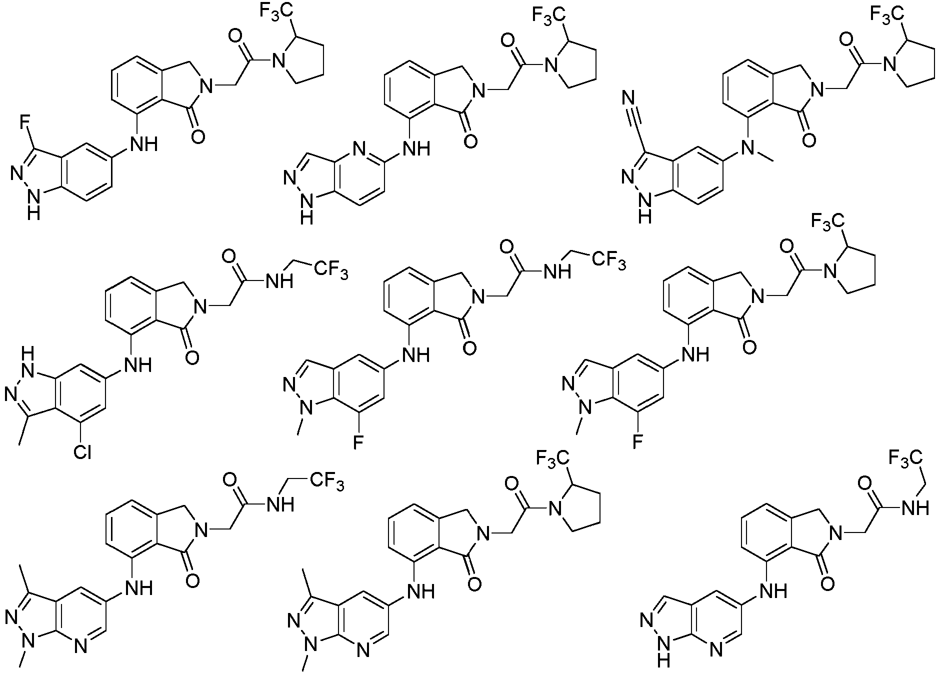

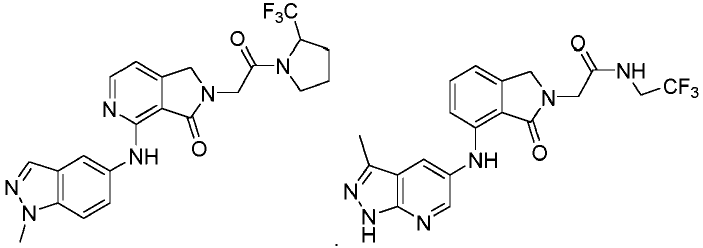

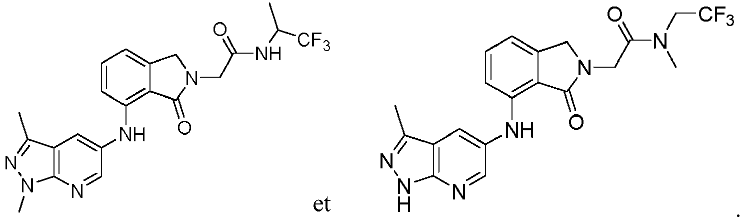

13. Composition pharmaceutique comprenant un composé de l'une quelconque des revendications 1 à 12 et un excipient pharmaceutiquement acceptable.

14. Composé de l'une quelconque des revendications 1 à 12 destiné à être utilisé comme médicament.

15. Composé de l'une quelconque des revendications 1 à 12 destiné à être utilisé dans le traitement d'une maladie ou d'un trouble choisi parmi les affections rénales, les affections hépatiques, les états inflammatoires, les affections cardiovasculaires, le rejet aigu et chronique de greffe d'organe, les maladies fibrotiques et le cancer.
